# EUROPEAN PATENT APPLICATION

(11) **EP 4 822 025 A1**
(43) Date of publication of application: **14.10.2026**
(21) Application number: 24899800.7
(22) Date of filing: 03.12.2024
(51) Int. Cl.: C12N 15/45, C07K 14/005, A61K 39/155, A61P 31/14

(54) **METAPNEUMOVIRUS (MPV) VACCINE**

(30) Priority: 04.12.2023 WO PCT/CN2023/136163
(71) Applicant: Shenzhen Shenxin Biotechnology Co., Ltd., Shenzhen, Guangdong 518000 (CN)
(72) Inventor: LI, Linxian, Shenzhen, Guangdong 518000 (CN); LI, Hui, Shenzhen, Guangdong 518000 (CN)
(74) Representative: Dentons Patent Solutions Rechtsanwaltsgesellschaft mbH
(86) International application number: PCT/CN2024/136380
(87) International publication number: WO 2025/119162

(57) **Abstract**

The present invention relates to a metapneumovirus (MPV) vaccine, wherein the nucleic acid comprises a polynucleotide encoding a mutant of an MPV F protein, and the mutant comprises disulfide bond mutations relative to a wild-type MPV F protein.

## Description

### Technical Field

The present invention belongs to the field of biotechnology, and relates to a metapneumovirus (MPV) vaccine.

### Background

Human metapneumovirus (hMPV) has been successively reported worldwide since it was first discovered in the Netherlands in 2001. hMPV is a single-stranded ribonucleic acid virus, and is one of the main pathogens causing acute lower respiratory tract infection. hMPV includes two genotypes A and B, and can be divided into four subtypes A1, A2, B1, and B2.

The initial infection of hMPV usually occurs under 5 years of age, and it is shown in epidemiological studies that in 2018 globally, there were, in children under 5 years of age, 1.42 millions of hMPV-associated acute lower respiratory tract infections occurred, 643 thousands of hospitalized cases associated with hMPV, 7,700 hospitalized deaths associated with hMPV, and 16,100 (hospital and community) acute lower respiratory tract infection deaths associated with hMPV. hMPV-induced immune protection is weak, and therefore, the infection can be repeated, and can cause serious diseases (such as bronchitis or pneumonia) in adults (especially in the elderly and immunodeficient patients), and the hospitalization rate of hMPV is comparable to RSV and influenza virus.

However, there are currently no prophylactic vaccines or potent antiviral medicament approved for hMPV. At present, the treatment of hMPV infection is only symptomatic and supportive treatment.

### Summary of the invention

The present disclosure provides a nucleic acid encoding a protein or polypeptide capable of inducing a specific immune response for preventing, or at least reducing, a metapneumovirus (MPV) infection.

In addition, the present disclosure also provides a delivery carrier , a pharmaceutical composition, an MPV vaccine comprising the above nucleic acid and use thereof in the preparation of a medicament.

A nucleic acid comprising a polynucleotide encoding a mutant of an MPV F protein, the mutant comprising an F1 polypeptide and an F2 polypeptide, the mutant comprising disulfide bond mutations relative to a wild-type MPV F protein, the disulfide bond mutations comprising one or more of:
A147C and A159C, E146C and T160C, F168C and F196C, L165C and F196C, N145C and A161C, S149C and V157C, T59C and N180C, T150C and R156C, V52C and L165C, V55C and V169C, L58C and T174C, V148C and L158C.

In some embodiments, the disulfide bond mutations comprises one or more of: A147C and A159C, L165C and F196C, N145C and A161C, S149C and V157C, T59C and N180C.

In some embodiments, the disulfide bond mutations are selected from one of A147C and A159C, L165C and F196C, N145C and A161C.

In some embodiments, the mutant is selected from one of the following (1) to (38):
(1) a mutant comprising an amino acid sequence set forth in SEQ ID NO: 1 or 2;
(2) a mutant comprising mutations A147C and A159C and comprising an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to the amino acid sequence set forth in SEQ ID NO: 1 or 2;
(3) a mutant comprising an amino acid sequence set forth in SEQ ID NO: 3 or 4;
(4) a mutant comprising mutations E146C and T160C and comprising an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to the amino acid sequence set forth in SEQ ID NO: 3 or 4;
(5) a mutant comprising an amino acid sequence set forth in SEQ ID NO: 5 or 6;
(6) a mutant comprising mutations F168C and F196C and comprising an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to the amino acid sequence set forth in SEQ ID NO: 5 or 6;
(7) a mutant comprising an amino acid sequence set forth in SEQ ID NO: 7 or 8;
(8) a mutant comprising mutations L165C and F196C and comprising an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to the amino acid sequence set forth in SEQ ID NO: 7 or 8;
(9) a mutant comprising an amino acid sequence set forth in SEQ ID NO: 9 or 10;
(10) a mutant comprising mutations N145C and A161C and comprising an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to the amino acid sequence set forth in SEQ ID NO: 9 or 10;
(11) a mutant comprising an amino acid sequence set forth in SEQ ID NO: 11 or 12;
(12) a mutant comprising mutations S149C and V157C and comprising an amino acid sequence having at least 85%, 88%, 90%, 91%, 92%, 93, 94, 95, 95.5%, 96, 96.5%, 97%, 97.5%, 98%, 98.5%, 99, or 99.5% identity to the amino acid sequence set forth in SEQ ID NO: 11or 12;
(13) a mutant comprising an amino acid sequence set forth in SEQ ID NO: 13 or 14;
(14) a mutant comprising mutations T59C and N180C and comprising an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to the amino acid sequence set forth in SEQ ID NO: 13 or 14;
(15) a mutant comprising an amino acid sequence set forth in SEQ ID NO: 15 or 16;
(16) a mutant comprising mutations T59C and N180C and comprising an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to the amino acid sequence set forth in SEQ ID NO: 15 or 16;
(17) a mutant comprising an amino acid sequence set forth in SEQ ID NO: 17 or 18;
(18) a mutant comprising mutations T59C and N180C and comprising an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to the amino acid sequence set forth in SEQ ID NO: 17 or 18;
(19) a mutant comprising an amino acid sequence set forth in SEQ ID NO: 19 or 20;
(20) a mutant comprising mutations T59C and N180C and comprising an amino acid sequence having at least 85%, 88%, 90%, 91%, 92%, 93%, 94%, 95%, 95.5%, 96%, 96.5%, 97%, 97.5%, 98%, 98.5%, 99%, or 99.5% identity to the amino acid sequence set forth in SEQ ID NO: 19 or 20;
(21) a mutant comprising an amino acid sequence set forth in SEQ ID NO: 21 or 22;
(22) a mutant comprising mutations T150C and R156C and comprising an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to the amino acid sequence set forth in SEQ ID NO: 21 or 22;
(23) a mutant comprising an amino acid sequence set forth in SEQ ID NO: 23 or 24;
(24) a mutant comprising mutations V52C and L165C and comprising an amino acid sequence having at least 85%, 88%, 90%, 91%, 92%, 93%, 94%, 95%, 95.5%, 96%, 96.5%, 97%, 97.5%, 98, 98.5%, 99%, or 99.5% identity to the amino acid sequence set forth in SEQ ID NO: 23 or 24;
(25) a mutant comprising an amino acid sequence set forth in SEQ ID NO: 25 or 26;
(26) a mutant comprising mutations V55C and V169C and comprising an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to the amino acid sequence set forth in SEQ ID NO: 25 or 26;
(27) a mutant comprising an amino acid sequence set forth in SEQ ID NO: 27 or 28;
(28) a mutant comprising mutations L58C and T174C and comprising an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to the amino acid sequence set forth in SEQ ID NO: 27 or 28;
(29) a mutant comprising an amino acid sequence set forth in SEQ ID NO: 29 or 30;
(30) a mutant comprising mutations V148C and L158C and comprising an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to the amino acid sequence set forth in SEQ ID NO: 29 or 30;
(31) a mutant comprising an amino acid sequence set forth in SEQ ID NO: 81 or 82;
(32) a mutant comprising mutations V148C and L158C and comprising an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to the amino acid sequence set forth in SEQ ID NO: 81 or 82;
(33) a mutant comprising an amino acid sequence set forth in SEQ ID NO: 83;
(34) a mutant comprising mutations A147C and A159C and comprising an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to the amino acid sequence set forth in SEQ ID NO: 83;
(35) a mutant comprising an amino acid sequence set forth in SEQ ID NO: 84;
(36) a mutant comprising mutations L165C and F196C and comprising an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to the amino acid sequence set forth in SEQ ID NO: 84;
(37) a mutant comprising the amino acid sequence set forth in SEQ ID NO: 85; and
(38) a mutant comprising mutations N145C and A161C and comprising an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to the amino acid sequence set forth in SEQ ID NO: 85.

In some embodiments, the wild-type MPV is subtype A or subtype B.

In some embodiments, the wild-type MPV is wild-type hMPV.

In some embodiments, the MPV F protein is hMPV F protein.

In some embodiments, the nucleic acid is RNA; preferably, the RNA is mRNA; more preferably, the mRNA comprises at least one of 5'-cap structure, 5'-UTR, 3'-UTR and poly(A) tail.

In some embodiments, the nucleic acid is DNA.

In some embodiments, the DNA is capable of being transcribed into RNA.

In some embodiments, the DNA sequence corresponding to the 5'-UTR is set forth in SEQ ID NO: 31;
and/or, the DNA sequence corresponding to the 3'-UTR is set forth in SEQ ID NO: 32;
and/or, the nucleotides constituting the poly(A) tail comprise at least 20, at least 40, at least 80, at least 100 or at least 120 A nucleotides; preferably, the nucleotides constituting the poly(A) tail comprise at least 20, at least 40, at least 80, at least 100 or at least 120 consecutive A nucleotides; preferably, the nucleotides constituting the poly(A) tail comprise one or more nucleotides other than the A nucleotide; more preferably, the DNA sequence corresponding to the poly(A) tail is set forth in SEQ ID NO: 33.

In some embodiments, the nucleic acid contains a modified nucleotide;
preferably, the nucleic acid contains a modified nucleoside; preferably, the modified nucleoside includes at least one of modified uridine, modified cytidine, modified adenosine and modified guanosine.

In some embodiments, the nucleic acid comprises a polynucleotide encoding one or more mutants of MPV F protein.

In some embodiments, the nucleic acid further comprises a polynucleotide encoding a protein or polypeptide other than the mutant of MPV F protein.

In some embodiments, the nucleic acid further comprises one or more of a polynucleotide encoding the transmembrane domain of MPV F protein, a polynucleotide encoding a ferritin polypeptide, a polynucleotide encoding a signal peptide, and a polynucleotide encoding a trimerization domain.

A genetic engineering vector, comprising any one of the above nucleic acids, or comprising a polynucleotide capable of being transcribed into any one of the above nucleic acids.

A host cell, comprising any one of the above genetic engineering vectors.

A mutant of MPV F protein encoded by any one of the above nucleic acids;

In some embodiments, the mutant is a trimer.

A delivery carrier comprising a nucleic acid composition, wherein the nucleic acid composition comprises a first nucleic acid or a first genetic engineering vector, the first nucleic acid is any one of the above nucleic acids, and the first genetic engineering vector is any one of the above genetic engineering vectors;
or, the nucleic acid composition comprises a plurality of first nucleic acids or a plurality of first genetic engineering vectors, the first nucleic acids are any of the above nucleic acids, the first genetic engineering vectors are any of the above genetic engineering vectors, and the plurality of first nucleic acids are independent of each other or the plurality of first genetic engineering vectors are independent of each other.

In some embodiments, the nucleic acid composition further comprises a second nucleic acid or a second vector, the second nucleic acid comprises a polynucleotide encoding a protein or polypeptide other than the mutant of MPV F protein, and the second vector comprises a polynucleotide encoding a protein or polypeptide other than the mutant of MPV F protein;
and/or, the first nucleic acid and the second nucleic acid are RNA; more preferably, the first nucleic acid and the second nucleic acid are mRNA.

In some embodiments, the delivery carrier is lipid nanoparticles (LNPs), cationic liposomes, cationic proteins or lipid multimers (LPP);

In some embodiments, the delivery carrier is a lipid nanoparticle comprising a cationic lipid comprising the following compound (IV), or a pharmaceutically acceptable salt or stereoisomer thereof: 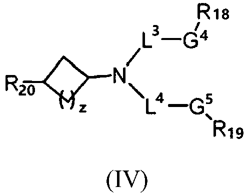 where:
L³ and L⁴ are the same or different, and are each independently C1-C12 alkylene, C2-C12 alkenylene or C2-C12 alkynylene; preferably L ³ and L⁴ are the same or different, and are each independently C3-C10 alkylene, C3-C10 alkenylene or C3-C10 alkynylene; in some embodiments, L³ and L⁴ are the same or different, and are each independently C3-C10 alkylene; in some embodiments, L³ and L⁴ are the same or different, and are each independently C5-C8 alkylene;
G⁴ and G⁵ are the same or different, and are each independently -O-(C=O)-, -(C=O)-O-, -C(=O)-, -O-, -C(=O)-S-or-S-C(=O)-; in some embodiments, G⁴ and G⁵ are the same or different, and are each independently -O-(C=O)-, -(C=O)-O-, -C(=O)- or -O-; in some embodiments, G⁴ and G⁵ are the same or different, and are each independently selected from -O-(C=O)- or -(C=O)-O-;
R₁₈ and R₁₉ are the same or different, and are each independently C5-C27 alkyl or C5-C27 alkenyl containing one or more double bonds; in some embodiments, R₁₈ and R₁₉ are the same or different, and are each independently C8-C20 alkyl or C8-C20 alkenyl containing one or more double bonds; in some embodiments, R₁₈ and R₁₉ are the same or different, and are each independently C9-C17 alkyl or C9-C18 alkenyl containing one or two double bonds; in some embodiments, R₁₈ and R₁₉ are the same or different, and are each independently 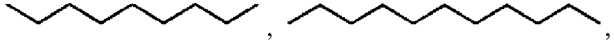 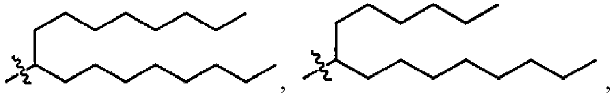 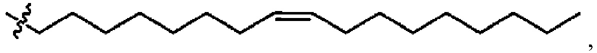 or 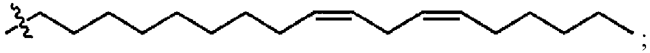
R₂₀ is halogen, hydroxyl, cyano, C1-C6 alkyl, nitro, C1-C6 alkoxy, C1-C6 alkylcarbonyloxy, C1-C6 alkoxycarbonyl, C1-C6 alkylaminocarbonyl or C1-C6 alkylcarbonylamino; in some embodiments, R₂₀ is halogen, hydroxyl, cyano, C1-C6 alkoxy, C1-C6 alkylcarbonyloxy, C1-C6 alkoxycarbonyl, C1-C6 alkylaminocarbonyl or C1-C6 alkylcarbonylamino; in some embodiments, R₂₀ is halogen, hydroxyl, cyano, C1-C4 alkoxy, C1-C4 alkylcarbonyloxy, C1-C4 alkoxycarbonyl, C1-C4 alkylaminocarbonyl or C1-C4 alkylcarbonylamino; in some embodiments, R₂₀ is fluorine, hydroxyl, cyano, methoxy, acetoxy, methoxycarbonyl, butylaminocarbonyl or acetamido;
z is 1, 2 or 3.

In some embodiments, the cationic lipid is the following compound (IV-1), or a pharmaceutically acceptable salt or stereoisomer thereof: 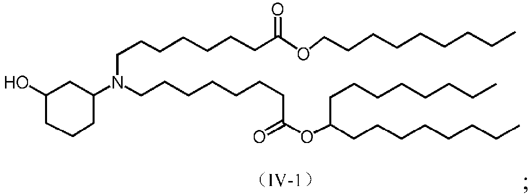 or, the cationic lipid is the following compound, or a pharmaceutically acceptable salt thereof: 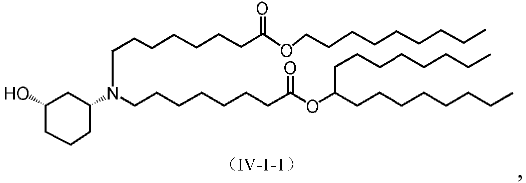 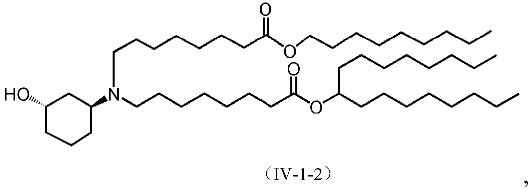 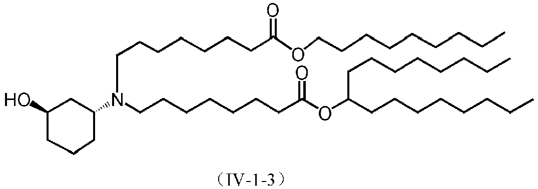 or

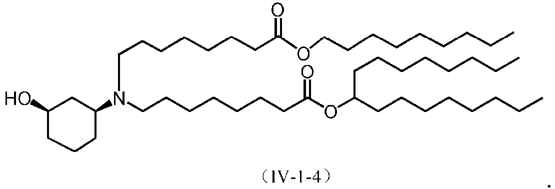

A pharmaceutical composition comprising any one of the above nucleic acids, any one of the above genetic engineering vectors, any one of the above host cells, any one of the above mutants or any one of the above delivery carriers, and a pharmaceutically acceptable carrier.

In some embodiments, the pharmaceutical composition comprises a plurality of the delivery carriers;
or, the pharmaceutical composition comprises a plurality of the nucleic acids.

In some embodiments, the pharmaceutical composition comprises two nucleic acids, the two nucleic acids are co-formulated or separately formulated in lipid nanoparticles, the two nucleic acids respectively encode a mutant of subtype A MPV F protein and a mutant of subtype B MPV F protein, and the two nucleic acids encode a mutant of subtype A MPV F protein and a mutant of subtype B MPV F protein selected from the group consisting of:
(1) a mutant comprising mutant A147C and A159C and an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to SEQ ID NO: 1, and a mutant comprising mutant A147C and A159C and an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to SEQ ID NO: 2; and
(2) a mutant comprising mutations A147C and A159C and an amino acid sequence having at least 85%, 88%, 90%, 91%, 92%, 93%, 94%, 95%, 95.5%, 96, 96.5%, 97%, 97.5%, 98%, 98.5%, 99%, or 99.5% identity to SEQ ID NO: 81, and a mutant comprising mutations A147C and A159C and an amino acid sequence having at least 85%, 88%, 90%, 91%, 92%, 93%, 94%, 95%, 95.5%, 96%, 96.5%, 97%, 97.5%, 98%, 98.5%, 99%, or 99.5% identity to SEQ ID NO: 82.

Use of any one of the above nucleic acids, any one of the above genetic engineering vectors, any one of the above host cells, any one of the above mutants, any one of the above delivery carriers or any one of the above pharmaceutical compositions in the preparation of a medicament;

In some embodiments, the medicament is used for preventing or treating MPV infection or a disease caused by MPV infection.

In some embodiments, the medicament is used for preventing or treating hMPV infection or a disease caused by hMPV infection.

In some embodiments, the medicament is a vaccine.

A vaccine comprising any one of the above nucleic acids, any one of the above genetic engineering vectors, any one of the above mutants or any one of the above delivery carriers.

### Brief description of the drawings

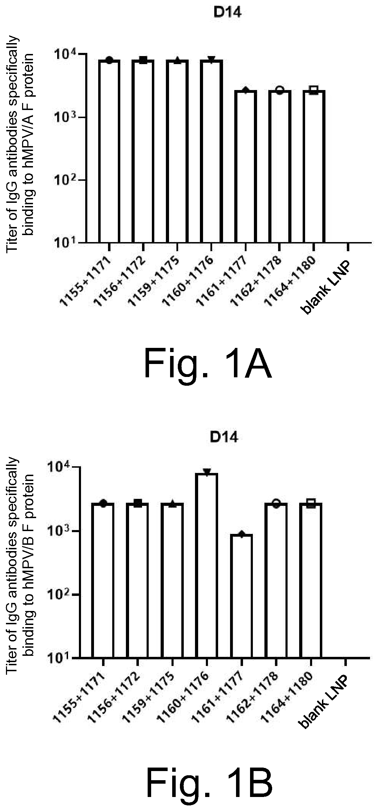

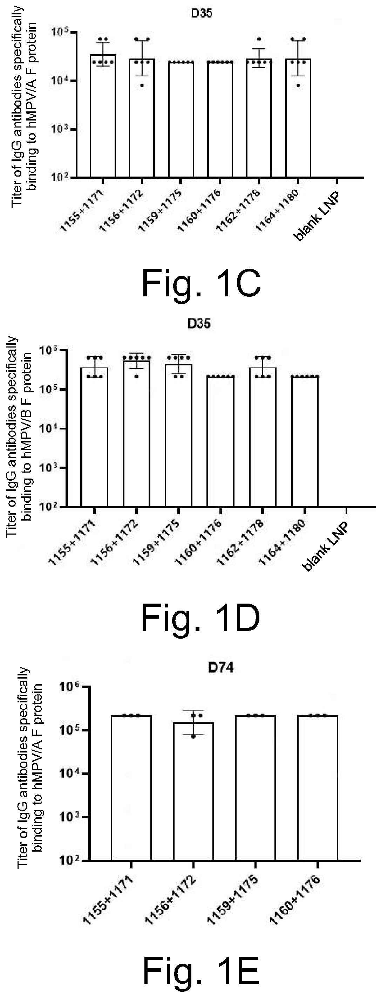

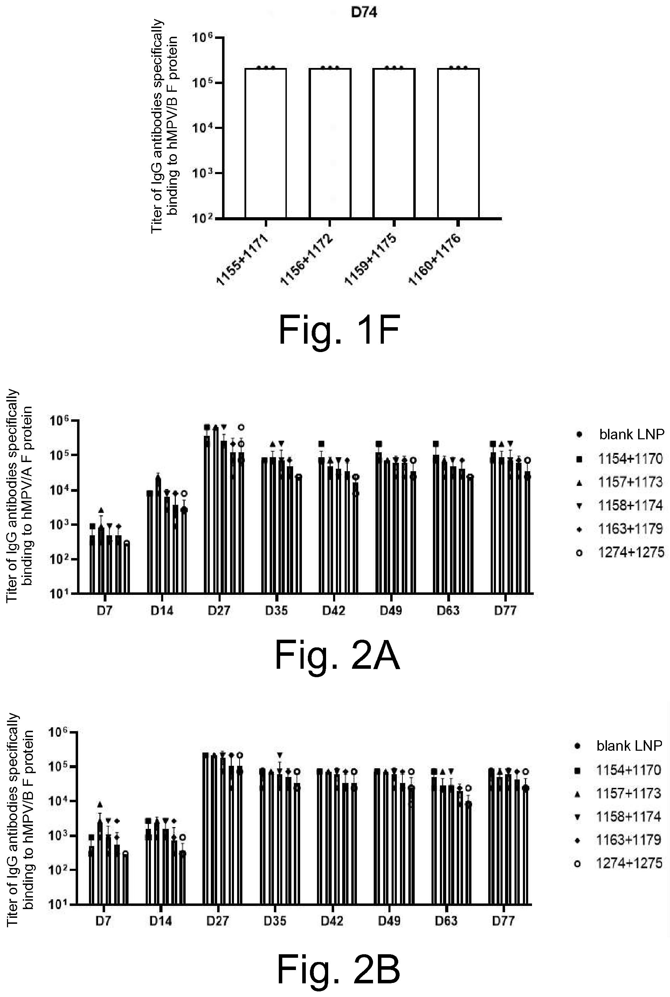

FIG. 1A to FIG. 1F show the detection of IgG antibody titers induced in mice immunized twice with different mRNA vaccines (FIG. 1C to FIG. 1F, GMT), wherein the day of the first immunization is recorded as day 0 (D0), and the second immunization is on day 22 (D22); in FIG. 1A to FIG. 1F, "hMPV/A F protein" represents the F protein of subtype A hMPV (the same applies to "hMPV/A F protein" present in other figures), "hMPV/B F protein" represents the F protein of subtype B hMPV (the same applies to "hMPV/B F protein" present in other figures), "1155+1171" represents a bivalent hMPV mRNA vaccine, which represents that the LNP formulation encapsulating mRNA numbered as 1155 and the LNP formulation encapsulating mRNA numbered as 1171 are mixed and injected into mice, the same applies to the bivalent hMPV mRNA vaccine represented by other groups, and the same applies to other unspecified figures.
FIG. 2A-FIG. 2B show the detection of IgG antibody titers induced in mice immunized twice with different mRNA vaccines (GMT), wherein the day of the first immunization is recorded as day 0 (D0), the second immunization is on day 21 (D21), FIG. 2A shows the titer of IgG antibodies specifically binding to hMPV/A F protein, and FIG. 2B shows the titer of IgG antibodies specifically binding to hMPV/B F protein.

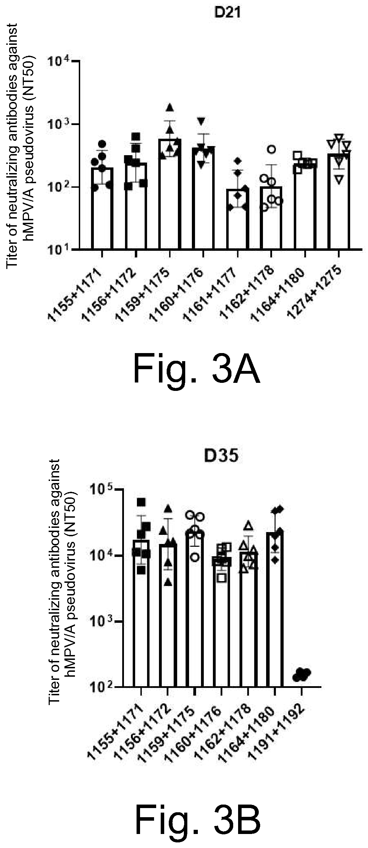

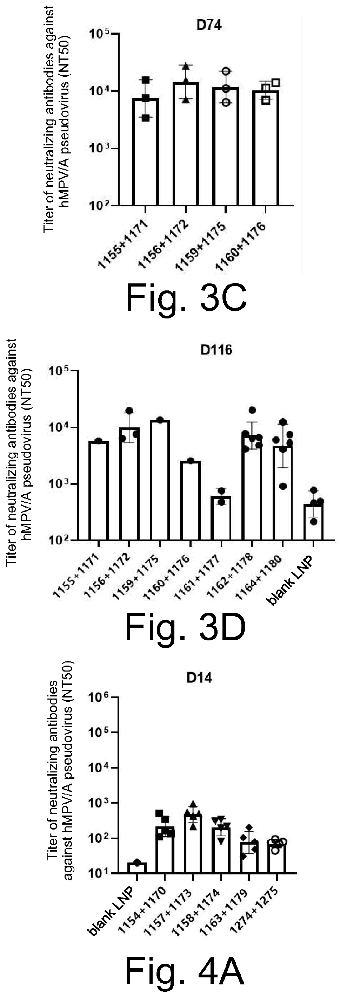

FIG. 3A to FIG. 3D show the detection of neutralizing antibodies against hMPV/A pseudovirus induced in mice immunized twice with different mRNA vaccines (GMT), wherein the antibody titer is NT50, wherein the day of the first immunization is recorded as D0, and the second immunization is on D22, "hMPV/A" represents subtype A hMPV.

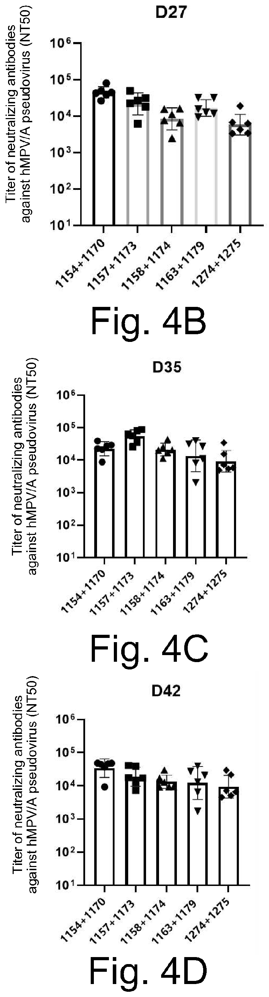

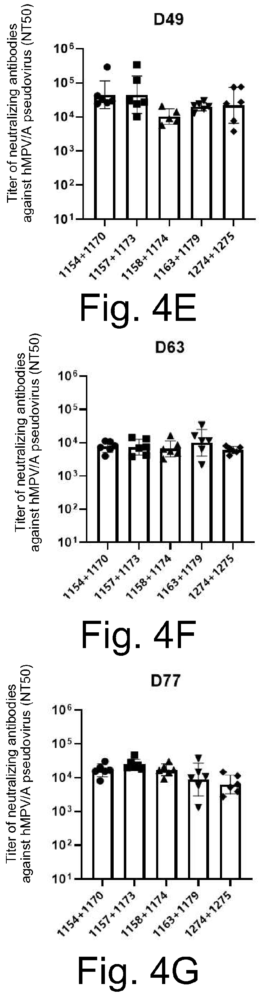

FIG. 4A to FIG. 4G show the detection of neutralizing antibody against hMPV/A pseudovirus induced in mice immunized twice with different mRNA vaccines (GMT), wherein the antibody titer is NT50, wherein the day of the first immunization is recorded as D0, and the second immunization is on D21, "hMPV/A" represents subtype A hMPV.

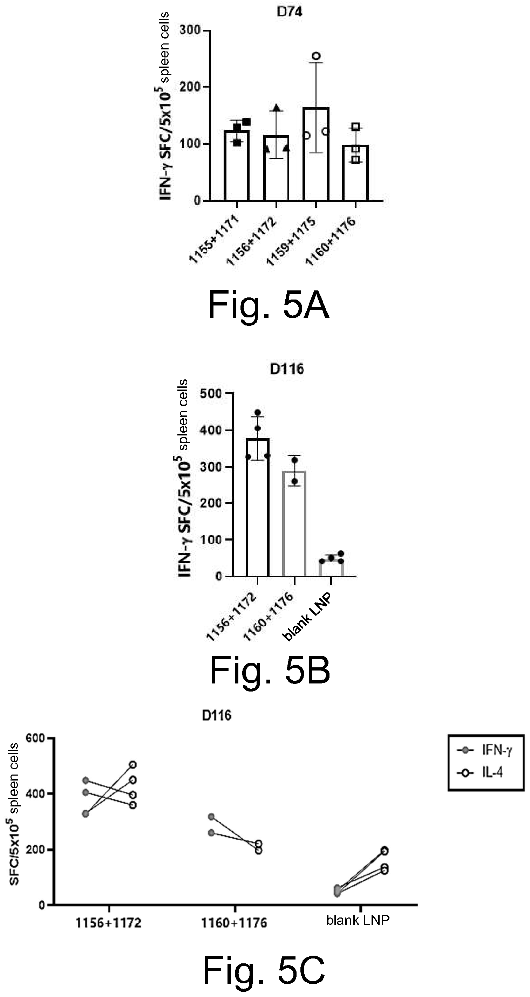

FIGS. 5A-5C show the ELISpot detection of spleen lymphocytes for mice immunized twice with different mRNA vaccines, wherein the day of the first immunization is recorded as D0, the second immunization is on D22, FIG. 5A shows the ELISpot detection of IFN-γ on D74, FIG. 5B shows the ELISpot detection of IFN-γ on D116, and FIG. 5C shows a comparison diagram of the ELISpot detections of IFN-γ and IL-4 on D116.

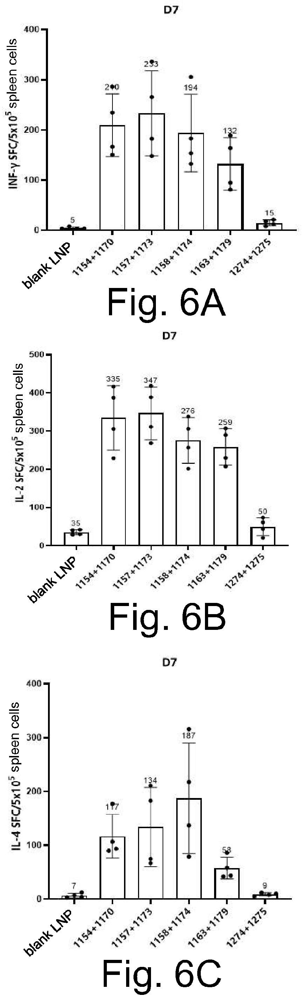

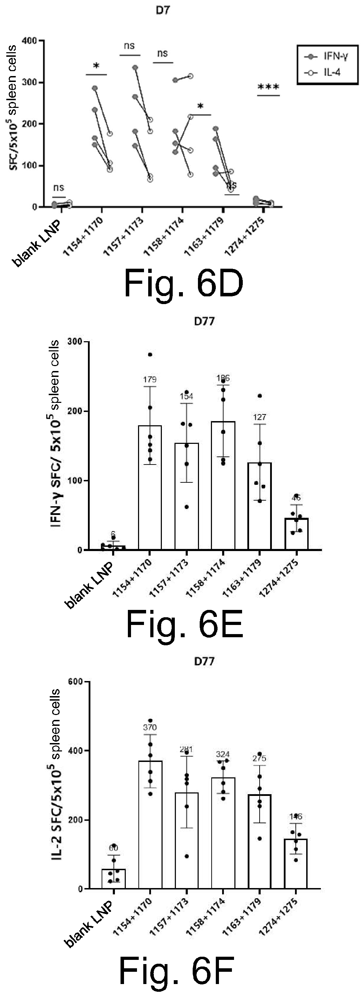

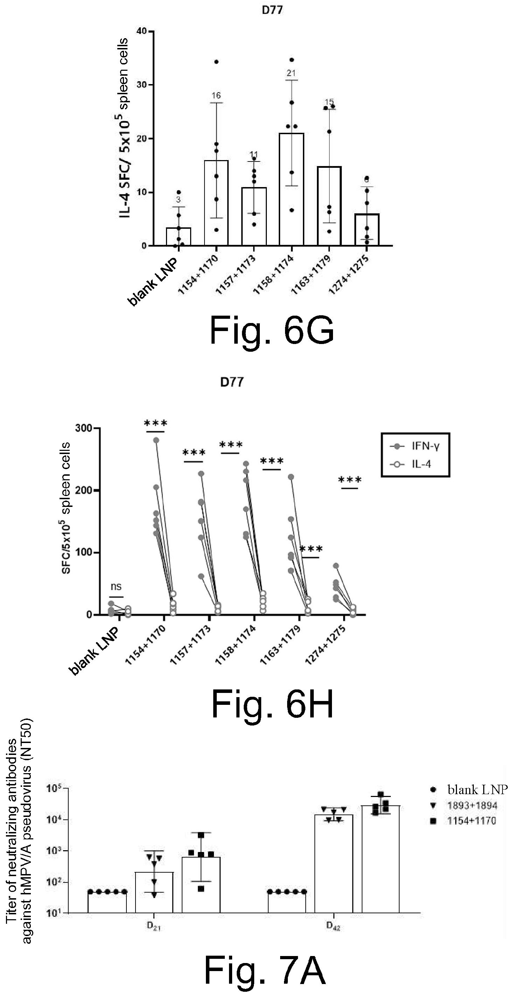

FIGS. 6A-6H show the ELISpot detection of spleen lymphocytes for mice immunized twice with different mRNA vaccines, wherein the day of the first immunization is recorded as D0, the second immunization is on D21, FIG. 6A is the ELISpot detection of IFN-γ on D7, FIG. 6B is the ELISpot detection of IL-2 on D7, FIG. 6C is the ELISpot detection of IL-4 on D7, FIG. 6D is a comparison diagram of the ELISpot detection of IFN-γ and IL-4 on D7, FIG. 6E is the ELISpot detection of IFN-γ on D77, FIG. 6F the ELISpot detection of IL-2 on D77, FIG. 6G is the ELISpot detection of IL-4 on D7, FIG. 6H is a comparison diagram of the ELISpot detection of IFN-γ and IL-4 on D77, "*" in FIGS. 6D and 6H represents P < 0.05, "***" represents P < 0.001, and "ns" represents P > 0.05.

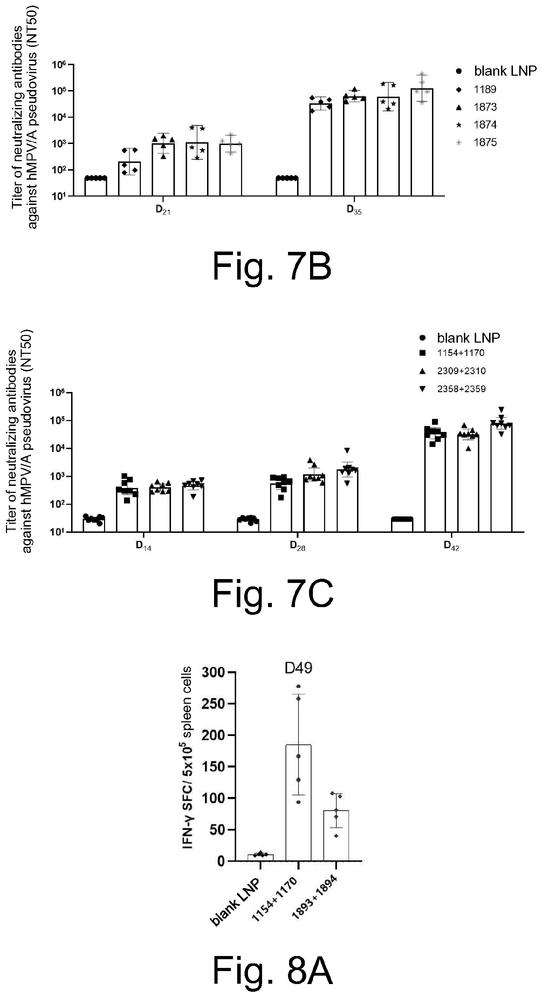

FIG. 7A to FIG. 7C show the detection of neutralizing antibodies against hMPV/A pseudovirus induced in mice immunized twice with different mRNA vaccines (GMT), wherein the antibody titer is NT50, wherein the day of the first immunization is recorded as D0, the second immunization is on D28, and hMPV/A represents subtype A hMPV.

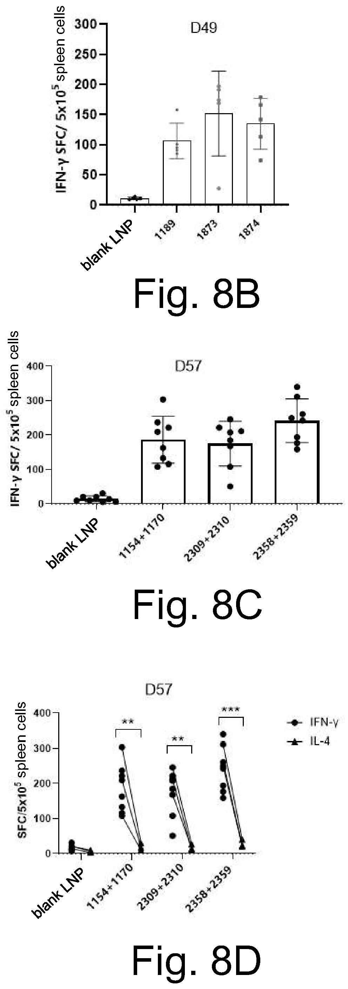

FIGS. 8A-8D show the ELISpot detection of spleen lymphocytes for mice immunized twice with different mRNA vaccines, wherein the day of the first immunization is recorded as D0, the second immunization is on D28, FIG. 8A and FIG. 8B are the ELISpot detection of IFN-γ on D49, FIG. 8C is the ELISpot detection of IFN-γ on D57, and FIG. 8D is a comparison diagram of the ELISpot detection of IFN-γ and IL-4 on D57.

### Detailed description of the invention

### I. Definition

All patents, patent applications, scientific publications, manufacturers' specifications and guidelines, etc., cited herein, whether supra or infra, are incorporated herein by reference in their entirety. Nothing herein is to be construed as an admission that the present disclosure is not entitled to antedate such disclosure.

Unless otherwise indicated, scientific and technical terms used herein have meanings commonly understood by those skilled in the art. Moreover, the terms related to protein and nucleic acid chemistry, molecular biology, cell and tissue culture and microbiology used herein are all terms widely used in the corresponding fields (see, for example, Molecular Cloning: A Laboratory Manual, 2nd Edition, J. Sambrook et al. eds., Cold Spring Harbor Laboratory Press, Cold Spring Harbor 1989). In addition, to better understand the present application, the following definitions and explanations of related terms are provided.

As used herein, the expressions "comprising," "including," "containing," and "having" are open-ended, meaning including the recited elements, steps, or components but not excluding other, unrecited elements, steps, or components. The expression "consisting of ... ..." excludes any unspecified element, step, or component. The expression "consisting essentially of ... ..." refers to ranges limited to the specified elements, steps, or components, plus the optional presence of elements, steps, or components that do not essentially affect the basic and novel nature of the claimed subject matter. It should be understood that the expressions "consisting essentially of" and "consisting of" are encompassed within the meaning of the expression "comprising."

As used herein, "a" and "an" and "the" and similar referents used in the context of describing the application (especially in the context of the following claims) are to be construed to cover both the singular and the plural, unless indicated otherwise in the context. The term "one or more" or "at least one" encompasses 1, 2, 3, 4, 5, 6, 7, 8, 9 or more. The term "at least one" or "one or more" encompasses 1, 2, 3, 4, 5, 6, 7, 8, 9 or more.

Numerical ranges recited herein are to be understood to encompass any and all sub-ranges subsumed therein. For example, a range of "1 to 10" should be understood to include not only the explicitly recited values 1 and 10, but also any single value (e.g., 2, 3, 4, 5, 6, 7, 8 and 9) and sub-range within the range of 1 to 10 (e.g., 1 to 2, 1.5 to 2.5, 1 to 3, 1.5 to 3.5, 2.5 to 4, 3 to 4.5, etc.). This principle also applies to ranges using only one numerical value as the minimum or maximum value.

As used herein, the terms "and/or," "any combination thereof," and grammatical equivalents thereof are used interchangeably. These terms may be expressed, specifically referring to any combination. For example, the following phrases "A, B, and/or C" or "A, B, C, or any combination thereof" may refer to "A alone; B alone; C alone; A and B; B and C; A and C; and A, B, and C".

As used herein, the term "naturally occurred" or "naturally occurring" refers to the fact that a substance is can be found in nature. For example, peptides, amino acids, proteins, or nucleic acids present in organisms, including viruses, and which can be isolated from natural sources and are not artificially modified in experiments are naturally occurring.

As used herein, the term "non-naturally occurring" as used herein in describing a nucleic acid is intended to mean that the nucleic acid is not found in nature. For example, a non-naturally occurring nucleic acid encoding a viral peptide or protein has at least one genetic alteration or chemical modification that is generally not found in a wild-type strain of the mentioned virus. Such genetic alteration includes, for example, the introduction of expressible nucleic acid sequences encoding peptide segments or polypeptides heterologous to the mentioned virus, other nucleic acid additions, nucleic acid deletions, nucleic acid substitutions, and/or other functional disruptions to the genetic material of the virus. Chemical modifications include, for example, one or more functional nucleotide analogs as described herein.

All methods described herein can be performed in any suitable order unless otherwise indicated.

As used herein, the term "wild-type" means that the sequence is naturally occurring and not artificially modified, including naturally occurring mutants.

The term "mutation" refers to the deletion, addition or substitution of an amino acid residue in the amino acid sequence of a protein or polypeptide compared to the amino acid sequence of a reference protein or polypeptide. Throughout the description and claims, an amino acid substitution at a particular position in a protein sequence is referred to with the designation "(amino acid residue in wild-type protein)(amino acid position)(amino acid residue in engineered protein)". For example, the designation A147C refers to the substitution of the alanine (A) residue at position 147 of the amino acid sequence of the reference protein with a cysteine (C) residue (in the mutant of the reference protein). In the presence of differences in amino acid residues at the same position between different wild-type sequences, the amino acid code preceding the position number may be omitted in the code, such as "147C".

As used herein, the term "% identity" or "% identity" refers to the percentage of nucleotides or amino acids that are identical in the optimal alignment between the sequences to be compared, the difference between the two sequences may be distributed over a local region (segment) or the entire length of the sequences to be compared. The identity between two sequences is typically determined after the optimal alignment to a segment or "comparison window." Optimal alignment may be performed manually or by means of algorithms known in the art. Algorithms known in the art include, but are not limited to, the local homology algorithms described by Smith and Waterman, 1981, Ads App. Math. 2, 482 and Neddleman and Wunsch, 1970, J. Mol. Biol. 48, 443, the similarity search methods described by Pearson and Lipman, 1988, Proc. Natl Acad. Sci. USA 88, 2444, or using computer programs such as GAP, BESTFIT, FASTA, BLAST P, BLAST N and T FASTA in the Wisconsin Genetics Software Package, Genetics Computer Group, 575 Science Drive, Madison, Wis. For example, the percentage identity of the two sequences may be determined using BLASTN or BLASTP algorithms publicly available to the National Center for Biotechnology Information (NCBI) website.

"% identity" or "% identity" can be obtained by determining the number of identical positions corresponding to the sequences to be compared, dividing this number by the number of positions compared (e.g., the number of positions in the reference sequence), and multiplying this result by 100 to obtain % identity. In some embodiments, a region of at least about 50%, at least about 55%, at least about 60%, at least about 65%, at least about 70%, at least about 75%, at least about 80%, at least about 85%, at least about 90%, at least about 95%, or about 100% gives a degree of identity. In some embodiments, the degree of identity is given for the entire length of the reference sequence. Alignment of sequence identity can be determined by tools known in the art, preferably optimal sequence alignment, for example, using Align, using standard settings, preferably EMBOSS:: needle, Matrix: Blosum62, Gap Open 10.0, Gap Extend 0.5.

As used herein, "nucleotide" includes deoxyribonucleotides, deoxyribonucleotides, deoxyribonucleotide derivatives, and ribonucleotide derivatives. As used herein, "ribonucleotide" is a substance constituting ribonucleic acid (RNA), which consists of a molecule of base, a molecule of pentose and a molecule of phosphate, which refers to a nucleotide having a hydroxyl group at the 2' position of the β-D-ribofuranosyl group and "deoxyribonucleotide" is a substance constituting deoxyribonucleic acid (DNA), which also consists of a molecule of base, a molecule of pentose and a molecule of phosphate, which refers to a nucleotide having a hydroxyl group at the 2' position of the β-D-ribofuranosyl group substituted by hydrogen, and is the main chemical component of a chromosome.

"Nucleotide" is generally referred to by a single letter representing the base therein, "A" or "A nucleotide" refers to an adenine deoxyribonucleotide or an adenine ribonucleotide containing adenine, "C" or "C nucleotide" refers to a cytosine deoxyribonucleotide or a cytosine ribonucleotide containing cytosine, "G" or "G nucleotide" refers to a guanine deoxyribonucleotide or a guanine ribonucleotide containing guanine, "U" or "U nucleotide" refers to a uracil ribonucleotide containing uracil, and "T" or "T nucleotide" refers to a thymine deoxyribonucleotide containing thymine.

As used herein, the term "nucleic acid" generally refers to any compound comprising a polymer of deoxyribonucleotides (deoxyribonucleic acid, abbreviated DNA) or a polymer of ribonucleotides (ribonucleic acid, abbreviated RNA), or a combination thereof. In addition, the nucleic acid herein also include a derivative of a nucleic acid. The term "derivative of a nucleic acid" includes chemical derivatization of a nucleic acid on the base of a nucleotide, on the saccharide, or on the phosphate, as well as nucleic acids containing non-native nucleotides and nucleotide analogs. In addition, herein, nucleic acids can be in the form of single or double stranded linear or covalent closed circular molecules.

"Polynucleotide sequence", "nucleic acid sequence" and "nucleotide sequence" can be used interchangeably to indicate the ordering of nucleotides in a polynucleotide. It should be understood by those skilled in the art that the coding strand (sense strand) of DNA and the RNA encoded thereby can be regarded as having the same nucleotide sequence, and the deoxythymidylate in the sequence of the coding strand of DNA corresponds to the uridylate in RNA sequence encoded thereby. RNA corresponding to DNA refers to a polynucleotide with T in DNA is completely replaced with U. mRNA corresponding to DNA refers to a polynucleotide with T in DNA is completely replaced with U.

A polynucleotide can comprise one or more segments (nucleic acid fragments) (e.g., 1, 2, 3, 4, 5, 6, 7, 8 segments). For example, a polynucleotide may comprise a segment encoding a polypeptide of interest. In particular embodiments, a polynucleotide may comprise a segment encoding a polypeptide of interest as well as regulatory segments including, but not limited to, segments for the regulation of transcription and the regulation of translation. In one embodiment, the regulatory segment comprises a polynucleotide corresponding to one or more of the following regulatory elements: a promoter, a 5' untranslated region (5'-UTR), a 3' untranslated region (3'-UTR), and a poly(A) tail.

As used herein, the term "promoter" refers to a polynucleotide located upstream of the 5' end of the coding region of a gene, which contains conserved sequences specific binded by RNA polymerase and required for the initiation of transcription, and can activate RNA polymerase, so that RNA polymerase can accurately bind to template DNA and has the specificity for the initiation of transcription. The promoter may be derived from viruses, bacteria, fungi, plants, insects, and animals. Representative examples of promoters include the phage T7 promoter, the phage T3 promoter, the SP6 promoter, the lac operon-promoter, the tac promoter, the SV40 late promoter, the SV40 early promoter, the RSV-LTR promoter, the CMV IE promoter, the SV40 early promoter, or the SV40 late promoter and the CMV IE promoter.

As used herein, the term "5' untranslated region" or "5'-UTR" may be an RNA sequence in mRNA that is upstream of the coding sequence and is not translated into a protein. The 5'-UTR in a gene typically starts at the transcription initiation site and ends at the nucleotide upstream of the translation initiation codon of the coding sequence. The 5'-UTR may comprise elements that control gene expression, such as a ribosome binding site, a 5'-terminal oligo pyrimidine bundle, and a translation initiation signal such as a Kozak sequence. mRNA can be post-transcriptional modified by adding a 5' cap. Thus, 5'-UTR in mature mRNA can also refer to the RNA sequence between the 5' cap and the start codon.

As used herein, the term "3' untranslated region" or "3'-UTR" may be an RNA sequence in mRNA that is downstream of the coding sequence and is not translated into a protein. The 3'-UTR in the mRNA is located between the stop codon of the coding sequence and the poly(A) sequence, e.g., starting at the nucleotide downstream of the stop codon and ending at the nucleotide upstream of the poly(A) sequence.

As used herein, the terms "polyadenylic acid," "poly(A) sequence," and "poly(A) tail" are used interchangeably, and the naturally occurring poly(A) sequences typically consists of adenine ribonucleotides. According to the present application, the term "modified poly(A) sequence" refers to a poly(A) sequence comprising a nucleotide or a segment of nucleotides other than adenine ribonucleotide. The poly(A) sequence is typically located at the 3' end of mRNA, e.g., the 3' end (downstream) of 3' -UTR .

As used herein, the term "5'-cap structure": 5'-cap structure is typically located at the 5' end of a mature mRNA. In some embodiments, the 5'-cap structure is linked to the 5'-end of the mRNA by a 5'-5'-triphosphate linkage. The 5'-cap structure is typically formed from modified (e.g., methylated) ribonucleotides, especially from guanine nucleotide derivatives. For example, m7GpppN (cap 0 or "cap0", which is a cap structure formed by the reaction of the 5' phosphate group of hnRNA with the 5' -phosphate group of m7GTP under the action of guanylyltransferase to form a 5',5'-phosphodiester bond), wherein N is the 5'-end nucleotide of the nucleic acid carrying the 5'-cap structure. In some embodiments, the 5'-cap structure includes, but is not limited to, cap 0, cap 1 (a cap structure formed by further methylation at 2' -OH of glycosyl in the nucleotide at position 1 of hnRNA on the basis of cap 0, or referred to as "cap1"), cap 2 (a cap structure formed by further methylation at 2' -OH of glycosyl in the nucleotide at position 2 of hnRNA on the basis of cap 1, or referred to as "cap2"), cap 4, cap 0 analog, cap 1 analog, cap 2 analog, or cap 4 analog.

As used herein, the term "expression" includes transcription and/or translation of a nucleotide sequence. Thus, expression may involve the production of transcripts and/or polypeptides. The term "transcription" refers to the process of transcribing the genetic codons in a DNA sequence into an RNA (transcript). The term "in vitro transcription" refers to the in vitro synthesis of RNA, particularly mRNA, in a cell-free system (e.g., in an appropriate cell extract) (see, e.g., Pardi N., Muramatsu H., Weissman D., Karikó K. (2013). In: Rabinovich P. (eds) Synthetic Messenger RNA and Cell Metabolism Modulation. Methods in Molecular Biology (Methods and Protocols), vol 969. Humana Press, Totowa, NJ.). A vector that can be used to produce a transcript are also referred to as "transcription vector" which contains regulatory sequences required for transcription. The term "transcription" encompasses "in vitro transcription."

As used herein, the term "host cell" refers to a cell for receiving, holding, replicating, expressing a polynucleotide or vector. The term "host cell" includes prokaryotic cells (e.g., E. coli) or eukaryotic cells (e.g., yeast cells and insect cells). For example, cells from human, mouse, hamster, pig, goat, primates. Cells can be derived from a variety types of tissues and include primary cells and cell lines. Some specific examples include keratinocytes, peripheral blood leukocytes, bone marrow stem cells, and embryonic stem cells. In other embodiments, the host cell is an antigen presenting cell, particularly a dendritic cell, a monocyte, or a macrophage. Nucleic acids can be present in a host cell as a single copy or as several copies. In some embodiments, the host cell may be a cell in which the polypeptide of the present application is expressed.

In the context of the present application, the term "plasmid" generally refers to a circular DNA molecule, but the term may also encompass linearized DNA molecules. Specifically, the term "plasmid" also encompasses molecules obtained by linearizing a circular plasmid by, for example, digesting the circular plasmid with a restriction enzyme, thereby converting the circular plasmid molecule into a linear molecule. Plasmids can be replicated, i.e., amplified in cells independently from the genetic information stored as chromosomal DNA, and can be used for cloning, i.e., for amplifying genetic information in bacterial cells. In an alternatively particular example, the DNA plasmid is a medium-copy or high-copy plasmid. In another alternatively particular example, the DNA plasmid is a high-copy plasmid. Examples of such high-copy plasmids include, for example, pUC and pTZ plasmids or any other plasmid (e.g., pMB1, p CoIE1) that includes an origin of replication that supports the high copy of the plasmid.

The term "vaccine" is typically understood to be a prophylactic or therapeutic material that provides at least one antigen or has an antigenic function, which can stimulate the adaptive immune system in the body to provide an adaptive immune response.

The term "treating" or the like is used herein to generally refer to obtaining a desired pharmacological and/or physiological effect. Thus, the treatment in the present application may involve the treatment of the condition of a certain disease, but may also involve prophylactic treatment in terms of the complete or partial prevention of the disease or the symptoms thereof. In some embodiments, the term "treating" should be understood to be therapeutic in partially or completely curing a disease and/or adverse effects and/or symptoms attributed to the disease. The treatment may also be a prophylactic or preventive treatment, i.e., a measure conducted to prevent a disease, e.g., to prevent an infection and/or the onset of a disease.

As used herein, the terms "subject" and "patient" may be used interchangeably. In certain embodiments, the subject is a mammal, such as human, non-human primates (e.g., simian, chimpanzee, monkey, and chimpanzee), domestic animals (including dog and cat and livestocks (e.g., horse, cow, pig, sheep, and goat)), or other mammals. Other mammals include, but are not limited to, mouse, rat, guinea pig, rabbit, hamster, etc. In particular embodiments, the subject is human. In one embodiment, the subject is a mammal (e.g., human) suffering from an infectious disease or a neoplastic disease . In another embodiment, the subject is a mammal (e.g., human) at risk of developing an infectious or neoplastic disease.

As used herein, the term "administering" refers to providing or administering an agent to a subject by any effective route. Exemplary routes of administration include, but are not limited to, one or more of the follows: injection (e.g., subcutaneous, intramuscular, intradermal, intraperitoneal, intrathecal, intracerebroventricular, or intravenous), oral, intrabile, sublingual, rectal, transdermal, intranasal, vaginal, and inhalation. When used to treat a disease, disorder, condition, or symptom thereof, the administration of the substance is typically performed after the onset of the disease, disorder, condition, or symptom thereof. When used to prevent a disease, disorder, condition or symptom, the administration of the substance is typically performed prior to the onset of the disease, disorder, condition or symptom.

Herein, some elements of the present application will be described. These elements are listed together with specific embodiments, however it should be understood that they can be combined in any manner and in any number to generate additional embodiments. The different described examples and preferred embodiments should not be construed as limiting the application to the explicitly described embodiments only. This specification should be understood to support and include embodiments that combine the explicitly described embodiments with any number of the disclosed and/or preferred elements. In addition, any arrangement and combination of all described elements in the present application should be construed as disclosed in the description of the present application unless indicated otherwise in the context. For example, in one embodiment, the nucleic acid comprises a polynucleotide encoding a mutant of MPV F protein, and the mutation of the mutant comprises: A147C and A159C relative to the wild-type MPV F protein, and in another embodiment, the nucleic acid is mRNA, then the following technical solution is also an embodiment claimed in the present application: a nucleic acid, comprising a polynucleotide encoding a mutant of MPV F protein, and the mutation of the mutant comprises: A147C and A159C relative to the wild-type MPV F protein, and the nucleic acid is mRNA.

### II. Nucleic Acid

The genome of hMPV is about 13kb in length, comprises 8 genes, and encodes the following 9 proteins in total: nucleoprotein (N protein for short), phosphoprotein (P protein for short), matrix protein (M protein for short), fusion protein (F protein for short), small hydrophobic protein (SH protein for short), attachment glycoprotein (G protein for short), RNA polymerase (L protein for short), transcription extension factor (M2-1 for short) and RNA synthesis regulatory factor (M2-2 for short) encoded by M2 gene, wherein F protein, G protein and SH protein are viral envelope glycoproteins, and F protein is essential for infection.

The F protein monomer encoded by the F gene is initially an inactive precursor F0, which is hydrolyzed into two subunits F1 and F2 under the action of a protease, and F1 and F2 are connected by a disulfide bond.

Given the substantial conservation of the amino acid sequence of the hMPV F protein, one of ordinary skill in the art can readily compare the amino acid positions between different native hMPV F protein sequences to determine the amino acid positions of the corresponding hMPV F protein between different hMPV strains and subtypes. Thus, the conservation of the amino acid sequence of the native hMPV F protein between strains and subtypes allows the amino acid sequence of a particular position in the hMPV F protein to be compared using the amino acid sequence of the reference hMPV F protein. For the purposes of the present disclosure (unless indicated otherwise in the context), the amino acid position of the hMPV F protein herein is given by reference to the sequence of hMPV F0 set forth in SEQ ID NO: 64 (i.e., the amino acid sequence of the full-length native F precursor polypeptide of the A2 subtype strain CAN97-83 (GenBank: AY297749.1)). However, those skilled in the art will understand that different hMPV F0s may have different numbering systems (e.g., there are additional amino acid residues added or deleted as compared to SEQ ID NO: 64). Thus, it should be understood that when a particular amino acid residue is referred to by a number, it is not limited to the amino acid residue located exactly at that numbered position when counted from a given amino acid sequence, but also includes any and all equivalent/corresponding amino acid residues in the amino acid sequence of the hMPV F protein, even if the amino acid residue is not at the same exactly numbered position (e.g., if the hMPV F protein sequence is shorter or longer than SEQ ID NO: 64, or has an insertion or deletion as compared to SEQ ID NO: 64).

One embodiment of the present disclosure provides a nucleic acid comprising a polynucleotide encoding a mutant of an MPV F protein, the mutant of MPV F protein (hereinafter referred to as "mutant") comprising or being disulfide bond mutations relative to a wild-type MPV F protein.

In some embodiments, the MPV F protein is hMPV F protein.

In some embodiments, the disulfide bond mutations comprise or are one or more of A147C and A159C, E146C and T160C, F168C and F196C, L165C and F196C, N145C and A161C, S149C and V157C, T59C and N180C, T150C and R156C, V52C and L165C, V55C and V169C, L58C and T174C, V148C and L158C. It should be noted that, in this specification, "a plurality of" means at least two, for example, two, three, four, five, or more.

In some embodiments, the disulfide bond mutations comprise or are one or more of A147C and A159C, L165C and F196C, N145C and A161C, S149C and V157C, T59C and N180C.

In some embodiments, the disulfide bond mutations are selected from one of A147C and A159C, L165C and F196C, N145C and A161C.

In some embodiments, the mutant is selected from one of the following (1) to (38):
(1) a mutant comprising an amino acid sequence set forth in SEQ ID NO: 1 or 2;
(2) a mutant comprising mutations A147C and A159C and comprising an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to the amino acid sequence set forth in SEQ ID NO: 1 or 2;
(3) a mutant comprising an amino acid sequence set forth in SEQ ID NO: 3 or 4;
(4) a mutant comprising mutations E146C and T160C and comprising an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to the amino acid sequence set forth in SEQ ID NO: 3 or 4;
(5) a mutant comprising an amino acid sequence set forth in SEQ ID NO: 5 or 6;
(6) a mutant comprising mutations F168C and F196C and comprising an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to the amino acid sequence set forth in SEQ ID NO: 5 or 6;
(7) a mutant comprising an amino acid sequence set forth in SEQ ID NO: 7 or 8;
(8) a mutant comprising mutations L165C and F196C and comprising an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to the amino acid sequence set forth in SEQ ID NO: 7 or 8;
(9) a mutant comprising an amino acid sequence set forth in SEQ ID NO: 9 or 10;
(10) a mutant comprising mutations N145C and A161C and comprising an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to the amino acid sequence set forth in SEQ ID NO: 9 or 10;
(11) a mutant comprising an amino acid sequence set forth in SEQ ID NO: 11 or 12;
(12) a mutant comprising mutations S149C and V157C and comprising an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to the amino acid sequence set forth in SEQ ID NO: 11or 12;
(13) a mutant comprising an amino acid sequence set forth in SEQ ID NO: 13 or 14;
(14) a mutant comprising mutations T59C and N180C and comprising an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to the amino acid sequence set forth in SEQ ID NO: 13 or 14;
(15) a mutant comprising an amino acid sequence set forth in SEQ ID NO: 15 or 16;
(16) a mutant comprising mutations T59C and N180C and comprising an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to the amino acid sequence set forth in SEQ ID NO: 15 or 16;
(17) a mutant comprising an amino acid sequence set forth in SEQ ID NO: 17 or 18;
(18) a mutant comprising mutations T59C and N180C and comprising an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to the amino acid sequence set forth in SEQ ID NO: 17 or 18;
(19) a mutant comprising an amino acid sequence set forth in SEQ ID NO: 19 or 20;
(20) a mutant comprising mutations T59C and N180C and comprising an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to the amino acid sequence set forth in SEQ ID NO: 19 or 20;
(21) a mutant comprising an amino acid sequence set forth in SEQ ID NO: 21 or 22;
(22) a mutant comprising mutations T150C and R156C and comprising an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to the amino acid sequence set forth in SEQ ID NO: 21 or 22;
(23) a mutant comprising an amino acid sequence set forth in SEQ ID NO: 23 or 24;
(24) a mutant comprising mutations V52C and L165C and comprising an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to the amino acid sequence set forth in SEQ ID NO: 23 or 24;
(25) a mutant comprising an amino acid sequence set forth in SEQ ID NO: 25 or 26;
(26) a mutant comprising mutations V55C and V169C and comprising an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to the amino acid sequence set forth in SEQ ID NO: 25 or 26;
(27) a mutant comprising an amino acid sequence set forth in SEQ ID NO: 27 or 28;
(28) a mutant comprising mutations L58C and T174C and comprising an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to the amino acid sequence set forth in SEQ ID NO: 27 or 28;
(29) a mutant comprising an amino acid sequence set forth in SEQ ID NO: 29 or 30;
(30) a mutant comprising mutations V148C and L158C and comprising an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to the amino acid sequence set forth in SEQ ID NO: 29 or 30;
(31) a mutant comprising an amino acid sequence set forth in SEQ ID NO: 81 or 82;
(32) a mutant comprising mutations V147C and L159C and comprising an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to the amino acid sequence set forth in SEQ ID NO: 81 or 82;
(33) a mutant comprising an amino acid sequence set forth in SEQ ID NO: 83;
(34) a mutant comprising mutations A147C and A159C and comprising an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to the amino acid sequence set forth in SEQ ID NO: 83;
(35) a mutant comprising an amino acid sequence set forth in SEQ ID NO: 84;
(36) a mutant comprising mutations L165C and F196C and comprising an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to the amino acid sequence set forth in SEQ ID NO: 84;
(37) a mutant comprising the amino acid sequence set forth in SEQ ID NO: 85; and
(38) a mutant comprising mutations N145C and A161C and comprising an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to the amino acid sequence set forth in SEQ ID NO: 85.

In some embodiments, the wild-type MPV is subtype A or subtype B.

In some embodiments, the wild-type MPV is wild-type hMPV.

In some embodiments, the wild-type hMPV is subtype A1, subtype A2, subtype B1, or subtype B2.

In some embodiments, the wild-type hMPV strain is CAN97-83 (GenBank: AY297749.1), NL/1/99 (GenBank: AY525843.1), NL/1/00 (GenBank: AF371337.2), CA/83/97 (GenBank: AY297749), JP/240/03 (GenBank: AY530095.1), CN/gz01/08 (GenBank: GQ153651.1). In an optional specific example, the amino acid sequence of the wild-type MPV F protein is set forth in SEQ ID NO: 64 or 65. It can be understood that in other embodiments, the wild-type MPV is not limited to those listed above, and may also be others.

In some embodiments, the mutant comprises an F1 polypeptide and an F2 polypeptide. The F1 and F2 polypeptides of the mutant of MPV protein, into which one or more mutations are introduced, may be derived from any wild-type MPV F protein known in the art or discovered in the future, including but not limited to the F proteins of MPV subtype A and subtype B (e.g., subtype A1, subtype A2, subtype B1, or subtype B2) or any other subtype. In addition, the F1 polypeptide and the F2 polypeptide may be from F proteins of MPV of the same genotype, or may be from F proteins of MPV of different genotypes. For example, F1 polypeptide is from F protein of subtype A MPV, F2 polypeptide is from F protein of subtype B MPV; or F1 polypeptide is from F protein of subtype B MPV, F2 polypeptide is from F protein of subtype A MPV.

In some embodiments, the mutant further comprises one or more of: a signal peptide, a ferritin polypeptide, a transmembrane domain (TM) of an MPV F protein (e.g., a transmembrane domain of a wild-type MPV F protein), and a trimerization domain (e.g., a foldon). As used herein, unless otherwise specified, a ferritin polypeptide refers to a polypeptide derived from ferritin, which may be a full-length ferritin, may be a mutated full-length ferritin, may be truncated ferritin, or may be a mutated truncated ferritin. Trimerization domains are used to promote the formation of a trimer of 3 F1/F2 heterodimers. The trimerization domain is not particularly limited, and may be, for example, a GCN4 leucine zipper, a trimerization motif from a pulmonary surfactant protein, a phage T4 fibrotin foldon, etc. In some embodiments, the foldon is located at the C-terminus of the F1 polypeptide. In an optional specific example, the amino acid sequence of the foldoner is: GYIPEAPRDGQAYVRKDGEWVLLSTFL (SEQ ID NO: 66). In some embodiments, the ferritin polypeptide is located at the C-terminus of the F1 polypeptide. In an optional specific example, the amino acid sequence of the ferritin polypeptide is set forth in SEQ ID NO: 67.

In some embodiments, the mutant further comprises a transmembrane domain (TM) of MPV F protein (e.g., the transmembrane domain of a wild-type MPV F protein).

In some embodiments, the mutant further comprises one or more of: a signal peptide, a transmembrane domain of MPV F protein, and a trimerization domain (e.g., a foldon). In some embodiments, the mutant comprises a signal peptide and a trimerization domain. In some embodiments, the mutant comprises a transmembrane domain of MPV F protein and a trimerization domain. In other embodiments, the mutant comprises a signal peptide, a transmembrane domain of MPV F protein and a trimerization domain.

In some embodiments, the mutant comprises one or more of: a signal peptide, a transmembrane domain of MPV F protein, and a ferritin polypeptide. In some embodiments, the mutant comprises a signal peptide and a ferritin polypeptide. In some embodiments, the mutant comprises a ferritin polypeptide and a transmembrane domain of MPV F protein. In other embodiments, the mutant comprises a signal peptide, a transmembrane domain of MPV F protein, and a ferritin polypeptide.

In some embodiments, the F1 polypeptide of the mutant has the same length as the full-length F1 polypeptide of the corresponding wild-type MPV F protein. The full-length F1 polypeptide of the mutant of MPV F protein corresponds to amino acid positions 103-539 of the native MPV F0 precursor, including (from N-terminal to C-terminal) the extracellular region (residues 103-488), the transmembrane domain (residues 489-513), and the cytoplasmic domain (residues 514-539).

In other embodiments, the F1 polypeptide of the mutant has a deletion relative to the full-length F1 polypeptide (or natural F1 polypeptide) of the corresponding wild-type MPV F protein. In some embodiments, the F1 polypeptide of the mutant has a deletion of one or more of partial extracellular region, partial transmembrane domain, the entire transmembrane domain, partial cytoplasmic domain, and the entire cytoplasmic domain relative to the full-length F1 polypeptide of the corresponding wild-type MPV F protein. For example, an optional sequence forward of amino acid residue 474 of a native F1 polypeptide may not be present in the F1 polypeptide of the mutant. For example, an optional sequence forward of amino acid residues 478 or 491 of the native F1 polypeptide may not be present in the F1 polypeptide of the mutant, i.e., the F1 polypeptide of the mutant is the one with 1-62 or 1-49 amino acid residues from the C-terminus of the native F1 polypeptide deleted. For example, the F1 polypeptide of the mutant lacks the entire cytoplasmic domain. For another example, the F1 polypeptide of the mutant lacks the cytoplasmic domain and a part or all of the transmembrane domain. For further example, the F1 polypeptide of the mutant has the amino acid residue at position 489, 490, 491, 510, 512, 513, 514, 515, 520, 525, or 530 to 539 deleted compared to the native F1 polypeptide. Typically, for mutants having a trimerization domain (such as a foldon) or a ferritin polypeptide attached thereto, amino acid residues at positions 489, 490, or 491 to 539 are absent. Thus, in some embodiments, the mutant is linked to a trimerization domain or ferritin polypeptide, and the F1 polypeptide of the mutant has the amino acid residues at positions 489, 490, or 491 to 539 deleted compared to the native F1 polypeptide. In other embodiments, the F1 polypeptide of the mutant comprises or consists of amino acid residues 103-490 of the native F0 polypeptide.

In some embodiments, the F2 polypeptide of the mutant may have the same length as the full-length F2 polypeptide of the corresponding wild-type MPV F protein, or may have a deletion, e.g., deletion of 1, 2, 3, 4, 5, 6, 7, 8, 9 or 10 amino acid residues at the N-terminus or C-terminus of the F2 polypeptide.

In addition, the above nucleic acid further comprises one or more of: a polynucleotide encoding a protease cleavage site (e.g., a thrombin cleavage site (LVPRGS, SEQ ID NO: 68)), a polynucleotide encoding a protein tag (e.g., a polynucleotide encoding a 6×His-tag (HHHHHH, SEQ ID NO: 69) and a streptomycin tag II (WSHPGFEK, SEQ ID NO: 70), a polynucleotide encoding a linker sequence (e.g., GG, SAIG, and GS)). Polypeptides encoded by these sequences are not necessary for the function of the MPV F protein (e.g., inducing an immune response). Such sequences are well known to those skilled in the art, and it is understood that these sequences are not limited to the above.

In some embodiments, the nucleic acid is an isolated nucleic acid.

In some embodiments, the nucleic acid is a non-naturally occurring nucleic acid.

In some embodiments, the nucleic acid is a codon-optimized polynucleotide. Methods for codon optimization are known in the art and may be used as provided herein. In some embodiments, codon optimization can be used to: match codon frequencies of the target and host organisms to ensure proper folding; bias GC content to increase mRNA stability or reduce secondary structure; minimize tandem repeat codons or base extension that can impair gene construction or expression; customize transcription and translation control regions; insert or remove protein transport sequences; remove/add post-translation modification sites in the encoded protein (e.g., glycosylation site); add, remove, or rearrange protein domains; insert or delete restriction sites; modify ribosome binding site and mRNA degradation site; modulate translation rate so that various domains of the protein can be properly folded; or reduce or eliminate problematic secondary structures within the polynucleotide. Codon optimization tools, algorithms, and services are known in the art, the non-limiting examples include services from GeneArt (Life Technologies), DNA2.0 (Menlo Park CA), and/or proprietary methods. In some embodiments, the sequence of open reading frame (ORF) is optimized using an optimization algorithm. In some embodiments, the codon-optimized nucleic acid has at least 50%, 55%, 60%, 65%, 70%, 75%, 80%, 85%, 90%, 95%, or 99% identity to the nucleic acid prior to codon optimization.

In some embodiments, the above nucleic acid is RNA. In some embodiments, the above nucleic acid is RNA, and the RNA contains an open reading frame (ORF).

In some embodiments, the above nucleic acid is RNA, and the RNA is mRNA.

In some embodiments, the above nucleic acid further comprises at least one of a 5'-cap structure, a 5'-UTR, a 3'-UTR, and a poly(A) tail. In an optional specific example, the nucleic acid further comprises a 5'-cap structure, 5'-UTR, 3'-UTR and a poly(A) tail.

In some embodiments, the 5'-cap structure is selected from at least one of m⁷GpppG, m₂^{7,3'}-^{O}GpppG, m⁷G ppp (5')N1 and m⁷Gppp(m^{2'}- ^{O})N 1; wherein "m⁷G" represents a 7-methyl guanosine cap nucleoside, "ppp" represents a triphosphate bond between the 5' carbon of the cap nucleoside and the first nucleotide of the primary RNA transcript, N1 is the 5' most nucleotide, "G" represents a guanosine, "7" represents a methyl group at the 7-position of guanosine, and "m^{2'}- ^{O}" represents a methyl group at the 2'-O position of nucleotide. In some embodiments, the 5'-cap structure is m⁷Gppp(5')N1 or m⁷Gppp(m^{2'}- ^{O})N1. It is understood that in other embodiments, the 5'-cap structure is not limited to the above.

In some embodiments, the DNA sequence corresponding to 5'-UTR is set forth in SEQ ID NO: 31; and/or, the DNA sequence corresponding to 3'-UTR is set forth in SEQ ID NO: 32. It should be noted that the "DNA sequence corresponding to 5'-UTR" refers to the nucleotide sequence of 5'-UTR in DNA form, and the same applies to the "DNA sequence corresponding to 3'-UTR" and the "DNA sequence corresponding to poly(A) tail" below.

It may be understood that, in another embodiment, the 5'-UTR and the 3'-UTR are not limited to the above, and may also be others, e.g., the 5'-UTRs and 3'-UTRs described in patents such as CN108291230A, CN104321432A, and CN107849574A.

In some embodiments, the nucleotides constituting the poly(A) tail comprise at least 20, at least 40, at least 80, at least 100, or at least 120 A nucleotides. In some embodiments, the nucleotides constituting the poly(A) tail comprise at least 20, at least 40, at least 80, at least 100, or at least 120 consecutive A nucleotides.

In some embodiments, the nucleotides constituting the poly(A) tail comprise one or more nucleotides other than the A nucleotide. In some embodiments, the poly(A) tail comprises two or more contiguous nucleotides other than the A nucleotide. In an optional specific example, the nucleotide DNA sequence corresponding to the poly(A) tail is set forth in SEQ ID NO: 33. It can be understood that the poly(A) tail contained in the nucleic acid of the present disclosure is not limited to the above, and may also be other poly(A) tails, such as the poly(A) tails described in patents such as US20170166905A1 and WO2020074642A1.

In some embodiments, the above nucleic acid does not contain a modified nucleotide.

In some embodiments, the above nucleic acid contains a modified nucleotide.

In some embodiments, the above nucleic acid contains a modified nucleoside. In some embodiments, the modified nucleoside included in the above nucleic acid includes at least one of a modified uridine, a modified cytidine, a modified adenosine, and a modified guanosine.

In some embodiments, the modified nucleoside in the nucleic acid is a modified uridine. In some embodiments, 0.1%~100% of the uridines in the nucleic acid described above are modified. In some embodiments, 80%~100% of the uridines are modified. In some embodiments, 100% of the uridines are modified. Exemplary modified uridine comprises pseudouridine (ψ), N1-methylpseudouridine, pyridin-4-one ribonucleoside, 5-aza-uridine, 6-aza-uridine, 2-thio-5-aza-uridine, 2-thio-uridine (s2U), 4-thio-uridine (s4U), 4-thio-uridine, 2-thio-pseudouridine, 5-hydroxy-uridine (ho5U), 5-aminoallyl-uridine, 5-halo-uridine (e.g., 5-iodo-uridine or 5-bromo-uridine), 3-methyl-uridine (m3U), 5-methoxy-uridine (mo5U), uridine-5-oxyacetic acid (cmo5U), uridine-5-oxyacetate methyl ester (mcmo5U), 5-carboxymethyl-uridine (cm5U), 1-carboxymethyl-pseudouridine, 5-carboxyhydroxymethyl-uridine (chm5U), 5-carboxyhydroxymethyl-uridine methyl ester (mchm5U), 5-methoxycarbonylmethyl-uridine (mcm5U), 5-methoxycarbonylmethyl-2-thio-uridine (mcm5s2U), 5-aminomethyl-2-thio-uridine (nm5s2U), 5-methylaminomethyl-uridine (mnm5U), 5-methylaminomethyl-2-thio-uridine (mnm5s2U), 5-methylaminomethyl-2-seleno-uridine (mnm5se2U), 5-carbamoylmethyl-uridine (ncm5U), 5-carboxymethylaminomethyl-uridine (cmnm5U), 5-carboxymethylaminomethyl-2-thio-uridine (cmnm5s2U), 5-propynyl-uridine, 1-propynyl-pseudouridine, 5-tauromethyl-uridine (τm5U), 1-Taurine methyl-pseudouridine, 5-Taurine methyl-2-thio-uridine (τm5s2U), 1-Taurine methyl-4-thio-uridine, 5-methyl-uridine (m5U, i.e., deoxythymine with nucleobase), 1-methyl-pseudouridine (m1ψ), 5-methyl-2-thio-uridine (m5s2U), 1-Methyl-4-thio-pseudouridine (m1s4ψ), 4-thio-1-methyl-pseudouridine, 3-methyl-pseudouridine (m3ψ), 2-thio-1-methyl-pseudouridine, 1-methyl-1-denitride-pseudouridine, 2-thio-1-methyl-1-denitride-pseudouridine, dihydrouridine (D), dihydropseudouridine, 5,6-dihydrouridine, 5-methyl-dihydrouridine (m5D), 2-thio-dihydrouridine, 2-thio-dihydrouridine, 2-methoxy-uridine, 2-methoxy-4-thio-uridine, 4-methoxy-uridine, 4-methoxy-2-thio-uridine, N1-methyl-uridine, 3-(3-amino-3-carboxypropyl) uridine (acp3U), 1-methyl-3-(3-amino-3-carboxypropyl) pseudouridine (acp3ψ), 5-(isopentenylaminomethyl) uridine (inm5U), 5- (isopentenylaminomethyl)-2-thio-uridine (inm5s2U), α-thio-uridine, 2'-O-methyl-uridine (Um), 5,2'-O- dimethyl-uridine (m5U m), 2'-O-methyl-pseudouridine (ψm), 2-thio-2'-O-methyl-uridine (s2Um), 5-methoxycarbonylmethyl-2'-O-methyl-uridine (mcm5Um), 5-carbamoylmethyl-2'-O-methyl-uridine (ncm5Um), 5-carboxymethylaminomethyl-2'-O-methyl-uridine (cmnm5Um), 3,2'-O-dimethyl-uridine (m3Um), 5-(isopentenylaminomethyl)-2'-O- methyl-uridine (inm5Um), 1-thio-uridine, deoxythymidine, 2'-F-ara-uridine, 2'-F-uridine, 2'-OH-ara-uridine, 5-(2-carbomethoxyvinyl) uridine, and 5-[3-(1-E-propenylamino) uridine.

In some embodiments, the modified nucleoside in the above nucleic acid is a modified cytidine. In some embodiments, 0.1%~100% of the cytidines in the above nucleic acid are modified. In some embodiments, 80%~100% of the cytidine are modified. In some embodiments, 100% of the cytidine are modified. Exemplary modified cytidine comprises 5-aza-cytidine, 6-aza-cytidine, pseudoisocytidine, 3-methyl-cytidine (m3C), N4-acetyl-cytidine (ac4C), 5-formyl-cytidine (f5C), N4-methyl-cytidine (m4C), 5-methyl-cytidine (m5C), 5-halo-cytidine (e.g., 5-iodo-cytidine), 5-hydroxymethyl-cytidine (hm5C), 1-methyl-pseudoisocytidine, pyrrolo-cytidine, pyrrolo-pseudoisocytidine, 2-thio-cytidine (s2C), 2-thio-5-methyl-cytidine, 4-thio-pseudoisocytidine, 4-thio-1-methyl-pseudoisocytidine, 4-thio-1-methyl-1-deaza-pseudoisocytidine, 1-methyl-1-deaza-pseudoisocytidine, zebularine, 5-azazebularine, 5-methyl-zebularine, 5-aza-2-thio-zebularine, 2-thio-zebularine, 2-methoxy-cytidine, 2-methoxy-5-methyl-cytidine, 4-methoxy-pseudoisocytidine, 4-ethoxy-1-methyl-pseudoisocytidine, Lysidine (K2C), α-thio-cytidine, 2'-O-methyl-cytidine (Cm), 5,2'-O-dimethyl-cytidine (m5Cm), N4-Acetyl-2'-O-methyl-cytidine (ac4Cm), N4,2'-O-dimethyl-cytidine (m4Cm), 5-formyl-2'-O-methyl-cytidine (f5Cm), N4,N4,2'-O-trimethyl-cytidine (m42Cm), 1-thio-cytidine, 2'-F-arabino-cytidine, 2'-F-cytidine, and 2'-OH-arabino-cytidine.

In some embodiments, the modified nucleoside in the above nucleic acid is a modified adenosine. In some embodiments, 0.1%~100% of the adenosines in above the nucleic acid are modified. In some embodiments, 80%~100% of the adenosines are modified. In some embodiments, 100% of the adenosines are modified. Exemplary modified adenosine comprises 2-amino-purine, 2,6-diaminopurine, 2-amino-6-halo-purine (e.g., 2-amino-6-chloro-purine), 6-halo-purine (e.g., 6-chloro-purine), 2-amino-6-methyl-purine, 8-azido-adenosine, 7-deaza-adenine, 7-deaza-8-aza-adenine, 7-deaza-2-amino-purine, 7-deaza-8-aza-2-amino-purine, 7-deaza-2,6-diaminopurine, 7-deaza-8-aza-2,6-diaminopurine, 1-methyl-adenosine (m1A), 2-methyl-adenine (m2A), N6-methyl-adenosine (m6A), 2-methylthio-N6-methyl-adenosine (ms2m6A), N6-isopentenyl-adenosine (i6A), 2-methylthio-N6-isopentenyl-adenosine (ms2i6A), N6- (cis-hydroxyisopentenyl) adenosine (io6A), 2-methylthio -N6- (cis-hydroxyisopentenyl) adenosine (ms2io6A), N6-glycinylcarbamoyl-adenosine (g6A), N6-threcarbamoyl-adenosine (t6A), N6-methyl -N6- threcarbamoyl-adenosine (m6t6A), 2-methylthio -N6-threyl carbamoyl-adenosine (ms2g6A), N6,N6-dimethyl-adenosine (m62A), N6-hydroxyvalylcarbamoyl-adenosine (hn6A), 2-methylthio-N6-hydroxyvalylcarbamoyl-adenosine (ms2hn6A), N6-acetyl-adenosine (ac6A), 7-methyl-adenine, 2-methylthio-adenine, 2-methoxy-adenine, α-thio-adenosine, 2'-O-methyl-adenosine (Am), N6,2'-O-dimethyl-adenosine (m6Am), N6,N6,2'-O- trimethyl-adenosine (m62Am), 1,2'-O-dimethyl-adenosine (m1Am), 2'-O-ribosyl-adenosine (phosphate) (Ar(p)), 2-amino-N6-methyl-purine, 1-thio-adenosine, 8-azido-adenosine, 2'-F-arabine-adenosine, 2'-F-adenosine, 2'-OH-arabine-adenosine, and N6-(19-amino-pentaoxa-nonadecyl)-adenosine.

In some embodiments, the modified nucleoside in the above nucleic acid is a modified guanosine. In some embodiments, 0.1%~100% of the guanosines in the above nucleic acid are modified. In some embodiments, 80%~100% of the guanosines are modified. In some embodiments, 100% of the guanosines are modified. Exemplary modified guanosine comprises inosine (I), 1-methyl-inosine (m1I), wyosine (imG), methyl wyosine (mimG), 4-demethyl-wyosine (imG-14), isowyosine (imG2), wybutosine (yW), peroxywybutosine (o2yW), hydroxywybutosine (OHyW), undermodified hydroxywybutosine (OHyW *), 7-deaza-guanosine, queuosine (Q), epoxy-queuosine (oQ), galactosyl-queuosine (galQ), mannosyl-queuosine (manQ), 7-cyano-7-deaza-guanosine (preQ0), 7-aminomethyl-7-deaza-guanosine (preQ1), archaeosine (G+), 7-deaza-8-aza-guanosine, 6-thio-guanosine, 6-thio-7-deaza-guanosine, 6-thio-7-deaza--8-aza-guanosine, 7-methyl-guanosine (m7G), 6-thio-7-methyl-guanosine, 7-methyl-inosine, 6-methoxy-guanosine, 1-methyl-guanosine (m1G), N2-methyl-guanosine (m2G), N2,N2-dimethyl-guanosine (m22G), N2,7-dimethyl-guanosine (m2,7G), N2,N2,7-trimethyl-guanosine (m2,2,7G), 8-oxo-guanosine, 7-methyl-8-oxo-guanosine, 1-methyl-6-thio-guanosine, N2-methyl-6-thio-guanosine, N2,N2-dimethyl-6-thioguanosine, α-thio-guanosine, 2'-O-methyl-guanosine (Gm), N2-methyl-2'-O-methylguanosine (m2Gm), N2,N2-dimethyl-2'-O-methyl-guanosine (m22Gm), 1-methyl-2'-O-methyl-guanosine (m1Gm), N2,7-dimethyl-2'-O-methyl-guanosine (M2,7Gm), 2'-O-methyl-inosine (Im), 1,2'-O-dimethyl-inosine (m1Im), 2'-O-ribosyl-guanosine (phosphate) (Gr(p)), 1-thio-guanosine, O6-methyl-guanosine, 2'-F-arabino-guanosine, and 2'-F-guanosine.

In some embodiments, the modified nucleotide in the above nucleic acid comprises a nucleotide containing an isotope.

In some embodiments, the above nucleic acid comprises a nucleotide containing an isotope of hydrogen. The isotope of hydrogen is not limited to deuterium, tritium. In addition, in some embodiments, the above nucleic acid further comprises or is a nucleotide containing an isotope of other elements than hydrogen, wherein the other elements include, but are not limited to, carbon, oxygen, nitrogen and phosphorus.

In some embodiments, the above nucleic acids comprise an RNA corresponding to a polynucleotide having at least 50%, 55%, 60%, 65%, 70%, 75%, 80%, 85%, 90%, 91%, 92%, 93%, 94%, 95%, 96%, 97%, 98% or 99% identity to a polynucleotide having a nucleotide sequence of one of SEQ ID NOs: 34-63 and 86-93 and encoding a corresponding MPV F protein set forth in SEQ ID NOs: 1-30 and 81-85. For example, the above nucleic acid comprises an RNA corresponding to a polynucleotide having at least 50%, 55%, 60%, 65%, 70%, 75%, 80%, 85%, 90%, 91%, 92%, 93%, 94%, 95%, 96%, 97%, 98% or 99% identity to a polynucleotide having a nucleotide sequence set forth in SEQ ID NO: 34 and encoding the MPV F protein set forth in SEQ ID NO: 1.

In some embodiments, the above nucleic acid comprises an RNA corresponding to a polynucleotide encoding a mutant having an amino acid sequence set forth in SEQ ID NO: 1 and having a nucleotide sequence having at least 50%, 55%, 60%, 65%, 70%, 75%, 80%, 85%, 90%, 91%, 92%, 93%, 94%, 95%, 96%, 97%, 98% or 99% identity to SEQ ID NO: 34 or 86.

In some embodiments, the above nucleic acid comprises an RNA corresponding to a polynucleotide encoding a mutant having an amino acid sequence set forth in SEQ ID NO: 2 and having a nucleotide sequence having at least 50%, 55%, 60%, 65%, 70%, 75%, 80%, 85%, 90%, 91%, 92%, 93%, 94%, 95%, 96%, 97%, 98% or 99% identity to SEQ ID NO: 35 or 87.

In some embodiments, the above nucleic acid comprises an RNA corresponding to a polynucleotide encoding a mutant having an amino acid sequence set forth in SEQ ID NO: 3 and having a nucleotide sequence having at least 50%, 55%, 60%, 65%, 70%, 75%, 80%, 85%, 90%, 91%, 92%, 93%, 94%, 95%, 96%, 97%, 98% or 99% identity to SEQ ID NO: 36.

In some embodiments, the above nucleic acid comprises an RNA corresponding to a polynucleotide encoding a mutant having an amino acid sequence set forth in SEQ ID NO: 4 and having a nucleotide sequence having at least 50%, 55%, 60%, 65%, 70%, 75%, 80%, 85%, 90%, 91%, 92%, 93%, 94%, 95%, 96%, 97%, 98% or 99% identity to SEQ ID NO: 37.

In some embodiments, the above nucleic acid comprises an RNA corresponding to a polynucleotide encoding a mutant having an amino acid sequence set forth in SEQ ID NO: 5 and having a nucleotide sequence having at least 50%, 55%, 60%, 65%, 70%, 75%, 80%, 85%, 90%, 91%, 92%, 93%, 94%, 95%, 96%, 97%, 98% or 99% identity to SEQ ID NO: 38.

In some embodiments, the above nucleic acid comprises an RNA corresponding to a polynucleotide encoding a mutant having an amino acid sequence set forth in SEQ ID NO: 6 and having a nucleotide sequence having at least 50%, 55%, 60%, 65%, 70%, 75%, 80%, 85%, 90%, 91%, 92%, 93%, 94%, 95%, 96%, 97%, 98% or 99% identity to SEQ ID NO: 39.

In some embodiments, the above nucleic acid comprises an RNA corresponding to a polynucleotide encoding a mutant having an amino acid sequence set forth in SEQ ID NO: 7 and having a nucleotide sequence having at least 50%, 55%, 60%, 65%, 70%, 75%, 80%, 85%, 90%, 91%, 92%, 93%, 94%, 95%, 96%, 97%, 98% or 99% identity to SEQ ID NO: 40.

In some embodiments, the above nucleic acid comprises an RNA corresponding to a polynucleotide encoding a mutant having an amino acid sequence set forth in SEQ ID NO: 8 and having a nucleotide sequence having at least 50%, 55%, 60%, 65%, 70%, 75%, 80%, 85%, 90%, 91%, 92%, 93%, 94%, 95%, 96%, 97%, 98% or 99% identity to SEQ ID NO: 41.

In some embodiments, the above nucleic acid comprises an RNA corresponding to a polynucleotide encoding a mutant having an amino acid sequence set forth in SEQ ID NO: 9 and having a nucleotide sequence having at least 50%, 55%, 60%, 65%, 70%, 75%, 80%, 85%, 90%, 91%, 92%, 93%, 94%, 95%, 96%, 97%, 98% or 99% identity to SEQ ID NO: 42.

In some embodiments, the above nucleic acid comprises an RNA corresponding to a polynucleotide encoding a mutant having an amino acid sequence set forth in SEQ ID NO: 10 and having a nucleotide sequence having at least 50%, 55%, 60%, 65%, 70%, 75%, 80%, 85%, 90%, 91%, 92%, 93%, 94%, 95%, 96%, 97%, 98% or 99% identity to SEQ ID NO: 43.

In some embodiments, the above nucleic acid comprises an RNA corresponding to a polynucleotide encoding a mutant having an amino acid sequence set forth in SEQ ID NO: 11 and having a nucleotide sequence having at least 50%, 55%, 60%, 65%, 70%, 75%, 80%, 85%, 90%, 91%, 92%, 93%, 94%, 95%, 96%, 97%, 98% or 99% identity to SEQ ID NO: 44.

In some embodiments, the above nucleic acid comprises an RNA corresponding to a polynucleotide encoding a mutant having an amino acid sequence set forth in SEQ ID NO: 12 and having a nucleotide sequence having at least 50%, 55%, 60%, 65%, 70%, 75%, 80%, 85%, 90%, 91%, 92%, 93%, 94%, 95%, 96%, 97%, 98% or 99% identity to SEQ ID NO: 45.

In some embodiments, the above nucleic acid comprises an RNA corresponding to a polynucleotide encoding a mutant having an amino acid sequence set forth in SEQ ID NO: 13 and having a nucleotide sequence having at least 50%, 55%, 60%, 65%, 70%, 75%, 80%, 85%, 90%, 91%, 92%, 93%, 94%, 95%, 96%, 97%, 98% or 99% identity to SEQ ID NO: 46.

In some embodiments, the above nucleic acid comprises an RNA corresponding to a polynucleotide encoding a mutant having an amino acid sequence set forth in SEQ ID NO: 14 and having a nucleotide sequence having at least 50%, 55%, 60%, 65%, 70%, 75%, 80%, 85%, 90%, 91%, 92%, 93%, 94%, 95%, 96%, 97%, 98% or 99% identity to SEQ ID NO: 47.

In some embodiments, the above nucleic acid comprises an RNA corresponding to a polynucleotide encoding a mutant having an amino acid sequence set forth in SEQ ID NO: 15 and having a nucleotide sequence having at least 50%, 55%, 60%, 65%, 70%, 75%, 80%, 85%, 90%, 91%, 92%, 93%, 94%, 95%, 96%, 97%, 98% or 99% identity to SEQ ID NO: 48.

In some embodiments, the above nucleic acid comprises an RNA corresponding to a polynucleotide encoding a mutant having an amino acid sequence set forth in SEQ ID NO: 16 and having a nucleotide sequence having at least 50%, 55%, 60%, 65%, 70%, 75%, 80%, 85%, 90%, 91%, 92%, 93%, 94%, 95%, 96%, 97%, 98% or 99% identity to SEQ ID NO: 49.

In some embodiments, the above nucleic acid comprises an RNA corresponding to a polynucleotide encoding a mutant having an amino acid sequence set forth in SEQ ID NO: 17 and having a nucleotide sequence having at least 50%, 55%, 60%, 65%, 70%, 75%, 80%, 85%, 90%, 91%, 92%, 93%, 94%, 95%, 96%, 97%, 98% or 99% identity to SEQ ID NO: 50.

In some embodiments, the above nucleic acid comprises an RNA corresponding to a polynucleotide encoding a mutant having an amino acid sequence set forth in SEQ ID NO: 18 and having a nucleotide sequence having at least 50%, 55%, 60%, 65%, 70%, 75%, 80%, 85%, 90%, 91%, 92%, 93%, 94%, 95%, 96%, 97%, 98% or 99% identity to SEQ ID NO: 51.

In some embodiments, the above nucleic acid comprises an RNA corresponding to a polynucleotide encoding a mutant having an amino acid sequence set forth in SEQ ID NO: 19 and having a nucleotide sequence having at least 50%, 55%, 60%, 65%, 70%, 75%, 80%, 85%, 90%, 91%, 92%, 93%, 94%, 95%, 96%, 97%, 98% or 99% identity to SEQ ID NO: 52.

In some embodiments, the above nucleic acid comprises an RNA corresponding to a polynucleotide encoding a mutant having an amino acid sequence set forth in SEQ ID NO: 20 and having a nucleotide sequence having at least 50%, 55%, 60%, 65%, 70%, 75%, 80%, 85%, 90%, 91%, 92%, 93%, 94%, 95%, 96%, 97%, 98% or 99% identity to SEQ ID NO: 53.

In some embodiments, the above nucleic acid comprises an RNA corresponding to a polynucleotide encoding a mutant having an amino acid sequence set forth in SEQ ID NO: 21 and having a nucleotide sequence having at least 50%, 55%, 60%, 65%, 70%, 75%, 80%, 85%, 90%, 91%, 92%, 93%, 94%, 95%, 96%, 97%, 98% or 99% identity to SEQ ID NO: 54.

In some embodiments, the above nucleic acid comprises an RNA corresponding to a polynucleotide encoding a mutant having an amino acid sequence set forth in SEQ ID NO: 22 and having a nucleotide sequence having at least 50%, 55%, 60%, 65%, 70%, 75%, 80%, 85%, 90%, 91%, 92%, 93%, 94%, 95%, 96%, 97%, 98% or 99% identity to SEQ ID NO: 55.

In some embodiments, the above nucleic acid comprises an RNA corresponding to a polynucleotide encoding a mutant having an amino acid sequence set forth in SEQ ID NO: 81 and having a nucleotide sequence having at least 50%, 55%, 60%, 65%, 70%, 75%, 80%, 85%, 90%, 91%, 92%, 93%, 94%, 95%, 96%, 97%, 98% or 99% identity to SEQ ID NO: 88.

In some embodiments, the above nucleic acid comprises an RNA corresponding to a polynucleotide encoding a mutant having an amino acid sequence set forth in SEQ ID NO: 82 and having a nucleotide sequence having at least 50%, 55%, 60%, 65%, 70%, 75%, 80%, 85%, 90%, 91%, 92%, 93%, 94%, 95%, 96%, 97%, 98% or 99% identity to SEQ ID NO: 89.

In some embodiments, the above nucleic acid comprises an RNA corresponding to a polynucleotide encoding a mutant having an amino acid sequence set forth in SEQ ID NO: 83 and having a nucleotide sequence having at least 50%, 55%, 60%, 65%, 70%, 75%, 80%, 85%, 90%, 91%, 92%, 93%, 94%, 95%, 96%, 97%, 98% or 99% identity to SEQ ID NO: 90 or 92.

In some embodiments, the above nucleic acid comprises an RNA corresponding to a polynucleotide encoding a mutant having an amino acid sequence set forth in SEQ ID NO: 84 and having a nucleotide sequence having at least 50%, 55%, 60%, 65%, 70%, 75%, 80%, 85%, 90%, 91%, 92%, 93%, 94%, 95%, 96%, 97%, 98% or 99% identity to SEQ ID NO: 91.

In some embodiments, the above nucleic acid comprises an RNA corresponding to a polynucleotide encoding a mutant having an amino acid sequence set forth in SEQ ID NO: 85 and having a nucleotide sequence having at least 50%, 55%, 60%, 65%, 70%, 75%, 80%, 85%, 90%, 91%, 92%, 93%, 94%, 95%, 96%, 97%, 98% or 99% identity to SEQ ID NO: 93.

In some embodiments, the above nucleic acid comprises an RNA corresponding to a polynucleotide having a nucleotide sequence set forth in any one of SEQ ID NOs: 34-63 and 86-93.

In some embodiments, the nucleic acid of any of the above embodiments is mRNA, and all of the uridines in the nucleic acid are replaced with N1-methylpseudouridine.

In some embodiments, the nucleic acid comprises an RNA corresponding to a polynucleotide having at least 50%, 55%, 60%, 65%, 70%, 75%, 80%, 85%, 90%, 91%, 92%, 93%, 94%, 95%, 96%, 97%, 98%, or 99% identity to a polynucleotide having a nucleotide sequence set forth in one of SEQ ID NOs: 34-63 and 86-93 and encoding a corresponding MPV F protein set forth in SEQ ID NOs: 1-30 and 81-85, and all of the uridines in the nucleic acid are replaced with N1-methylpseudouridine.

In some embodiments, the above nucleic acid comprises an RNA corresponding to a polynucleotide having a nucleotide sequence set forth in any one of SEQ ID NOs: 34-63 and 86-93, and all of the uridines in the nucleic acid is replaced with N1-methylpseudouridine.

In some embodiments, the above nucleic acid is mRNA, which comprises an RNA corresponding to a polynucleotide having a nucleotide sequence set forth in any one of SEQ ID NOs: 34-63 and 86-93, and all of the uridines in the nucleic acid are replaced with N1-methylpseudouridine.

In other embodiments, the above nucleic acid is DNA.

In some embodiments, the DNA is capable of being transcribed into RNA in vitro.

In some embodiments, the above nucleic acid comprises a polynucleotide having at least 50%, 55%, 60%, 65%, 70%, 75%, 80%, 85%, 90%, 91%, 92%, 93%, 94%, 95%, 96%, 97%, 98% or 99% identity to a polynucleotide having a nucleotide sequence set forth in one of SEQ ID NOs: 34-63 and 86-93 and encoding a corresponding MPV F protein set forth in SEQ ID NOs: 1-30 and 81-85.

In some embodiments, the above nucleic acid comprises a polynucleotide having a nucleotide sequence set forth in one of SEQ ID NOs: 34-63 and 86-93.

In some embodiments, the above nucleic acid comprises a polynucleotide encoding a mutant of MPV F protein. It should be noted that "a mutant of MPV F protein" here and below refers to any one of the plurality of mutants of MPV F proteins above.

In other embodiments, the above nucleic acid comprises a polynucleotide encoding a plurality of mutants of MPV F protein. At this point, the coding strand of the plurality of mutants of MPV F protein are located on the same nucleic acid strand, and the individual polynucleotides encoding the plurality of mutants of MPV F protein is directly linked or indirectly linked through a linking element. It should be noted that "a plurality of mutants of MPV F protein" here and below refers to any plurality (e.g., two, three, four or more) of the mutants of the plurality of MPV F proteins above.

For example, the plurality of mutants of MPV F protein are different MPV F proteins from the same subtype strain but with different mutations. Further for example, the plurality of mutants of MPV F protein are two mutants, the first mutant is MPV F protein derived from strain CAN97-83 and having A147C and A159C mutations, and the second mutant is MPV F protein derived from strain CAN97-83 and having L165C and F196C mutations. Correspondingly, the above nucleic acid comprises a polynucleotide encoding an MPV F protein derived from strain CAN97-83 and having A147C and A159C mutations and a polynucleotide encoding an MPV F protein derived from strain CAN97-83 and having L165C and F196C mutations.

For another example, the plurality of mutants of MPV F protein are different MPV F proteins from different subtype strains but with the same mutation. Further for example, the plurality of mutants of MPV F protein are two mutants, the first mutant is MPV F protein derived from strain CAN97-83 and having A147C and A159C mutations, and the second mutant is MPV F protein derived from strain NL/1/99 and having A147C and A159C mutations. Correspondingly, the above nucleic acid comprises a polynucleotide encoding an MPV F protein derived from strain CAN97-83 and having A147C and A159C mutations and a polynucleotide encoding an MPV F protein derived from strain NL/1/99 and having A147C and A159C mutations.

For another example, the plurality of mutants of MPV F protein are different MPV F proteins from different subtype strains and with different mutations. Further for example, the plurality of mutants of MPV F protein are two mutants, the first mutant is MPV F protein derived from strain CAN97-83 and having A147C and A159C mutations, and the second mutant is MPV F protein derived from strain NL/1/99 and having L165C and F196C mutations. Correspondingly, the above nucleic acid comprises a nucleic acid encoding a polynucleotide derived from the MPV F protein of strain CAN97-83 and having A147C and A159C mutations and a polynucleotide derived from the MPV F protein of strain NL/1/99 and having L165C and F196C mutations.

For another example, the plurality of mutants of MPV F protein are three mutants, the first mutant is a polynucleotide of MPV F protein derived from strain CAN97-83 and having A147C and A159C mutations, the second mutant is a polynucleotide of MPV F protein derived from strain NL/1/99 and having L165C and F196C mutations, and the third mutant is a polynucleotide of MPV F protein derived from strain CAN97-83 and having N145C and A161C mutations. Correspondingly, the above nucleic acid comprises a polynucleotide encoding an MPV F protein derived from strain CAN97-83 and having A147C and A159C mutations, a polynucleotide encoding an MPV F protein derived from strain NL/1/99 and having L165C and F196 C mutations, and a polynucleotide encoding an MPV F protein derived from strain CAN97-83 and having N145C and A161C mutations.

In some embodiments, the above nucleic acid further comprises a polynucleotide encoding a protein or polypeptide other than the mutant of MPV F protein. It can be understood that the protein or polypeptide other than the mutant of MPV F protein refers to a protein or polypeptide with biological significance (e.g., immunogenicity), such as another protein or polypeptide of MPV, or a mutant thereof (e.g., G protein of MPV or a mutant thereof), or a protein or polypeptide of another virus, or a mutant thereof.

In addition, it can be understood that in the above nucleic acids, whether the polynucleotide encoding one mutant of MPV F protein or the polynucleotides encoding the plurality of mutants of MPV F protein can have a plurality of (e.g., two or three) repeated units on the same strand. For example, for a polynucleotide encoding one of the mutants of the MPV F protein, the polynucleotide encoding one of the mutants of the MPV F protein is referred to as "A fragment" for short, and the same nucleic acid may contain a plurality of A fragments. For another example, for a polynucleotide encoding the plurality of mutants of MPV F protein, the polynucleotide encoding one of the mutants of MPV F protein is referred to as "A fragment" for short, and the polynucleotide encoding another mutant of MPV F protein is referred to as "B fragment" for short, then the same nucleic acid may contain a plurality of A fragments and a plurality of B fragments, may contain one A fragment and a plurality of B fragments, or may contain a plurality of A fragments and one B fragment.

In some embodiments, the above nucleic acid is any mRNA in Table 1. The mRNAs in Table 1 are mRNAs encoding a mutant of hMPV F protein, which comprises a Cap1 cap structure, 5'-UTR corresponding to the sequence set forth in SEQ ID NO: 31, 3'-UTR corresponding to the sequence set forth in SEQ ID NO: 32, a poly(A) tail corresponding to the sequence set forth in SEQ ID NO: 33, and a coding region for F protein corresponding to one of SEQ ID NOs: 34-63 and 86-93, wherein the coding region for F protein is RNA, and all of the uridines in all of the mRNAs in Table 1 are replaced with N1-methylpseudouridine. For example, the mRNA numbered as 1154 comprises a Cap1 cap structure, 5'-UTR corresponding to the sequence set forth in SEQ ID NO: 31, 3'-UTR corresponding to the sequence set forth in SEQ ID NO: 32, a poly(A) tail corresponding to the sequence set forth in SEQ ID NO: 33, and a coding region for F protein corresponding to the sequence set forth in SEQ ID NO: 34, and all of the uridines in the mRNA numbered as 1154 are replaced with N1-methylpseudouridine. In addition, in Table 1, all of the mRNAs in G7 group further contain a polynucleotide encoding Ferritin with an amino acid sequence set forth in SEQ ID NO: 67 at their C-terminus; and all of the mRNAs in G8 group further contain a polynucleotide encoding a foldon derived from T4 phage fibrin set forth in SEQ ID NO: 66 at their C-terminus.

**Table 1**

| Groups | mRNA number | Mutations contained in the mutant of the corresponding hMPV F protein of the mRNA | Amino acid sequence of the mutant of hMPV F protein (SEQ ID NO:) | DNA sequence corresponding to the coding region for hMPV F protein (SEQ ID NO:) |
|---|---|---|---|---|
| G1 | 1154 | A147C and A159C | 1 | 34 |
| | 1170 | A147C and A159C | 2 | 35 |
| G2 | 1155 | E146C and T160C | 3 | 36 |
| | 1171 | E146C and T160C | 4 | 37 |
| G3 | 1156 | F168C and F196C | 5 | 38 |
| | 1172 | F168C and F196C | 6 | 39 |
| G4 | 1157 | L165C and F196C | 7 | 40 |
| | 1173 | L165C and F196C | 8 | 41 |
| G5 | 1158 | N145C and A161C | 9 | 42 |
| | 1174 | N145C and A161C | 10 | 43 |
| G6 | 1159 | S149C and V157C | 11 | 44 |
| | 1175 | S149C and V157C | 12 | 45 |
| G7 | 1160 | T59C and N180C | 13 | 46 |
| | 1176 | T59C and N180C | 14 | 47 |
| G8 | 1161 | T59C and N180C | 15 | 48 |
| | 1177 | T59C and N180C | 16 | 49 |
| G9 | 1162 | T59C and N180C | 17 | 50 |
| | 1178 | T59C and N180C | 18 | 51 |
| G10 | 1163 | T59C and N180C | 19 | 52 |
| | 1179 | T59C and N180C | 20 | 53 |
| G11 | 1164 | T150C and R156C | 21 | 54 |
| | 1180 | T150C and R156C | 22 | 55 |
| G12 | 1165 | V52C and L165C | 23 | 56 |
| | 1169 | V52C and L165C | 24 | 57 |
| G13 | 1166 | V55C and V169C | 25 | 58 |
| | 1181 | V55C and V169C | 26 | 59 |
| G14 | 1182 | L58C and T174C | 27 | 60 |
| | 1167 | L58C and T174C | 28 | 61 |
| G15 | 1168 | V148C and L158C | 29 | 62 |
| | 1183 | V148C and L158C | 30 | 63 |
| G16 | 2309 | A147C and A159C | 1 | 86 |
| | 2310 | A147C and A159C | 2 | 87 |
| G17 | 2358 | A147C and A159C | 81 | 88 |
| | 2359 | A147C and A159C | 82 | 89 |
| G18 | 2311 | A147C and A159C | 83 | 90 |
| G19 | 1873 | L165C and F196C | 84 | 91 |
| G20 | 1874 | A147C and A159C | 83 | 92 |
| G21 | 1875 | N145C and A161C | 85 | 93 |

In some embodiments, the above nucleic acid comprises a stop codon. It is understood that, in other embodiments, the above nucleic acid does not contain a stop codon. When using the above nucleic acid that does not contain a stop codon, one of ordinary skill in the art knows that a stop codon (e.g., UGA or TGA) should be added at the appropriate location. It can be understood that the above nucleic acid may comprise one or more stop codons.

The nucleic acid comprises a polynucleotide encoding a mutant of MPV F protein with disulfide bond mutations, capable of inducing a specific immune response to prevent or at least reduce human metapneumovirus infection.

In some embodiments, the above nucleic acid or the mutant of MPV F protein encoded by the same can induce the generation of a significant humoral immune response by body, so that the body can produce a high level of IgG antibodies and neutralizing antibodies against MPV.

In some embodiments, the above nucleic acid or the mutant of MPV F protein encoded by the same can induce a significant IFN-γ positive cellular immune response. In some embodiments, the above nucleic acid or the mutant of MPV F protein encoded by the same can induce a significant IFN-γ positive cellular immune response, which is Th1 biased.

In some embodiments, the above nucleic acid or the mutant of MPV F protein encoded by the same can induce the production of neutralizing antibodies against subtype A hMPV.

In some embodiments, the above nucleic acid or the mutant of MPV F protein encoded by the same can induce the production of neutralizing antibodies against subtype B hMPV.

In some embodiments, the above nucleic acid or the mutant of MPV F protein encoded by the same can induce the production of neutralizing antibodies against subtype A hMPV and subtype B hMPV.

### III. Genetic Engineering Vector, Method for Preparing Nucleic Acid, Host Cell and Protein Mutant

The present disclosure also provides a genetic engineering vector comprising the nucleic acid of any of the above embodiments, or the genetic engineering vector comprises a polynucleotide capable of being transcribed into the nucleic acid of any of the above embodiments. In some embodiments, the genetic engineering vector is a plasmid, cosmid, virus, phage, or another vector conventionally used in genetic engineering. In an optional specific example, the genetic engineering vector is a plasmid. In some embodiments, the genetic engineering vector further comprises at least one or more of an origin of replication (ORI), a marker gene or fragment thereof, a reporter gene or fragment thereof, and a restriction site allowing the insertion of a DNA element. Restriction sites in the form of multiple cloning sites (MCS) are preferred.

In some embodiments, the genetic engineering vector is an expression vector. In some embodiments, the genetic engineering vector comprises a promoter, a 5'-UTR, a coding region for the mutant of the MPV F protein of any of the above embodiments (referred to as "mutant coding region"), a 3' -UTR, and a poly(A) tail, wherein the poly(A) tail, the promoter, the 5' - UTR, the mutant coding region, and the 3'-UTR are operably linked to each other.

In other embodiments, the genetic engineering vector is a cloning vector. It can be understood that the nucleic acid contained in the genetic engineering vector does not contain a 5'-cap structure.

The present disclosure also provides a method for preparing the nucleic acid of any one of the above embodiments, which comprises a step of introducing the genetic engineering vector of any one of the above embodiments (e.g., in the form of a plasmid) into a host cell (e.g., E. coli) and then culturing the host cell containing the nucleic acid.

In addition, the present disclosure also provides another method for preparing the above nucleic acid, which includes a step of preparing the nucleic acid according to the nucleotide sequence of the nucleic acid of any one of the above embodiments by chemical synthesis. It can be understood that the particular method for chemical synthesis may be a method known in the art, such as a solid phase phosphoramidite method.

It can be understood that the method for preparing the nucleic acid of any of the above embodiments is not limited to the above, and may also be other methods.

In addition, the present disclosure also provides a host cell comprising the nucleic acid of any of the above embodiments or the genetic engineering vector of any of the above embodiments.

In some embodiments, the host cell is an isolated cell.

In some embodiments, the host cell is used to store and/or amplify the nucleic acid described above.

In some embodiments, the host cell is a bacterial cell. Bacterial host cells include E. coli cells which are well known to those skilled in the art.

The host cell of the present disclosure can be prepared by transforming the genetic engineering vector of any of the above embodiments into a competent host cell. Competent host cells are cells that have the ability to uptake free extracellular genetic material (e.g., a DNA plasmid) independent of sequence. A variety of bacterial cells known to those skilled in the art are naturally capable of uptaking exogenous DNA from the environment and thus can serve as bacterial host cells according to the present disclosure. In addition, it is known to those skilled in the art that competent bacterial host cells can be obtained from natural non-competent bacterial cells using, for example, electroporation or chemicals (e.g., treatment with calcium ions with high temperature exposure). Upon uptake, the exogenous DNA is preferably neither degraded nor integrated in the genome of the bacterial host cell.

In addition, the present disclosure also provides a mutant of MPV F protein, which is encoded by the nucleic acid of any of the above embodiments.

In some embodiments, the mutant of MPV F protein is a single molecule or a multi-molecule complex (e.g., trimer).

In addition, the present disclosure also provides a MPV immunogen comprising a mutant of MPV F protein encoded by the nucleic acid of any of the above embodiments.

In some embodiments, the above MPV immunogen is a trimer. In some embodiments, the mutations of the monomers of the trimer are identical. In other embodiments, the mutations of the three monomers of the trimer are partially the same or completely different.

In addition, the present disclosure also provides a method of preparing a mutant of MPV F protein, comprising: transcribing a nucleic acid comprising a polynucleotide encoding the mutant of MPV F protein into mRNA; and translating the transcribed mRNA into a polypeptide or protein.

In some embodiments, the method of preparing the mutant of MPV F protein is performed completely or partially in vitro.

### IV. RNA and Methods for Preparing RNA

The present disclosure also provides a method of preparing RNA, comprising a step of transcription using the genetic engineering vector of any of the above embodiments of the present disclosure.

In some embodiments, the method of preparing RNA is an in vitro method. In some embodiments, the method of preparing RNA comprises contacting the genetic engineering vector (e.g., plasmid) of any of the above embodiments with an RNA polymerase. In some embodiments, the method of preparing RNA further comprises the step of linearizing the genetic engineering vector (e.g., plasmid). In some embodiments, prior to the linearization, the superhelical rate of the genetic engineering vector (e.g., plasmid) is at least about 85% (e.g., 85%, 86%, 87%, 88%, 89%, 90%, 91%, 92%, 93%, etc.). In some embodiments, the method of preparing RNA further comprises a step of purifying the linearized genetic engineering vector. In some embodiments, the method of preparing RNA further comprises a step of purifying RNA.

The present disclosure further provides another method of preparing RNA, comprising a step of preparing the RNA according to the nucleotide sequence of the RNA or DNA corresponding to any of the above embodiments by chemical synthesis. It can be understood that the chemical synthesis method may be a method known in the art, such as a solid phase phosphoramidite method.

In some embodiments, the nucleic acid is RNA.

In some embodiments, any of the above methods of preparing RNA further comprises a step of capping and optionally purifying the capping product. In some embodiments, the cap is a Cap1 cap. The Cap1 cap structure is as follows:
cap G¹G² = m⁷G-5'-ppp-5'-Gm2'-3'-p-[m7 = 7-CH₃; m2' = 2'-O-CH₃; -ppp- = -PO₂H-O-PO₂H-O-PO₂H)-; -p- = -PO₂H-].

The capping reaction is as follows:
pppN1(p)Nx-OH(3') → ppN1(pN)x-OH(3') + Pi
ppN1(pN)x-OH(3') + GTP → G(5')ppp(5')N1(pN)x-OH(3') + PPi
G(5')ppp(5')N1(pN)x-OH(3') + AdoMet → m7G(5')ppp(5')N1(pN)x-OH(3') + AdoHyc
m7GpppN1(pN)x-OH(3') + AdoMet → m7Gppp[m2'-O]N1(pN)x-OH(3') + AdoHyc.

In other embodiments, the method of preparing RNA is partially an in vitro method. The method of preparing RNA, at this moment, comprises the following steps: preparing the genetic engineering vector of any of the above embodiments in vitro; and introducing the genetic engineering vector into an organism (e.g., in the form of a plasmid). In some embodiments, the genetic engineering vector is encapsulated in a delivery carrier. At this moment, the genetic engineering vector is delivered into the organism via the delivery carrier.

In some embodiments, the RNA prepared in the method of preparing RNA according to any one of the above embodiments comprises a modified nucleoside or a modified nucleotide. Correspondingly, the raw material for preparing the RNA comprises one or more modified nucleosides or nucleotides. It can be understood that the amount and type of modified nucleosides or nucleotides correspond to the RNA to be prepared.

In addition, the present disclosure further provides an RNA prepared by the method of preparing an RNA according to any one of the above embodiments. In some embodiments, the RNA is mRNA.

### V. Nucleic Acid Composition

The present disclosure also provides a nucleic acid composition comprising a first nucleic acid or a first genetic engineering vector, the first nucleic acid is the nucleic acid of any of the above embodiments, and the first genetic engineering vector is the genetic engineering vector of any of the above embodiments.

In some embodiments, the first nucleic acid is RNA. In some embodiments, the first nucleic acid is mRNA.

In some embodiments, the nucleic acid composition comprises a first nucleic acid or a first genetic engineering vector, wherein the first nucleic acid is a nucleic acid comprising a polynucleotide encoding any one or more of the plurality of mutants of MPV F protein, and the first genetic engineering vector is a genetic engineering vector comprising a polynucleotide encoding any one or more of the plurality of mutants of MPV F protein.

In some embodiments, the nucleic acid composition comprises a first nucleic acid or a first genetic engineering vector, the first nucleic acid is a nucleic acid comprising a polynucleotide encoding one of the mutants of MPV F protein, and the first genetic engineering vector is a genetic engineering vector comprising a polynucleotide encoding one of the mutants of MPV F protein.

In other embodiments, the nucleic acid composition comprises a first nucleic acid or a first genetic engineering vector, wherein the first nucleic acid is a nucleic acid comprising polynucleotides encoding a plurality of mutants of the plurality of mutants of MPV F protein, and the first genetic engineering vector is a genetic engineering vector comprising polynucleotides encoding a plurality of mutants of the plurality of mutants of MPV F protein, wherein the polynucleotides encoding the plurality of mutants of the plurality of mutants of MPV F protein are located on the same nucleic acid strand.

In some embodiments, the nucleic acid composition comprises a plurality of first nucleic acids or a plurality of first genetic engineering vectors, the plurality of first nucleic acids are independent of each other or the plurality of first genetic engineering vectors are independent of each other, the plurality of first nucleic acids are used for encoding a plurality of mutants of the MPV F protein, and the plurality of first genetic engineering vectors are used for cloning or expressing a plurality of polynucleotides encoding the MPV F protein. It should be noted that "independent of each other" herein means that the plurality of first nucleic acids are not linked with a linking element, or the plurality of first genetic engineering vectors are not linked with a linking element. In this case, the polynucleotides encoding the plurality of mutants of MPV F protein are not on the same nucleic acid strand.

In some embodiments, the plurality of mutants of MPV F proteins corresponding to the plurality of first nucleic acids are MPV F proteins derived from different subtype strains and having the same mutation, MPV F proteins derived from different subtype strains and having different mutations, and/or MPV F proteins derived from the same subtype strain and having different mutations.

For example, the plurality of first nucleic acids are a nucleic acid (or referred to as first first nucleic acids) comprising a polynucleotide encoding MPV F proteins derived from strain CAN97-83 and having A147C and A159C mutations, and a nucleic acid (or referred to as second first nucleic acids) comprising a polynucleotide encoding MPV F proteins derived from strain NL/1/99 and having A147C and A159C mutations. In other words, the nucleic acid composition comprises two first nucleic acids, the first first nucleic acid is a nucleic acid comprising a polynucleotide encoding an MPV F protein derived from strain CAN97-83 and having A147C and A159C mutations, and the second first nucleic acid is a nucleic acid comprising a polynucleotide encoding an MPV F protein derived from strain NL/1/99 and having A147C and A159C mutations.

For example, the plurality of first nucleic acids are a nucleic acid comprising a polynucleotide encoding MPV F proteins derived from strain CAN97-83 and having A147C and A159C mutations (first first nucleic acids), and a nucleic acid comprising a polynucleotide encoding MPV F proteins derived from strain CAN97-83 and having L165C and F196C mutations (second first nucleic acids). In other words, the nucleic acid composition comprises two first nucleic acids, the first first nucleic acid is a nucleic acid comprising a polynucleotide encoding an MPV F protein derived from strain CAN97-83 and having A147C and A159C mutations, and the second first nucleic acid is a nucleic acid comprising a polynucleotide comprising an MPV F protein derived from strain CAN97-83 and having L165C and F196C mutations.

For example, the plurality of first nucleic acids are nucleic acids comprising polynucleotides encoding MPV F proteins derived from strain CAN97-83 and having A147C and A159C mutations (first first nucleic acids), and polynucleotides comprising MPV F proteins derived from strain NL/1/99 and having L165C and F196C mutations (second first nucleic acids). In other words, the nucleic acid composition comprises two first nucleic acids, the first first nucleic acid is a nucleic acid comprising a polynucleotide encoding an MPV F protein derived from strain CAN97-83 and having A147C and A159C mutations, and the second first nucleic acid is a nucleic acid comprising a polynucleotide comprising an MPV F protein derived from strain NL/1/99 and having L165C and F196C mutations.

For another example, the plurality of first nucleic acids are a nucleic acid comprising a polynucleotide encoding MPV F proteins derived from strain CAN97-83 and having A147C and A159C mutations, a nucleic acid comprising a polynucleotide encoding MPV F proteins derived from strain NL/1/99 and having A147C and A159C mutations, and a nucleic acid comprising a polynucleotide encoding MPV F proteins derived from strain CAN97-83 and having L165C and F196C mutations. In other words, the nucleic acid composition comprises three first nucleic acids, the first first nucleic acid is a nucleic acid comprising a polynucleotide encoding an MPV F protein derived from strain CAN97-83 and having A147C and A159C mutations, the second first nucleic acid is a nucleic acid comprising a polynucleotide encoding an MPV F protein derived from strain NL/1/99 and having A147C and A159C mutations, and the third first nucleic acid is a nucleic acid comprising a polynucleotide of an MPV F protein derived from strain CAN97-83 and having L165C and F196C mutations.

It can be understood that the nucleic acid composition is not limited to include one or more first nucleic acids or first genetic engineering vectors, but also includes other nucleic acids (such as nucleic acids encoding other immunogens) and/or other substances (such as buffers or lyoprotectants).

In some embodiments, the nucleic acid composition further comprises a second nucleic acid or a second vector, the second nucleic acid comprises a polynucleotide encoding a protein or polypeptide other than the mutant of MPV F protein, and the second vector comprises a polynucleotide encoding a protein or polypeptide other than the mutant of MPV F protein. The protein or polypeptide other than the mutant of MPV F protein are as described above. In some embodiments, the second nucleic acid is RNA. In some embodiments, the second nucleic acid is mRNA. It can be understood that the second nucleic acid contained in the nucleic acid composition is not limited to one, and may also be multiple. For example, the nucleic acid composition may further comprise a second nucleic acid comprising a polynucleotide encoding a protein or polypeptide other than the mutant of MPV F protein; or the nucleic acid composition may further comprise a plurality of second nucleic acids independent of each other and comprising a plurality of polynucleotides encoding a protein or polypeptide other than the mutant of MPV F protein. The second vector is also not limited to one, but may also be multiple.

### VI. Delivery carrier, Pharmaceutical Composition and Use thereof

The present disclosure also provides a delivery carrier comprising the nucleic acid of any of the above embodiments, the RNA of any of the above embodiments, the genetic engineering vector of any of the above embodiments, or the nucleic acid composition of any of the above embodiments.

In some embodiments, the nucleic acid is RNA. In some embodiments, the nucleic acid is mRNA.

In some embodiments, the delivery carrier is selected from one or a complex of several of the follows: lipid nanoparticles (LNPs), liposomes, cationic proteins, vesicles, microparticles, polymers, and micelles. In some embodiments, the delivery carrier is selected from one of lipid nanoparticles, liposomes, cationic proteins, vesicles, microparticles, polymers, and micelles.

In some embodiments, the delivery carrier is lipid nanoparticles (LNPs) comprising the nucleic acid of any of the above embodiments, the RNA of any of the above embodiments, the genetic engineering vector of any of the above embodiments, or the nucleic acid composition of any of the above embodiments.

In some embodiments, lipid nanoparticles refer to particles at the order of nanometers (e.g., 1nm-1000nm) that include one or more lipids.

In some embodiments, the lipid nanoparticles have an average diameter of 20nm~800nm, 20nm~500nm, 20nm~400nm, 20nm~300nm, 20nm~200nm, 20nm~100nm, 30nm~700nm, 30nm~500nm, 30nm~300nm, 30nm~200nm, 30nm~100nm, 40nm~800nm, 40nm~600nm, 40nm~500nm, 40nm~300nm, 40nm~200nm, 40nm~100nm, 50nm~800nm, 50nm~600nm, 50nm~500nm, 50nm~400nm, 50nm~300nm, 50nm~200nm, 50nm~100nm, 60nm~800nm, 60nm~600nm, 60nm~500nm, 60nm~400nm, 60nm~300nm, 60nm~200nm or 60nm~100nm. In some alternative specific examples, the lipid nanoparticles have an average diameter of 26nm, 31nm, 36nm, 41nm, 46nm, 51nm, 56nm, 61nm, 66nm, 71nm, 76nm, 81nm, 86nm, 91nm, 96nm, 101nm, 106nm, 111nm, 116nm, 121nm, 126nm, 131nm, 136nm, 141nm, 146nm, 151nm, 156nm, 161nm, 166nm, 171nm, 176nm, 181nm, 186nm, 191nm, 196nm, 201nm, 206nm, 211nm, 216nm, 221nm, 226nm, 231nm, 236nm, 241nm, 246nm or 249nm. Herein, the average diameter of the lipid nanoparticles may be expressed as a z-average determined by dynamic light scattering.

In some embodiments, the lipid nanoparticles comprise one of the follows: cationic lipid nanoparticles, solid lipid nanoparticles (SLN), nanostructured lipid carriers (NLC), and non-lamellar lipid nanoparticles. In an optional specific example, the lipid nanoparticles are cationic lipid nanoparticles.

In some embodiments, the lipid nanoparticles contain one or more of the following substances: cationic lipids, helper lipids, structural lipids, and polymer-lipids.

The term "cationic lipid" refers to a lipid that becomes positively charged by lowering the pH below the pKa of the ionizable group of the lipid, but becomes gradually neutral at higher pH values, where the positively charged lipid is able to bind to negatively charged nucleic acids. In certain embodiments, the cationic lipid comprises a zwitterionic lipid.

In some embodiments, the cationic lipid comprises the following compound (I), N-oxide thereof, salt thereof or isomer thereof: 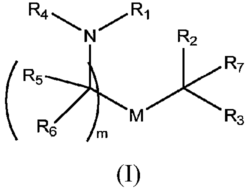 where:
R₁ is selected from the group consisting of C₅-C₃₀ alkyl, C₅-C₂₀ alkenyl, -R*YR", -YR", and -R"'M'R';
R₂ and R₃ are independently selected from the group consisting of H, C₁-C₁₄ alkyl, C₂-C₁₄ alkenyl, -R*YR", -YR", and -R*OR", or R₂ and R₃ together with the atom to which they are attached form a heterocyclic or carbocyclic ring;
R₄ is selected from the group consisting of hydrogen, C₃-C₆ carbocycle, -(CH₂)ₙQ, - (CH₂)ₙCHQR, -(CH₂)ₒC(R₁₀)₂(CH₂)ₙ₋ₒQ, -CHQR, -CQ(R)₂, and unsubstituted C₁-C₆ alkyl, wherein Q is selected from carbocycle, heterocycle, -OR, -O(CH₂)ₙN(R)₂, -C(O)OR, - OC(O)R, -CX₃, -CX₂H, -CXH₂, -CN, -N(R)₂, -C(O)N(R)₂, -N(R)C(O)R, -N(R)S(O)₂R, - N(R)C(O)N(R)₂, -N(R)C(S)N(R)₂, N(R)R₈, -N(R)S(O)₂R₈, -O(CH₂)ₙOR, -N(R)C( = NR₉)N(R)₂, -N(R)C( = CHR₉)N(R)₂, -OC(O)N(R)₂, -N(R)C(O)OR, -N(OR)C(O)R, - N(OR)S(O)₂R, -N(OR)C(O)OR, -N(OR)C(O)N(R)₂, -N(OR)C(S)N(R)₂, -N(OR)C( = NR₉)N(R)₂, -N(OR)C(=CHR₉)N(R)₂, -C(=NR₉)N(R)₂, -C(=NR₉)R, -C(O)N(R)OR and - C(R)N(R)₂C(O)OR, each o is independently selected from 1, 2, 3 and 4, and each n is independently selected from 1, 2, 3, 4 and 5;
each R₅ is independently selected from the group consisting of OH, C₁-C₃ alkyl, C₂-C₃ alkenyl, and H;
each R₆ is independently selected from the group consisting of OH, C₁-C₃ alkyl, C₂-C₃ alkenyl, and H;
M and M ' are independently selected from the group consisting of -C(O)O-, -OC(O)-, - OC(O)-M"-C(O)O-, -C(O)N(R')-, -N(R')C(O)-, -C(O)-, -C(S)-, -C(S)S-, -SC(S)-, -CH(OH)-, -P(O)(OR')O-, -S(O)₂-, -S-S-, aryl groups, and heteroaryl groups, wherein M" is a bond, C₁-C₁₃ alkyl, or C₂₋-C₁₃ alkenyl;
R₇ is selected from the group consisting of C₁₋₃ alkyl, C₂-C₃ alkenyl, and H;
R₈ is selected from the group consisting of C₃₋₆ carbocyclic and heterocyclic;
R₉ is selected from the group consisting of H, CN, NO₂, C₁-C₆ alkyl, -OR, -S(O)₂R, - S(O)₂N(R)₂, C₂-C₆ alkenyl, C₃-C₆ carbocyclic, and heterocyclic;
R₁₀ is selected from the group consisting of H, C₁-C₃ alkyl, and C₂-C₃ alkenyl;
each R is independently selected from the group consisting of C₁-C₃ alkyl, C₂-C₃ alkenyl, (CH₂)_{q}OR**, and H,
and each q is independently selected from 1, 2 and 3;
each R' is independently selected from the group consisting of C₁-C₁₈ alkyl, C₂-C₁₈ alkenyl, -R*YR", -YR", Hand 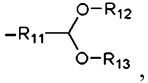 and R₁₁ is selected from the group consisting of C₁-C₁₂ alkylene and C₂-C₁₂ alkenylene, R₁₂ and R₁₃ are each independently selected from the group consisting of C₁-C₁₂ alkyl and C₂-C₁₂ alkenyl;
each R" is independently selected from the group consisting of C₃-C₁₅ alkyl and C₃-C₁₅ alkenyl;
each R"' is independently selected from the group consisting of C₃-C₁₅ alkylene and C₃-C₁₅ alkenylene;
each R* is independently selected from the group consisting of absent, C1-C12 alkylene, and C₂-C₁₂ alkenylene;
each R** is independently selected from the group consisting of absent, C₁-C₁₂ alkyl, and C₂-C₁₂ alkenyl;
each Y is independently a C₃-C₆ carbocyclic ring;
each X is independently selected from the group consisting of F, Cl, Br, and I; and
m is selected from 5, 6, 7, 8, 9, 10, 11, 12 and 13; and wherein when R₄ is -(CH₂)ₙQ, - (CH₂)ₙCHQR, -CHQR, or -CQ(R)₂, then (i) when n is 1, 2, 3, 4 or 5, Q is not -N(R)₂; or (ii) when n is 1 or 2, Q is not 5, 6 or 7-membered heterocycloalkyl.

In an optional specific example, the cationic lipid is the following compound (I), N-oxide thereof, salt thereof or isomer thereof: 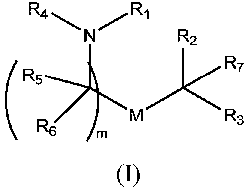 R₁-R₇, M and m are as defined above.

In some embodiments, the cationic lipid comprises the following compound (II), N-oxide thereof, salt thereof or isomer thereof: 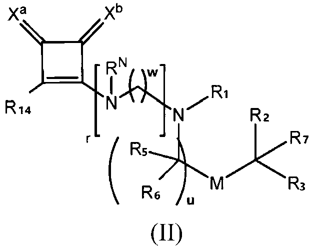 where:
R₁, R₂, R₃, R₅, R₆, M and R₇ are as described above,
R^{N} is H or C₁-C₃ alkyl;
X^{a} and X^{b} are each independently O or S;
R₁₄ is selected from the group consisting of H, halogen, -OH, R^{b}, -N(R^{b})₂, -CN, -N₃, - C(O)OH, -C(O)OR^{b}, -OC(O)R^{b}, -OR^{b}, -SR^{b}, -S(O)R^{b}, -S(O)OR^{b}, -S(O)₂OR^{b}, -NO₂, - S(O)₂N(R^{b})₂, -N(R^{b})S(O)₂R^{b}, -NH(CH₂)ₜ₁N(R^{b})₂, -NH(CH₂)ₚ₁O(CH₂)_{q1}N(R^{b})₂, - NH(CH₂)ₛ₁OR^{b}, -N((CH₂)_{S}OR^{b})₂ -N(R^{b})-carbocycle, -N(R^{b})-heterocycle, N(R^{b})-aryl, -N(R^{b})-heteroaryl, -N(R^{b})(CH₂)ₜ-carbocycle, -N(R^{b})(CH₂)ₜ₁-heterocycle, -N(R^{b})(CH₂)ₜ₁-aryl, - N(R^{b})(CH₂)ₜ₁-heteroaryl, carbocycle, heterocycle, aryl and heteroaryl;
each R^{d} is independently selected from the group consisting of C₁-C₃ alkyl, C₂-C₃ alkenyl, and H;
u is 5, 6, 7, 8, 9, 10, 11, 12 or 13;
w is 1, 2, 3, 4, 5, 6, 7, 8, 9 or 10;
r is 0 or 1;
t¹ is 1, 2, 3, 4 or 5;
p¹ is 1, 2, 5, 4 or 5;
q¹ is 1, 2, 5, 4 or 5; and
s¹ is 1, 2, 3, 4 or 5.

In an optional specific example, the cationic lipid is the following compound (II), its N-oxide, its salt or its isomer: 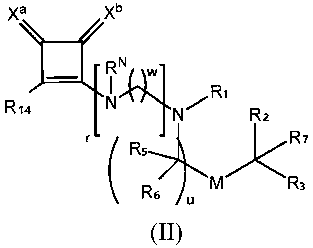 where R₁-R₃, R₅-R₇, R₁₄, X^{a}, X^{b}, R^{N}, M, u, w and r are as defined above.

In some embodiments, the cationic lipid comprises the following compound (III), N-oxide thereof, salt thereof or isomer thereof: 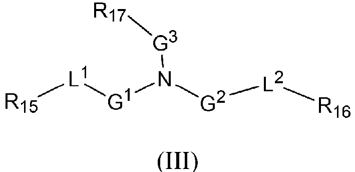 where:
one of L¹ or L² is -O(C=O)-, -(C=O)O-, -C(=O)-, -O-, -S(O)ₓ-, -S-S-, -C(=O)S-, - SC(=O)-, -NR^{a}C(=O)-, -C(=O)NR^{a}-, NR^{a}C(=O)NR^{a}-, -OC(=O)NR^{a}- or -NR^{a}C(=O)O-, and the other of L¹ or L² is -O(C=O)-, -(C=O)O-, -C(=O)-, -O-, -S(O)ₓ-, -S-S-, -C(=O)S-, SC(=O)-, -NR^{a}C(=O)-, -C(=O)NR'-, NR^{a}C(=O)NR^{a}-, -OC(=O)NR^{a}- or -NR^{a}C(=O)O- or a bond;
G¹ and G² are each independently unsubstituted C₁-C₁₂ alkylene or C₁-C₁₂ alkenylene;
G³ is C₁-C₂₄ alkylene, C₁-C₂₄ alkenylene, C₃-C₈ cycloalkylene, C₃-C₈ cycloalkenylene;
R^{a} is H or C₁-C₁₂ hydrocarbyl;
R₁₅ and R₁₆ are each independently C₆-C₂₄ alkyl or C₆-C₂₄ alkenyl;
R₁₇ is H, OR₁₈, CN, -C(=O)OR₁₉, -OC(=O)R₁₉ or -NR₁₈C(=O)R₁₉;
R₁₉ is a C₁-C₁₂ hydrocarbyl group;
R₁₈ is H or C₁-C₆ hydrocarbyl; and
x is 0, 1 or 2.

In an optional specific example, the cationic lipid is the following compound (III), its N-oxide, its salt or its isomer: 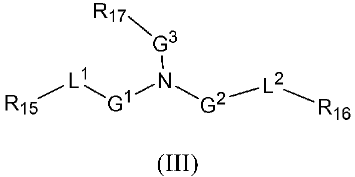 R₁₅-R₁₇, G¹-G³, L¹-L² are as defined above.

In some embodiments, the cationic lipid comprises the following compound (IV), or a pharmaceutically acceptable salt or stereoisomer thereof: 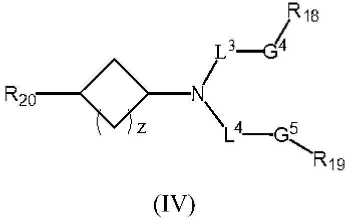 where:
L³ and L⁴ are the same or different, and are each independently C1-C12 alkylene, C2-C12 alkenylene or C₂-C₁₂ alkynylene; in some embodiments, L³ and L⁴ are the same or different, and are each independently C3-C10 alkylene, C3-C10 alkenylene or C3-C10 alkynylene; in some embodiments, L³ and L⁴ are the same or different, and are each independently C3-C10 alkylene; in some embodiments, L³ and L⁴ are the same or different, and are each independently C5-C8 alkylene;
G⁴ and G⁵ are the same or different, and are each independently -O-(C=O)-, -(C=O)-O-, -C(=O)-, -O-, -C(=O)-S-or-S-C(=O)-; in some embodiments, G⁴ and G⁵ are the same or different, and are each independently -O-(C=O)-, -(C=O)-O-, -C(=O)- or -O-; in some embodiments, G⁴ and G⁵ are the same or different, and are each independently selected from -O-(C=O)- or -(C=O)-O-;
R₁₈ and R₁₉ are the same or different, and are each independently C5-C27 alkyl or C5-C27 alkenyl containing one or more double bonds; in some embodiments, R₁₈ and R₁₉ are the same or different, and are each independently C8-C20 alkyl or C8-C20 alkenyl containing one or more double bonds; in some embodiments, R₁₈ and R₁₉ are the same or different, and are each independently C9-C17 alkyl or C9-C18 alkenyl containing one or two double bonds; in some embodiments, R₁₈ and R₁₉ are the same or different, and are each independently 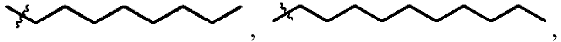 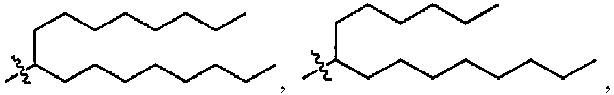 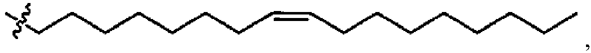 or 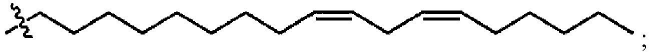
R₂₀ is halogen, hydroxyl, cyano, C1-C6 alkyl, nitro, C1-C6 alkoxy, C1-C6 alkylcarbonyloxy, C1-C6 alkoxycarbonyl, C1-C6 alkylaminocarbonyl or C1-C6 alkylcarbonylamino; in some embodiments, R₂₀ is halogen, hydroxyl, cyano, C1-C6 alkoxy, C1-C6 alkylcarbonyloxy, C1-C6 alkoxycarbonyl, C1-C6 alkylaminocarbonyl or C1-C6 alkylcarbonylamino; in some embodiments, R₂₀ is halogen, hydroxyl, cyano, C1-C4 alkoxy, C1-C4 alkylcarbonyloxy, C1-C4 alkoxycarbonyl, C1-C4 alkylaminocarbonyl or C1-C4 alkylcarbonylamino; in some embodiments, R₂₀ is fluorine, hydroxyl, cyano, methoxy, acetoxy, methoxycarbonyl, butylaminocarbonyl or acetamido;
z is 1, 2 or 3.

In some embodiments, the cationic lipid is the following compound (IV), or a pharmaceutically acceptable salt or stereoisomer thereof: 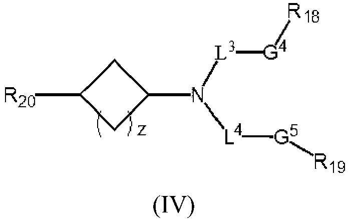 R₁₈-R₂₀, G⁴-G⁵, L³-L⁴ and z are as defined above.

In some embodiments, the cationic lipid comprises the following compound (IV-1), or a pharmaceutically acceptable salt or stereoisomer thereof:

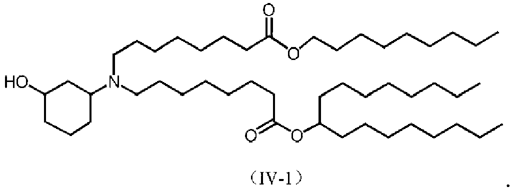

In some embodiments, the cationic lipid includes the following compounds, or pharmaceutically acceptable salts thereof: 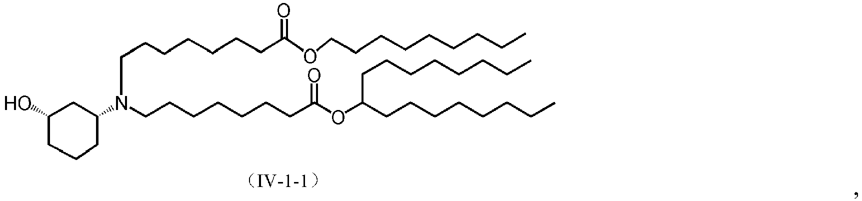 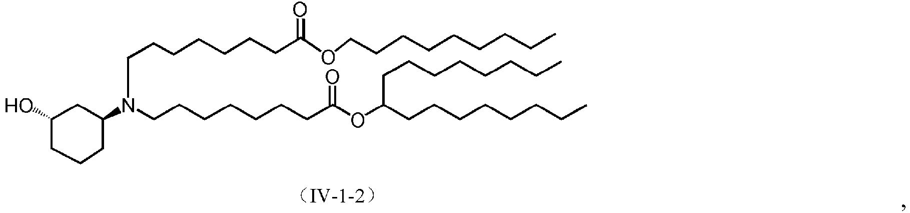 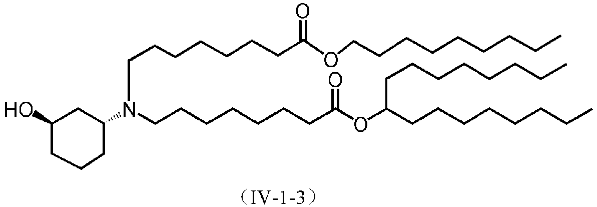 or

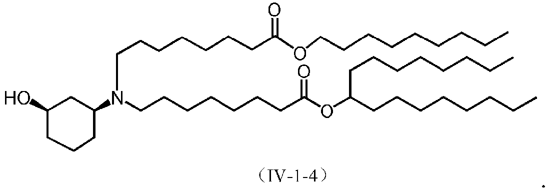

In some embodiments, the cationic lipid comprises one or more of ALC-0315 (CAS No. 2036272-55-4), SM-102 (CAS No. 2089251-47-6), and

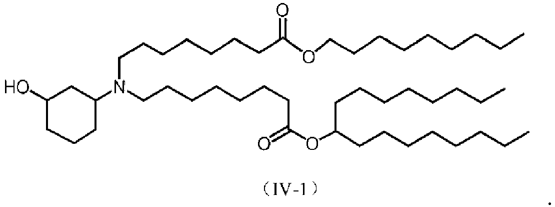

In some embodiments, the helper lipid of the lipid nanoparticle comprises a phospholipid. A phospholipid is generally semi-synthetic and may be naturally derived or chemically modified. In an optional specific example, the helper lipid of the lipid nanoparticle is a phospholipid. In some embodiments, the phospholipids of the lipid nanoparticles include one or more of the following: DSPC (distearoyl phosphatidylcholine), DOPE (dioleoyl phosphatidylethanolamine), DOPC (dioleoyl phosphatidylcholine), DOPS (dioleoyl phosphatidylserine), DSPG (1,2-dioctadecanoyl-sn-glycero-3-phospho-(1'-rac-glycerol)), DPPG (dipalmitoyl phosphatidylglycerol), DPPC (dipalmitoyl phosphatidylcholine), DGTS (1,2-dipalmitoyl-sn-glycero-3-O-4'-(N,N,N-trimethyl) homoserine), and lysophospholipids. In some embodiments, the helper lipid of the lipid nanoparticle is one or more selected from the group consisting of DSPC, DOPE, DOPC, and DOPS. In some embodiments, the helper lipid of the lipid nanoparticle is DSPC and/or DOPE.

In some embodiments, the structural lipid of the lipid nanoparticle includes a sterol. In an optional specific example, the structural lipid of the lipid nanoparticle is a sterol. In some embodiments, the sterol of the lipid nanoparticle includes one or more of the follows: 20α-hydroxycholesterol, cholesterol, cholesterol esters, sterols hormones, sterols vitamins, bile acids, cholesterol, ergosterol, β-sitosterol, and oxidized cholesterol derivatives. In some embodiments, the structural lipid of the lipid nanoparticle comprises at least one of cholesterol, cholesterol ester, sterol hormone, sterol vitamin, and bile acid. In some embodiments, the structural lipid of the lipid nanoparticles is cholesterol. In an alternative specific example, the structural lipid of the lipid nanoparticles is high purity cholesterol, in particular injection-grade high purity cholesterol, such as CHO-HP (produced by AVT). In other embodiments, the structural lipid is 20α-hydroxycholesterol.

A polymer-lipid refers to a conjugate comprising a polymer and a lipid coupled to the polymer. The polymer-lipid (e.g., polyethylene glycol-lipid) in the lipid nanoparticles can improve the stability of the lipid nanoparticles in vivo.

In some embodiments, the lipids of the polymer-lipid used to form the lipid nanoparticles include one or more of 1,2-dimyristoyl-sn-glycerol (DMG), distearoyl-phosphatidylethanolamine (DSPE), diacylglycerol (DAG), dialkyloxypropyl (DAA), phospholipids, ceramide (Cer), 1,2-distearoyl-rac-glycerol (DSG), and 1,2-dipalmitoyl-rac-glycero (DPG).

In some embodiments, the polymer-lipid polymer used to form the lipid nanoparticles includes one or both of a hydrophilic polymer and a amphoteric polymer .

In some embodiments, the polymer-lipid polymer used to form the lipid nanoparticles is a hydrophilic polymer. In other embodiments, the polymer-lipid polymer used to form the lipid nanoparticles is a amphoteric polymer .

In some embodiments, the hydrophilic polymer includes one or more of the following: polyethylene glycol (PEG), poly (oxazolines)(POX), poly (glycerols) (PGs), poly (hydroxypropyl methacrylate) (PHPMA), poly (2-hydroxy ethyl methacrylate) (PHEMA), poly (N- (2-hydroxy propyl) methacrylamide) (HPMA), poly (vinylpyrrolidone) (PVP), poly(N,N-dimethyl acrylamide) (PDMA), poly(N-acryloyl morpholine) (PAcM), polyaminoacids, glycosaminoglycans (GAGs), heparin, hyaluronic acid (HA), polysialic acid (PSA), elastin-like polypeptides (ELPs), and serum albumin and CD47.

Correspondingly, polymer-lipids include one or more of the follows: polyethylene glycol-lipids (PEG-lipids), polyoxazoline-lipids, polyglycerol-lipids, polyhydroxypropyl methacrylate-lipids, poly(2-hydroxyethyl methacrylate)-lipids, poly(N-(2-hydroxypropyl) methacrylamide)-lipids, polyvinylpyrrolidone-lipids, poly(N,N-dimethylacrylamide)-lipids, poly(N-acryloylmorpholine)-lipids, glycosaminoglycan-lipids, heparin-lipids, hyaluronic acid-lipids, polysialyl-lipids, elastidin-like lipids, serum albumin-lipids, and CD47-lipids. It should be noted that "PEG-lipid" is a conjugate of polyethylene glycol and lipid, "polyoxazoline-lipid" refers to a conjugate formed by coupling polyoxazoline with lipid, "polyglycerol-lipid" refers to a conjugate formed by coupling polyglycerol with lipid, and the same applies to other polymer-lipids. In an optional specific example, the hydrophilic polymer comprises polyethylene glycol.

In some embodiments, the polymer-lipid comprises a PEG-lipid. In an alternative specific example, the polymer-lipid is a PEG-lipid. In some embodiments, PEG- lipids include one or more of PEG-myristoyl diglyceride (PEG-DMG), PEG-distearoyl phosphatidylethanolamine (PEG-DSPE), PEG-diacylglycerol (PEG-DAG), PEG-dialkyloxypropyl (PEG-DAA), PEG-phospholipids, PEG-ceramide (PEG-Cer), PEG-1,2-distearoyl-rac-glycerol (PEG-DSG), and PEG-1,2-dipalmitoyl-rac-glycerol (PEG-DPG). The PEG-lipid is preferably PEG-DMG, PEG-DSG, PEG-DPG. PEG-DMG is a polyethylene glycol derivative of glycerol 1,2-dimyristate. In some embodiments, the average molecular weight of the PEG in the PEG-lipid is about 2000-5000. In an alternative specific example, the average molecular weight of PEG in PEG-lipid is about 2000. In some embodiments, the amphoteric polymer includes one or more of poly(carboxybetaine) (pCB), poly(sulfobetaine) (pSB), phosphobetaine-base polymers, and phosphorylcholine polymers. In some embodiments, the amphoteric polymer includes one or more of the following: polycarboxybetaine acrylamide , (pCBAA), poly(carboxybetaine methacrylate), poly(sulfobetaine methacrylate), poly(methacryloyloxyethyl phosphorylcholine), poly(vinyl-pyridinio propanesulfonate), poly(carboxybetaine) based on vinylimidazole, poly(sulfobetaine) based on vinylimidazole, poly(sulfobetaine) based on vinylpyridine.

Correspondingly, the polymer-lipid comprises one or more of the following: polyhydroxybetaine-lipid, polysulfobetaine-lipid, phosphobetaine-based polymer-lipid, and phosphocholine polymer-lipid. In some embodiments, the polymer-lipid comprises one or more of poly (carboxybetaine acrylamide)-lipid, poly (carboxybetaine methacrylate)-lipid, poly (sulfobetaine methacrylate)-lipid, poly (methacryloxyethyl phosphorylcholine)-lipid, poly (vinyl pyridyl propanesulfonate)-lipid, polyvinylimidazolyl betaine-lipid, polyvinylimidazolyl sulfobetaine-lipid, polyvinypyridyl sulfobetaine-lipid.

In addition, in some embodiments, the polymers applied to nanoparticles in Hoang Thi, Thai Thanh, et al., "The Importance of Poly (ethylene glycol) Alternatives for Overcoming PEG Immunogenicity in Drug Delivery and Bioconjugate." Polymers vol. 12,2298, are also incorporated herein.

In some embodiments, the lipid nanoparticle contains a cationic lipid, a helper lipid, a structural lipid, and a polymer-lipid. In some embodiments, the lipid nanoparticles comprise about 25.0%~75.0%, such as about 25.0%~28.0%, 28.0%~32.0%, 32.0%~35.0%, 35.0%~40.0%, 4.0% to 42.0%, 42.0% to 45.0%, 45.0% to 46.3%, 46.3% to 48.0%, 48.0% to 49.5%, 49.5% to 50.0%, 50.0% to 55.0%, 55.0% to 60.0%, 60.0% to 65.0%, or 65.0% to 75.0%, based on the total amount of cationic lipid, helper lipid, structural lipid, and polymer-lipid.

In some embodiments, the lipid nanoparticle comprises a cationic lipid, a helper lipid, a structural lipid, and a polymer-lipid, the cationic lipid accounts for 25 mol% to 75 mol% of the total lipids present in the lipid nanoparticle, the helper lipid accounts for 0 mol% to 45 mol% of the total lipids present in the lipid nanoparticle, the structural lipid accounts for 0 mol% to 60 mol% of the total lipids present in the lipid nanoparticle, and the polymer-lipid accounts for 0.5 mol% to 5 mol% of the total lipids present in the lipid nanoparticle.

In some embodiments, the cationic lipid in the lipid nanoparticle accounts for 25 mol% to 75 mol% of the total lipid present in the lipid nanoparticle. For example, 25mol%, 28mol%, 30mol%, 31mol%, 32mol%, 33mol%, 34mol%, 35mol%, 36mol%, 37mol%, 38mol%, 39mol%, 40mol%, 41mol%, 42mol%, 43mol%, 44mol%, 45mol%, 45.5mol%, 46mol%, 46.5mol%, 47mol%, 47.5mol%, 48mol%, 48.5mol%, 49mol%, 49.5mol%, 50mol%, 50.5mol%, 51mol%, 52.5mol%, 53mol%, 53.5mol%, 54mol%, 54.5mol%, 55mol%, 55.5mol%, 56mol%, 56.5mol%, 57mol%, 57.5mol%, 58mol%, 58.5mol%, 59mol%, 59.5mol%, 60mol%, 60.5mol%, 61mol%, 61.5mol%, 62mol%, 62.5mol%, 63mol%, 63.5mol%, 64mol%, 64.5mol%, 65mol%, 68mol%, 70mol% or 75mol%. In some embodiments, the cationic lipid in the above lipid nanoparticle accounts for 30 mol% to 65 mol%, 30 mol% to 60 mol%, 35 mol% to 60 mol%, 40 mol% to 60 mol%, 45 mol% to 55 mol%, or 50 mol% to 55 mol% of the total lipid present in the lipid nanoparticle.

In some embodiments, the helper lipid (e.g., DSPC) in the above lipid nanoparticle accounts for 0 mol% to 45 mol% of the total lipid present in the lipid nanoparticles. For example, 0.5 mol%, 1 mol%, 3 mol%, 5 mol%, 7.5 mol%, 8 mol%, 8.5 mol%, 9 mol%, 9.5 mol%, 10 mol%, 10.5 mol%, 11 mol%, 11.5 mol%, 12 mol%, 12.5 mol%, 13 mol%, 13.5 mol%, 14 mol%, 14.5 mol%, 15 mol%, 15.5 mol%, 16 mol%, 16.5 mol%, 17 mol%, 17.5 mol%, 18 mol%, 18.5 mol%, 19 mol%, 19.5 mol%, 20 mol%, 20.5 mol%, 21 mol%, 21.5 mol%, 22 mol%, 22.5 mol%, 23 mol%, 23.5 mol%, 24 mol%, 24.5 mol%, 25 mol%, 25.5 mol%, 26 mol%, 26.5 mol%, 27 mol%, 27.5 mol%, 28 mol%, 28.5 mol%, 29 mol%, 29.5 mol%, 30 mol%, 34 mol%, 35 mol%, 36 mol%, 38 mol%, 40 mol%, 42 mol%, 44 mol% or 45 mol%. In some embodiments, the helper lipid (e.g., DSPC) in the above lipid nanoparticle accounts for 1 mol% to 40 mol%, 5 mol% to 40 mol%, 5 mol% to 35 mol%, 5 mol% to 30 mol%, or 5 mol% to 25 mol% of the total lipid present in the lipid nanoparticle.

In some embodiments, the structural lipid (e.g., cholesterol) in the above lipid nanoparticle accounts for 0 mol% to 60 mol% of the total lipid present in the lipid nanoparticle. For example, 0mol%, 1mol%, 5mol%, 8mol%, 10mol%, 12mol%, 14mol%, 15mol%, 16mol%, 18mol%, 20mol%, 22mol%, 24mol%, 25mol%, 27mol%, 27.5mol%, 28mol%, 28.5mol%, 29mol%, 29.5mol%, 30mol%, 30.5mol%, 31mol%, 31.5mol%, 32mol%, 32.5mol%, 33mol%, 33.5mol%, 34mol%, 34.5mol%, 35mol%, 35.5mol%, 36mol%, 36.5mol%, 37mol%, 37.5mol%, 38mol%, 38.5mol%, 39mol%, 39.5mol%, 40mol%, 40.5mol%, 41mol%, 41.5mol%, 42mol%, 42.5mol%, 43mol%, 44mol%, 45mol%, 46mol%, 47mol%, 48mol%, 49mol%, 50mol%, 51mol%, 52mol%, 53mol%, 54mol%, 55mol%, 56mol%, 57mol%, 58mol%, 59mol% or 60mol%. In some embodiments, the structural lipid (e.g., cholesterol) in the above lipid nanoparticle accounts for 1 mol% to 60 mol%, 1 mol% to 55 mol%, 5 mol% to 55 mol%, 10 mol% to 50 mol%, 15 mol% to 50 mol%, 15 mol% to 45 mol%, 20 mol% to 45 mol%, and 25 mol% to 40 mol% of the total lipid present in the lipid nanoparticle.

In some embodiments, the polymer-lipid (e.g., PEG-lipid) in the above lipid nanoparticle accounts for 0.5 mol% to 5 mol% of the total lipid present in the lipid nanoparticle. For example, 0.5 mol%, 1 mol%, 1.5 mol%, 2 mol%, 2.5 mol%, 3 mol%, 3.5 mol%, 4 mol%, 4.5 mol% or 5 mol%. In some embodiments, the polymer-lipid (e.g., PEG-lipid) in the above lipid nanoparticle accounts for 0.5 mol% to 4.5 mol%, 1 mol% to 4.5 mol%, 1 mol% to 4 mol%, 1.5 mol% to 4 mol%, 1.5 mol% to 3.5 mol%, or 1.5 mol% to 3 mol% of the total lipid present in the lipid nanoparticle. In some embodiments, the non-lamellar lipid nanoparticles are selected from one of: alcoholic liposomes and echogenic liposomes.

In some embodiments, the above delivery carrier is a liposome comprising the nucleic acid of any of the above embodiments, the RNA of any of above the embodiments, the genetic engineering vector of any of the above embodiments, or the nucleic acid composition of any of the above embodiments. The liposome encapsulates the nucleic acid, RNA, vector or nucleic acid composition of any of the above embodiments with a vesicle formed by a phospholipid bilayer membrane. In some embodiments, the composition of the liposome comprises a phospholipid and cholesterol.

In some embodiments, the above delivery carrier is a cationic protein loaded with the nucleic acid of any of the above embodiments, the RNA of any of the above embodiments, the genetic engineering vector of any of the above embodiments, or the nucleic acid composition of any of the above embodiments. In some embodiments, the cationic protein includes, but is not limited to, protamine.

In some embodiments, the above delivery carrier is a polymer comprising the nucleic acid of any of the above embodiments, the RNA of any of the above embodiments, the genetic engineering vector of any of the above embodiments, or the nucleic acid composition of any of the above embodiments. In some embodiments, the polymer is a lipopolyplex (LPP) and/or a hyaluronic acid polymer (e.g., a hyaluronic acid gel) comprising the nucleic acid, RNA, genetic engineering vector or nucleic acid composition of any of the above embodiments. In an optional specific example, the polymer is a lipopolyplex or a hyaluronic acid gel. A lipopolyplex is a bilayer structure a polymer-encapsulated nucleic acid (e.g., mRNA) as the core and a lipid (e.g., phospholipid) encapsulated as the shell.

It can be understood that the delivery carrier suitable for the present disclosure is not limited to the above, and may also be other substances capable of transporting the nucleic acid of any of the above embodiments, the RNA of any of the above embodiments, the genetic engineering vector of any of the above embodiments or the nucleic acid composition of any of the above embodiments into an organism. For example, vesicles (e.g., exosomes) and the like.

In addition, the present disclosure further provides a preparation method for the above compound (IV), and the reaction of the preparation method is as follows: 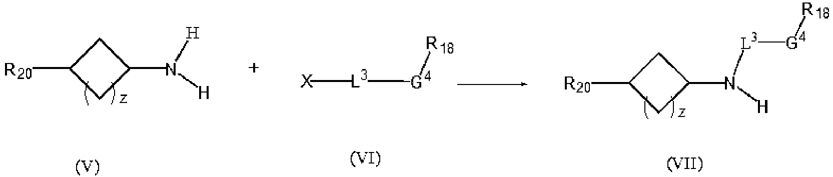 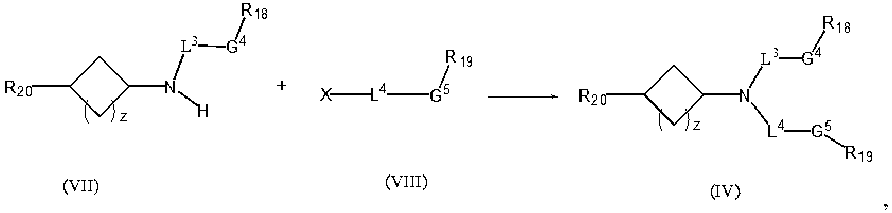 where, R₁₈-R₂₀, G⁴-G⁵, L³-L⁴ and z are as defined above, and X is halogen, preferably bromine.

The preparation method includes step S11 and step S12.

Step S11: subjecting the intermediate compound (V) and the intermediate compound (VI) to a substitution reaction at room temperature (16° C. to 30° C., the same applies below) in an organic solvent in the presence of an acid-binding agent to obtain an intermediate compound (VII). The organic solvent in step S1 is selected from one or more of nitrile organic solvents, alcohol organic solvents, halogenated hydrocarbon organic solvents, amide organic solvents, and aromatic hydrocarbon organic solvents, for example, the organic solvent in step S1 is selected from one or more of acetonitrile, methanol, ethanol, dichloromethane, and dichloroethane (DCE). The acid-binding agent in step S1 is selected from one or more of an organic base and an inorganic base. For example, the acid-binding agent in step S1 is selected from one or more of sodium hydroxide, potassium hydroxide, sodium carbonate, potassium carbonate, triethylamine, and DIPEA.

Step S12: subjecting the intermediate compound (VII) and the intermediate compound (VIII) to a substitution reaction at room temperature in an organic solvent in the presence or absence of an acid-binding agent and an iodide to obtain the compound of formula (IV). The organic solvent in step S2 is selected from one or more of nitrile organic solvents, alcohol organic solvents, halogenated hydrocarbon organic solvents, amide organic solvents and aromatic hydrocarbon organic solvents. For example, the organic solvent in step S2 is selected from one or more of acetonitrile, methanol, ethanol, dichloromethane and dichloroethane. The acid-binding agent is selected from one or more of organic bases and inorganic bases. For example, the acid-binding agent is selected from one or more of sodium hydroxide, potassium hydroxide, sodium carbonate, potassium carbonate, triethylamine and DIPEA. Iodides are common iodides, such as potassium iodide.

In addition, the present disclosure also provides a preparation method for the optical isomer (IV-1- i) of the above compound (IV-1), and the reaction of the preparation method is as follows: 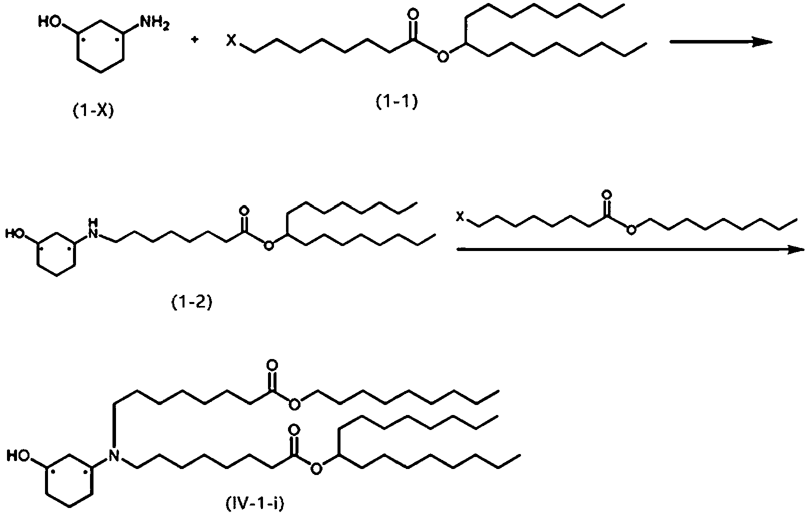 where X is halogen, preferably bromine.

Specifically, the preparation steps for the optical isomer (IV-1- i) of compound (IV-1) of the present disclosure include step S21 and step S22.
Step S21: subjecting compound (1-X) and compound (1-1) to an N-alkylation reaction at 30° C to 50° C in a solvent (such as nitrile, alcohol, halogenated hydrocarbon, amide, aromatic hydrocarbon solvent, specifically acetonitrile, methanol, ethanol, dichloromethane, dichloroethane (DCE), etc.) to prepare compound (1-2);
Step S22: subjecting the compound (1-2) and nonyl 8-halooctanoate to an N-alkylation reaction at 60° C to 110° C in a solvent (such as nitriles, alcohols, halogenated hydrocarbons, amides, aromatic hydrocarbons, ether solvents, specifically acetonitrile, methanol, ethanol, dichloromethane, dichloroethane (DCE), cyclopentylether, methyl tert-butyl ether, etc.), in the presence or absence of an acid binding agent and a catalyst to prepare the compound (IV-1-i), wherein the acid binding agent is selected from one or more of organic bases and inorganic bases. For example, one or more selected from the group consisting of sodium hydroxide, potassium hydroxide, sodium carbonate, potassium carbonate, triethylamine and DIPEA; the catalyst is an iodide, preferably KI.

In some embodiments, according to the above method, when the compound (1-X) as used is (1S,3R)-3-aminocyclohexanol, the compound (1-2) is as shown in structural formula (1-2-1): 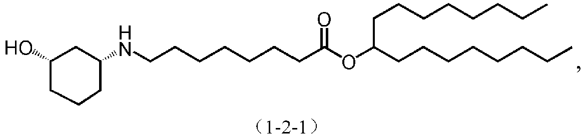 the optical isomer compound (IV-1-1) of the compound (IV-1) was obtained:

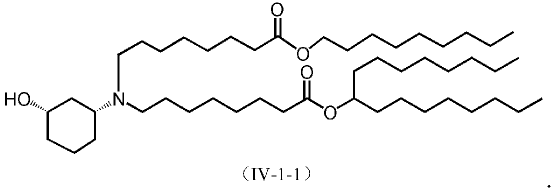

When the compound (1-X) is (1S,3S)-3-aminocyclohexanol, and the compound (1-2) is shown in the structural formula (1-2-2): 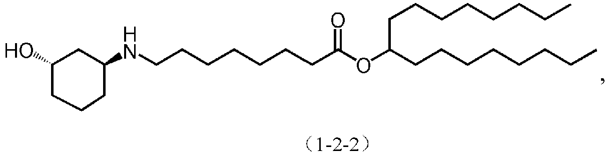 the optical isomer compound (IV-1-2) of the compound (IV-1) was obtained:

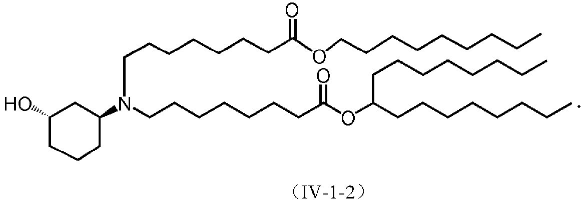

When the compound (1-X) is (1R,3R)-3-aminocyclohexanol, and the compound (1-2) is shown in the structural formula (1-2-3): 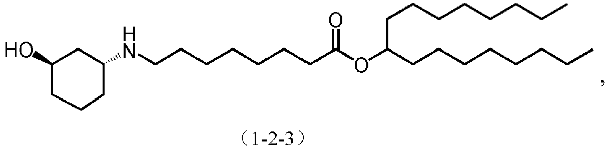 the optical isomer compound (IV-1-3) of the compound (IV-1) was obtained:

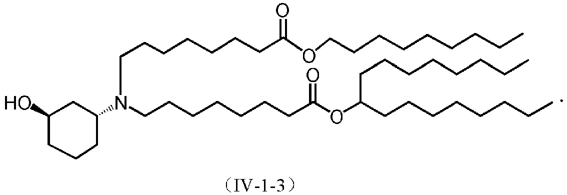

When the compound (1-X) is (1R,3S)-3-aminocyclohexanol, and the compound (1-2) is shown in the structural formula (1-2-4): 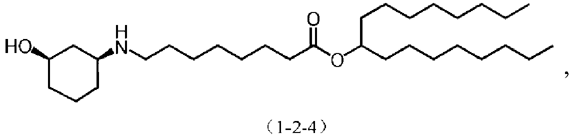 the optical isomer compound (IV-1-4) of the compound (IV-1) was obtained:

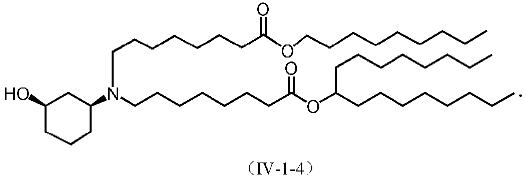

In addition, the present disclosure also provides a pharmaceutical composition comprising the nucleic acid of any of the above embodiments, the RNA of any of the above embodiments, the genetic engineering vector of any of the above embodiments, the host cell of any of the above embodiments, the nucleic acid composition of any of the above embodiments, the immunogen of any of the above embodiments, the mutant of MPV F protein of any of the above embodiments or the delivery carrier of any of the above embodiments, and a pharmaceutically acceptable carrier.

In some embodiments, the above pharmaceutical composition comprises one or more nucleic acids of any one of the above embodiments.

In some embodiments, the above pharmaceutical composition comprises one or more nucleic acids of any of the above embodiments, which are formulated (e.g., encapsulated) separately or co-formulated in a delivery vehicle.

In some embodiments, the above pharmaceutical composition comprises a nucleic acid of any one of the above embodiments.

In some embodiments, the above pharmaceutical composition comprises a nucleic acid of any of the above embodiments formulated (e.g., encapsulated) in a delivery vector.

In some embodiments, the pharmaceutical composition comprises a nucleic acid formulated (e.g., encapsulated) in a delivery carrier (e.g., lipid nanoparticle), the nucleic acid encoding a mutant of MPV F protein, the mutant of MPV F protein encoded by the nucleic acid being selected from one of:
(1) a mutant comprising mutations A147C and A159C and having an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to SEQ ID NO: 1, 2, 81, 82 or 83;
(2) a mutant comprising mutations E146C and T160C and having an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to SEQ ID NO: 3 or 4;
(3) a mutant comprising mutations F168C and F196C and having an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to SEQ ID NO: 5 or 6;
(4) a mutant comprising mutations L165C and F196C and having an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to SEQ ID NO: 7, 8 or 84;
(5) a mutant comprising mutations N145C and A161C and having an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to SEQ ID NO: 9, 10 or 85;
(6) a mutant comprising mutations S149C and V157C and having an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to SEQ ID NO: 11 or 12;
(7) a mutant comprising mutations T59C and N180C and having an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to any one of SEQ ID NOs: 13-20; and
(8) a mutant comprising mutations T150C and R156C and having an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to SEQ ID NO: 21 or 22.

In some embodiments, the above pharmaceutical composition comprises the above nucleic acid formulated (e.g., encapsulated) in a delivery carrier, and the amino acid sequence of the mutant of the MPV F protein encoded by the nucleic acid is set forth in any one of SEQ ID NOs: 1-22 and 81-85.

In some embodiments, the pharmaceutical composition comprises a nucleic acid formulated (e.g., encapsulated) in a delivery carrier (e.g., lipid nanoparticle), the nucleic acid being selected from one of:
(1) an mRNA corresponding to a DNA comprising a nucleotide sequence encoding a mutant having an amino acid sequence set forth in SEQ ID NO: 1 and having at least 70%, 75%, 80%, 85%, 88%, 90%, 91%, 92%, 93%, 94%, 95%, 96%, 97%, 98% or 99% identical to SEQ ID NO: 34 or 86;
(2) an mRNA corresponding to a DNA comprising a nucleotide sequence encoding a mutant having an amino acid sequence set forth in SEQ ID NO: 2 and having at least 70%, 75%, 80%, 85%, 88%, 90%, 91%, 92%, 93%, 94%, 95%, 96%, 97%, 98% or 99% identical to SEQ ID NO: 35 or 87;
(3) an mRNA corresponding to a DNA comprising a nucleotide sequence encoding a mutant having an amino acid sequence set forth in SEQ ID NO: 3 and having at least 70%, 75%, 80%, 85%, 88%, 90%, 91%, 92%, 93%, 94%, 95%, 96%, 97%, 98% or 99% identical to SEQ ID NO: 36;
(4) an mRNA corresponding to a DNA comprising a nucleotide sequence encoding a mutant having an amino acid sequence set forth in SEQ ID NO: 4 and having at least 70%, 75%, 80%, 85%, 88%, 90%, 91%, 92%, 93%, 94%, 95%, 96%, 97%, 98% or 99% identical to SEQ ID NO: 37;
(5) an mRNA corresponding to a DNA comprising a nucleotide sequence encoding a mutant having an amino acid sequence set forth in SEQ ID NO: 5 and having at least 70%, 75%, 80%, 85%, 88%, 90%, 91%, 92%, 93%, 94%, 95%, 96%, 97%, 98% or 99% identical to SEQ ID NO: 38;
(6) an mRNA corresponding to a DNA comprising a nucleotide sequence encoding a mutant having an amino acid sequence set forth in SEQ ID NO: 6 and having at least 70%, 75%, 80%, 85%, 88%, 90%, 91%, 92%, 93%, 94%, 95%, 96%, 97%, 98% or 99% identical to SEQ ID NO: 39;
(7) an mRNA corresponding to a DNA comprising a nucleotide sequence encoding a mutant having an amino acid sequence set forth in SEQ ID NO: 7 and having at least 70%, 75%, 80%, 85%, 88%, 90%, 91%, 92%, 93%, 94%, 95%, 96%, 97%, 98% or 99% identical to SEQ ID NO: 40;
(8) an mRNA corresponding to a DNA comprising a nucleotide sequence encoding a mutant having an amino acid sequence set forth in SEQ ID NO: 8 and having at least 70%, 75%, 80%, 85%, 88%, 90%, 91%, 92%, 93%, 94%, 95%, 96%, 97%, 98% or 99% identical to SEQ ID NO: 41;
(9) an mRNA corresponding to a DNA comprising a nucleotide sequence encoding a mutant having an amino acid sequence set forth in SEQ ID NO: 9 and having at least 70%, 75%, 80%, 85%, 88%, 90%, 91%, 92%, 93%, 94%, 95%, 96%, 97%, 98% or 99% identical to SEQ ID NO: 42;
(10) an mRNA corresponding to a DNA comprising a nucleotide sequence encoding a mutant having an amino acid sequence set forth in SEQ ID NO: 10 and having at least 70%, 75%, 80%, 85%, 88%, 90%, 91%, 92%, 93%, 94%, 95%, 96%, 97%, 98% or 99% identical to SEQ ID NO: 43;
(11) an mRNA corresponding to a DNA comprising a nucleotide sequence encoding a mutant having an amino acid sequence set forth in SEQ ID NO: 11 and having at least 70%, 75%, 80%, 85%, 88%, 90%, 91%, 92%, 93%, 94%, 95%, 96%, 97%, 98% or 99% identical to SEQ ID NO: 44;
(12) an mRNA corresponding to a DNA comprising a nucleotide sequence encoding a mutant having an amino acid sequence set forth in SEQ ID NO: 12 and having at least 70%, 75%, 80%, 85%, 88%, 90%, 91%, 92%, 93%, 94%, 95%, 96%, 97%, 98% or 99% identical to SEQ ID NO: 45;
(13) an mRNA corresponding to a DNA comprising a nucleotide sequence encoding a mutant having an amino acid sequence set forth in SEQ ID NO: 13 and having at least 70%, 75%, 80%, 85%, 88%, 90%, 91%, 92%, 93%, 94%, 95%, 96%, 97%, 98% or 99% identical to SEQ ID NO: 46;
(14) an mRNA corresponding to a DNA comprising a nucleotide sequence encoding a mutant having an amino acid sequence set forth in SEQ ID NO: 14 and having at least 70%, 75%, 80%, 85%, 88%, 90%, 91%, 92%, 93%, 94%, 95%, 96%, 97%, 98% or 99% identical to SEQ ID NO: 47;
(15) an mRNA corresponding to a DNA comprising a nucleotide sequence encoding a mutant having an amino acid sequence set forth in SEQ ID NO: 15 and having at least 70%, 75%, 80%, 85%, 88%, 90%, 91%, 92%, 93%, 94%, 95%, 96%, 97%, 98% or 99% identical to SEQ ID NO: 48;
(16) an mRNA corresponding to a DNA comprising a nucleotide sequence encoding a mutant having an amino acid sequence set forth in SEQ ID NO: 16 and having at least 70%, 75%, 80%, 85%, 88%, 90%, 91%, 92%, 93%, 94%, 95%, 96%, 97%, 98% or 99% identical to SEQ ID NO: 49;
(17) an mRNA corresponding to a DNA comprising a nucleotide sequence encoding a mutant having an amino acid sequence set forth in SEQ ID NO: 17 and having at least 70%, 75%, 80%, 85%, 88%, 90%, 91%, 92%, 93%, 94%, 95%, 96%, 97%, 98% or 99% identical to SEQ ID NO: 50;
(18) an mRNA corresponding to a DNA comprising a nucleotide sequence encoding a mutant having an amino acid sequence set forth in SEQ ID NO: 18 and having at least 70%, 75%, 80%, 85%, 88%, 90%, 91%, 92%, 93%, 94%, 95%, 96%, 97%, 98% or 99% identical to SEQ ID NO: 51;
(19) an mRNA corresponding to a DNA comprising a nucleotide sequence encoding a mutant having an amino acid sequence set forth in SEQ ID NO: 19 and having at least 70%, 75%, 80%, 85%, 88%, 90%, 91%, 92%, 93%, 94%, 95%, 96%, 97%, 98% or 99% identical to SEQ ID NO: 52;
(20) an mRNA corresponding to a DNA comprising a nucleotide sequence encoding a mutant having an amino acid sequence set forth in SEQ ID NO: 20 and having at least 70%, 75%, 80%, 85%, 88%, 90%, 91%, 92%, 93%, 94%, 95%, 96%, 97%, 98% or 99% identical to SEQ ID NO: 53;
(21) an mRNA corresponding to a DNA comprising a nucleotide sequence encoding a mutant having an amino acid sequence set forth in SEQ ID NO: 21 and having at least 70%, 75%, 80%, 85%, 88%, 90%, 91%, 92%, 93%, 94%, 95%, 96%, 97%, 98% or 99% identical to SEQ ID NO: 54;
(22) an mRNA corresponding to a DNA comprising a nucleotide sequence encoding a mutant having an amino acid sequence set forth in SEQ ID NO: 22 and having at least 70%, 75%, 80%, 85%, 88%, 90%, 91%, 92%, 93%, 94%, 95%, 96%, 97%, 98% or 99% identical to SEQ ID NO: 55;
(23) an mRNA corresponding to a DNA comprising a nucleotide sequence encoding a mutant having an amino acid sequence set forth in SEQ ID NO: 81 and having at least 70%, 75%, 80%, 85%, 88%, 90%, 91%, 92%, 93%, 94%, 95%, 96%, 97%, 98% or 99% identical to SEQ ID NO: 88;
(24) an mRNA corresponding to a DNA comprising a nucleotide sequence encoding a mutant having an amino acid sequence set forth in SEQ ID NO: 82 and having at least 70%, 75%, 80%, 85%, 88%, 90%, 91%, 92%, 93%, 94%, 95%, 96%, 97%, 98% or 99% identical to SEQ ID NO: 89;
(25) an mRNA corresponding to a DNA comprising a nucleotide sequence encoding a mutant having an amino acid sequence set forth in SEQ ID NO: 83 and having at least 70%, 75%, 80%, 85%, 88%, 90%, 91%, 92%, 93%, 94%, 95%, 96%, 97%, 98% or 99% identical to SEQ ID NO: 90 or 92;
(26) an mRNA corresponding to a DNA comprising a nucleotide sequence encoding a mutant having an amino acid sequence set forth in SEQ ID NO: 84 and having at least 70%, 75%, 80%, 85%, 88%, 90%, 91%, 92%, 93%, 94%, 95%, 96%, 97%, 98% or 99% identical to SEQ ID NO: 91; and
(27) an mRNA corresponding to a DNA comprising a nucleotide sequence encoding a mutant having an amino acid sequence set forth in SEQ ID NO: 85 and having at least 70%, 75%, 80%, 85%, 88%, 90%, 91%, 92%, 93%, 94%, 95%, 96%, 97%, 98% or 99% identical to SEQ ID NO: 93.

In some embodiments, the pharmaceutical composition comprises a nucleic acid formulated (e.g., encapsulated) in a delivery carrier (e.g., lipid nanoparticle), the nucleic acid being an mRNA corresponding to a DNA comprising a nucleotide sequence set forth in any one of SEQ ID NOs: 34-55 and 86-93.

In some embodiments, the above pharmaceutical composition comprises a plurality of nucleic acids of any of the above embodiments.

In some embodiments, the above pharmaceutical composition comprises a plurality of nucleic acids of any of the above embodiments, the plurality of nucleic acids are co-formulated in a delivery carrier or the plurality of nucleic acids are separately formulated in delivery carriers. "Co-formulated" means that a single delivery carrier contains (e.g., encapsulates) a plurality of nucleic acids, and "separately formulated" means that a single delivery carrier contains (e.g., encapsulates) one nucleic acid, and the same applies hereinafter.

In some embodiments, the pharmaceutical composition comprises two nucleic acids co-formulated in a delivery carrier (e.g., lipid nanoparticle) or separately formulated in delivery carriers (e.g., lipid nanoparticles).

In some embodiments, the pharmaceutical composition comprises two nucleic acids co-formulated in a delivery carrier (e.g., lipid nanoparticle) or separately formulated in delivery carriers (e.g., lipid nanoparticles), the two nucleic acids encoding a mutant of subtype A MPV F protein and a mutant of subtype B MPV F protein, respectively, the mutant of subtype A MPV F protein and the mutant of subtype B MPV F protein encoded by the two nucleic acids are selected from the group consisting of:
(1) a mutant comprising mutations A147C and A159C and having an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to SEQ ID NO: 1, and a mutant comprising mutations A147C and A159C and having an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to SEQ ID NO: 2;
(2) a mutant comprising mutations E146C and T160C and having an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to SEQ ID NO: 3, and a mutant comprising mutations E146C and T160C and having an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to SEQ ID NO: 4;
(3) a mutant comprising mutations F168C and F196C and having an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to SEQ ID NO: 5, and a mutant comprising mutations F168C and F196C and having an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to SEQ ID NO: 6;
(4) a mutant comprising mutations L165C and F196C and having an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to SEQ ID NO: 7, and a mutant comprising mutations L165C and F196C and having an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to SEQ ID NO: 8;
(5) a mutant comprising mutations N145C and A161C and having an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to SEQ ID NO: 9, and a mutant comprising mutations N145C and A161C and having an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to SEQ ID NO: 10;
(6) a mutant comprising mutations S149C and V157C and having an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to SEQ ID NO: 11, and a mutant comprising mutations S149C and V157C and having an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to SEQ ID NO: 12;
(7) a mutant comprising mutations T59C and N180C and having an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to SEQ ID NO: 13, and a mutant comprising mutations T59C and N180C and having an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to SEQ ID NO: 14;
(8) a mutant comprising mutations T59C and N180C and having an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to SEQ ID NO: 15, and a mutant comprising mutations T59C and N180C and having an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to SEQ ID NO: 16;
(9) a mutant comprising mutations T59C and N180C and having an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to SEQ ID NO: 17, and a mutant comprising mutations T59C and N180C and having an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to SEQ ID NO: 18;
(10) a mutant comprising mutations T59C and N180C and having an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to SEQ ID NO: 19, and a mutant comprising mutations T59C and N180C and having an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to SEQ ID NO: 20;
(11) a mutant comprising mutations T150C and R156C and having an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to SEQ ID NO: 21, and a mutant comprising mutations T150C and R156C and having an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to SEQ ID NO: 22;
(12) a mutant comprising mutations A147C and A159C and having an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to SEQ ID NO: 81, and a mutant comprising mutations A147C and A159C and having an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to SEQ ID NO: 82;

In some embodiments, the pharmaceutical composition comprises two nucleic acids co-formulated in a delivery carrier (e.g., lipid nanoparticle) or separately formulated in delivery carriers (e.g., lipid nanoparticles), the two nucleic acids encoding a mutant of subtype A MPV F protein and a mutant of subtype B MPV F protein, respectively, the mutant of subtype A MPV F protein and the mutant of subtype B MPV F protein encoded by the two nucleic acids are selected from the group consisting of:
(1) a mutant having an amino acid sequence set forth in SEQ ID NO: 1 and a mutant having an amino acid sequence set forth in SEQ ID NO: 2;
(2) a mutant having an amino acid sequence set forth in SEQ ID NO: 3 and a mutant having an amino acid sequence set forth in SEQ ID NO: 4;
(3) a mutant having an amino acid sequence set forth in SEQ ID NO: 5 and a mutant having an amino acid sequence set forth in SEQ ID NO: 6;
(4) a mutant having an amino acid sequence set forth in SEQ ID NO: 7 and a mutant having an amino acid sequence set forth in SEQ ID NO: 8;
(5) a mutant having an amino acid sequence set forth in SEQ ID NO: 9 and a mutant having an amino acid sequence set forth in SEQ ID NO: 10;
(6) a mutant having an amino acid sequence set forth in SEQ ID NO: 11 and a mutant having an amino acid sequence set forth in SEQ ID NO: 12;
(7) a mutant having an amino acid sequence set forth in SEQ ID NO: 13 and a mutant having an amino acid sequence set forth in SEQ ID NO: 14;
(8) a mutant having an amino acid sequence set forth in SEQ ID NO: 15 and a mutant having an amino acid sequence set forth in SEQ ID NO: 16;
(9) a mutant having an amino acid sequence set forth in SEQ ID NO: 17 and a mutant having an amino acid sequence set forth in SEQ ID NO: 18;
(10) a mutant having an amino acid sequence set forth in SEQ ID NO: 19 and a mutant having an amino acid sequence set forth in SEQ ID NO: 20;
(11) a mutant having an amino acid sequence set forth in SEQ ID NO: 21 and a mutant having an amino acid sequence set forth in SEQ ID NO: 22; and
(12) a mutant having an amino acid sequence set forth in SEQ ID NO: 81 and a mutant having an amino acid sequence set forth in SEQ ID NO: 82.

In some embodiments, the above pharmaceutical composition comprises two nucleic acids co-formulated in a delivery carrier (e.g., lipid nanoparticle) or separately formulated in delivery carriers (e.g., lipid nanoparticles), the two nucleic acids encoding a mutant of subtype A MPV F protein and a mutant of subtype B MPV F protein, respectively, the two nucleic acids being selected from the group consisting of:
(1) an mRNA corresponding to a DNA comprising a nucleotide sequence encoding a mutant having an amino acid sequence set forth in SEQ ID NO: 1 and having at least 70%, 75%, 80%, 85%, 88%, 90%, 91%, 92%, 93%, 94%, 95%, 96%, 97%, 98% or 99% identity to SEQ ID NO: 34, and an mRNA corresponding to a DNA comprising a nucleotide sequence encoding a mutant having an amino acid sequence set forth in SEQ ID NO: 2 and having at least 70%, 75%, 80%, 85%, 88%, 90%, 91%, 92%, 93%, 94%, 95%, 96%, 97%, 98% or 99% identity to SEQ ID NO: 35;
(2) an mRNA corresponding to a DNA comprising a nucleotide sequence encoding a mutant having an amino acid sequence set forth in SEQ ID NO: 1 and having at least 70%, 75%, 80%, 85%, 88%, 90%, 91%, 92%, 93%, 94%, 95%, 96%, 97%, 98% or 99% identity to SEQ ID NO: 86, and an mRNA corresponding to a DNA comprising a nucleotide sequence encoding a mutant having an amino acid sequence set forth in SEQ ID NO: 2 and having at least 70%, 75%, 80%, 85%, 88%, 90%, 91%, 92%, 93%, 94%, 95%, 96%, 97%, 98% or 99% identity to SEQ ID NO: 87;
(3) an mRNA corresponding to a DNA comprising a nucleotide sequence encoding a mutant having an amino acid sequence set forth in SEQ ID NO: 3 and having at least 70%, 75%, 80%, 85%, 88%, 90%, 91%, 92%, 93%, 94%, 95%, 96%, 97%, 98% or 99% identity to SEQ ID NO: 36, and an mRNA corresponding to a DNA comprising a nucleotide sequence encoding a mutant having an amino acid sequence set forth in SEQ ID NO: 4 and having at least 70%, 75%, 80%, 85%, 88%, 90%, 91%, 92%, 93%, 94%, 95%, 96%, 97%, 98% or 99% identity to SEQ ID NO: 37;
(4) an mRNA corresponding to a DNA comprising a nucleotide sequence encoding a mutant having an amino acid sequence set forth in SEQ ID NO: 5 and having at least 70%, 75%, 80%, 85%, 88%, 90%, 91%, 92%, 93%, 94%, 95%, 96%, 97%, 98% or 99% identity to SEQ ID NO: 38, and an mRNA corresponding to a DNA comprising a nucleotide sequence encoding a mutant having an amino acid sequence set forth in SEQ ID NO: 6 and having at least 70%, 75%, 80%, 85%, 88%, 90%, 91%, 92%, 93%, 94%, 95%, 96%, 97%, 98% or 99% identity to SEQ ID NO: 39;
(5) an mRNA corresponding to a DNA comprising a nucleotide sequence encoding a mutant having an amino acid sequence set forth in SEQ ID NO: 7 and having at least 70%, 75%, 80%, 85%, 88%, 90%, 91%, 92%, 93%, 94%, 95%, 96%, 97%, 98% or 99% identity to SEQ ID NO: 40, and an mRNA corresponding to a DNA comprising a nucleotide sequence encoding a mutant having an amino acid sequence set forth in SEQ ID NO: 8 and having at least 70%, 75%, 80%, 85%, 88%, 90%, 91%, 92%, 93%, 94%, 95%, 96%, 97%, 98% or 99% identity to SEQ ID NO: 41;
(6) an mRNA corresponding to a DNA comprising a nucleotide sequence encoding a mutant having an amino acid sequence set forth in SEQ ID NO: 9 and having at least 70%, 75%, 80%, 85%, 88%, 90%, 91%, 92%, 93%, 94%, 95%, 96%, 97%, 98% or 99% identity to SEQ ID NO: 42, and an mRNA corresponding to a DNA comprising a nucleotide sequence encoding a mutant having an amino acid sequence set forth in SEQ ID NO: 10 and having at least 70%, 75%, 80%, 85%, 88%, 90%, 91%, 92%, 93%, 94%, 95%, 96%, 97%, 98% or 99% identity to SEQ ID NO: 43;
(7) an mRNA corresponding to a DNA comprising a nucleotide sequence encoding a mutant having an amino acid sequence set forth in SEQ ID NO: 11 and having at least 70%, 75%, 80%, 85%, 88%, 90%, 91%, 92%, 93%, 94%, 95%, 96%, 97%, 98% or 99% identity to SEQ ID NO: 44, and an mRNA corresponding to a DNA comprising a nucleotide sequence encoding a mutant having an amino acid sequence set forth in SEQ ID NO: 12 and having at least 70%, 75%, 80%, 85%, 88%, 90%, 91%, 92%, 93%, 94%, 95%, 96%, 97%, 98% or 99% identity to SEQ ID NO: 45;
(8) an mRNA corresponding to a DNA comprising a nucleotide sequence encoding a mutant having an amino acid sequence set forth in SEQ ID NO: 13 and having at least 70%, 75%, 80%, 85%, 88%, 90%, 91%, 92%, 93%, 94%, 95%, 96%, 97%, 98% or 99% identity to SEQ ID NO: 46, and an mRNA corresponding to a DNA comprising a nucleotide sequence encoding a mutant having an amino acid sequence set forth in SEQ ID NO: 14 and having at least 70%, 75%, 80%, 85%, 88%, 90%, 91%, 92%, 93%, 94%, 95%, 96%, 97%, 98% or 99% identity to SEQ ID NO: 47;
(9) an mRNA corresponding to a DNA comprising a nucleotide sequence encoding a mutant having an amino acid sequence set forth in SEQ ID NO: 15 and having at least 70%, 75%, 80%, 85%, 88%, 90%, 91%, 92%, 93%, 94%, 95%, 96%, 97%, 98% or 99% identity to SEQ ID NO: 48, and an mRNA corresponding to a DNA comprising a nucleotide sequence encoding a mutant having an amino acid sequence set forth in SEQ ID NO: 16 and having at least 70%, 75%, 80%, 85%, 88%, 90%, 91%, 92%, 93%, 94%, 95%, 96%, 97%, 98% or 99% identity to SEQ ID NO: 49;
(10) an mRNA corresponding to a DNA comprising a nucleotide sequence encoding a mutant having an amino acid sequence set forth in SEQ ID NO: 17 and having at least 70%, 75%, 80%, 85%, 88%, 90%, 91%, 92%, 93%, 94%, 95%, 96%, 97%, 98% or 99% identity to SEQ ID NO: 50, and an mRNA corresponding to a DNA comprising a nucleotide sequence encoding a mutant having an amino acid sequence set forth in SEQ ID NO: 18 and having at least 70%, 75%, 80%, 85%, 88%, 90%, 91%, 92%, 93%, 94%, 95%, 96%, 97%, 98% or 99% identity to SEQ ID NO: 51;
(11) an mRNA corresponding to a DNA comprising a nucleotide sequence encoding a mutant having an amino acid sequence set forth in SEQ ID NO: 19 and having at least 70%, 75%, 80%, 85%, 88%, 90%, 91%, 92%, 93%, 94%, 95%, 96%, 97%, 98% or 99% identity to SEQ ID NO: 52, and an mRNA corresponding to a DNA comprising a nucleotide sequence encoding a mutant having an amino acid sequence set forth in SEQ ID NO: 20 and having at least 70%, 75%, 80%, 85%, 88%, 90%, 91%, 92%, 93%, 94%, 95%, 96%, 97%, 98% or 99% identity to SEQ ID NO: 53;
(12) an mRNA corresponding to a DNA comprising a nucleotide sequence encoding an amino acid sequence set forth in SEQ ID NO: 21 and having at least 70%, 75%, 80%, 85%, 88%, 90%, 91%, 92%, 93%, 94%, 95%, 96%, 97%, 98% or 99% identity to SEQ ID NO: 54, and an mRNA corresponding to a DNA comprising a nucleotide sequence encoding an amino acid sequence set forth in SEQ ID NO: 22 and having at least 70%, 75%, 80%, 85%, 88%, 90%, 91%, 92%, 93%, 94%, 95%, 96%, 97%, 98% or 99% identity to SEQ ID NO: 55; and
(13) an mRNA corresponding to a DNA comprising a nucleotide sequence encoding an amino acid sequence set forth in SEQ ID NO: 81 and having at least 70%, 75%, 80%, 85%, 88%, 90%, 91%, 92%, 93%, 94%, 95%, 96%, 97%, 98% or 99% identity to SEQ ID NO: 88, and an mRNA corresponding to a DNA comprising a nucleotide sequence encoding an amino acid sequence set forth in SEQ ID NO: 82 and having at least 70%, 75%, 80%, 85%, 88%, 90%, 91%, 92%, 93%, 94%, 95%, 96%, 97%, 98% or 99% identity to SEQ ID NO: 89.

In some embodiments, the above pharmaceutical composition comprises two nucleic acids co-formulated in a delivery carrier (e.g., lipid nanoparticle) or separately formulated in delivery carriers (e.g., lipid nanoparticles), the two nucleic acids being selected from the group consisting of:
(1) an mRNA corresponding to the DNA comprising a nucleotide sequence set forth in SEQ ID NO: 34, and an mRNA corresponding to the DNA comprising a nucleotide sequence set forth in SEQ ID NO: 35;
(2) an mRNA corresponding to the DNA comprising a nucleotide sequence set forth in SEQ ID NO: 36, and an mRNA corresponding to the DNA comprising a nucleotide sequence set forth in SEQ ID NO: 37;
(3) an mRNA corresponding to the DNA comprising a nucleotide sequence set forth in SEQ ID NO: 38, and an mRNA corresponding to the DNA comprising a nucleotide sequence set forth in SEQ ID NO: 39;
(4) an mRNA corresponding to the DNA comprising a nucleotide sequence set forth in SEQ ID NO: 40 and an mRNA corresponding to the DNA comprising a nucleotide sequence set forth in SEQ ID NO: 41;
(5) an mRNA corresponding to the DNA comprising a nucleotide sequence set forth in SEQ ID NO: 42, and an mRNA corresponding to the DNA comprising a nucleotide sequence set forth in SEQ ID NO: 43;
(6) an mRNA corresponding to the DNA comprising a nucleotide sequence set forth in SEQ ID NO: 44, and an mRNA corresponding to the DNA comprising a nucleotide sequence set forth in SEQ ID NO: 45;
(7) an mRNA corresponding to the DNA comprising a nucleotide sequence set forth in SEQ ID NO: 46 and an mRNA corresponding to the DNA comprising a nucleotide sequence set forth in SEQ ID NO: 47;
(8) an mRNA corresponding to the DNA comprising a nucleotide sequence set forth in SEQ ID NO: 48, and an mRNA corresponding to the DNA comprising a nucleotide sequence set forth in SEQ ID NO: 49;
(9) an mRNA corresponding to the DNA comprising a nucleotide sequence set forth in SEQ ID NO: 50, and an mRNA corresponding to the DNA comprising a nucleotide sequence set forth in SEQ ID NO: 51;
(10) an mRNA corresponding to the DNA comprising a nucleotide sequence set forth in SEQ ID NO: 52 and an mRNA corresponding to the DNA comprising a nucleotide sequence set forth in SEQ ID NO: 53;
(11) mRNA corresponding to the DNA comprising a nucleotide sequence set forth in SEQ ID NO: 54, and mRNA corresponding to the DNA comprising a nucleotide sequence set forth in SEQ ID NO: 55
(12) an mRNA corresponding to the DNA comprising a nucleotide sequence set forth in SEQ ID NO: 86 and an mRNA corresponding to the DNA comprising a nucleotide sequence set forth in SEQ ID NO: 87; and
(13) mRNA corresponding to the DNA comprising a nucleotide sequence set forth in SEQ ID NO: 88, and mRNA corresponding to the DNA comprising a nucleotide sequence set forth in SEQ ID NO: 89.

In some embodiments, the nucleic acid contained in the above pharmaceutical composition is mRNA.

In some embodiments, the nucleic acid contained in the above pharmaceutical composition is mRNA, and all of the uridines in the mRNA are replaced with N1-methylpseudouridine.

In some embodiments, the above pharmaceutical composition comprises a nucleic acid of any one of the above embodiments, the nucleic acid is formulated in a delivery carrier, and the nucleic acid is mRNA.

In some embodiments, the above pharmaceutical composition comprises a plurality of nucleic acids of any one of the above-mentioned embodiments, the plurality of nucleic acids are co-formulated in a delivery carrier (e.g., lipid nanoparticle) or the plurality of nucleic acids are separately formulated in delivery carriers (e.g., lipid nanoparticles), and the plurality of nucleic acids are all mRNA.

In some embodiments, the above pharmaceutical composition comprises two nucleic acids co-formulated in a delivery carrier (e.g., lipid nanoparticle) or separately formulated in delivery carriers (e.g., lipid nanoparticles), the two nucleic acids are both mRNA. In some embodiments, the above pharmaceutical composition comprises a plurality of delivery carriers, and the mutants of MPV F protein encoded by the nucleic acids contained in the plurality of delivery carriers are from the same subtype strain but have different mutations, from different subtype strains but have the same mutation, or from different subtype strains and have different mutations.

For example, the above pharmaceutical composition comprises a first delivery carrier and a second delivery carrier, the first delivery carrier comprises a first nucleic acid, the second delivery carrier comprises a second nucleic acid, the first nucleic acid is a nucleic acid comprising a polynucleotide encoding an MPV F protein derived from strain CAN97-83 and having A147C and A159C mutations, and the second nucleic acid is a nucleic acid comprising a polynucleotide encoding an MPV F protein derived from strain NL/1/99 and having A147C and A159C mutations.

For another example, the pharmaceutical composition comprises a first delivery carrier and a second delivery carrier, the first delivery carrier comprises a first nucleic acid, the second delivery carrier comprises a second nucleic acid, the first nucleic acid is a nucleic acid comprising a polynucleotide encoding an MPV F protein derived from strain CAN97-83 and having A147C and A159C mutations, and the second nucleic acid is a nucleic acid comprising a polynucleotide of an MPV F protein derived from strain CAN97-83 and having L165C and F196C mutations.

For another example, the pharmaceutical composition comprises a delivery carrier, the delivery carrier comprises a first nucleic acid and a second nucleic acid, the first nucleic acid is a nucleic acid comprising a polynucleotide encoding an MPV F protein derived from strain CAN97-83 and having A147C and A159C mutations, and the second nucleic acid is a nucleic acid comprising a polynucleotide encoding an MPV F protein derived from strain NL/1/99 and having A147C and A159C mutations.

In some embodiments, the delivery carrier of the pharmaceutical composition of any of the above embodiments is a lipid nanoparticle.

In some embodiments, the above pharmaceutical composition comprises a plurality of nucleic acids of any of the above embodiments, a plurality of RNAs of any of the above embodiments, a plurality of genetic engineering vectors of any of the above embodiments, or a plurality of host cells of any of the above embodiments.

In some embodiments, the pharmaceutical composition of any of the above embodiments is a vaccine.

As used herein, the term "pharmaceutically acceptable" means the approval for use in animals and/or human by a regulatory agency, such as China Food and Drug Administration (CFDA), the Food and Drug Administration (FDA) of US), or a recognized pharmacopoeia, such as the Chinese Pharmacopoeia, European Pharmacopeia. The term "pharmaceutically acceptable carrier" refers to a substance that can be administered with the nucleic acid, genetic engineering vector, nucleic acid composition, protein mutant or delivery carrier of the present disclosure, including but not limited to diluents, sweeteners, flavoring agents, wetting agents, adjuvants, glidants, preservatives, dyes/colorants, surfactants, dispersants, suspending agents, stabilizers, isotonic agents, solvents or emulsifiers.

In some embodiments, the above pharmaceutical composition does not comprise an adjuvant.

In some embodiments, the above pharmaceutical composition further comprises an adjuvant.

In some embodiments, the adjuvant comprises a flagellin adjuvant. In some embodiments, the flagellin adjuvant is mRNA encoding flagellin. In addition, the present disclosure also provides use of the nucleic acid of any of the above embodiments, the genetic engineering vector of any of the above embodiments, the host cell of any of the above embodiments, the nucleic acid composition of any of the above embodiments, the mutant of the MPV F protein of any of the above embodiments, the RNA of any of the above embodiments, the immunogen of any of the above embodiments, the delivery carrier of any of the above embodiments or the pharmaceutical composition of any of the above embodiments in the preparation of a medicament.

In some embodiments, the medicament is used for preventing or treating MPV infection or a disease caused by MPV infection.

In some embodiments, the medicament is used for preventing or treating hMPV infection or a disease caused by hMPV infection.

In some embodiments, the medicament is a vaccine.

### VII. Vaccine

The present disclosure also provides a vaccine comprising the nucleic acid of any of the above embodiments, the genetic engineering vector of any of the above embodiments, the nucleic acid composition of any of the above embodiments, the mutant of MPV F protein of any of the above embodiments, the RNA of any of the above embodiments, the immunogen of any of the above embodiments, or the delivery carrier of any of the above embodiments.

In some embodiments, the vaccine is a nucleic acid vaccine or a protein vaccine.

In some embodiments, the above vaccine is a MPV vaccine.

In some embodiments, the above vaccine is a multivalent vaccine.

In some embodiments, the above vaccine comprises any plurality of the above nucleic acids or any plurality of the above mutants of MPV F protein.

In some embodiments, the above vaccine is a combined vaccine.

In some embodiments, the above vaccine is a combined nucleic acid vaccine. In some embodiments, in addition to any one or more of the nucleic acids comprising the polynucleotide encoding the mutant of MPV F protein, the vaccine further comprises a nucleic acid encoding a protein or polypeptide of the MPV other than the F protein or a protein or polypeptide of other viruses and capable of acting as an immunogen. Of course, the other viruses may be a virus subtype, or a plurality of viruses or a plurality of virus subtypes.

Alternatively, the vaccine is a combined protein vaccine. In some embodiments, in addition to any one or more of the above mutants of MPV F protein, the vaccine further comprises a protein or polypeptide of other viruses that can be used as an immunogen. Of course, there may be one or more other viruses.

In some embodiments, the above vaccine does not comprise an adjuvant.

In some embodiments, the above vaccine further comprises an adjuvant.

It can be understood that the dosage form of the vaccine is not particularly limited. In some embodiments, the above vaccine is an mRNA vaccine.

In some embodiments, the above vaccine is an mRNA vaccine, and the dosage form of the vaccine is nasal, transtracheal or injectable (e.g., intravenous, intraocular, intravitreal, intramuscular, intradermal, intracardiac, intraperitoneal and subcutaneous).

### VIII. Methods for Prevention or Treatment

The present disclosure also provides a method for preventing or treating MPV viral infection, comprising administering to a subject the nucleic acid of any of the above embodiments, the genetic engineering vector of any of the above embodiments, the nucleic acid composition of any of the above embodiments, the mutant of MPV F protein of any of the above embodiments, the RNA of any of the above embodiments, the immunogen of any of the above embodiments, the delivery carrier of any of the above embodiments, the pharmaceutical composition of any of the above embodiments, or the vaccine of any of the above embodiments.

In some embodiments, the subject is a mammal (e.g., a human, a non-human primate (e.g., simian, chimpanzee, monkey, and chimpanzee)), a domestic animal (e.g., dog, cat, and livestock (e.g., horse, cow, pig, sheep, and goat)), or other mammals. Other mammals include, but are not limited to, mouse, rat, guinea pig, rabbit, hamster, etc. In an optional specific example, the subject is a human.

In some embodiments, the frequency of administrations is one, two, three, four, or more.

In addition, the present disclosure also provides a method of eliciting an immune response against MPV in a subject. In some embodiments, the method comprises administering to a subject the nucleic acid of any of the above embodiments, the genetic engineering vector of any of the above embodiments, the nucleic acid composition of any of the above embodiments, the mutant of MPV F protein of any of the above embodiments, the RNA of any of the above embodiments, the immunogen of any of the above embodiments, the delivery carrier of any of the above embodiments, the pharmaceutical composition of any of the above embodiments, or the vaccine of any of the above embodiments. After vaccination with the vaccine of the present disclosure, the anti-antigen antibody titer in the subject is increased as compared to the anti-antigen antibody titer in the subject vaccinated with a prophylactically effective dose of a traditional vaccine against MPV. An "anti-antigen antibody" is a seral antibody that specifically binds to an antigen.

In addition, the present disclosure further provides use of the nucleic acid of any one of the above embodiments, the genetic engineering vector of any one of the above embodiments, the nucleic acid composition of any one of the above embodiments, the mutant of MPV F protein of any one of the above embodiments, the RNA of any one of the above embodiments, the immunogen of any one of the above embodiments, the delivery carrier of any one of the above embodiments, or the pharmaceutical composition of any one of the above embodiments in the preparation of a detection reagent or kit for an MPV F protein-binding antibody.

In addition, the present disclosure also provides a detection reagent or kit for an MPV F protein-binding antibody, comprising the nucleic acid of any of the above embodiments, the genetic engineering vector of any of the above embodiments, the nucleic acid composition of any of the above embodiments, the mutant of MPV F protein of any of the above embodiments, the RNA of any of the above embodiments, the immunogen of any of the above embodiments, the delivery carrier of any of the above embodiments, or the composition of the medicament of any of the above embodiments.

In addition, the present disclosure also provides a method for detecting or isolating an MPV F protein-binding antibody in a subject, the method comprising: providing an effective amount of the nucleic acid of any one of the above embodiments, the genetic engineering vector of any one of the above embodiments, the nucleic acid composition of any one of the above embodiments, the mutant of MPV F protein of any one of the above embodiments, the immunogen of any one of the above embodiments, or the pharmaceutical composition or vaccine containing the mutant of MPV F protein; contacting a biological sample from the subject with the mutant of MPV F protein under conditions sufficient to form an immune complex between the mutant and the MPVF binding antibody; and detecting the immune complex, thereby detecting or isolating the MPVF binding antibody in the subject.

### Examples

To make the objectives and technical solutions of the present disclosure clearer, they are described in details below in view of the particular Examples. Apparently, the described Examples are only a part of the embodiments of the present disclosure, rather than all of the embodiments. All other embodiments obtained by a person of ordinary skill in the art based on the Examples of the present disclosure without creative efforts shall fall within the protection scope of the present disclosure.

### Example 1

### I. Synthesis of Compound (IV-1)

### Step 1): Synthesis of nonyl 8-((3-ydroxycyclohexyl) amino)octanoate (Compound (IV-1-p1))

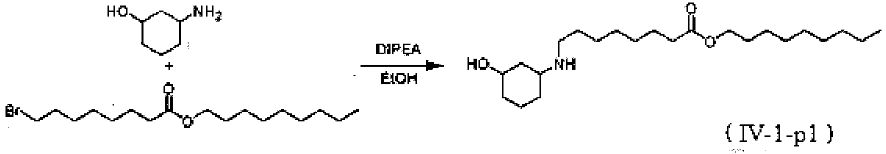

In a 100 mL reaction flask, nonyl 8-bromooctanoate (3.50 g, 10 mmol), 3-aminocyclohexanol (11.5 g, 100 mmol), and 30 mL of ethanol were added in sequence, stirred and dissolved, then N, N-diisopropylethylamine (2.58 g, 20 mmol) was added, reacted at room temperature for 24 h, 100 mL of dichloromethane was added, washed with water for 3 times, dried with anhydrous sodium sulfate, concentrated, and purified by a rapid column chromatography system (dichloromethane: methanol = 20:1 to 5:1) to obtain nonyl 8-((3-hydroxycyclohexyl)amino)octanoate.

¹H NMR (600 MHz, CDCl₃) δ 4.20-4.13 (m, 0.5H), 4.05 (t, 2H), 3.83 (m, 0.5H), 3.09 (m, 0.5H), 2.87 (m, 0.5H), 2.76-2.59 (m, 2H), 2.29 (t, 2H), 2.00 (m, 0.5H), 1.93-1.78 (m, 1.5H), 1.78-1.65 (m, 2H), 1.65-1.46 (m, 8H), 1.40-1.18 (m, 20H), 0.88 (t, 3H).

LCMS:384.3[M+H]⁺ .

### Step 2): Synthesis of Compound (IV-1)

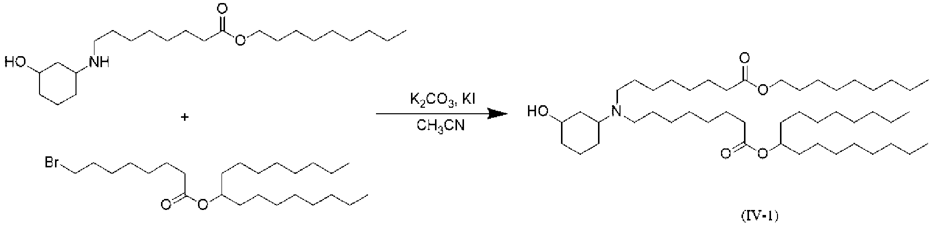

In a 100 mL reaction flask, nonyl 8- ((3-hydroxycyclohexyl) amino) octanoate (383 mg, 1 mmol), 1-octylnonyl 8-bromooctanoate (554 mg, 1.2 mmol), and 20 mL of acetonitrile were sequentially added, stirred and dissolved, then potassium carbonate (276 mg, 2 mmol) and potassium iodide (166 mg, 1 mmol) were added, reacted at room temperature for 24 h, 100 mL of dichloromethane was added, washed with water for 3 times, dried over anhydrous sodium sulfate, concentrated, and purified by a flash column chromatography system (dichloromethane: methanol = 20:1 to 5:1) to obtain compound (IV-1).

¹H NMR (600 MHz, CDCl₃) δ 4.95-4.81 (m, 1H), 4.30-4.12 (m, 0.5H), 4.07 (t, 2H), 3.72-3.61 (m, 0.5H), 2.97 (m, 0.5H), 2.64-2.52 (m, 0.5H), 2.48-2.37 (m, 4H), 2.30 (q, 4H), 1.93-1.81 (m, 2H), 1.72-1.57 (m, 8H), 1.51 (t, 4H), 1.43-1.18 (m, 56H), 0.89 (m, 9H).

LCMS:765.3[M+H]⁺ .

### II. Preparation of Compound (IV-1-1)

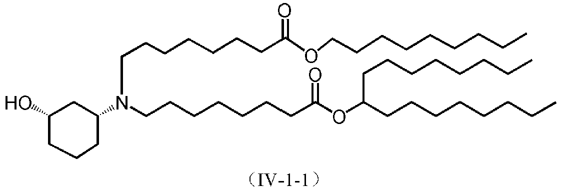

To a 250 mL single-necked flask, 8g (17.3 mmol) of 9-heptadecyl-8-bromooctanoate, 10 g (76.5 mmol) of (1S, 3R)-3-aminocyclohexanol and 100 mL of ethanol were added, and reacted at 50° C for 15 h, and the raw material 9-heptadecyl-8-bromooctanoate was completely reacted. After the solvent was removed by rotary evaporation, the obtained crude product was added with 200 mL of ethyl acetate (EA), washed twice with 100 mL of water, (1S, 3R)-3-aminocyclohexanol was completely washed off, and the EA phase was rotary dried to obtain the crude product of compound (IV-1-1-p) (i.e., 9-heptadecyl-8-(((1R, 3S)-3-hydroxycyclohexyl)amino) octanoate). The crude product was purified by column chromatography (dichloromethane: methanol = 20:1 to 5:1) to obtain compound (IV-1-1-p).

### NMR data:

¹H NMR (600 MHz, CDCl₃): δ 4.91-4.81 (m, 1H), 3.86-3.82 (m, 1H), 2.84-2.81 (m, 1H), 2.69-2.54 (qt, J = 11.2, 7.3 Hz, 2H), 2.29-2.26 (t, J = 7.5 Hz, 2H), 1.93-1.86 (m, 1H), 1.77-1.74(d, J = 12.0 Hz, 1H), 1.72-1.57 (m, 6H), 1.54-1.45 (m, 6H), 1.37-1.30 (m, 8H), 1.30-1.22 (m, 24H), 0.88 (t, J = 7.0 Hz, 6H).

LCMS:496.7[M+H]⁺ .

6g (12.1 mmol) of the above compound (IV-1-1-p) was added into a 250 mL single-necked flask, 90 mL of acetonitrile and 60 mL of cyclopentylmethyl ether were added, then 6.7 g of potassium carbonate powder (48.4 mmol), 2 g of potassium iodide (12.1 mmol) and 5 g of nonyl 8-bromooctanoate (17.5 mmol) were added, the reaction was carried out at 90° C for 24 h, the raw material compound ((IV-1-1-p)) was completely reacted, the reaction was removed from the oil bath and cooled to room temperature, the solid was removed by suction filtration, the filtrate was rotary dried to obtain a crude product, and finally purified by column chromatography (dichloromethane: methanol = 20:1 to 5:1) to obtain compound (IV-1-1).

### NMR data:

¹H NMR (600 MHz, CDCl₃): δ 4.93-4.84 (m, 1H), 4.09 (t, J = 6.8 Hz, 2H), 3.68-3.61 (m, 1H), 2.62-2.54 (m, 1H), 2.53-2.41 (m, 4H), 2.31-2.26 (q, J = 7.4 Hz, 4H), 1.97 (m, 1H), 1.91-1.78 (m, 2H), 1.71-1.61 (m, 7H), 1.54 (d, J = 6.0 Hz, 4H), 1.43-1.37 (m, 4H), 1.39-1.23 (m, 52H), 0.91 (m, 9H).
Specific rotation:+4.3°
LCMS:765.2[M+H]⁺ .

### Example 2 Design of Mutants of hMPV F Protein and Coding Sequence Thereof

The mutants of hMPV F protein as shown in Table 1 was designed, and the codon-optimized DNA sequence (the DNA sequence corresponding to the coding region of hMPV F protein in Table 1) was further designed according to the amino acid sequence of the mutant, and the synthesis was entrusted to GenScript Biotech, and the detection showed that all DNA sequences were correct. In addition, the synthesis of DNA sequences corresponding to mRNAs numbered as 1191, 1192, 1274 and 1275 (see Table 2) was also entrusted to GenScript Biotech, and the detection showed that all of the DNA sequences were correct, among which the mRNAs numbered as 1191 and 1192 encoded hMPV post F proteins, and the mRNAs numbered as 1274 and 1275 encoded hMPV pre F proteins; and the mRNAs numbered as 1191, 1192, 1274 and 1275 used the same 5'-UTR, 3'-UTR and poly(A) tails as the mRNAs in Table 1.

**Table 2**

| **mRNA number** | **Aminod acid sequence of the encoded protein (SEQ ID NO:)** | **DNA sequence corresponding to the coding region for the protein (SEQ ID NO:)** |
|---|---|---|
| 1191 | 71 | 75 |
| 1192 | 72 | 76 |
| 1274 | 73 | 77 |
| 1275 | 74 | 78 |

### Example 3 Construction of Engineered Plasmids

The construction method is as follows:
1, Construction of Plasmid B: The Amp resistance gene in plasmid pcDNA3.1 was replaced with the Kana resistance gene, new restriction sites HindIII, BamHI, KpnI and ApaI were further added subsequent to T7 promoter, and the NeoR/KanR sequence starting from 2136 bp to 2930 bp were deleted to obtain plasmid B, which was conducted by GENEWIZ.
2, Construction of Plasmid C: Plasmid B was linearized with Apal, and plasmid C was obtained by the homologous recombination of the linearized Plasmid B with the nucleic acid fragment having a nucleotide sequence as shown in SEQ ID NO: 33, which was synthesized by IDT (Integrated DNA Technologies).
3. Construction of plasmid D: Plasmid C was double-digested with KpnI and ApaI, the large fragment of about 4.7 kb was recovered by agarose gel electrophoresis, and then ligated with a nucleic acid fragment having a nucleotide sequence set forth in SEQ ID NO: 32, which was synthesized by IDT, with T4 enzyme to obtain plasmid D.
4, Construction of Plasmid F: Plasmid D was double-digested with BamHI and HindIII, a large fragment of about 4.8 kb was recovered by agarose gel electrophoresis, and then ligated with a nucleic acid fragment having a nucleotide sequence as shown in SEQ ID NO: 31, which was synthesized by IDT, with T4 enzyme to obtain plasmid F.
5, Construction of engineered plasmids: homologous arm sequences were introduced at the ends of the DNA sequences corresponding to the protein coding regions of different mutants of hMPV F protein (see Tables 1 and 2 for details) by PCR, and then the PCR products were subjected to homologous recombination with the large fragment of about 4.8 kb recovered by gel extraction of the double enzyme digest of plasmid F by BamHI and KpnI, respectively, and finally a variety of engineered plasmids were obtained, which were completely correct in sequencing.

### Example 4 Preparation of mRNA Encoding Mutants of hMPV F Protein

1. The engineered plasmid was extracted, ensuring that the superhelical rate of the plasmid was 85% or more.
2. Linearization of Plasmid
(1) Linearization by Enzymatic Digestion: 20µg of different engineered plasmids containing the coding sequence for the mutants of hMPV F protein as prepared in Example 3 were taken to prepare the reaction systems according to Table 3, which were blended, and placed in an incubator at 37° C for enzymatic digestion reaction for 16 h.

**Table 3**

| | |
|---|---|
| Plasmid | 20µg |
| Buffer | 8µL |
| Endonuclease (BsaI) | 2µL |
| H₂O | Supplemented to 80µL |

(2) Purification of Linearized Plasmid : After the determination of complete enzymatic digestion, the linearized plasmid was recovered using a DNA fragment purification kit (Takara, Cat. No. 9761).
(3) Identification: 100 ng of purified linear plasmid and original circular plasmid were subjected to 1% agarose gel electrophoresis to confirm whether the plasmid was completely linearized.
3. In Vitro Transcription of mRNA
(1) The IVT reaction system was prepared according to Table 4.

**Table 4**

| H₂O | Supplemented to 100µL | Final concentration |
|---|---|---|
| Linearized plasmid | 10nM | 0.1nM/µL |
| 10 x Reaction Buffer | 10µL | 1X |
| NTP(200mM) | 40mM in total (according to the ratio of nucleotides) | / |
| Pyrophosphatase, Inorganic(0.1U/µL) | 2.5µL | 0.0025U/µL |
| RNase Inhibitor(40U/µL) | 0.75µL | 0.3U/µL |
| T7 Enzyme(50U/µL) | 10µL | 5U/µL |

(2) The reaction system was reacted at 37° C. for 2 h, 5µL of DNase was added to the reaction system at the end of the reaction, and incubated at 37° C for 15min (removing the linearized plasmid template with DNase).
(3) The above IVT product was subjected to magnetic bead purification according to the instructions of magnetic beads for purification (Hieff NGS ^{®} RNA Cleaner, YEASEN, Cat. No. 12602 ES56).
(4) Capping:
The mRNA Cap1-type capping reaction:
The structure and reaction principle of the Cap1 type cap are as follows:
pppN1(p)Nx-OH(3') → ppN1(pN)x-OH(3') + Pi
ppN1(pN)x-OH(3') + GTP → G(5')ppp(5')N1(pN)x-OH(3') + PPi
G(5')ppp(5')N1(pN)x-OH(3') + AdoMet → m7G(5')ppp(5')N1(pN)x-OH(3') + AdoHyc
m7GpppN1(pN)x-OH(3') + AdoMet → m7Gppp[m2'-O]N1(pN)x-OH(3') + AdoHyc
5'-Cap1 cap structure:
   cap G¹G² = m⁷G-5'-ppp-5'-Gm^{2'}-3'-p- [m⁷ = 7-CH₃; m^{2'} = 2'-O-CH₃; -ppp- = -PO₂H-O-PO₂H-O-PO₂H)-; -p- = -PO₂H-].

58µL of the aqueous solution of mRNA (125 pmol capless mRNA supplemented with water to 58µL) was preheated at 65°C for 5 min, then mixed with a mixture containing 10µL of 10× Capping Buffer, 10µL of 10 mM GTP, 1.88µL of 32 mM SAM, 0.187µL of RNase Inhibitor, 5µL of 2'-O-Methyltransferase (50U/µL) and 15µL of ScriptCap Capping Enzyme (10U/µL), and incubated at 42° C for 1 h. The above reagents for capping reaction were purchased from Novoprotein.

### (5) Purification of Capped Product:

The capped product was purified by magnetic beads according to the instructions of magnetic beads for purification (Hieff NGS ^{®} RNA Cleaner, YEASEN, Cat. No. 12602 ES56). Characterization data: the concentration was determined by onedrop or Qubit.

Results of Experiment: mRNAs encoding different mutants of hMPV F protein containing Cap1 cap structure, 5'-UTR, 3'-UTR, poly(A) tail with all uridines replaced by N1-methylpseudouridine were obtained, and the amino acid sequences of the mutants of hMPV F protein upon the translation of the coding region for protein in each mRNA were shown in Tables 1 and 2.

### Example 5 Immunization Experiment on Mice with Encapsulated hMPV mRNA Vaccine

(1) Encapsulation: mRNA was encapsulated by using a microsyringe and a microfluidic chip (SN. 000038) at a speed of 9 mL/min in an aqueous phase and 3 mL/min in an alcohol phase, to prepare a crude product of LNP formulations containing mRNA encoding the mutant of hMPV F protein , where the LNP formulations differs from each other in the encapsulated mRNA only, the mRNA used in this Example was prepared according to the method described in Example 4, and the compositions of the aqueous phase and the alcohol phase of each LNP formulation are shown in Table 5 below.

**Table 5**

| Aqueous phase (mRNA ) | Mass/mg | Concentration of mRNA sample | Volume of mRNA/ µL | Volume of Acetic acid-sodium acetate buffer (pH5.0)/ µL | Volume of DEPC water/µL | Total volume of Aqueous phase /µL |
|---|---|---|---|---|---|---|
| | 1 | 2.78µg/µL | 360 | 469 | 2921 | 3750 |
| Alcohol phase | Composition | Concentration of stock solution (in ethyl alcohol) | Molar mass% | Mass/mg | Required volume of working solution /µL | Total volume of alcohol phase /µL |
| | Compound (IV-1-1) of Example 1 (MW:764.27) | 20.0mg/mL | 49.5% | 10 | 500 | 1250 |
| | DSPC (MW: 790.14) | 10.0mg/mL | 10% | 2.09 | 209 | |
| | CHO-HP(MW: 386.7) | 20.0mg/mL | 39% | 4.0 | 200 | |
| | M-DMG2000 (MW: 2650) | 25.0mg/mL | 1.5% | 1.05 | 42 | |
| | EtOH (MW: 46.07) | | NA | NA | 299 | |

### (2) Solution Exchange by Dialysis:

a. Preparation of dialysate: Tris+8% (m/V) sucrose solution: 1.22g of Tris and 4.7g of Tris-HCL were weighed, dissolved in 2L of Watsons distilled water and blended, 160 g of sucrose was added, and blended to obtain Tris+8% (m/V) sucrose solution.

(b) Dialysis: the encapsulated crude products were respectively loaded into a 100 KD dialysis bag, immersed in a beaker containing 1L of dialysis solution, which was wrapped with aluminum foil paper and the dialysis was performed at 100 rpm for 1h at room temperature, and then the dialysis was continued for 1h after replacing the dialysis solution. Upon dialysis, the preparation samples were sterilized and filtered using a 0.22µm disposable filter membrane to prepare various LNP formulations encapsulating mRNA encoding mutants of hMPV F protein, wherein the mRNA concentration in the LNP formulation was 0.2µg/µL, the mass ratio of mRNA: Lipid was 1:10, the particle size was 80 nm-130 nm, and the encapsulation rate was 85% or more.

(3) In vivo Immunization: 7-9 weeks old Balb/c female mice (17g-22 g, adaptive feeding in SPF environment) were divided into a blank LNP group and a test group. The tested samples were intramuscularly injected at 10µg/mouse, wherein each tested sample included an equal amount of LNP formulation encapsulating mRNA encoding a mutant of subtype A hMPV F protein and LNP formulation encapsulating mRNA encoding a mutant of subtype B hMPV F protein with the same mutations, for example, 5µg of LNP formulation encapsulating mRNA numbered as 1154 and 5µg of LNP formulation encapsulating mRNA numbered as 1170 were mixed and injected into mice twice in total, the first injection time was recorded as Day 0 (D0), the second injection time was D21 or D22, and orbital blood collection was performeded on Day 7, Day 14, Day 21, Day 27, Day 35, Day 42, Day 49, Day 63, Day 74 (or Day 77) and Day 116 after immunization (injection), the blood was collected with a non-anticoagulant tube, placed on ice for 30 min, centrifuged at 4° C and 3,500 rpm for 10 min, and the upper pale yellow liquid was carefully pipetted after stratification to prepare immunoserum.

Some test batches of mice were subjected to spleen collection on day 74 and day 116 or day 7 and day 77, respectively, for subsequent ELISpot detection (Example 8).

### Example 6 Experiment for IgG Antibody Titer

The immunoserum used in this Example was provided by the experiment in Example 5.

Reagents: hMPV subtype A F protein (having an amino acid sequence set forth in SEQ ID NO: 79), hMPV subtype B F protein (having an amino acid sequence set forth in SEQ ID NO: 80), mouse serum inactivated by incubation at 56° C for 30 min, Goat anti-Mouse IgG (H+L) HRP Conjugate, PBS, FBS, single-component TMB developing solution, and stop solution.

The operation steps include:
(1) The F proteins of hMPV of subtype A or hMPV of B subtype were diluted to 1µg/mL with a coating solution and blended for standby; added to a 96-well ELISA plate at 100µL per well, sealed with a plate sealing membrane, and placed at 2-8°C overnight (16-20 hours).
(2) After incubation, the plate was subjected to machine wash, 300µL/well, for 3 times with washing solution, and drying on clean paper.
(3) The ELISA plate was added with a blocking solution at 250µL/well, sealed with a sealing membrane, and incubated at 37° C for 60 min.
(4) After blocking, the plate was subjected to machine wash, 300µL/well, for 3 times with washing solution, and drying on clean paper.
(5) The serum separated in advance was taken out and mixed by vortex for IgG titer detection.
(6) The separated serum was taken, the initial dilution factor was determined according to different immunization times, and the dilution factor was used as the first dilution factor for gradient dilution. The diluted serum was added to an ELISA plate, 100µL/well, and incubated at 37°C for 1 h.
(7) After incubation, the plate was subjected to machine wash, 300µL/well, for 3 times with washing solution, and drying on clean paper.
(8) The enzyme-labeled secondary antibody was diluted by 10,000 folds with the sample diluting solution, 100µL/well, and incubated at 37° C for 1h.
(9) After incubation, the plate was subjected to machine wash, 300µL/well, for 3 times with washing solution, and drying on clean paper.
(10) The TMB single-component developing solution was equilibrated to room temperature in advance, 100µL was added to each well, and incubated at room temperature in the dark for 15 min.
(11) At the end of development, 50µL/well of stop solution was added.
(12) A detection wavelength of 450 nm and a reference wavelength of 630 nm were selected for reading and analysis in a microplate reader.

The detection of IgG antibody titers for hMPV mRNA vaccines containing different mRNAs were shown in FIG. 1A to FIG. 2B, and all mRNA vaccines induced IgG antibodies against hMPV subtype A F protein and/or hMPV subtype B F protein.

### Example 7 MPV-GFP1 Pseudovirus Neutralization Experiment

The immunoserum required for the pseudovirus neutralization assay was provided by the experiment of Example 5.

Reagents: MPV-GFP1 pseudovirus (7.0 log₁₀TCID 50/mL), mouse serum inactivated by incubation at 56° C for 30 min, LLC-MK₂ cells, Opti-MEM, FBS.

The operation steps include:
(1) LLC-MK₂ cells were added to the 96-well plate (2.5×10⁴ cells/well) and cultured in an Opti-MEM medium containing 5% FBS for 24h.
(2) The separated serum was taken, the initial dilution factor was determined according to different immunization times, and the dilution factor was used as the first dilution factor for a 3-fold gradient dilution. 310µL of serum-free Opti-MEM medium was added to H7-H12 of 96-well deep well plate as cell control (CC), and 155µL of DMEM medium was added to H1-H6 as virus control (VC).
(3) 233µL of diluted serum sample was added to A1-A12 as the initial diluent of the sample, and 155µL of Opti-MEM serum-free medium was added to 1-12 of B, C, D, E, F, and G. 78µL of volume was taken from A1 to A12 respectively to the next row and blended, then 3 folds gradient dilution was performed, there were 6 gradients in total, and the excess 78µL of liquid in the last row was discarded.
(4) 155µL of diluted virus was added to each well except H7-H12 and mixed, the titer of the mixed virus was diluted to 1250 TCID50/mL, and incubated at 37° C for 1 h;
(5) 93µL of mixed solution was added to the 96-well plate with LLC-MK₂ cells coated one day in advance (the cells were washed once in advance with PBS), and cultured for 48h, three replicate wells were arranged for each dilution .
(6) The 96-well plate was taken out, the culture medium was pipetted from the well plate, 100µL of PBS was added for washing, followed by reading the fluorescence intensity at an excitation wavelength of 485 nm and an emission wavelength of 535 nm with a microplate reader.

The detection of neutralizing antibody titers of hMPV mRNA vaccines containing different mRNA combinations were shown in FIG. 3A-FIG. 4G, except for the hMPV mRNA vaccine represented by 1191+ 1192, all of other hMPV mRNA vaccines could induce high levels of neutralizing antibodies.

### Example 8 ELISpot Experiment on Mouse Spleen cells

Reagents: RPMI Medium 1640 basic (1×), mouse lymphocyte isolation solution, positive reference PMA+Ionomycin, ELISpot Plus Mouse IFN-gamma (HRP), hMPV F protein peptide library, ELISpot Plus Mouse IL-4 (HRP), erythrocyte lysis solution.

The spleens of mice used in the ELISpot experiment were from the experiment in Example 5.

The specific operation steps of the ELISpot experiment include:
(1) The spleens were added to a 6-well cell culture plate, 7 mL of mouse lymphocyte separation solution was added to each well (recovered to room temperature and shaken up before use). During grinding with a syringe piston, the upward rebound force of the cell sieve was used to control the grinding force and minimize possible mechanical damage to the cells.
(2) The isolation solution with spleen cells suspended therein was immediately transferred to a 15 mL centrifugation tube, and 10 mL of RPMI 1640 medium was slowly added, keeping the liquid level boundary obvious.
(3) Centrifuging with a horizontal rotor at 800g for 30 min at room temperature.
(4) The lymphocyte layer was pepitted, and then, 10 mL of RPMI 1640 medium was added for washing by overturning the tube. Cells were collected by centrifugation at 250g for 10 min at room temperature.
(5) The erythrocytes were lysed according to the instructions of the erythrocyte lysis solution. 2 mL of the erythrocyte lysis solution was added to each tube for resuspending. After placing at 4° C for 2 minutes, 10 mL of DPBS was added to terminate the erythrocyte lysis reaction. After centrifugation at 250g of 4°C for 10 minutes, the cells were collected.
(6) 10 mL of RPMI 1640 medium was added to the cells collected in the above step for washing by overturning the tube. Cells were collected by centrifugation at 250g for 10 min at room temperature.
(7) Finally, the cells were resuspended with culture medium, counted and diluted.
(8) The cell suspension at an adjusted concentration was added to each test well, 5×10⁵ cells/100µL/well.
(9) Stimulators (PMA+Ionomycin working solution (with concentrations, PMA 250 ng/mL+Ionomycin 5µg/mL) and F protein peptide library (2µg/mL)) were added, 10µL/well.
(10) After all samples and stimulators were added, the plate was covered with a lid. The cells were incubated in a 37° C, 5% CO₂ incubator for 20h.
(11) The liquid was thrown out of the well, PBS buffer was added, 200µL/well, which was repeated five times with drying on absorbent paper each time.
(12) The detection antibody was diluted to 1µg/mL with the solution for diluting sample, 100µL/well. The incubation was performed at room temperature for 2h.
(13) The above plate washing operation was repeated.
(14) Enzyme-labeled avidin (Streptavidin-HRP) was diluted for 1000 folds with the solution for diluting sample, 100µL/well, The incubation was performed at room temperature for 1h.
(15) The above plate washing operation was repeated.
(16) The TMB substrate developing solution was equilibrated to room temperature in advance, 100µL was added to each well, followed by incubation at room temperature in dark for 10 min.
(17) The liquid in the wells was poured, the base of the plate was opened, and the front and back surfaces and the base were washed with deionized water for 3-5 times to terminate the development. The plate was placed in a shady and dark place at room temperature, and the base was closed after the plate was naturally dried.
(18) The plate was read with an enzyme-linked immunospot analyzer for various parameters of the spots and statistical analysis was performs.

The Elispot detection of hMPV mRNA vaccines containing different mRNAs was shown in FIG. 5A to FIG. 6H, the hMPV mRNA vaccines containing different mRNAs could induce the generation of significant T cell immune responses in the body, and the frequency of F protein-specific cells secreting IFN-γ was significantly higher than the frequency of F protein-specific cells secreting IL-4, indicating that the immune response is Th1 biased rather than Th2 biased.

### Example 9

1. The LNP formulation encapsulating mRNA numbered as 1154, 1170, 2309, 2310, 2358, 2359, 2311, 1873, 1874, 1875, 1189, 1893 or 1894 was prepared by reference to Examples 1-5, wherein: the mRNA numbered as 1189 encoded the hMPV F protein having an amino acid sequence set forth in SEQ ID NO: 94, and the DNA sequence corresponding to the coding region therein for F protein was set forth in SEQ ID NO: 97; the mRNA numbered as 1893 encoded the hMPV F protein having an amino acid sequence set forth in SEQ ID NO: 95, and the DNA sequence corresponding to the coding region therein for F protein was set forth in SEQ ID NO: 98; the mRNA numbered as 1894 encoded the hMPV F protein having an amino acid sequence set forth in SEQ ID NO: 96, and the DNA sequence corresponding to the coding region therein for F protein was set forth in SEQ ID NO: 99; the mRNAs numbered as 1189, 1893 and 1894 used the same 5'-UTR, 3'-UTR and poly(A) tail as the mRNA of Table 1.
2. 7-9 weeks old Balb/c female mice were immunized (intramuscularly injected) twice with the LNP formulation encapsulating mRNA by reference to Example 5, the first immunization time was D0, the second immunization time was D28, orbital blood collection was performeded on D14, D21, D28 (performed before the second intramuscular injection), D35 and D42 to obtain immune serum, and some batches of mice were subjected to spleen collection on D49 or D57, respectively, for subsequent ELISpot detection.
3. The immunoserum obtained in step 2 was subjected to MPV- GFP1 pseudovirus neutralization experiment by reference to Example 7, and the results were shown in FIG. 7A-FIG. 7C. It can be seen from FIG. 7A-FIG. 7C that the introduction of mutations (A147C and A159C, L165C and F196C, or N145C and A161C) could enhance the humoral immune response of the antigen.
4. The spleens obtained in step 2 were subjected to ELISpot experiment by reference to Example 8, and the results were shown in FIG. 8A-FIG. 8D. It can be seen from FIG. 8A-FIG. 8D that the introduction of mutations (A147C and A 159 C, L165C and F196C, or N145C and A161C) can enhance the cellular immune response of antigen.

### SEQUENCES

SEQ ID NO:1 
MSWKVVIIFSLLITPQHGLKESYLEESCSTITEGYLSVLRTGWYTNVFTLEVGDVENLTCSD
GPSLIKTELDLTKSALRELKTVSADQLAREEQIENPRQSRFVLGAIALGVATAAAVTAGVAI
AKTIRLESEVTAIKNALKTTNECVSTLGNGVRVLCTAVRELKDFVSKNLTRAINKNKCDIDD
LKMAVSFSQFNRRFLNVVRQFSDNAGITPAISLDLMTDAELARAVSNMPTSAGQIKLMLENR
AMVRRKGFGILIGVYGSSVIYMVQLPIFGVIDTPCWIVKAAPSCSGKKGNYACLLREDQGWY
CQNAGSTVYYPNEKDCETRGDHVFCDTAAGINVAEQSKECNINISTTNYPCKVSTGRHPISM
VALSPLGALVACYKGVSCSIGSNRVGIIKQLNKGCSYITNQDADTVTIDNTVYQLSKVEGEQ
HVIKGRPVSSSFDPIKFPEDQFNVALDQVFENIENSQALVDQSNRILSSAEKGNTGFIIVII
LIAVLGSSMILVSIFII
SEQ ID NO:2 
MSWKVMIIISLLITPQHGLKESYLEESCSTITEGYLSVLRTGWYTNVFTLEVGDVENLTCTD
GPSLIKTELDLTKSALRELKTVSADQLAREEQIENPRQSRFVLGAIALGVATAAAVTAGIAI
AKTIRLESEVNAIKGALKQTNECVSTLGNGVRVLCTAVRELKEFVSKNLTSAINRNKCDIAD
LKMAVSFSQFNRRFLNVVRQFSDNAGITPAISLDLMTDAELARAVSYMPTSAGQIKLMLENR
AMVRRKGFGILIGVYGSSVIYMVQLPIFGVIDTPCWIIKAAPSCSEKNGNYACLLREDQGWY
CKNAGSTVYYPNEKDCETRGDHVFCDTAAGINVAEQSRECNINISTTNYPCKVSTGRHPISM
VALSPLGALVACYKGVSCSIGSNWVGIIKQLPKGCSYITNQDADTVTIDNTVYQLSKVEGEQ
HVIKGRPVSSSFDPIKFPEDQFNVALDQVFESIENSQALVDQSNKILNSAEKGNTGFIIVVI
LVAVLGLTMISVSIIII
SEQ ID NO:3 
MSWKVVIIFSLLITPQHGLKESYLEESCSTITEGYLSVLRTGWYTNVFTLEVGDVENLTCSD
GPSLIKTELDLTKSALRELKTVSADQLAREEQIENPRQSRFVLGAIALGVATAAAVTAGVAI
AKTIRLESEVTAIKNALKTTNCAVSTLGNGVRVLACAVRELKDFVSKNLTRAINKNKCDIDD
LKMAVSFSQFNRRFLNVVRQFSDNAGITPAISLDLMTDAELARAVSNMPTSAGQIKLMLENR
AMVRRKGFGILIGVYGSSVIYMVQLPIFGVIDTPCWIVKAAPSCSGKKGNYACLLREDQGWY
CQNAGSTVYYPNEKDCETRGDHVFCDTAAGINVAEQSKECNINISTTNYPCKVSTGRHPISM
VALSPLGALVACYKGVSCSIGSNRVGIIKQLNKGCSYITNQDADTVTIDNTVYQLSKVEGEQ
HVIKGRPVSSSFDPIKFPEDQFNVALDQVFENIENSQALVDQSNRILSSAEKGNTGFIIVII
LIAVLGSSMILVSIFII
SEQ ID NO:4 
MSWKVMIIISLLITPQHGLKESYLEESCSTITEGYLSVLRTGWYTNVFTLEVGDVENLTCTD
GPSLIKTELDLTKSALRELKTVSADQLAREEQIENPRQSRFVLGAIALGVATAAAVTAGIAI
AKTIRLESEVNAIKGALKQTNCAVSTLGNGVRVLACAVRELKEFVSKNLTSAINRNKCDIAD
LKMAVSFSQFNRRFLNVVRQFSDNAGITPAISLDLMTDAELARAVSYMPTSAGQIKLMLENR
AMVRRKGFGILIGVYGSSVIYMVQLPIFGVIDTPCWIIKAAPSCSEKNGNYACLLREDQGWY
CKNAGSTVYYPNEKDCETRGDHVFCDTAAGINVAEQSRECNINISTTNYPCKVSTGRHPISM
VALSPLGALVACYKGVSCSIGSNWVGIIKQLPKGCSYITNQDADTVTIDNTVYQLSKVEGEQ
HVIKGRPVSSSFDPIKFPEDQFNVALDQVFESIENSQALVDQSNKILNSAEKGNTGFIIVVI
LVAVLGLTMISVSIIII
SEQ ID NO:5 
MSWKVVIIFSLLITPQHGLKESYLEESCSTITEGYLSVLRTGWYTNVFTLEVGDVENLTCSD
GPSLIKTELDLTKSALRELKTVSADQLAREEQIENPRQSRFVLGAIALGVATAAAVTAGVAI
AKTIRLESEVTAIKNALKTTNEAVSTLGNGVRVLATAVRELKDCVSKNLTRAINKNKCDIDD
LKMAVSFSQCNRRFLNVVRQFSDNAGITPAISLDLMTDAELARAVSNMPTSAGQIKLMLENR
AMVRRKGFGILIGVYGSSVIYMVQLPIFGVIDTPCWIVKAAPSCSGKKGNYACLLREDQGWY
CQNAGSTVYYPNEKDCETRGDHVFCDTAAGINVAEQSKECNINISTTNYPCKVSTGRHPISM
VALSPLGALVACYKGVSCSIGSNRVGIIKQLNKGCSYITNQDADTVTIDNTVYQLSKVEGEQ
HVIKGRPVSSSFDPIKFPEDQFNVALDQVFENIENSQALVDQSNRILSSAEKGNTGFIIVII
LIAVLGSSMILVSIFII
SEQ ID NO:6 MSWKVMIIISLLITPQHGLKESYLEESCSTITEGYLSVLRTGWYTNVFTLEVGDVENLTCTD 
GPSLIKTELDLTKSALRELKTVSADQLAREEQIENPRQSRFVLGAIALGVATAAAVTAGIAI
AKTIRLESEVNAIKGALKQTNEAVSTLGNGVRVLATAVRELKECVSKNLTSAINRNKCDIAD
LKMAVSFSQCNRRFLNVVRQFSDNAGITPAISLDLMTDAELARAVSYMPTSAGQIKLMLENR
AMVRRKGFGILIGVYGSSVIYMVQLPIFGVIDTPCWIIKAAPSCSEKNGNYACLLREDQGWY
CKNAGSTVYYPNEKDCETRGDHVFCDTAAGINVAEQSRECNINISTTNYPCKVSTGRHPISM
VALSPLGALVACYKGVSCSIGSNWVGIIKQLPKGCSYITNQDADTVTIDNTVYQLSKVEGEQ
HVIKGRPVSSSFDPIKFPEDQFNVALDQVFESIENSQALVDQSNKILNSAEKGNTGFIIVVI
LVAVLGLTMISVSIIII
SEQ ID NO:7 
MSWKVVIIFSLLITPQHGLKESYLEESCSTITEGYLSVLRTGWYTNVFTLEVGDVENLTCSD
GPSLIKTELDLTKSALRELKTVSADQLAREEQIENPRQSRFVLGAIALGVATAAAVTAGVAI
AKTIRLESEVTAIKNALKTTNEAVSTLGNGVRVLATAVRECKDFVSKNLTRAINKNKCDIDD
LKMAVSFSQCNRRFLNVVRQFSDNAGITPAISLDLMTDAELARAVSNMPTSAGQIKLMLENR
AMVRRKGFGILIGVYGSSVIYMVQLPIFGVIDTPCWIVKAAPSCSGKKGNYACLLREDQGWY
CQNAGSTVYYPNEKDCETRGDHVFCDTAAGINVAEQSKECNINISTTNYPCKVSTGRHPISM
VALSPLGALVACYKGVSCSIGSNRVGIIKQLNKGCSYITNQDADTVTIDNTVYQLSKVEGEQ
HVIKGRPVSSSFDPIKFPEDQFNVALDQVFENIENSQALVDQSNRILSSAEKGNTGFIIVII
LIAVLGSSMILVSIFII
SEQ ID NO:8 
MSWKVMIIISLLITPQHGLKESYLEESCSTITEGYLSVLRTGWYTNVFTLEVGDVENLTCTD
GPSLIKTELDLTKSALRELKTVSADQLAREEQIENPRQSRFVLGAIALGVATAAAVTAGIAI
AKTIRLESEVNAIKGALKQTNEAVSTLGNGVRVLATAVRECKEFVSKNLTSAINRNKCDIAD
LKMAVSFSQCNRRFLNVVRQFSDNAGITPAISLDLMTDAELARAVSYMPTSAGQIKLMLENR
AMVRRKGFGILIGVYGSSVIYMVQLPIFGVIDTPCWIIKAAPSCSEKNGNYACLLREDQGWY
CKNAGSTVYYPNEKDCETRGDHVFCDTAAGINVAEQSRECNINISTTNYPCKVSTGRHPISM
VALSPLGALVACYKGVSCSIGSNWVGIIKQLPKGCSYITNQDADTVTIDNTVYQLSKVEGEQ
HVIKGRPVSSSFDPIKFPEDQFNVALDQVFESIENSQALVDQSNKILNSAEKGNTGFIIVVI
LVAVLGLTMISVSIIII
SEQ ID NO:9 
MSWKVVIIFSLLITPQHGLKESYLEESCSTITEGYLSVLRTGWYTNVFTLEVGDVENLTCSD
GPSLIKTELDLTKSALRELKTVSADQLAREEQIENPRQSRFVLGAIALGVATAAAVTAGVAI
AKTIRLESEVTAIKNALKTTCEAVSTLGNGVRVLATCVRELKDFVSKNLTRAINKNKCDIDD
LKMAVSFSQFNRRFLNVVRQFSDNAGITPAISLDLMTDAELARAVSNMPTSAGQIKLMLENR
AMVRRKGFGILIGVYGSSVIYMVQLPIFGVIDTPCWIVKAAPSCSGKKGNYACLLREDQGWY
CQNAGSTVYYPNEKDCETRGDHVFCDTAAGINVAEQSKECNINISTTNYPCKVSTGRHPISM
VALSPLGALVACYKGVSCSIGSNRVGIIKQLNKGCSYITNQDADTVTIDNTVYQLSKVEGEQ
HVIKGRPVSSSFDPIKFPEDQFNVALDQVFENIENSQALVDQSNRILSSAEKGNTGFIIVII
LIAVLGSSMILVSIFII
SEQ ID NO:10 
MSWKVMIIISLLITPQHGLKESYLEESCSTITEGYLSVLRTGWYTNVFTLEVGDVENLTCTD
GPSLIKTELDLTKSALRELKTVSADQLAREEQIENPRQSRFVLGAIALGVATAAAVTAGIAI
AKTIRLESEVNAIKGALKQTCEAVSTLGNGVRVLATCVRELKEFVSKNLTSAINRNKCDIAD
LKMAVSFSQFNRRFLNVVRQFSDNAGITPAISLDLMTDAELARAVSYMPTSAGQIKLMLENR
AMVRRKGFGILIGVYGSSVIYMVQLPIFGVIDTPCWIIKAAPSCSEKNGNYACLLREDQGWY
CKNAGSTVYYPNEKDCETRGDHVFCDTAAGINVAEQSRECNINISTTNYPCKVSTGRHPISM
VALSPLGALVACYKGVSCSIGSNWVGIIKQLPKGCSYITNQDADTVTIDNTVYQLSKVEGEQ
HVIKGRPVSSSFDPIKFPEDQFNVALDQVFESIENSQALVDQSNKILNSAEKGNTGFIIVVI
LVAVLGLTMISVSIIII
SEQ ID NO:11 
MSWKVVIIFSLLITPQHGLKESYLEESCSTITEGYLSVLRTGWYTNVFTLEVGDVENLTCSD
GPSLIKTELDLTKSALRELKTVSADQLAREEQIENPRQSRFVLGAIALGVATAAAVTAGVAI
AKTIRLESEVTAIKNALKTTNEAVCTLGNGVRCLATAVRELKDFVSKNLTRAINKNKCDIDD
LKMAVSFSQFNRRFLNVVRQFSDNAGITPAISLDLMTDAELARAVSNMPTSAGQIKLMLENR
AMVRRKGFGILIGVYGSSVIYMVQLPIFGVIDTPCWIVKAAPSCSGKKGNYACLLREDQGWY
 
CQNAGSTVYYPNEKDCETRGDHVFCDTAAGINVAEQSKECNINISTTNYPCKVSTGRHPISM
VALSPLGALVACYKGVSCSIGSNRVGIIKQLNKGCSYITNQDADTVTIDNTVYQLSKVEGEQ
HVIKGRPVSSSFDPIKFPEDQFNVALDQVFENIENSQALVDQSNRILSSAEKGNTGFIIVII
LIAVLGSSMILVSIFII
SEQ ID NO:12 
MSWKVMIIISLLITPQHGLKESYLEESCSTITEGYLSVLRTGWYTNVFTLEVGDVENLTCTD
GPSLIKTELDLTKSALRELKTVSADQLAREEQIENPRQSRFVLGAIALGVATAAAVTAGIAI
AKTIRLESEVNAIKGALKQTNEAVCTLGNGVRCLATAVRELKEFVSKNLTSAINRNKCDIAD
LKMAVSFSQFNRRFLNVVRQFSDNAGITPAISLDLMTDAELARAVSYMPTSAGQIKLMLENR
AMVRRKGFGILIGVYGSSVIYMVQLPIFGVIDTPCWIIKAAPSCSEKNGNYACLLREDQGWY
CKNAGSTVYYPNEKDCETRGDHVFCDTAAGINVAEQSRECNINISTTNYPCKVSTGRHPISM
VALSPLGALVACYKGVSCSIGSNWVGIIKQLPKGCSYITNQDADTVTIDNTVYQLSKVEGEQ
HVIKGRPVSSSFDPIKFPEDQFNVALDQVFESIENSQALVDQSNKILNSAEKGNTGFIIVVI
LVAVLGLTMISVSIIII
SEQ ID NO:13 
MSWKVVIIFSLLITPQHGLKESYLEESCSTITEGYLSVLRTGWYTNVFTLEVGDVENLCCSD
GPSLIKTELDLTKSALRELKTVSADQLAREEQIENPRQSRFVLGAIALGVATAAAVTAGVAI
AKTIRLESEVTAIKNALKTTNEAVSTLGNGVRVLATAVRELKDFVSKNLTRAINKCKCDIDD
LKMAVSFSQFNRRFLNVVRQFSDNAGITPAISLDLMTDAELARAVSNMPTSAGQIKLMLENR
AMVRRKGFGILIGVYGSSVIYMVQLPIFGVIDTPCWIVKAAPSCSGKKGNYACLLREDQGWY
CQNAGSTVYYPNEKDCETRGDHVFCDTAAGINVAEQSKECNINISTTNYPCKVSTGRHPISM
VALSPLGALVACYKGVSCSIGSNRVGIIKQLNKGCSYITNQDADTVTIDNTVYQLSKVEGEQ
HVIKGRPVSSSFDPIKFPEDQFNVALDQVFENIENSQALVDQSNRILSSAEKGNTG
SEQ ID NO:14 
MSWKVMIIISLLITPQHGLKESYLEESCSTITEGYLSVLRTGWYTNVFTLEVGDVENLCCTD
GPSLIKTELDLTKSALRELKTVSADQLAREEQIENPRQSRFVLGAIALGVATAAAVTAGIAI
AKTIRLESEVNAIKGALKQTNEAVSTLGNGVRVLATAVRELKEFVSKNLTSAINRCKCDIAD
LKMAVSFSQFNRRFLNVVRQFSDNAGITPAISLDLMTDAELARAVSYMPTSAGQIKLMLENR
AMVRRKGFGILIGVYGSSVIYMVQLPIFGVIDTPCWIIKAAPSCSEKNGNYACLLREDQGWY
CKNAGSTVYYPNEKDCETRGDHVFCDTAAGINVAEQSRECNINISTTNYPCKVSTGRHPISM
VALSPLGALVACYKGVSCSIGSNWVGIIKQLPKGCSYITNQDADTVTIDNTVYQLSKVEGEQ
HVIKGRPVSSSFDPIKFPEDQFNVALDQVFESIENSQALVDQSNKILNSAEKGNTG
SEQ ID NO:15 
MSWKVVIIFSLLITPQHGLKESYLEESCSTITEGYLSVLRTGWYTNVFTLEVGDVENLCCSD
GPSLIKTELDLTKSALRELKTVSADQLAREEQIENPRQSRFVLGAIALGVATAAAVTAGVAI
AKTIRLESEVTAIKNALKTTNEAVSTLGNGVRVLATAVRELKDFVSKNLTRAINKCKCDIDD
LKMAVSFSQFNRRFLNVVRQFSDNAGITPAISLDLMTDAELARAVSNMPTSAGQIKLMLENR
AMVRRKGFGILIGVYGSSVIYMVQLPIFGVIDTPCWIVKAAPSCSGKKGNYACLLREDQGWY
CQNAGSTVYYPNEKDCETRGDHVFCDTAAGINVAEQSKECNINISTTNYPCKVSTGRHPISM
VALSPLGALVACYKGVSCSIGSNRVGIIKQLNKGCSYITNQDADTVTIDNTVYQLSKVEGEQ
HVIKGRPVSSSFDPIKFPEDQFNVALDQVFENIENSQALVDQSNRILSSAEKGNTG
SEQ ID NO:16 
MSWKVMIIISLLITPQHGLKESYLEESCSTITEGYLSVLRTGWYTNVFTLEVGDVENLCCTD
GPSLIKTELDLTKSALRELKTVSADQLAREEQIENPRQSRFVLGAIALGVATAAAVTAGIAI
AKTIRLESEVNAIKGALKQTNEAVSTLGNGVRVLATAVRELKEFVSKNLTSAINRCKCDIAD
LKMAVSFSQFNRRFLNVVRQFSDNAGITPAISLDLMTDAELARAVSYMPTSAGQIKLMLENR
AMVRRKGFGILIGVYGSSVIYMVQLPIFGVIDTPCWIIKAAPSCSEKNGNYACLLREDQGWY
CKNAGSTVYYPNEKDCETRGDHVFCDTAAGINVAEQSRECNINISTTNYPCKVSTGRHPISM
VALSPLGALVACYKGVSCSIGSNWVGIIKQLPKGCSYITNQDADTVTIDNTVYQLSKVEGEQ
HVIKGRPVSSSFDPIKFPEDQFNVALDQVFESIENSQALVDQSNKILNSAEKGNTG
SEQ ID NO:17 
MSWKVVIIFSLLITPQHGLKESYLEESCSTITEGYLSVLRTGWYTNVFTLEVGDVENLCCSD
GPSLIKTELDLTKSALRELKTVSADQLAREEQIENPRQSRFVLGAIALGVATAAAVTAGVAI
 
AKTIRLESEVTAIKNALKTTNEAVSTLGNGVRVLATAVRELKDFVSKNLTRAINKCKCDIDD
LKMAVSFSQFNRRFLNVVRQFSDNAGITPAISLDLMTDAELARAVSNMPTSAGQIKLMLENR
AMVRRKGFGILIGVYGSSVIYMVQLPIFGVIDTPCWIVKAAPSCSGKKGNYACLLREDQGWY
CQNAGSTVYYPNEKDCETRGDHVFCDTAAGINVAEQSKECNINISTTNYPCKVSTGRHPISM
VALSPLGALVACYKGVSCSIGSNRVGIIKQLNKGCSYITNQDADTVTIDNTVYQLSKVEGEQ
HVIKGRPVSSSFDPIKFPEDQFNVALDQVFENIENSQALVDQSNRILSSAEKGNTGFIIVII
LIAVLGSSMILVSIFIIIKKTKKPTGAPPELSGVTNNGFIPHS
SEQ ID NO:18 
MSWKVMIIISLLITPQHGLKESYLEESCSTITEGYLSVLRTGWYTNVFTLEVGDVENLCCTD
GPSLIKTELDLTKSALRELKTVSADQLAREEQIENPRQSRFVLGAIALGVATAAAVTAGIAI
AKTIRLESEVNAIKGALKQTNEAVSTLGNGVRVLATAVRELKEFVSKNLTSAINRCKCDIAD
LKMAVSFSQFNRRFLNVVRQFSDNAGITPAISLDLMTDAELARAVSYMPTSAGQIKLMLENR
AMVRRKGFGILIGVYGSSVIYMVQLPIFGVIDTPCWIIKAAPSCSEKNGNYACLLREDQGWY
CKNAGSTVYYPNEKDCETRGDHVFCDTAAGINVAEQSRECNINISTTNYPCKVSTGRHPISM
VALSPLGALVACYKGVSCSIGSNWVGIIKQLPKGCSYITNQDADTVTIDNTVYQLSKVEGEQ
HVIKGRPVSSSFDPIKFPEDQFNVALDQVFESIENSQALVDQSNKILNSAEKGNTGFIIVVI
LVAVLGLTMISVSIIIIIKKTRKPTGAPPELNGVTNGGFIPHS
SEQ ID NO:19 
MSWKVVIIFSLLITPQHGLKESYLEESCSTITEGYLSVLRTGWYTNVFTLEVGDVENLCCSD
GPSLIKTELDLTKSALRELKTVSADQLAREEQIENPRQSRFVLGAIALGVATAAAVTAGVAI
AKTIRLESEVTAIKNALKTTNEAVSTLGNGVRVLATAVRELKDFVSKNLTRAINKCKCDIDD
LKMAVSFSQFNRRFLNVVRQFSDNAGITPAISLDLMTDAELARAVSNMPTSAGQIKLMLENR
AMVRRKGFGILIGVYGSSVIYMVQLPIFGVIDTPCWIVKAAPSCSGKKGNYACLLREDQGWY
CQNAGSTVYYPNEKDCETRGDHVFCDTAAGINVAEQSKECNINISTTNYPCKVSTGRHPISM
VALSPLGALVACYKGVSCSIGSNRVGIIKQLNKGCSYITNQDADTVTIDNTVYQLSKVEGEQ
HVIKGRPVSSSFDPIKFPEDQFNVALDQVFENIENSQALVDQSNRILSSAEKGNTGFIIVII
LIAVLGSSMILVSIFII
SEQ ID NO:20 
MSWKVMIIISLLITPQHGLKESYLEESCSTITEGYLSVLRTGWYTNVFTLEVGDVENLCCTD
GPSLIKTELDLTKSALRELKTVSADQLAREEQIENPRQSRFVLGAIALGVATAAAVTAGIAI
AKTIRLESEVNAIKGALKQTNEAVSTLGNGVRVLATAVRELKEFVSKNLTSAINRCKCDIAD
LKMAVSFSQFNRRFLNVVRQFSDNAGITPAISLDLMTDAELARAVSYMPTSAGQIKLMLENR
AMVRRKGFGILIGVYGSSVIYMVQLPIFGVIDTPCWIIKAAPSCSEKNGNYACLLREDQGWY
CKNAGSTVYYPNEKDCETRGDHVFCDTAAGINVAEQSRECNINISTTNYPCKVSTGRHPISM
VALSPLGALVACYKGVSCSIGSNWVGIIKQLPKGCSYITNQDADTVTIDNTVYQLSKVEGEQ
HVIKGRPVSSSFDPIKFPEDQFNVALDQVFESIENSQALVDQSNKILNSAEKGNTGFIIVVI
LVAVLGLTMISVSIIII
SEQ ID NO:21 
MSWKVVIIFSLLITPQHGLKESYLEESCSTITEGYLSVLRTGWYTNVFTLEVGDVENLTCSD
GPSLIKTELDLTKSALRELKTVSADQLAREEQIENPRQSRFVLGAIALGVATAAAVTAGVAI
AKTIRLESEVTAIKNALKTTNEAVSCLGNGVCVLATAVRELKDFVSKNLTRAINKNKCDIDD
LKMAVSFSQFNRRFLNVVRQFSDNAGITPAISLDLMTDAELARAVSNMPTSAGQIKLMLENR
AMVRRKGFGILIGVYGSSVIYMVQLPIFGVIDTPCWIVKAAPSCSGKKGNYACLLREDQGWY
CQNAGSTVYYPNEKDCETRGDHVFCDTAAGINVAEQSKECNINISTTNYPCKVSTGRHPISM
VALSPLGALVACYKGVSCSIGSNRVGIIKQLNKGCSYITNQDADTVTIDNTVYQLSKVEGEQ
HVIKGRPVSSSFDPIKFPEDQFNVALDQVFENIENSQALVDQSNRILSSAEKGNTGFIIVII
LIAVLGSSMILVSIFII
SEQ ID NO:22 
MSWKVMIIISLLITPQHGLKESYLEESCSTITEGYLSVLRTGWYTNVFTLEVGDVENLTCTD
GPSLIKTELDLTKSALRELKTVSADQLAREEQIENPRQSRFVLGAIALGVATAAAVTAGIAI
AKTIRLESEVNAIKGALKQTNEAVSCLGNGVCVLATAVRELKEFVSKNLTSAINRNKCDIAD
LKMAVSFSQFNRRFLNVVRQFSDNAGITPAISLDLMTDAELARAVSYMPTSAGQIKLMLENR
AMVRRKGFGILIGVYGSSVIYMVQLPIFGVIDTPCWIIKAAPSCSEKNGNYACLLREDQGWY
CKNAGSTVYYPNEKDCETRGDHVFCDTAAGINVAEQSRECNINISTTNYPCKVSTGRHPISM
 
VALSPLGALVACYKGVSCSIGSNWVGIIKQLPKGCSYITNQDADTVTIDNTVYQLSKVEGEQ
HVIKGRPVSSSFDPIKFPEDQFNVALDQVFESIENSQALVDQSNKILNSAEKGNTGFIIVVI
LVAVLGLTMISVSIIII
SEQ ID NO:23 
MSWKVVIIFSLLITPQHGLKESYLEESCSTITEGYLSVLRTGWYTNVFTLECGDVENLTCSD
GPSLIKTELDLTKSALRELKTVSADQLAREEQIENPRQSRFVLGAIALGVATAAAVTAGVAI
AKTIRLESEVTAIKNALKTTNEAVSTLGNGVRVLATAVRECKDFVSKNLTRAINKNKCDIDD
LKMAVSFSQFNRRFLNVVRQFSDNAGITPAISLDLMTDAELARAVSNMPTSAGQIKLMLENR
AMVRRKGFGILIGVYGSSVIYMVQLPIFGVIDTPCWIVKAAPSCSGKKGNYACLLREDQGWY
CQNAGSTVYYPNEKDCETRGDHVFCDTAAGINVAEQSKECNINISTTNYPCKVSTGRHPISM
VALSPLGALVACYKGVSCSIGSNRVGIIKQLNKGCSYITNQDADTVTIDNTVYQLSKVEGEQ
HVIKGRPVSSSFDPIKFPEDQFNVALDQVFENIENSQALVDQSNRILSSAEKGNTGFIIVII
LIAVLGSSMILVSIFII
SEQ ID NO:24 
MSWKVMIIISLLITPQHGLKESYLEESCSTITEGYLSVLRTGWYTNVFTLECGDVENLTCTD
GPSLIKTELDLTKSALRELKTVSADQLAREEQIENPRQSRFVLGAIALGVATAAAVTAGIAI
AKTIRLESEVNAIKGALKQTNEAVSTLGNGVRVLATAVRECKEFVSKNLTSAINRNKCDIAD
LKMAVSFSQFNRRFLNVVRQFSDNAGITPAISLDLMTDAELARAVSYMPTSAGQIKLMLENR
AMVRRKGFGILIGVYGSSVIYMVQLPIFGVIDTPCWIIKAAPSCSEKNGNYACLLREDQGWY
CKNAGSTVYYPNEKDCETRGDHVFCDTAAGINVAEQSRECNINISTTNYPCKVSTGRHPISM
VALSPLGALVACYKGVSCSIGSNWVGIIKQLPKGCSYITNQDADTVTIDNTVYQLSKVEGEQ
HVIKGRPVSSSFDPIKFPEDQFNVALDQVFESIENSQALVDQSNKILNSAEKGNTGFIIVVI
LVAVLGLTMISVSIIII
SEQ ID NO:25 
MSWKVVIIFSLLITPQHGLKESYLEESCSTITEGYLSVLRTGWYTNVFTLEVGDCENLTCSD
GPSLIKTELDLTKSALRELKTVSADQLAREEQIENPRQSRFVLGAIALGVATAAAVTAGVAI
AKTIRLESEVTAIKNALKTTNEAVSTLGNGVRVLATAVRELKDFCSKNLTRAINKNKCDIDD
LKMAVSFSQFNRRFLNVVRQFSDNAGITPAISLDLMTDAELARAVSNMPTSAGQIKLMLENR
AMVRRKGFGILIGVYGSSVIYMVQLPIFGVIDTPCWIVKAAPSCSGKKGNYACLLREDQGWY
CQNAGSTVYYPNEKDCETRGDHVFCDTAAGINVAEQSKECNINISTTNYPCKVSTGRHPISM
VALSPLGALVACYKGVSCSIGSNRVGIIKQLNKGCSYITNQDADTVTIDNTVYQLSKVEGEQ
HVIKGRPVSSSFDPIKFPEDQFNVALDQVFENIENSQALVDQSNRILSSAEKGNTGFIIVII
LIAVLGSSMILVSIFII
SEQ ID NO:26 
MSWKVMIIISLLITPQHGLKESYLEESCSTITEGYLSVLRTGWYTNVFTLEVGDCENLTCTD
GPSLIKTELDLTKSALRELKTVSADQLAREEQIENPRQSRFVLGAIALGVATAAAVTAGIAI
AKTIRLESEVNAIKGALKQTNEAVSTLGNGVRVLATAVRELKEFCSKNLTSAINRNKCDIAD
LKMAVSFSQFNRRFLNVVRQFSDNAGITPAISLDLMTDAELARAVSYMPTSAGQIKLMLENR
AMVRRKGFGILIGVYGSSVIYMVQLPIFGVIDTPCWIIKAAPSCSEKNGNYACLLREDQGWY
CKNAGSTVYYPNEKDCETRGDHVFCDTAAGINVAEQSRECNINISTTNYPCKVSTGRHPISM
VALSPLGALVACYKGVSCSIGSNWVGIIKQLPKGCSYITNQDADTVTIDNTVYQLSKVEGEQ
HVIKGRPVSSSFDPIKFPEDQFNVALDQVFESIENSQALVDQSNKILNSAEKGNTGFIIVVI
LVAVLGLTMISVSIIII
SEQ ID NO:27 
MSWKVMIIISLLITPQHGLKESYLEESCSTITEGYLSVLRTGWYTNVFTLEVGDVENCTCTD
GPSLIKTELDLTKSALRELKTVSADQLAREEQIENPRQSRFVLGAIALGVATAAAVTAGIAI
AKTIRLESEVNAIKGALKQTNEAVSTLGNGVRVLATAVRELKEFVSKNLCSAINRNKCDIAD
LKMAVSFSQFNRRFLNVVRQFSDNAGITPAISLDLMTDAELARAVSYMPTSAGQIKLMLENR
AMVRRKGFGILIGVYGSSVIYMVQLPIFGVIDTPCWIIKAAPSCSEKNGNYACLLREDQGWY
CKNAGSTVYYPNEKDCETRGDHVFCDTAAGINVAEQSRECNINISTTNYPCKVSTGRHPISM
VALSPLGALVACYKGVSCSIGSNWVGIIKQLPKGCSYITNQDADTVTIDNTVYQLSKVEGEQ
HVIKGRPVSSSFDPIKFPEDQFNVALDQVFESIENSQALVDQSNKILNSAEKGNTGFIIVVI
LVAVLGLTMISVSIIII
SEQ ID NO:28 
MSWKVVIIFSLLITPQHGLKESYLEESCSTITEGYLSVLRTGWYTNVFTLEVGDVENCTCSD
GPSLIKTELDLTKSALRELKTVSADQLAREEQIENPRQSRFVLGAIALGVATAAAVTAGVAI
AKTIRLESEVTAIKNALKTTNEAVSTLGNGVRVLATAVRELKDFVSKNLCRAINKNKCDIDD
LKMAVSFSQFNRRFLNVVRQFSDNAGITPAISLDLMTDAELARAVSNMPTSAGQIKLMLENR
AMVRRKGFGILIGVYGSSVIYMVQLPIFGVIDTPCWIVKAAPSCSGKKGNYACLLREDQGWY
CQNAGSTVYYPNEKDCETRGDHVFCDTAAGINVAEQSKECNINISTTNYPCKVSTGRHPISM
VALSPLGALVACYKGVSCSIGSNRVGIIKQLNKGCSYITNQDADTVTIDNTVYQLSKVEGEQ
HVIKGRPVSSSFDPIKFPEDQFNVALDQVFENIENSQALVDQSNRILSSAEKGNTGFIIVII
LIAVLGSSMILVSIFII
SEQ ID NO:29 
MSWKVVIIFSLLITPQHGLKESYLEESCSTITEGYLSVLRTGWYTNVFTLEVGDVENLTCSD
GPSLIKTELDLTKSALRELKTVSADQLAREEQIENPRQSRFVLGAIALGVATAAAVTAGVAI
AKTIRLESEVTAIKNALKTTNEACSTLGNGVRVCATAVRELKDFVSKNLTRAINKNKCDIDD
LKMAVSFSQFNRRFLNVVRQFSDNAGITPAISLDLMTDAELARAVSNMPTSAGQIKLMLENR
AMVRRKGFGILIGVYGSSVIYMVQLPIFGVIDTPCWIVKAAPSCSGKKGNYACLLREDQGWY
CQNAGSTVYYPNEKDCETRGDHVFCDTAAGINVAEQSKECNINISTTNYPCKVSTGRHPISM
VALSPLGALVACYKGVSCSIGSNRVGIIKQLNKGCSYITNQDADTVTIDNTVYQLSKVEGEQ
HVIKGRPVSSSFDPIKFPEDQFNVALDQVFENIENSQALVDQSNRILSSAEKGNTGFIIVII
LIAVLGSSMILVSIFII
SEQ ID NO:30 
MSWKVMIIISLLITPQHGLKESYLEESCSTITEGYLSVLRTGWYTNVFTLEVGDVENLTCTD
GPSLIKTELDLTKSALRELKTVSADQLAREEQIENPRQSRFVLGAIALGVATAAAVTAGIAI
AKTIRLESEVNAIKGALKQTNEACSTLGNGVRVCATAVRELKEFVSKNLTSAINRNKCDIAD
LKMAVSFSQFNRRFLNVVRQFSDNAGITPAISLDLMTDAELARAVSYMPTSAGQIKLMLENR
AMVRRKGFGILIGVYGSSVIYMVQLPIFGVIDTPCWIIKAAPSCSEKNGNYACLLREDQGWY
CKNAGSTVYYPNEKDCETRGDHVFCDTAAGINVAEQSRECNINISTTNYPCKVSTGRHPISM
VALSPLGALVACYKGVSCSIGSNWVGIIKQLPKGCSYITNQDADTVTIDNTVYQLSKVEGEQ
HVIKGRPVSSSFDPIKFPEDQFNVALDQVFESIENSQALVDQSNKILNSAEKGNTGFIIVVI
LVAVLGLTMISVSIIII
SEQ ID NO:31 
TAATACGACTCACTATAGGACAGATCGCCTGGAGACGCCATCCACGCTGTTTTGACCTCCAT
AGAAGACACCGGGACCGATCCAGCCTCCGCGGCCGGGAACGGTGCATTGGAACGCGGATTCC
CCGTGCCAAGAGTGACGTAAGTACCGCAGTCACCGTCCTTGACACG
SEQ ID NO:32 
TCTTCACCCAGTCTGTCTCTTAGAATTTATTTCCCACTGACACAAACAAGCCATTTTGTCTG
CTTGTATAACCC
SEQ ID NO:33 
AAAAAAAAAAAAGAAAAAAAAAAAAAAAAAAGAAAGAAAAAAAAAAAAAAAAAAAAAAAAAA
AAAAAAAAAAAAAAAAAAAAAAAAAAAAAAAAAAAAAAAAAAAA
SEQ ID NO:34 
ATGAGCTGGAAGGTGGTCATCATCTTCAGCCTGCTGATCACCCCTCAGCACGGCCTGAAAGA
GAGCTACCTGGAAGAGTCCTGCAGCACCATCACAGAGGGCTACCTGTCTGTGCTGAGAACCG
GCTGGTACACCAACGTGTTCACACTGGAAGTGGGCGACGTGGAAAACCTGACATGCTCTGAT
GGCCCCAGCCTGATCAAGACCGAGCTGGATCTGACAAAGAGCGCCCTGAGAGAACTGAAAAC
CGTGTCTGCCGATCAGCTGGCCAGAGAGGAACAGATCGAGAACCCCAGACAGAGCAGATTCG
TGCTGGGAGCTATTGCCCTGGGAGTTGCTACAGCTGCTGCTGTGACAGCTGGCGTGGCCATT
GCCAAGACCATCAGACTGGAAAGCGAAGTGACCGCCATCAAGAACGCCCTGAAAACCACCAA
CGAGTGCGTGTCTACACTCGGCAATGGCGTTAGAGTGCTGTGCACAGCAGTGCGCGAGCTGA
AGGACTTCGTGTCCAAGAACCTGACCAGAGCCATCAACAAGAACAAGTGCGACATCGACGAC
CTGAAGATGGCCGTGTCCTTTAGCCAGTTCAACCGGCGGTTTCTGAACGTCGTGCGGCAGTT
TTCTGACAACGCCGGCATCACACCAGCCATCAGCCTGGACCTGATGACAGATGCTGAGCTGG
CTAGAGCCGTGTCCAACATGCCTACATCTGCCGGCCAGATCAAGCTGATGCTGGAAAACAGA
 
GCCATGGTCCGACGGAAAGGCTTCGGCATCCTGATCGGCGTGTACGGCAGCAGCGTGATCTA
TATGGTGCAGCTGCCTATCTTCGGCGTGATCGACACCCCTTGCTGGATTGTGAAGGCCGCTC
CTAGCTGTAGCGGCAAGAAGGGCAATTACGCCTGCCTGCTGAGAGAGGACCAAGGCTGGTAT
TGTCAGAATGCCGGCAGCACCGTGTACTACCCCAACGAGAAGGATTGCGAGACACGGGGCGA
TCACGTGTTCTGTGATACAGCCGCCGGAATCAATGTGGCCGAGCAGAGCAAAGAGTGCAACA
TCAACATCAGCACCACGAACTACCCCTGCAAGGTGTCCACAGGCAGACACCCTATCTCTATG
GTGGCCCTGTCTCCTCTGGGAGCCCTGGTTGCTTGTTACAAGGGCGTGTCCTGTAGCATCGG
CAGCAACAGAGTGGGCATCATCAAGCAGCTGAACAAGGGCTGCAGCTACATCACCAACCAGG
ACGCCGATACCGTGACCATCGACAACACCGTGTATCAGCTGAGCAAGGTGGAAGGCGAACAG
CACGTGATCAAGGGCAGACCTGTGTCCAGCAGCTTCGACCCCATCAAGTTCCCCGAGGATCA
GTTCAACGTGGCCCTGGACCAGGTGTTCGAGAACATCGAGAATAGCCAGGCTCTGGTGGACC
AGTCCAACAGAATCCTGTCTAGCGCCGAGAAGGGAAACACCGGCTTCATCATCGTGATCATC
CTGATTGCCGTGCTGGGCTCCAGCATGATCCTGGTGTCCATCTTTATCATC
SEQ ID NO:35 
ATGAGCTGGAAAGTCATGATCATCATCAGCCTGCTGATCACCCCTCAGCACGGCCTGAAAGA
GAGCTACCTGGAAGAGTCCTGCAGCACCATCACAGAGGGCTACCTGTCTGTGCTGAGAACCG
GCTGGTACACCAACGTGTTCACACTGGAAGTGGGCGACGTGGAAAACCTGACCTGTACAGAT
GGCCCCAGCCTGATCAAGACCGAGCTGGATCTGACAAAGAGCGCCCTGAGAGAACTGAAAAC
CGTGTCTGCCGATCAGCTGGCCAGAGAGGAACAGATCGAGAACCCCAGACAGAGCAGATTCG
TGCTGGGAGCTATTGCCCTGGGAGTTGCTACAGCTGCTGCCGTGACAGCCGGAATCGCCATT
GCCAAGACCATCCGGCTGGAAAGCGAAGTGAATGCCATCAAGGGCGCACTGAAGCAGACCAA
CGAATGCGTGTCTACCCTCGGCAACGGTGTTAGAGTGCTGTGCACAGCAGTGCGCGAGCTGA
AAGAATTTGTGTCCAAGAACCTGACCAGCGCCATCAACCGGAACAAGTGCGACATTGCCGAC
CTGAAGATGGCCGTGTCCTTCAGCCAGTTCAACCGGCGGTTTCTGAACGTCGTGCGGCAGTT
TTCTGACAACGCCGGCATCACACCCGCCATTAGCCTGGACCTGATGACAGATGCTGAGCTGG
CTCGGGCCGTGTCTTACATGCCTACATCTGCCGGCCAGATCAAGCTGATGCTGGAAAACAGA
GCCATGGTCCGACGGAAAGGCTTCGGCATCCTGATCGGCGTGTACGGCAGCTCCGTGATCTA
TATGGTGCAGCTGCCTATCTTCGGCGTGATCGACACCCCTTGCTGGATCATTAAGGCCGCTC
CTAGCTGCAGCGAGAAGAACGGCAATTACGCCTGCCTGCTGAGAGAGGACCAAGGCTGGTAC
TGCAAGAATGCCGGCAGCACCGTGTACTACCCCAACGAGAAGGATTGCGAGACACGGGGCGA
TCACGTGTTCTGTGATACAGCCGCCGGAATCAATGTGGCCGAGCAGAGCAGAGAGTGCAACA
TCAACATCAGCACCACAAACTACCCCTGCAAGGTGTCCACAGGCAGACACCCTATCTCTATG
GTGGCCCTGTCTCCTCTGGGAGCCCTGGTTGCTTGTTACAAGGGCGTGTCCTGTAGCATCGG
CAGCAACTGGGTCGGAATCATCAAGCAGCTGCCCAAGGGCTGCTCCTACATCACCAATCAGG
ACGCCGACACCGTGACCATCGACAACACAGTGTATCAGCTGAGCAAGGTGGAAGGCGAGCAG
CACGTTATCAAGGGCAGACCTGTGTCCAGCAGCTTCGACCCCATCAAGTTCCCCGAGGATCA
GTTCAACGTGGCCCTGGATCAGGTGTTCGAGAGCATCGAGAATAGCCAGGCTCTGGTGGACC
AGTCCAACAAGATCCTGAATAGCGCCGAGAAGGGCAACACCGGCTTCATCATCGTGGTTATC
CTGGTGGCTGTGCTGGGCCTGACAATGATCAGCGTGTCCATCATCATTATC
SEQ ID NO:36 
ATGAGCTGGAAGGTGGTCATCATCTTCAGCCTGCTGATCACCCCTCAGCACGGCCTGAAAGA
GAGCTACCTGGAAGAGTCCTGCAGCACCATCACAGAGGGCTACCTGTCTGTGCTGAGAACCG
GCTGGTACACCAACGTGTTCACACTGGAAGTGGGCGACGTGGAAAACCTGACATGCTCTGAT
GGCCCCAGCCTGATCAAGACCGAGCTGGATCTGACAAAGAGCGCCCTGAGAGAACTGAAAAC
CGTGTCTGCCGATCAGCTGGCCAGAGAGGAACAGATCGAGAACCCCAGACAGAGCAGATTCG
TGCTGGGAGCTATTGCCCTGGGAGTTGCTACAGCTGCTGCTGTGACAGCTGGCGTGGCCATT
GCCAAGACCATCAGACTGGAAAGCGAAGTGACCGCCATCAAGAACGCCCTGAAAACCACCAA
CTGCGCCGTGTCTACACTCGGCAATGGCGTTAGAGTGCTGGCCTGCGCAGTGCGCGAGCTGA
AGGACTTCGTGTCCAAGAACCTGACCAGAGCCATCAACAAGAACAAGTGCGACATCGACGAC
CTGAAGATGGCCGTGTCCTTTAGCCAGTTCAACCGGCGGTTTCTGAACGTCGTGCGGCAGTT
TTCTGACAACGCCGGCATCACACCAGCCATCAGCCTGGACCTGATGACAGATGCTGAGCTGG
CTAGAGCCGTGTCCAACATGCCTACATCTGCCGGCCAGATCAAGCTGATGCTGGAAAACAGA
GCCATGGTCCGACGGAAAGGCTTCGGCATCCTGATCGGCGTGTACGGCAGCAGCGTGATCTA
TATGGTGCAGCTGCCTATCTTCGGCGTGATCGACACCCCTTGCTGGATTGTGAAGGCCGCTC
CTAGCTGTAGCGGCAAGAAGGGCAATTACGCCTGCCTGCTGAGAGAGGACCAAGGCTGGTAT
TGTCAGAATGCCGGCAGCACCGTGTACTACCCCAACGAGAAGGATTGCGAGACACGGGGCGA
TCACGTGTTCTGTGATACAGCCGCCGGAATCAATGTGGCCGAGCAGAGCAAAGAGTGCAACA
 
TCAACATCAGCACCACGAACTACCCCTGCAAGGTGTCCACAGGCAGACACCCTATCTCTATG
GTGGCCCTGTCTCCTCTGGGAGCCCTGGTTGCTTGTTACAAGGGCGTGTCCTGTAGCATCGG
CAGCAACAGAGTGGGCATCATCAAGCAGCTGAACAAGGGCTGCAGCTACATCACCAACCAGG
ACGCCGATACCGTGACCATCGACAACACCGTGTATCAGCTGAGCAAGGTGGAAGGCGAACAG
CACGTGATCAAGGGCAGACCTGTGTCCAGCAGCTTCGACCCCATCAAGTTCCCCGAGGATCA
GTTCAACGTGGCCCTGGACCAGGTGTTCGAGAACATCGAGAATAGCCAGGCTCTGGTGGACC
AGTCCAACAGAATCCTGTCTAGCGCCGAGAAGGGAAACACCGGCTTCATCATCGTGATCATC
CTGATTGCCGTGCTGGGCTCCAGCATGATCCTGGTGTCCATCTTTATCATC
SEQ ID NO:37 
ATGAGCTGGAAAGTCATGATCATCATCAGCCTGCTGATCACCCCTCAGCACGGCCTGAAAGA
GAGCTACCTGGAAGAGTCCTGCAGCACCATCACAGAGGGCTACCTGTCTGTGCTGAGAACCG
GCTGGTACACCAACGTGTTCACACTGGAAGTGGGCGACGTGGAAAACCTGACCTGTACAGAT
GGCCCCAGCCTGATCAAGACCGAGCTGGATCTGACAAAGAGCGCCCTGAGAGAACTGAAAAC
CGTGTCTGCCGATCAGCTGGCCAGAGAGGAACAGATCGAGAACCCCAGACAGAGCAGATTCG
TGCTGGGAGCTATTGCCCTGGGAGTTGCTACAGCTGCTGCCGTGACAGCCGGAATCGCCATT
GCCAAGACCATCCGGCTGGAAAGCGAAGTGAATGCCATCAAGGGCGCACTGAAGCAGACCAA
CTGCGCCGTGTCTACCCTCGGCAACGGTGTTAGAGTGCTGGCCTGCGCAGTGCGCGAGCTGA
AAGAATTTGTGTCCAAGAACCTGACCAGCGCCATCAACCGGAACAAGTGCGACATTGCCGAC
CTGAAGATGGCCGTGTCCTTCAGCCAGTTCAACCGGCGGTTTCTGAACGTCGTGCGGCAGTT
TTCTGACAACGCCGGCATCACACCCGCCATTAGCCTGGACCTGATGACAGATGCTGAGCTGG
CTCGGGCCGTGTCTTACATGCCTACATCTGCCGGCCAGATCAAGCTGATGCTGGAAAACAGA
GCCATGGTCCGACGGAAAGGCTTCGGCATCCTGATCGGCGTGTACGGCAGCTCCGTGATCTA
TATGGTGCAGCTGCCTATCTTCGGCGTGATCGACACCCCTTGCTGGATCATTAAGGCCGCTC
CTAGCTGCAGCGAGAAGAACGGCAATTACGCCTGCCTGCTGAGAGAGGACCAAGGCTGGTAC
TGCAAGAATGCCGGCAGCACCGTGTACTACCCCAACGAGAAGGATTGCGAGACACGGGGCGA
TCACGTGTTCTGTGATACAGCCGCCGGAATCAATGTGGCCGAGCAGAGCAGAGAGTGCAACA
TCAACATCAGCACCACAAACTACCCCTGCAAGGTGTCCACAGGCAGACACCCTATCTCTATG
GTGGCCCTGTCTCCTCTGGGAGCCCTGGTTGCTTGTTACAAGGGCGTGTCCTGTAGCATCGG
CAGCAACTGGGTCGGAATCATCAAGCAGCTGCCCAAGGGCTGCTCCTACATCACCAATCAGG
ACGCCGACACCGTGACCATCGACAACACAGTGTATCAGCTGAGCAAGGTGGAAGGCGAGCAG
CACGTTATCAAGGGCAGACCTGTGTCCAGCAGCTTCGACCCCATCAAGTTCCCCGAGGATCA
GTTCAACGTGGCCCTGGATCAGGTGTTCGAGAGCATCGAGAATAGCCAGGCTCTGGTGGACC
AGTCCAACAAGATCCTGAATAGCGCCGAGAAGGGCAACACCGGCTTCATCATCGTGGTTATC
CTGGTGGCTGTGCTGGGCCTGACAATGATCAGCGTGTCCATCATCATTATC
SEQ ID NO:38 
ATGAGCTGGAAGGTGGTCATCATCTTCAGCCTGCTGATCACCCCTCAGCACGGCCTGAAAGA
GAGCTACCTGGAAGAGTCCTGCAGCACCATCACAGAGGGCTACCTGTCTGTGCTGAGAACCG
GCTGGTACACCAACGTGTTCACACTGGAAGTGGGCGACGTGGAAAACCTGACATGCTCTGAT
GGCCCCAGCCTGATCAAGACCGAGCTGGATCTGACAAAGAGCGCCCTGAGAGAACTGAAAAC
CGTGTCTGCCGATCAGCTGGCCAGAGAGGAACAGATCGAGAACCCCAGACAGAGCAGATTCG
TGCTGGGAGCTATTGCCCTGGGAGTTGCTACAGCTGCTGCTGTGACAGCTGGCGTGGCCATT
GCCAAGACCATCAGACTGGAAAGCGAAGTGACCGCCATCAAGAACGCCCTGAAAACCACCAA
CGAGGCCGTGTCTACACTCGGCAATGGCGTTAGAGTGCTGGCCACAGCAGTGCGCGAGCTGA
AGGACTGCGTGTCCAAGAACCTGACCAGAGCCATCAACAAGAACAAGTGCGACATCGACGAC
CTGAAGATGGCCGTGTCCTTTAGCCAGTGCAACCGGCGGTTTCTGAACGTCGTGCGGCAGTT
TTCTGACAACGCCGGCATCACACCAGCCATCAGCCTGGACCTGATGACAGATGCTGAGCTGG
CTAGAGCCGTGTCCAACATGCCTACATCTGCCGGCCAGATCAAGCTGATGCTGGAAAACAGA
GCCATGGTCCGACGGAAAGGCTTCGGCATCCTGATCGGCGTGTACGGCAGCAGCGTGATCTA
TATGGTGCAGCTGCCTATCTTCGGCGTGATCGACACCCCTTGCTGGATTGTGAAGGCCGCTC
CTAGCTGTAGCGGCAAGAAGGGCAATTACGCCTGCCTGCTGAGAGAGGACCAAGGCTGGTAT
TGTCAGAATGCCGGCAGCACCGTGTACTACCCCAACGAGAAGGATTGCGAGACACGGGGCGA
TCACGTGTTCTGTGATACAGCCGCCGGAATCAATGTGGCCGAGCAGAGCAAAGAGTGCAACA
TCAACATCAGCACCACGAACTACCCCTGCAAGGTGTCCACAGGCAGACACCCTATCTCTATG
GTGGCCCTGTCTCCTCTGGGAGCCCTGGTTGCTTGTTACAAGGGCGTGTCCTGTAGCATCGG
CAGCAACAGAGTGGGCATCATCAAGCAGCTGAACAAGGGCTGCAGCTACATCACCAACCAGG
ACGCCGATACCGTGACCATCGACAACACCGTGTATCAGCTGAGCAAGGTGGAAGGCGAACAG
CACGTGATCAAGGGCAGACCTGTGTCCAGCAGCTTCGACCCCATCAAGTTCCCCGAGGATCA
 
GTTCAACGTGGCCCTGGACCAGGTGTTCGAGAACATCGAGAATAGCCAGGCTCTGGTGGACC
AGTCCAACAGAATCCTGTCTAGCGCCGAGAAGGGAAACACCGGCTTCATCATCGTGATCATC
CTGATTGCCGTGCTGGGCTCCAGCATGATCCTGGTGTCCATCTTTATCATC
SEQ ID NO:39 
ATGAGCTGGAAAGTCATGATCATCATCAGCCTGCTGATCACCCCTCAGCACGGCCTGAAAGA
GAGCTACCTGGAAGAGTCCTGCAGCACCATCACAGAGGGCTACCTGTCTGTGCTGAGAACCG
GCTGGTACACCAACGTGTTCACACTGGAAGTGGGCGACGTGGAAAACCTGACCTGTACAGAT
GGCCCCAGCCTGATCAAGACCGAGCTGGATCTGACAAAGAGCGCCCTGAGAGAACTGAAAAC
CGTGTCTGCCGATCAGCTGGCCAGAGAGGAACAGATCGAGAACCCCAGACAGAGCAGATTCG
TGCTGGGAGCTATTGCCCTGGGAGTTGCTACAGCTGCTGCCGTGACAGCCGGAATCGCCATT
GCCAAGACCATCCGGCTGGAAAGCGAAGTGAATGCCATCAAGGGCGCACTGAAGCAGACCAA
CGAAGCCGTGTCTACCCTCGGCAACGGTGTTAGAGTGCTGGCCACAGCAGTGCGCGAGCTGA
AAGAATGCGTGTCCAAGAACCTGACCAGCGCCATCAACCGGAACAAGTGCGACATTGCCGAC
CTGAAGATGGCCGTGTCCTTCAGCCAGTGCAACCGGCGGTTTCTGAACGTCGTGCGGCAGTT
TTCTGACAACGCCGGCATCACACCCGCCATTAGCCTGGACCTGATGACAGATGCTGAGCTGG
CTCGGGCCGTGTCTTACATGCCTACATCTGCCGGCCAGATCAAGCTGATGCTGGAAAACAGA
GCCATGGTCCGACGGAAAGGCTTCGGCATCCTGATCGGCGTGTACGGCAGCTCCGTGATCTA
TATGGTGCAGCTGCCTATCTTCGGCGTGATCGACACCCCTTGCTGGATCATTAAGGCCGCTC
CTAGCTGCAGCGAGAAGAACGGCAATTACGCCTGCCTGCTGAGAGAGGACCAAGGCTGGTAC
TGCAAGAATGCCGGCAGCACCGTGTACTACCCCAACGAGAAGGATTGCGAGACACGGGGCGA
TCACGTGTTCTGTGATACAGCCGCCGGAATCAATGTGGCCGAGCAGAGCAGAGAGTGCAACA
TCAACATCAGCACCACAAACTACCCCTGCAAGGTGTCCACAGGCAGACACCCTATCTCTATG
GTGGCCCTGTCTCCTCTGGGAGCCCTGGTTGCTTGTTACAAGGGCGTGTCCTGTAGCATCGG
CAGCAACTGGGTCGGAATCATCAAGCAGCTGCCCAAGGGCTGCTCCTACATCACCAATCAGG
ACGCCGACACCGTGACCATCGACAACACAGTGTATCAGCTGAGCAAGGTGGAAGGCGAGCAG
CACGTTATCAAGGGCAGACCTGTGTCCAGCAGCTTCGACCCCATCAAGTTCCCCGAGGATCA
GTTCAACGTGGCCCTGGATCAGGTGTTCGAGAGCATCGAGAATAGCCAGGCTCTGGTGGACC
AGTCCAACAAGATCCTGAATAGCGCCGAGAAGGGCAACACCGGCTTCATCATCGTGGTTATC
CTGGTGGCTGTGCTGGGCCTGACAATGATCAGCGTGTCCATCATCATTATC
SEQ ID NO:40 
ATGAGCTGGAAGGTGGTCATCATCTTCAGCCTGCTGATCACCCCTCAGCACGGCCTGAAAGA
GAGCTACCTGGAAGAGTCCTGCAGCACCATCACAGAGGGCTACCTGTCTGTGCTGAGAACCG
GCTGGTACACCAACGTGTTCACACTGGAAGTGGGCGACGTGGAAAACCTGACATGCTCTGAT
GGCCCCAGCCTGATCAAGACCGAGCTGGATCTGACAAAGAGCGCCCTGAGAGAACTGAAAAC
CGTGTCTGCCGATCAGCTGGCCAGAGAGGAACAGATCGAGAACCCCAGACAGAGCAGATTCG
TGCTGGGAGCTATTGCCCTGGGAGTTGCTACAGCTGCTGCTGTGACAGCTGGCGTGGCCATT
GCCAAGACCATCAGACTGGAAAGCGAAGTGACCGCCATCAAGAACGCCCTGAAAACCACCAA
CGAGGCCGTGTCTACACTCGGCAATGGCGTTAGAGTGCTGGCCACAGCAGTGCGCGAGTGCA
AGGACTTCGTGTCCAAGAACCTGACCAGAGCCATCAACAAGAACAAGTGCGACATCGACGAC
CTGAAGATGGCCGTGTCCTTTAGCCAGTGCAACCGGCGGTTTCTGAACGTCGTGCGGCAGTT
TTCTGACAACGCCGGCATCACACCAGCCATCAGCCTGGACCTGATGACAGATGCTGAGCTGG
CTAGAGCCGTGTCCAACATGCCTACATCTGCCGGCCAGATCAAGCTGATGCTGGAAAACAGA
GCCATGGTCCGACGGAAAGGCTTCGGCATCCTGATCGGCGTGTACGGCAGCAGCGTGATCTA
TATGGTGCAGCTGCCTATCTTCGGCGTGATCGACACCCCTTGCTGGATTGTGAAGGCCGCTC
CTAGCTGTAGCGGCAAGAAGGGCAATTACGCCTGCCTGCTGAGAGAGGACCAAGGCTGGTAT
TGTCAGAATGCCGGCAGCACCGTGTACTACCCCAACGAGAAGGATTGCGAGACACGGGGCGA
TCACGTGTTCTGTGATACAGCCGCCGGAATCAATGTGGCCGAGCAGAGCAAAGAGTGCAACA
TCAACATCAGCACCACGAACTACCCCTGCAAGGTGTCCACAGGCAGACACCCTATCTCTATG
GTGGCCCTGTCTCCTCTGGGAGCCCTGGTTGCTTGTTACAAGGGCGTGTCCTGTAGCATCGG
CAGCAACAGAGTGGGCATCATCAAGCAGCTGAACAAGGGCTGCAGCTACATCACCAACCAGG
ACGCCGATACCGTGACCATCGACAACACCGTGTATCAGCTGAGCAAGGTGGAAGGCGAACAG
CACGTGATCAAGGGCAGACCTGTGTCCAGCAGCTTCGACCCCATCAAGTTCCCCGAGGATCA
GTTCAACGTGGCCCTGGACCAGGTGTTCGAGAACATCGAGAATAGCCAGGCTCTGGTGGACC
AGTCCAACAGAATCCTGTCTAGCGCCGAGAAGGGAAACACCGGCTTCATCATCGTGATCATC
CTGATTGCCGTGCTGGGCTCCAGCATGATCCTGGTGTCCATCTTTATCATC
SEQ ID NO:41 
ATGAGCTGGAAAGTCATGATCATCATCAGCCTGCTGATCACCCCTCAGCACGGCCTGAAAGA
GAGCTACCTGGAAGAGTCCTGCAGCACCATCACAGAGGGCTACCTGTCTGTGCTGAGAACCG
GCTGGTACACCAACGTGTTCACACTGGAAGTGGGCGACGTGGAAAACCTGACCTGTACAGAT
GGCCCCAGCCTGATCAAGACCGAGCTGGATCTGACAAAGAGCGCCCTGAGAGAACTGAAAAC
CGTGTCTGCCGATCAGCTGGCCAGAGAGGAACAGATCGAGAACCCCAGACAGAGCAGATTCG
TGCTGGGAGCTATTGCCCTGGGAGTTGCTACAGCTGCTGCCGTGACAGCCGGAATCGCCATT
GCCAAGACCATCCGGCTGGAAAGCGAAGTGAATGCCATCAAGGGCGCACTGAAGCAGACCAA
CGAAGCCGTGTCTACCCTCGGCAACGGTGTTAGAGTGCTGGCCACAGCAGTGCGCGAGTGCA
AGAATTTGTGTCCAAGAACCTGACCAGCGCCATCAACCGGAACAAGTGCGACATTGCCGAC
CTGAAGATGGCCGTGTCCTTCAGCCAGTGCAACCGGCGGTTTCTGAACGTCGTGCGGCAGTT
TTCTGACAACGCCGGCATCACACCCGCCATTAGCCTGGACCTGATGACAGATGCTGAGCTGG
CTCGGGCCGTGTCTTACATGCCTACATCTGCCGGCCAGATCAAGCTGATGCTGGAAAACAGA
GCCATGGTCCGACGGAAAGGCTTCGGCATCCTGATCGGCGTGTACGGCAGCTCCGTGATCTA
TATGGTGCAGCTGCCTATCTTCGGCGTGATCGACACCCCTTGCTGGATCATTAAGGCCGCTC
CTAGCTGCAGCGAGAAGAACGGCAATTACGCCTGCCTGCTGAGAGAGGACCAAGGCTGGTAC
TGCAAGAATGCCGGCAGCACCGTGTACTACCCCAACGAGAAGGATTGCGAGACACGGGGCGA
TCACGTGTTCTGTGATACAGCCGCCGGAATCAATGTGGCCGAGCAGAGCAGAGAGTGCAACA
TCAACATCAGCACCACAAACTACCCCTGCAAGGTGTCCACAGGCAGACACCCTATCTCTATG
GTGGCCCTGTCTCCTCTGGGAGCCCTGGTTGCTTGTTACAAGGGCGTGTCCTGTAGCATCGG
CAGCAACTGGGTCGGAATCATCAAGCAGCTGCCCAAGGGCTGCTCCTACATCACCAATCAGG
ACGCCGACACCGTGACCATCGACAACACAGTGTATCAGCTGAGCAAGGTGGAAGGCGAGCAG
CACGTTATCAAGGGCAGACCTGTGTCCAGCAGCTTCGACCCCATCAAGTTCCCCGAGGATCA
GTTCAACGTGGCCCTGGATCAGGTGTTCGAGAGCATCGAGAATAGCCAGGCTCTGGTGGACC
AGTCCAACAAGATCCTGAATAGCGCCGAGAAGGGCAACACCGGCTTCATCATCGTGGTTATC
CTGGTGGCTGTGCTGGGCCTGACAATGATCAGCGTGTCCATCATCATTATC
SEQ ID NO:42 
ATGAGCTGGAAGGTGGTCATCATCTTCAGCCTGCTGATCACCCCTCAGCACGGCCTGAAAGA
GAGCTACCTGGAAGAGTCCTGCAGCACCATCACAGAGGGCTACCTGTCTGTGCTGAGAACCG
GCTGGTACACCAACGTGTTCACACTGGAAGTGGGCGACGTGGAAAACCTGACATGCTCTGAT
GGCCCCAGCCTGATCAAGACCGAGCTGGATCTGACAAAGAGCGCCCTGAGAGAACTGAAAAC
CGTGTCTGCCGATCAGCTGGCCAGAGAGGAACAGATCGAGAACCCCAGACAGAGCAGATTCG
TGCTGGGAGCTATTGCCCTGGGAGTTGCTACAGCTGCTGCTGTGACAGCTGGCGTGGCCATT
GCCAAGACCATCAGACTGGAAAGCGAAGTGACCGCCATCAAGAACGCCCTGAAAACCACCTG
CGAGGCCGTGTCTACACTCGGCAATGGCGTTAGAGTGCTGGCCACATGCGTGCGCGAGCTGA
AGGACTTCGTGTCCAAGAACCTGACCAGAGCCATCAACAAGAACAAGTGCGACATCGACGAC
CTGAAGATGGCCGTGTCCTTTAGCCAGTTCAACCGGCGGTTTCTGAACGTCGTGCGGCAGTT
TTCTGACAACGCCGGCATCACACCAGCCATCAGCCTGGACCTGATGACAGATGCTGAGCTGG
CTAGAGCCGTGTCCAACATGCCTACATCTGCCGGCCAGATCAAGCTGATGCTGGAAAACAGA
GCCATGGTCCGACGGAAAGGCTTCGGCATCCTGATCGGCGTGTACGGCAGCAGCGTGATCTA
TATGGTGCAGCTGCCTATCTTCGGCGTGATCGACACCCCTTGCTGGATTGTGAAGGCCGCTC
CTAGCTGTAGCGGCAAGAAGGGCAATTACGCCTGCCTGCTGAGAGAGGACCAAGGCTGGTAT
TGTCAGAATGCCGGCAGCACCGTGTACTACCCCAACGAGAAGGATTGCGAGACACGGGGCGA
TCACGTGTTCTGTGATACAGCCGCCGGAATCAATGTGGCCGAGCAGAGCAAAGAGTGCAACA
TCAACATCAGCACCACGAACTACCCCTGCAAGGTGTCCACAGGCAGACACCCTATCTCTATG
GTGGCCCTGTCTCCTCTGGGAGCCCTGGTTGCTTGTTACAAGGGCGTGTCCTGTAGCATCGG
CAGCAACAGAGTGGGCATCATCAAGCAGCTGAACAAGGGCTGCAGCTACATCACCAACCAGG
ACGCCGATACCGTGACCATCGACAACACCGTGTATCAGCTGAGCAAGGTGGAAGGCGAACAG
CACGTGATCAAGGGCAGACCTGTGTCCAGCAGCTTCGACCCCATCAAGTTCCCCGAGGATCA
GTTCAACGTGGCCCTGGACCAGGTGTTCGAGAACATCGAGAATAGCCAGGCTCTGGTGGACC
AGTCCAACAGAATCCTGTCTAGCGCCGAGAAGGGAAACACCGGCTTCATCATCGTGATCATC
CTGATTGCCGTGCTGGGCTCCAGCATGATCCTGGTGTCCATCTTTATCATC
SEQ ID NO:43 
ATGAGCTGGAAAGTCATGATCATCATCAGCCTGCTGATCACCCCTCAGCACGGCCTGAAAGA
GAGCTACCTGGAAGAGTCCTGCAGCACCATCACAGAGGGCTACCTGTCTGTGCTGAGAACCG
GCTGGTACACCAACGTGTTCACACTGGAAGTGGGCGACGTGGAAAACCTGACCTGTACAGAT
GGCCCCAGCCTGATCAAGACCGAGCTGGATCTGACAAAGAGCGCCCTGAGAGAACTGAAAAC
CGTGTCTGCCGATCAGCTGGCCAGAGAGGAACAGATCGAGAACCCCAGACAGAGCAGATTCG
 
TGCTGGGAGCTATTGCCCTGGGAGTTGCTACAGCTGCTGCCGTGACAGCCGGAATCGCCATT
GCCAAGACCATCCGGCTGGAAAGCGAAGTGAATGCCATCAAGGGCGCACTGAAGCAGACCTG
CGAAGCCGTGTCTACCCTCGGCAACGGTGTTAGAGTGCTGGCCACATGCGTGCGCGAGCTGA
AAGAATTTGTGTCCAAGAACCTGACCAGCGCCATCAACCGGAACAAGTGCGACATTGCCGAC
CTGAAGATGGCCGTGTCCTTCAGCCAGTTCAACCGGCGGTTTCTGAACGTCGTGCGGCAGTT
TTCTGACAACGCCGGCATCACACCCGCCATTAGCCTGGACCTGATGACAGATGCTGAGCTGG
CTCGGGCCGTGTCTTACATGCCTACATCTGCCGGCCAGATCAAGCTGATGCTGGAAAACAGA
GCCATGGTCCGACGGAAAGGCTTCGGCATCCTGATCGGCGTGTACGGCAGCTCCGTGATCTA
TATGGTGCAGCTGCCTATCTTCGGCGTGATCGACACCCCTTGCTGGATCATTAAGGCCGCTC
CTAGCTGCAGCGAGAAGAACGGCAATTACGCCTGCCTGCTGAGAGAGGACCAAGGCTGGTAC
TGCAAGAATGCCGGCAGCACCGTGTACTACCCCAACGAGAAGGATTGCGAGACACGGGGCGA
TCACGTGTTCTGTGATACAGCCGCCGGAATCAATGTGGCCGAGCAGAGCAGAGAGTGCAACA
TCAACATCAGCACCACAAACTACCCCTGCAAGGTGTCCACAGGCAGACACCCTATCTCTATG
GTGGCCCTGTCTCCTCTGGGAGCCCTGGTTGCTTGTTACAAGGGCGTGTCCTGTAGCATCGG
CAGCAACTGGGTCGGAATCATCAAGCAGCTGCCCAAGGGCTGCTCCTACATCACCAATCAGG
ACGCCGACACCGTGACCATCGACAACACAGTGTATCAGCTGAGCAAGGTGGAAGGCGAGCAG
CACGTTATCAAGGGCAGACCTGTGTCCAGCAGCTTCGACCCCATCAAGTTCCCCGAGGATCA
GTTCAACGTGGCCCTGGATCAGGTGTTCGAGAGCATCGAGAATAGCCAGGCTCTGGTGGACC
AGTCCAACAAGATCCTGAATAGCGCCGAGAAGGGCAACACCGGCTTCATCATCGTGGTTATC
CTGGTGGCTGTGCTGGGCCTGACAATGATCAGCGTGTCCATCATCATTATC
SEQ ID NO:44 
ATGAGCTGGAAGGTGGTCATCATCTTCAGCCTGCTGATCACCCCTCAGCACGGCCTGAAAGA
GAGCTACCTGGAAGAGTCCTGCAGCACCATCACAGAGGGCTACCTGTCTGTGCTGAGAACCG
GCTGGTACACCAACGTGTTCACACTGGAAGTGGGCGACGTGGAAAACCTGACATGCTCTGAT
GGCCCCAGCCTGATCAAGACCGAGCTGGATCTGACAAAGAGCGCCCTGAGAGAACTGAAAAC
CGTGTCTGCCGATCAGCTGGCCAGAGAGGAACAGATCGAGAACCCCAGACAGAGCAGATTCG
TGCTGGGAGCTATTGCCCTGGGAGTTGCTACAGCTGCTGCTGTGACAGCTGGCGTGGCCATT
GCCAAGACCATCAGACTGGAAAGCGAAGTGACCGCCATCAAGAACGCCCTGAAAACCACCAA
CGAGGCCGTGTGCACACTCGGCAATGGCGTTAGATGCCTGGCCACAGCAGTGCGCGAGCTGA
AGGACTTCGTGTCCAAGAACCTGACCAGAGCCATCAACAAGAACAAGTGCGACATCGACGAC
CTGAAGATGGCCGTGTCCTTTAGCCAGTTCAACCGGCGGTTTCTGAACGTCGTGCGGCAGTT
TTCTGACAACGCCGGCATCACACCAGCCATCAGCCTGGACCTGATGACAGATGCTGAGCTGG
CTAGAGCCGTGTCCAACATGCCTACATCTGCCGGCCAGATCAAGCTGATGCTGGAAAACAGA
GCCATGGTCCGACGGAAAGGCTTCGGCATCCTGATCGGCGTGTACGGCAGCAGCGTGATCTA
TATGGTGCAGCTGCCTATCTTCGGCGTGATCGACACCCCTTGCTGGATTGTGAAGGCCGCTC
CTAGCTGTAGCGGCAAGAAGGGCAATTACGCCTGCCTGCTGAGAGAGGACCAAGGCTGGTAT
TGTCAGAATGCCGGCAGCACCGTGTACTACCCCAACGAGAAGGATTGCGAGACACGGGGCGA
TCACGTGTTCTGTGATACAGCCGCCGGAATCAATGTGGCCGAGCAGAGCAAAGAGTGCAACA
TCAACATCAGCACCACGAACTACCCCTGCAAGGTGTCCACAGGCAGACACCCTATCTCTATG
GTGGCCCTGTCTCCTCTGGGAGCCCTGGTTGCTTGTTACAAGGGCGTGTCCTGTAGCATCGG
CAGCAACAGAGTGGGCATCATCAAGCAGCTGAACAAGGGCTGCAGCTACATCACCAACCAGG
ACGCCGATACCGTGACCATCGACAACACCGTGTATCAGCTGAGCAAGGTGGAAGGCGAACAG
CACGTGATCAAGGGCAGACCTGTGTCCAGCAGCTTCGACCCCATCAAGTTCCCCGAGGATCA
GTTCAACGTGGCCCTGGACCAGGTGTTCGAGAACATCGAGAATAGCCAGGCTCTGGTGGACC
AGTCCAACAGAATCCTGTCTAGCGCCGAGAAGGGAAACACCGGCTTCATCATCGTGATCATC
CTGATTGCCGTGCTGGGCTCCAGCATGATCCTGGTGTCCATCTTTATCATC
SEQ ID NO:45 
ATGAGCTGGAAAGTCATGATCATCATCAGCCTGCTGATCACCCCTCAGCACGGCCTGAAAGA
GAGCTACCTGGAAGAGTCCTGCAGCACCATCACAGAGGGCTACCTGTCTGTGCTGAGAACCG
GCTGGTACACCAACGTGTTCACACTGGAAGTGGGCGACGTGGAAAACCTGACCTGTACAGAT
GGCCCCAGCCTGATCAAGACCGAGCTGGATCTGACAAAGAGCGCCCTGAGAGAACTGAAAAC
CGTGTCTGCCGATCAGCTGGCCAGAGAGGAACAGATCGAGAACCCCAGACAGAGCAGATTCG
TGCTGGGAGCTATTGCCCTGGGAGTTGCTACAGCTGCTGCCGTGACAGCCGGAATCGCCATT
GCCAAGACCATCCGGCTGGAAAGCGAAGTGAATGCCATCAAGGGCGCACTGAAGCAGACCAA
CGAAGCCGTGTGCACCCTCGGCAACGGTGTTAGATGCCTGGCCACAGCAGTGCGCGAGCTGA
AAGAATTTGTGTCCAAGAACCTGACCAGCGCCATCAACCGGAACAAGTGCGACATTGCCGAC
CTGAAGATGGCCGTGTCCTTCAGCCAGTTCAACCGGCGGTTTCTGAACGTCGTGCGGCAGTT
 
TTCTGACAACGCCGGCATCACACCCGCCATTAGCCTGGACCTGATGACAGATGCTGAGCTGG
CTCGGGCCGTGTCTTACATGCCTACATCTGCCGGCCAGATCAAGCTGATGCTGGAAAACAGA
GCCATGGTCCGACGGAAAGGCTTCGGCATCCTGATCGGCGTGTACGGCAGCTCCGTGATCTA
TATGGTGCAGCTGCCTATCTTCGGCGTGATCGACACCCCTTGCTGGATCATTAAGGCCGCTC
CTAGCTGCAGCGAGAAGAACGGCAATTACGCCTGCCTGCTGAGAGAGGACCAAGGCTGGTAC
TGCAAGAATGCCGGCAGCACCGTGTACTACCCCAACGAGAAGGATTGCGAGACACGGGGCGA
TCACGTGTTCTGTGATACAGCCGCCGGAATCAATGTGGCCGAGCAGAGCAGAGAGTGCAACA
TCAACATCAGCACCACAAACTACCCCTGCAAGGTGTCCACAGGCAGACACCCTATCTCTATG
GTGGCCCTGTCTCCTCTGGGAGCCCTGGTTGCTTGTTACAAGGGCGTGTCCTGTAGCATCGG
CAGCAACTGGGTCGGAATCATCAAGCAGCTGCCCAAGGGCTGCTCCTACATCACCAATCAGG
ACGCCGACACCGTGACCATCGACAACACAGTGTATCAGCTGAGCAAGGTGGAAGGCGAGCAG
CACGTTATCAAGGGCAGACCTGTGTCCAGCAGCTTCGACCCCATCAAGTTCCCCGAGGATCA
GTTCAACGTGGCCCTGGATCAGGTGTTCGAGAGCATCGAGAATAGCCAGGCTCTGGTGGACC
AGTCCAACAAGATCCTGAATAGCGCCGAGAAGGGCAACACCGGCTTCATCATCGTGGTTATC
CTGGTGGCTGTGCTGGGCCTGACAATGATCAGCGTGTCCATCATCATTATC
SEQ ID NO:46 
ATGAGCTGGAAGGTGGTCATCATCTTCAGCCTGCTGATCACCCCTCAGCACGGCCTGAAAGA
GAGCTACCTGGAAGAGTCCTGCAGCACCATCACAGAGGGCTACCTGTCTGTGCTGAGAACCG
GCTGGTACACCAACGTGTTCACACTGGAAGTGGGCGACGTGGAAAACCTGTGCTGCTCTGAT
GGCCCCAGCCTGATCAAGACCGAGCTGGATCTGACAAAGAGCGCCCTGAGAGAACTGAAAAC
CGTGTCTGCCGATCAGCTGGCCAGAGAGGAACAGATCGAGAACCCCAGACAGAGCAGATTCG
TGCTGGGAGCTATTGCCCTGGGAGTTGCTACAGCTGCTGCTGTGACAGCTGGCGTGGCCATT
GCCAAGACCATCAGACTGGAAAGCGAAGTGACCGCCATCAAGAACGCCCTGAAAACCACCAA
CGAGGCCGTGTCTACACTCGGCAATGGCGTTAGAGTGCTGGCCACAGCAGTGCGCGAGCTGA
AGGACTTCGTGTCCAAGAACCTGACCAGAGCCATCAACAAGTGCAAGTGCGACATCGACGAC
CTGAAGATGGCCGTGTCCTTTAGCCAGTTCAACCGGCGGTTTCTGAACGTCGTGCGGCAGTT
TTCTGACAACGCCGGCATCACACCAGCCATCAGCCTGGACCTGATGACAGATGCTGAGCTGG
CTAGAGCCGTGTCCAACATGCCTACATCTGCCGGCCAGATCAAGCTGATGCTGGAAAACAGA
GCCATGGTCCGACGGAAAGGCTTCGGCATCCTGATCGGCGTGTACGGCAGCAGCGTGATCTA
TATGGTGCAGCTGCCTATCTTCGGCGTGATCGACACCCCTTGCTGGATTGTGAAGGCCGCTC
CTAGCTGTAGCGGCAAGAAGGGCAATTACGCCTGCCTGCTGAGAGAGGACCAAGGCTGGTAT
TGTCAGAATGCCGGCAGCACCGTGTACTACCCCAACGAGAAGGATTGCGAGACACGGGGCGA
TCACGTGTTCTGTGATACAGCCGCCGGAATCAATGTGGCCGAGCAGAGCAAAGAGTGCAACA
TCAACATCAGCACCACGAACTACCCCTGCAAGGTGTCCACAGGCAGACACCCTATCTCTATG
GTGGCCCTGTCTCCTCTGGGAGCCCTGGTTGCTTGTTACAAGGGCGTGTCCTGTAGCATCGG
CAGCAACAGAGTGGGCATCATCAAGCAGCTGAACAAGGGCTGCAGCTACATCACCAACCAGG
ACGCCGATACCGTGACCATCGACAACACCGTGTATCAGCTGAGCAAGGTGGAAGGCGAACAG
CACGTGATCAAGGGCAGACCTGTGTCCAGCAGCTTCGACCCCATCAAGTTCCCCGAGGATCA
GTTCAACGTGGCCCTGGACCAGGTGTTCGAGAACATCGAGAATAGCCAGGCTCTGGTGGACC
AGTCCAACAGAATCCTGTCTAGCGCCGAGAAGGGAAACACCGGC
SEQ ID NO:47 
ATGAGCTGGAAAGTCATGATCATCATCAGCCTGCTGATCACCCCTCAGCACGGCCTGAAAGA
GAGCTACCTGGAAGAGTCCTGCAGCACCATCACAGAGGGCTACCTGTCTGTGCTGAGAACCG
GCTGGTACACCAACGTGTTCACACTGGAAGTGGGCGACGTGGAAAACCTGTGCTGTACAGAT
GGCCCCAGCCTGATCAAGACCGAGCTGGATCTGACAAAGAGCGCCCTGAGAGAACTGAAAAC
CGTGTCTGCCGATCAGCTGGCCAGAGAGGAACAGATCGAGAACCCCAGACAGAGCAGATTCG
TGCTGGGAGCTATTGCCCTGGGAGTTGCTACAGCTGCTGCCGTGACAGCCGGAATCGCCATT
GCCAAGACCATCCGGCTGGAAAGCGAAGTGAATGCCATCAAGGGCGCACTGAAGCAGACCAA
CGAAGCCGTGTCTACCCTCGGCAACGGTGTTAGAGTGCTGGCCACAGCAGTGCGCGAGCTGA
AAGAATTTGTGTCCAAGAACCTGACCAGCGCCATCAACCGGTGCAAGTGCGACATTGCCGAC
CTGAAGATGGCCGTGTCCTTCAGCCAGTTCAACCGGCGGTTTCTGAACGTCGTGCGGCAGTT
TTCTGACAACGCCGGCATCACACCCGCCATTAGCCTGGACCTGATGACAGATGCTGAGCTGG
CTCGGGCCGTGTCTTACATGCCTACATCTGCCGGCCAGATCAAGCTGATGCTGGAAAACAGA
GCCATGGTCCGACGGAAAGGCTTCGGCATCCTGATCGGCGTGTACGGCAGCTCCGTGATCTA
TATGGTGCAGCTGCCTATCTTCGGCGTGATCGACACCCCTTGCTGGATCATTAAGGCCGCTC
CTAGCTGCAGCGAGAAGAACGGCAATTACGCCTGCCTGCTGAGAGAGGACCAAGGCTGGTAC
TGCAAGAATGCCGGCAGCACCGTGTACTACCCCAACGAGAAGGATTGCGAGACACGGGGCGA
 
TCACGTGTTCTGTGATACAGCCGCCGGAATCAATGTGGCCGAGCAGAGCAGAGAGTGCAACA
TCAACATCAGCACCACAAACTACCCCTGCAAGGTGTCCACAGGCAGACACCCTATCTCTATG
GTGGCCCTGTCTCCTCTGGGAGCCCTGGTTGCTTGTTACAAGGGCGTGTCCTGTAGCATCGG
CAGCAACTGGGTCGGAATCATCAAGCAGCTGCCCAAGGGCTGCTCCTACATCACCAATCAGG
ACGCCGACACCGTGACCATCGACAACACAGTGTATCAGCTGAGCAAGGTGGAAGGCGAGCAG
CACGTTATCAAGGGCAGACCTGTGTCCAGCAGCTTCGACCCCATCAAGTTCCCCGAGGATCA
GTTCAACGTGGCCCTGGATCAGGTGTTCGAGAGCATCGAGAATAGCCAGGCTCTGGTGGACC
AGTCCAACAAGATCCTGAATAGCGCCGAGAAGGGCAACACCGGC
SEQ ID NO:48 
ATGAGCTGGAAGGTGGTCATCATCTTCAGCCTGCTGATCACCCCTCAGCACGGCCTGAAAGA
GAGCTACCTGGAAGAGTCCTGCAGCACCATCACAGAGGGCTACCTGTCTGTGCTGAGAACCG
GCTGGTACACCAACGTGTTCACACTGGAAGTGGGCGACGTGGAAAACCTGTGCTGCTCTGAT
GGCCCCAGCCTGATCAAGACCGAGCTGGATCTGACAAAGAGCGCCCTGAGAGAACTGAAAAC
CGTGTCTGCCGATCAGCTGGCCAGAGAGGAACAGATCGAGAACCCCAGACAGAGCAGATTCG
TGCTGGGAGCTATTGCCCTGGGAGTTGCTACAGCTGCTGCTGTGACAGCTGGCGTGGCCATT
GCCAAGACCATCAGACTGGAAAGCGAAGTGACCGCCATCAAGAACGCCCTGAAAACCACCAA
CGAGGCCGTGTCTACACTCGGCAATGGCGTTAGAGTGCTGGCCACAGCAGTGCGCGAGCTGA
AGGACTTCGTGTCCAAGAACCTGACCAGAGCCATCAACAAGTGCAAGTGCGACATCGACGAC
CTGAAGATGGCCGTGTCCTTTAGCCAGTTCAACCGGCGGTTTCTGAACGTCGTGCGGCAGTT
TTCTGACAACGCCGGCATCACACCAGCCATCAGCCTGGACCTGATGACAGATGCTGAGCTGG
CTAGAGCCGTGTCCAACATGCCTACATCTGCCGGCCAGATCAAGCTGATGCTGGAAAACAGA
GCCATGGTCCGACGGAAAGGCTTCGGCATCCTGATCGGCGTGTACGGCAGCAGCGTGATCTA
TATGGTGCAGCTGCCTATCTTCGGCGTGATCGACACCCCTTGCTGGATTGTGAAGGCCGCTC
CTAGCTGTAGCGGCAAGAAGGGCAATTACGCCTGCCTGCTGAGAGAGGACCAAGGCTGGTAT
TGTCAGAATGCCGGCAGCACCGTGTACTACCCCAACGAGAAGGATTGCGAGACACGGGGCGA
TCACGTGTTCTGTGATACAGCCGCCGGAATCAATGTGGCCGAGCAGAGCAAAGAGTGCAACA
TCAACATCAGCACCACGAACTACCCCTGCAAGGTGTCCACAGGCAGACACCCTATCTCTATG
GTGGCCCTGTCTCCTCTGGGAGCCCTGGTTGCTTGTTACAAGGGCGTGTCCTGTAGCATCGG
CAGCAACAGAGTGGGCATCATCAAGCAGCTGAACAAGGGCTGCAGCTACATCACCAACCAGG
ACGCCGATACCGTGACCATCGACAACACCGTGTATCAGCTGAGCAAGGTGGAAGGCGAACAG
CACGTGATCAAGGGCAGACCTGTGTCCAGCAGCTTCGACCCCATCAAGTTCCCCGAGGATCA
GTTCAACGTGGCCCTGGACCAGGTGTTCGAGAACATCGAGAATAGCCAGGCTCTGGTGGACC
AGTCCAACAGAATCCTGTCTAGCGCCGAGAAGGGAAACACCGGC
SEQ ID NO:49 
ATGAGCTGGAAAGTCATGATCATCATCAGCCTGCTGATCACCCCTCAGCACGGCCTGAAAGA
GAGCTACCTGGAAGAGTCCTGCAGCACCATCACAGAGGGCTACCTGTCTGTGCTGAGAACCG
GCTGGTACACCAACGTGTTCACACTGGAAGTGGGCGACGTGGAAAACCTGTGCTGTACAGAT
GGCCCCAGCCTGATCAAGACCGAGCTGGATCTGACAAAGAGCGCCCTGAGAGAACTGAAAAC
CGTGTCTGCCGATCAGCTGGCCAGAGAGGAACAGATCGAGAACCCCAGACAGAGCAGATTCG
TGCTGGGAGCTATTGCCCTGGGAGTTGCTACAGCTGCTGCCGTGACAGCCGGAATCGCCATT
GCCAAGACCATCCGGCTGGAAAGCGAAGTGAATGCCATCAAGGGCGCACTGAAGCAGACCAA
CGAAGCCGTGTCTACCCTCGGCAACGGTGTTAGAGTGCTGGCCACAGCAGTGCGCGAGCTGA
AAGAATTTGTGTCCAAGAACCTGACCAGCGCCATCAACCGGTGCAAGTGCGACATTGCCGAC
CTGAAGATGGCCGTGTCCTTCAGCCAGTTCAACCGGCGGTTTCTGAACGTCGTGCGGCAGTT
TTCTGACAACGCCGGCATCACACCCGCCATTAGCCTGGACCTGATGACAGATGCTGAGCTGG
CTCGGGCCGTGTCTTACATGCCTACATCTGCCGGCCAGATCAAGCTGATGCTGGAAAACAGA
GCCATGGTCCGACGGAAAGGCTTCGGCATCCTGATCGGCGTGTACGGCAGCTCCGTGATCTA
TATGGTGCAGCTGCCTATCTTCGGCGTGATCGACACCCCTTGCTGGATCATTAAGGCCGCTC
CTAGCTGCAGCGAGAAGAACGGCAATTACGCCTGCCTGCTGAGAGAGGACCAAGGCTGGTAC
TGCAAGAATGCCGGCAGCACCGTGTACTACCCCAACGAGAAGGATTGCGAGACACGGGGCGA
TCACGTGTTCTGTGATACAGCCGCCGGAATCAATGTGGCCGAGCAGAGCAGAGAGTGCAACA
TCAACATCAGCACCACAAACTACCCCTGCAAGGTGTCCACAGGCAGACACCCTATCTCTATG
GTGGCCCTGTCTCCTCTGGGAGCCCTGGTTGCTTGTTACAAGGGCGTGTCCTGTAGCATCGG
CAGCAACTGGGTCGGAATCATCAAGCAGCTGCCCAAGGGCTGCTCCTACATCACCAATCAGG
ACGCCGACACCGTGACCATCGACAACACAGTGTATCAGCTGAGCAAGGTGGAAGGCGAGCAG
CACGTTATCAAGGGCAGACCTGTGTCCAGCAGCTTCGACCCCATCAAGTTCCCCGAGGATCA
GTTCAACGTGGCCCTGGATCAGGTGTTCGAGAGCATCGAGAATAGCCAGGCTCTGGTGGACC
 AGTCCAACAAGATCCTGAATAGCGCCGAGAAGGGCAACACCGGC
SEQ ID NO:50 
ATGAGCTGGAAGGTGGTCATCATCTTCAGCCTGCTGATCACCCCTCAGCACGGCCTGAAAGA
GAGCTACCTGGAAGAGTCCTGCAGCACCATCACAGAGGGCTACCTGTCTGTGCTGAGAACCG
GCTGGTACACCAACGTGTTCACACTGGAAGTGGGCGACGTGGAAAACCTGTGCTGCTCTGAT
GGCCCCAGCCTGATCAAGACCGAGCTGGATCTGACAAAGAGCGCCCTGAGAGAACTGAAAAC
CGTGTCTGCCGATCAGCTGGCCAGAGAGGAACAGATCGAGAACCCCAGACAGAGCAGATTCG
TGCTGGGAGCTATTGCCCTGGGAGTTGCTACAGCTGCTGCTGTGACAGCTGGCGTGGCCATT
GCCAAGACCATCAGACTGGAAAGCGAAGTGACCGCCATCAAGAACGCCCTGAAAACCACCAA
CGAGGCCGTGTCTACACTCGGCAATGGCGTTAGAGTGCTGGCCACAGCAGTGCGCGAGCTGA
AGGACTTCGTGTCCAAGAACCTGACCAGAGCCATCAACAAGTGCAAGTGCGACATCGACGAC
CTGAAGATGGCCGTGTCCTTTAGCCAGTTCAACCGGCGGTTTCTGAACGTCGTGCGGCAGTT
TTCTGACAACGCCGGCATCACACCAGCCATCAGCCTGGACCTGATGACAGATGCTGAGCTGG
CTAGAGCCGTGTCCAACATGCCTACATCTGCCGGCCAGATCAAGCTGATGCTGGAAAACAGA
GCCATGGTCCGACGGAAAGGCTTCGGCATCCTGATCGGCGTGTACGGCAGCAGCGTGATCTA
TATGGTGCAGCTGCCTATCTTCGGCGTGATCGACACCCCTTGCTGGATTGTGAAGGCCGCTC
CTAGCTGTAGCGGCAAGAAGGGCAATTACGCCTGCCTGCTGAGAGAGGACCAAGGCTGGTAT
TGTCAGAATGCCGGCAGCACCGTGTACTACCCCAACGAGAAGGATTGCGAGACACGGGGCGA
TCACGTGTTCTGTGATACAGCCGCCGGAATCAATGTGGCCGAGCAGAGCAAAGAGTGCAACA
TCAACATCAGCACCACGAACTACCCCTGCAAGGTGTCCACAGGCAGACACCCTATCTCTATG
GTGGCCCTGTCTCCTCTGGGAGCCCTGGTTGCTTGTTACAAGGGCGTGTCCTGTAGCATCGG
CAGCAACAGAGTGGGCATCATCAAGCAGCTGAACAAGGGCTGCAGCTACATCACCAACCAGG
ACGCCGATACCGTGACCATCGACAACACCGTGTATCAGCTGAGCAAGGTGGAAGGCGAACAG
CACGTGATCAAGGGCAGACCTGTGTCCAGCAGCTTCGACCCCATCAAGTTCCCCGAGGATCA
GTTCAACGTGGCCCTGGACCAGGTGTTCGAGAACATCGAGAATAGCCAGGCTCTGGTGGACC
AGTCCAACAGAATCCTGTCTAGCGCCGAGAAGGGAAACACCGGCTTCATCATCGTGATCATC
CTGATTGCCGTGCTGGGCTCCAGCATGATCCTGGTGTCCATCTTTATCATCATCAAAAAGAC
CAAGAAGCCCACAGGCGCCCCTCCAGAACTGAGCGGAGTGACCAACAATGGCTTCATCCCTC
ACAGC
SEQ ID NO:51 
ATGAGCTGGAAAGTCATGATCATCATCAGCCTGCTGATCACCCCTCAGCACGGCCTGAAAGA
GAGCTACCTGGAAGAGTCCTGCAGCACCATCACAGAGGGCTACCTGTCTGTGCTGAGAACCG
GCTGGTACACCAACGTGTTCACACTGGAAGTGGGCGACGTGGAAAACCTGTGCTGTACAGAT
GGCCCCAGCCTGATCAAGACCGAGCTGGATCTGACAAAGAGCGCCCTGAGAGAACTGAAAAC
CGTGTCTGCCGATCAGCTGGCCAGAGAGGAACAGATCGAGAACCCCAGACAGAGCAGATTCG
TGCTGGGAGCTATTGCCCTGGGAGTTGCTACAGCTGCTGCCGTGACAGCCGGAATCGCCATT
GCCAAGACCATCCGGCTGGAAAGCGAAGTGAATGCCATCAAGGGCGCACTGAAGCAGACCAA
CGAAGCCGTGTCTACCCTCGGCAACGGTGTTAGAGTGCTGGCCACAGCAGTGCGCGAGCTGA
AAGAATTTGTGTCCAAGAACCTGACCAGCGCCATCAACCGGTGCAAGTGCGACATTGCCGAC
CTGAAGATGGCCGTGTCCTTCAGCCAGTTCAACCGGCGGTTTCTGAACGTCGTGCGGCAGTT
TTCTGACAACGCCGGCATCACACCCGCCATTAGCCTGGACCTGATGACAGATGCTGAGCTGG
CTCGGGCCGTGTCTTACATGCCTACATCTGCCGGCCAGATCAAGCTGATGCTGGAAAACAGA
GCCATGGTCCGACGGAAAGGCTTCGGCATCCTGATCGGCGTGTACGGCAGCTCCGTGATCTA
TATGGTGCAGCTGCCTATCTTCGGCGTGATCGACACCCCTTGCTGGATCATTAAGGCCGCTC
CTAGCTGCAGCGAGAAGAACGGCAATTACGCCTGCCTGCTGAGAGAGGACCAAGGCTGGTAC
TGCAAGAATGCCGGCAGCACCGTGTACTACCCCAACGAGAAGGATTGCGAGACACGGGGCGA
TCACGTGTTCTGTGATACAGCCGCCGGAATCAATGTGGCCGAGCAGAGCAGAGAGTGCAACA
TCAACATCAGCACCACAAACTACCCCTGCAAGGTGTCCACAGGCAGACACCCTATCTCTATG
GTGGCCCTGTCTCCTCTGGGAGCCCTGGTTGCTTGTTACAAGGGCGTGTCCTGTAGCATCGG
CAGCAACTGGGTCGGAATCATCAAGCAGCTGCCCAAGGGCTGCTCCTACATCACCAATCAGG
ACGCCGACACCGTGACCATCGACAACACAGTGTATCAGCTGAGCAAGGTGGAAGGCGAGCAG
CACGTTATCAAGGGCAGACCTGTGTCCAGCAGCTTCGACCCCATCAAGTTCCCCGAGGATCA
GTTCAACGTGGCCCTGGATCAGGTGTTCGAGAGCATCGAGAATAGCCAGGCTCTGGTGGACC
AGTCCAACAAGATCCTGAATAGCGCCGAGAAGGGCAACACCGGCTTCATCATCGTGGTTATC
CTGGTGGCTGTGCTGGGCCTGACAATGATCAGCGTGTCCATCATCATTATCATCAAGAAAAC
CCGGAAGCCTACAGGCGCCCCTCCTGAACTGAATGGCGTGACCAATGGCGGCTTCATTCCCC
ACTCT
SEQ ID NO:52 
ATGAGCTGGAAGGTGGTCATCATCTTCAGCCTGCTGATCACCCCTCAGCACGGCCTGAAAGA
GAGCTACCTGGAAGAGTCCTGCAGCACCATCACAGAGGGCTACCTGTCTGTGCTGAGAACCG
GCTGGTACACCAACGTGTTCACACTGGAAGTGGGCGACGTGGAAAACCTGTGCTGCTCTGAT
GGCCCCAGCCTGATCAAGACCGAGCTGGATCTGACAAAGAGCGCCCTGAGAGAACTGAAAAC
CGTGTCTGCCGATCAGCTGGCCAGAGAGGAACAGATCGAGAACCCCAGACAGAGCAGATTCG
TGCTGGGAGCTATTGCCCTGGGAGTTGCTACAGCTGCTGCTGTGACAGCTGGCGTGGCCATT
GCCAAGACCATCAGACTGGAAAGCGAAGTGACCGCCATCAAGAACGCCCTGAAAACCACCAA
CGAGGCCGTGTCTACACTCGGCAATGGCGTTAGAGTGCTGGCCACAGCAGTGCGCGAGCTGA
AGGACTTCGTGTCCAAGAACCTGACCAGAGCCATCAACAAGTGCAAGTGCGACATCGACGAC
CTGAAGATGGCCGTGTCCTTTAGCCAGTTCAACCGGCGGTTTCTGAACGTCGTGCGGCAGTT
TTCTGACAACGCCGGCATCACACCAGCCATCAGCCTGGACCTGATGACAGATGCTGAGCTGG
CTAGAGCCGTGTCCAACATGCCTACATCTGCCGGCCAGATCAAGCTGATGCTGGAAAACAGA
GCCATGGTCCGACGGAAAGGCTTCGGCATCCTGATCGGCGTGTACGGCAGCAGCGTGATCTA
TATGGTGCAGCTGCCTATCTTCGGCGTGATCGACACCCCTTGCTGGATTGTGAAGGCCGCTC
CTAGCTGTAGCGGCAAGAAGGGCAATTACGCCTGCCTGCTGAGAGAGGACCAAGGCTGGTAT
TGTCAGAATGCCGGCAGCACCGTGTACTACCCCAACGAGAAGGATTGCGAGACACGGGGCGA
TCACGTGTTCTGTGATACAGCCGCCGGAATCAATGTGGCCGAGCAGAGCAAAGAGTGCAACA
TCAACATCAGCACCACGAACTACCCCTGCAAGGTGTCCACAGGCAGACACCCTATCTCTATG
GTGGCCCTGTCTCCTCTGGGAGCCCTGGTTGCTTGTTACAAGGGCGTGTCCTGTAGCATCGG
CAGCAACAGAGTGGGCATCATCAAGCAGCTGAACAAGGGCTGCAGCTACATCACCAACCAGG
ACGCCGATACCGTGACCATCGACAACACCGTGTATCAGCTGAGCAAGGTGGAAGGCGAACAG
CACGTGATCAAGGGCAGACCTGTGTCCAGCAGCTTCGACCCCATCAAGTTCCCCGAGGATCA
GTTCAACGTGGCCCTGGACCAGGTGTTCGAGAACATCGAGAATAGCCAGGCTCTGGTGGACC
AGTCCAACAGAATCCTGTCTAGCGCCGAGAAGGGAAACACCGGCTTCATCATCGTGATCATC
CTGATTGCCGTGCTGGGCTCCAGCATGATCCTGGTGTCCATCTTTATCATC
SEQ ID NO:53 
ATGAGCTGGAAAGTCATGATCATCATCAGCCTGCTGATCACCCCTCAGCACGGCCTGAAAGA
GAGCTACCTGGAAGAGTCCTGCAGCACCATCACAGAGGGCTACCTGTCTGTGCTGAGAACCG
GCTGGTACACCAACGTGTTCACACTGGAAGTGGGCGACGTGGAAAACCTGTGCTGTACAGAT
GGCCCCAGCCTGATCAAGACCGAGCTGGATCTGACAAAGAGCGCCCTGAGAGAACTGAAAAC
CGTGTCTGCCGATCAGCTGGCCAGAGAGGAACAGATCGAGAACCCCAGACAGAGCAGATTCG
TGCTGGGAGCTATTGCCCTGGGAGTTGCTACAGCTGCTGCCGTGACAGCCGGAATCGCCATT
GCCAAGACCATCCGGCTGGAAAGCGAAGTGAATGCCATCAAGGGCGCACTGAAGCAGACCAA
CGAAGCCGTGTCTACCCTCGGCAACGGTGTTAGAGTGCTGGCCACAGCAGTGCGCGAGCTGA
AAGAATTTGTGTCCAAGAACCTGACCAGCGCCATCAACCGGTGCAAGTGCGACATTGCCGAC
CTGAAGATGGCCGTGTCCTTCAGCCAGTTCAACCGGCGGTTTCTGAACGTCGTGCGGCAGTT
TTCTGACAACGCCGGCATCACACCCGCCATTAGCCTGGACCTGATGACAGATGCTGAGCTGG
CTCGGGCCGTGTCTTACATGCCTACATCTGCCGGCCAGATCAAGCTGATGCTGGAAAACAGA
GCCATGGTCCGACGGAAAGGCTTCGGCATCCTGATCGGCGTGTACGGCAGCTCCGTGATCTA
TATGGTGCAGCTGCCTATCTTCGGCGTGATCGACACCCCTTGCTGGATCATTAAGGCCGCTC
CTAGCTGCAGCGAGAAGAACGGCAATTACGCCTGCCTGCTGAGAGAGGACCAAGGCTGGTAC
TGCAAGAATGCCGGCAGCACCGTGTACTACCCCAACGAGAAGGATTGCGAGACACGGGGCGA
TCACGTGTTCTGTGATACAGCCGCCGGAATCAATGTGGCCGAGCAGAGCAGAGAGTGCAACA
TCAACATCAGCACCACAAACTACCCCTGCAAGGTGTCCACAGGCAGACACCCTATCTCTATG
GTGGCCCTGTCTCCTCTGGGAGCCCTGGTTGCTTGTTACAAGGGCGTGTCCTGTAGCATCGG
CAGCAACTGGGTCGGAATCATCAAGCAGCTGCCCAAGGGCTGCTCCTACATCACCAATCAGG
ACGCCGACACCGTGACCATCGACAACACAGTGTATCAGCTGAGCAAGGTGGAAGGCGAGCAG
CACGTTATCAAGGGCAGACCTGTGTCCAGCAGCTTCGACCCCATCAAGTTCCCCGAGGATCA
GTTCAACGTGGCCCTGGATCAGGTGTTCGAGAGCATCGAGAATAGCCAGGCTCTGGTGGACC
AGTCCAACAAGATCCTGAATAGCGCCGAGAAGGGCAACACCGGCTTCATCATCGTGGTTATC
CTGGTGGCTGTGCTGGGCCTGACAATGATCAGCGTGTCCATCATCATTATC
SEQ ID NO:54 
ATGAGCTGGAAGGTGGTCATCATCTTCAGCCTGCTGATCACCCCTCAGCACGGCCTGAAAGA
GAGCTACCTGGAAGAGTCCTGCAGCACCATCACAGAGGGCTACCTGTCTGTGCTGAGAACCG
GCTGGTACACCAACGTGTTCACACTGGAAGTGGGCGACGTGGAAAACCTGACATGCTCTGAT
 
GGCCCCAGCCTGATCAAGACCGAGCTGGATCTGACAAAGAGCGCCCTGAGAGAACTGAAAAC
CGTGTCTGCCGATCAGCTGGCCAGAGAGGAACAGATCGAGAACCCCAGACAGAGCAGATTCG
TGCTGGGAGCTATTGCCCTGGGAGTTGCTACAGCTGCTGCTGTGACAGCTGGCGTGGCCATT
GCCAAGACCATCAGACTGGAAAGCGAAGTGACCGCCATCAAGAACGCCCTGAAAACCACCAA
CGAGGCCGTGTCTTGCCTCGGCAATGGCGTTTGCGTGCTGGCCACAGCAGTGCGCGAGCTGA
AGGACTTCGTGTCCAAGAACCTGACCAGAGCCATCAACAAGAACAAGTGCGACATCGACGAC
CTGAAGATGGCCGTGTCCTTTAGCCAGTTCAACCGGCGGTTTCTGAACGTCGTGCGGCAGTT
TTCTGACAACGCCGGCATCACACCAGCCATCAGCCTGGACCTGATGACAGATGCTGAGCTGG
CTAGAGCCGTGTCCAACATGCCTACATCTGCCGGCCAGATCAAGCTGATGCTGGAAAACAGA
GCCATGGTCCGACGGAAAGGCTTCGGCATCCTGATCGGCGTGTACGGCAGCAGCGTGATCTA
TATGGTGCAGCTGCCTATCTTCGGCGTGATCGACACCCCTTGCTGGATTGTGAAGGCCGCTC
CTAGCTGTAGCGGCAAGAAGGGCAATTACGCCTGCCTGCTGAGAGAGGACCAAGGCTGGTAT
TGTCAGAATGCCGGCAGCACCGTGTACTACCCCAACGAGAAGGATTGCGAGACACGGGGCGA
TCACGTGTTCTGTGATACAGCCGCCGGAATCAATGTGGCCGAGCAGAGCAAAGAGTGCAACA
TCAACATCAGCACCACGAACTACCCCTGCAAGGTGTCCACAGGCAGACACCCTATCTCTATG
GTGGCCCTGTCTCCTCTGGGAGCCCTGGTTGCTTGTTACAAGGGCGTGTCCTGTAGCATCGG
CAGCAACAGAGTGGGCATCATCAAGCAGCTGAACAAGGGCTGCAGCTACATCACCAACCAGG
ACGCCGATACCGTGACCATCGACAACACCGTGTATCAGCTGAGCAAGGTGGAAGGCGAACAG
CACGTGATCAAGGGCAGACCTGTGTCCAGCAGCTTCGACCCCATCAAGTTCCCCGAGGATCA
GTTCAACGTGGCCCTGGACCAGGTGTTCGAGAACATCGAGAATAGCCAGGCTCTGGTGGACC
AGTCCAACAGAATCCTGTCTAGCGCCGAGAAGGGAAACACCGGCTTCATCATCGTGATCATC
CTGATTGCCGTGCTGGGCTCCAGCATGATCCTGGTGTCCATCTTTATCATC
SEQ ID NO:55 
ATGAGCTGGAAAGTCATGATCATCATCAGCCTGCTGATCACCCCTCAGCACGGCCTGAAAGA
GAGCTACCTGGAAGAGTCCTGCAGCACCATCACAGAGGGCTACCTGTCTGTGCTGAGAACCG
GCTGGTACACCAACGTGTTCACACTGGAAGTGGGCGACGTGGAAAACCTGACCTGTACAGAT
GGCCCCAGCCTGATCAAGACCGAGCTGGATCTGACAAAGAGCGCCCTGAGAGAACTGAAAAC
CGTGTCTGCCGATCAGCTGGCCAGAGAGGAACAGATCGAGAACCCCAGACAGAGCAGATTCG
TGCTGGGAGCTATTGCCCTGGGAGTTGCTACAGCTGCTGCCGTGACAGCCGGAATCGCCATT
GCCAAGACCATCCGGCTGGAAAGCGAAGTGAATGCCATCAAGGGCGCACTGAAGCAGACCAA
CGAAGCCGTGTCTTGCCTCGGCAACGGTGTTTGCGTGCTGGCCACAGCAGTGCGCGAGCTGA
AAGAATTTGTGTCCAAGAACCTGACCAGCGCCATCAACCGGAACAAGTGCGACATTGCCGAC
CTGAAGATGGCCGTGTCCTTCAGCCAGTTCAACCGGCGGTTTCTGAACGTCGTGCGGCAGTT
TTCTGACAACGCCGGCATCACACCCGCCATTAGCCTGGACCTGATGACAGATGCTGAGCTGG
CTCGGGCCGTGTCTTACATGCCTACATCTGCCGGCCAGATCAAGCTGATGCTGGAAAACAGA
GCCATGGTCCGACGGAAAGGCTTCGGCATCCTGATCGGCGTGTACGGCAGCTCCGTGATCTA
TATGGTGCAGCTGCCTATCTTCGGCGTGATCGACACCCCTTGCTGGATCATTAAGGCCGCTC
CTAGCTGCAGCGAGAAGAACGGCAATTACGCCTGCCTGCTGAGAGAGGACCAAGGCTGGTAC
TGCAAGAATGCCGGCAGCACCGTGTACTACCCCAACGAGAAGGATTGCGAGACACGGGGCGA
TCACGTGTTCTGTGATACAGCCGCCGGAATCAATGTGGCCGAGCAGAGCAGAGAGTGCAACA
TCAACATCAGCACCACAAACTACCCCTGCAAGGTGTCCACAGGCAGACACCCTATCTCTATG
GTGGCCCTGTCTCCTCTGGGAGCCCTGGTTGCTTGTTACAAGGGCGTGTCCTGTAGCATCGG
CAGCAACTGGGTCGGAATCATCAAGCAGCTGCCCAAGGGCTGCTCCTACATCACCAATCAGG
ACGCCGACACCGTGACCATCGACAACACAGTGTATCAGCTGAGCAAGGTGGAAGGCGAGCAG
CACGTTATCAAGGGCAGACCTGTGTCCAGCAGCTTCGACCCCATCAAGTTCCCCGAGGATCA
GTTCAACGTGGCCCTGGATCAGGTGTTCGAGAGCATCGAGAATAGCCAGGCTCTGGTGGACC
AGTCCAACAAGATCCTGAATAGCGCCGAGAAGGGCAACACCGGCTTCATCATCGTGGTTATC
CTGGTGGCTGTGCTGGGCCTGACAATGATCAGCGTGTCCATCATCATTATC
SEQ ID NO:56 
ATGAGCTGGAAGGTGGTCATCATCTTCAGCCTGCTGATCACCCCTCAGCACGGCCTGAAAGA
GAGCTACCTGGAAGAGTCCTGCAGCACCATCACAGAGGGCTACCTGTCTGTGCTGAGAACCG
GCTGGTACACCAACGTGTTCACACTGGAATGCGGCGACGTGGAAAACCTGACATGCTCTGAT
GGCCCCAGCCTGATCAAGACCGAGCTGGATCTGACAAAGAGCGCCCTGAGAGAACTGAAAAC
CGTGTCTGCCGATCAGCTGGCCAGAGAGGAACAGATCGAGAACCCCAGACAGAGCAGATTCG
TGCTGGGAGCTATTGCCCTGGGAGTTGCTACAGCTGCTGCTGTGACAGCTGGCGTGGCCATT
GCCAAGACCATCAGACTGGAAAGCGAAGTGACCGCCATCAAGAACGCCCTGAAAACCACCAA
CGAGGCCGTGTCTACACTCGGCAATGGCGTTAGAGTGCTGGCCACAGCAGTGCGCGAGTGCA
 
AGGACTTCGTGTCCAAGAACCTGACCAGAGCCATCAACAAGAACAAGTGCGACATCGACGAC
CTGAAGATGGCCGTGTCCTTTAGCCAGTTCAACCGGCGGTTTCTGAACGTCGTGCGGCAGTT
TTCTGACAACGCCGGCATCACACCAGCCATCAGCCTGGACCTGATGACAGATGCTGAGCTGG
CTAGAGCCGTGTCCAACATGCCTACATCTGCCGGCCAGATCAAGCTGATGCTGGAAAACAGA
GCCATGGTCCGACGGAAAGGCTTCGGCATCCTGATCGGCGTGTACGGCAGCAGCGTGATCTA
TATGGTGCAGCTGCCTATCTTCGGCGTGATCGACACCCCTTGCTGGATTGTGAAGGCCGCTC
CTAGCTGTAGCGGCAAGAAGGGCAATTACGCCTGCCTGCTGAGAGAGGACCAAGGCTGGTAT
TGTCAGAATGCCGGCAGCACCGTGTACTACCCCAACGAGAAGGATTGCGAGACACGGGGCGA
TCACGTGTTCTGTGATACAGCCGCCGGAATCAATGTGGCCGAGCAGAGCAAAGAGTGCAACA
TCAACATCAGCACCACGAACTACCCCTGCAAGGTGTCCACAGGCAGACACCCTATCTCTATG
GTGGCCCTGTCTCCTCTGGGAGCCCTGGTTGCTTGTTACAAGGGCGTGTCCTGTAGCATCGG
CAGCAACAGAGTGGGCATCATCAAGCAGCTGAACAAGGGCTGCAGCTACATCACCAACCAGG
ACGCCGATACCGTGACCATCGACAACACCGTGTATCAGCTGAGCAAGGTGGAAGGCGAACAG
CACGTGATCAAGGGCAGACCTGTGTCCAGCAGCTTCGACCCCATCAAGTTCCCCGAGGATCA
GTTCAACGTGGCCCTGGACCAGGTGTTCGAGAACATCGAGAATAGCCAGGCTCTGGTGGACC
AGTCCAACAGAATCCTGTCTAGCGCCGAGAAGGGAAACACCGGCTTCATCATCGTGATCATC
CTGATTGCCGTGCTGGGCTCCAGCATGATCCTGGTGTCCATCTTTATCATC
SEQ ID NO:57 
ATGAGCTGGAAAGTCATGATCATCATCAGCCTGCTGATCACCCCTCAGCACGGCCTGAAAGA
GAGCTACCTGGAAGAGTCCTGCAGCACCATCACAGAGGGCTACCTGTCTGTGCTGAGAACCG
GCTGGTACACCAACGTGTTCACACTGGAATGCGGCGACGTGGAAAACCTGACCTGTACAGAT
GGCCCCAGCCTGATCAAGACCGAGCTGGATCTGACAAAGAGCGCCCTGAGAGAACTGAAAAC
CGTGTCTGCCGATCAGCTGGCCAGAGAGGAACAGATCGAGAACCCCAGACAGAGCAGATTCG
TGCTGGGAGCTATTGCCCTGGGAGTTGCTACAGCTGCTGCCGTGACAGCCGGAATCGCCATT
GCCAAGACCATCCGGCTGGAAAGCGAAGTGAATGCCATCAAGGGCGCACTGAAGCAGACCAA
CGAAGCCGTGTCTACCCTCGGCAACGGTGTTAGAGTGCTGGCCACAGCAGTGCGCGAGTGCA
AGAATTTGTGTCCAAGAACCTGACCAGCGCCATCAACCGGAACAAGTGCGACATTGCCGAC
CTGAAGATGGCCGTGTCCTTCAGCCAGTTCAACCGGCGGTTTCTGAACGTCGTGCGGCAGTT
TTCTGACAACGCCGGCATCACACCCGCCATTAGCCTGGACCTGATGACAGATGCTGAGCTGG
CTCGGGCCGTGTCTTACATGCCTACATCTGCCGGCCAGATCAAGCTGATGCTGGAAAACAGA
GCCATGGTCCGACGGAAAGGCTTCGGCATCCTGATCGGCGTGTACGGCAGCTCCGTGATCTA
TATGGTGCAGCTGCCTATCTTCGGCGTGATCGACACCCCTTGCTGGATCATTAAGGCCGCTC
CTAGCTGCAGCGAGAAGAACGGCAATTACGCCTGCCTGCTGAGAGAGGACCAAGGCTGGTAC
TGCAAGAATGCCGGCAGCACCGTGTACTACCCCAACGAGAAGGATTGCGAGACACGGGGCGA
TCACGTGTTCTGTGATACAGCCGCCGGAATCAATGTGGCCGAGCAGAGCAGAGAGTGCAACA
TCAACATCAGCACCACAAACTACCCCTGCAAGGTGTCCACAGGCAGACACCCTATCTCTATG
GTGGCCCTGTCTCCTCTGGGAGCCCTGGTTGCTTGTTACAAGGGCGTGTCCTGTAGCATCGG
CAGCAACTGGGTCGGAATCATCAAGCAGCTGCCCAAGGGCTGCTCCTACATCACCAATCAGG
ACGCCGACACCGTGACCATCGACAACACAGTGTATCAGCTGAGCAAGGTGGAAGGCGAGCAG
CACGTTATCAAGGGCAGACCTGTGTCCAGCAGCTTCGACCCCATCAAGTTCCCCGAGGATCA
GTTCAACGTGGCCCTGGATCAGGTGTTCGAGAGCATCGAGAATAGCCAGGCTCTGGTGGACC
AGTCCAACAAGATCCTGAATAGCGCCGAGAAGGGCAACACCGGCTTCATCATCGTGGTTATC
CTGGTGGCTGTGCTGGGCCTGACAATGATCAGCGTGTCCATCATCATTATC
SEQ ID NO:58 
ATGAGCTGGAAGGTGGTCATCATCTTCAGCCTGCTGATCACCCCTCAGCACGGCCTGAAAGA
GAGCTACCTGGAAGAGTCCTGCAGCACCATCACAGAGGGCTACCTGTCTGTGCTGAGAACCG
GCTGGTACACCAACGTGTTCACACTGGAAGTGGGCGACTGCGAAAACCTGACATGCTCTGAT
GGCCCCAGCCTGATCAAGACCGAGCTGGATCTGACAAAGAGCGCCCTGAGAGAACTGAAAAC
CGTGTCTGCCGATCAGCTGGCCAGAGAGGAACAGATCGAGAACCCCAGACAGAGCAGATTCG
TGCTGGGAGCTATTGCCCTGGGAGTTGCTACAGCTGCTGCTGTGACAGCTGGCGTGGCCATT
GCCAAGACCATCAGACTGGAAAGCGAAGTGACCGCCATCAAGAACGCCCTGAAAACCACCAA
CGAGGCCGTGTCTACACTCGGCAATGGCGTTAGAGTGCTGGCCACAGCAGTGCGCGAGCTGA
AGGACTTCTGCTCCAAGAACCTGACCAGAGCCATCAACAAGAACAAGTGCGACATCGACGAC
CTGAAGATGGCCGTGTCCTTTAGCCAGTTCAACCGGCGGTTTCTGAACGTCGTGCGGCAGTT
TTCTGACAACGCCGGCATCACACCAGCCATCAGCCTGGACCTGATGACAGATGCTGAGCTGG
CTAGAGCCGTGTCCAACATGCCTACATCTGCCGGCCAGATCAAGCTGATGCTGGAAAACAGA
GCCATGGTCCGACGGAAAGGCTTCGGCATCCTGATCGGCGTGTACGGCAGCAGCGTGATCTA
 
TATGGTGCAGCTGCCTATCTTCGGCGTGATCGACACCCCTTGCTGGATTGTGAAGGCCGCTC
CTAGCTGTAGCGGCAAGAAGGGCAATTACGCCTGCCTGCTGAGAGAGGACCAAGGCTGGTAT
TGTCAGAATGCCGGCAGCACCGTGTACTACCCCAACGAGAAGGATTGCGAGACACGGGGCGA
TCACGTGTTCTGTGATACAGCCGCCGGAATCAATGTGGCCGAGCAGAGCAAAGAGTGCAACA
TCAACATCAGCACCACGAACTACCCCTGCAAGGTGTCCACAGGCAGACACCCTATCTCTATG
GTGGCCCTGTCTCCTCTGGGAGCCCTGGTTGCTTGTTACAAGGGCGTGTCCTGTAGCATCGG
CAGCAACAGAGTGGGCATCATCAAGCAGCTGAACAAGGGCTGCAGCTACATCACCAACCAGG
ACGCCGATACCGTGACCATCGACAACACCGTGTATCAGCTGAGCAAGGTGGAAGGCGAACAG
CACGTGATCAAGGGCAGACCTGTGTCCAGCAGCTTCGACCCCATCAAGTTCCCCGAGGATCA
GTTCAACGTGGCCCTGGACCAGGTGTTCGAGAACATCGAGAATAGCCAGGCTCTGGTGGACC
AGTCCAACAGAATCCTGTCTAGCGCCGAGAAGGGAAACACCGGCTTCATCATCGTGATCATC
CTGATTGCCGTGCTGGGCTCCAGCATGATCCTGGTGTCCATCTTTATCATC
SEQ ID NO:59 
ATGAGCTGGAAAGTCATGATCATCATCAGCCTGCTGATCACCCCTCAGCACGGCCTGAAAGA
GAGCTACCTGGAAGAGTCCTGCAGCACCATCACAGAGGGCTACCTGTCTGTGCTGAGAACCG
GCTGGTACACCAACGTGTTCACACTGGAAGTGGGCGACTGCGAAAACCTGACCTGTACAGAT
GGCCCCAGCCTGATCAAGACCGAGCTGGATCTGACAAAGAGCGCCCTGAGAGAACTGAAAAC
CGTGTCTGCCGATCAGCTGGCCAGAGAGGAACAGATCGAGAACCCCAGACAGAGCAGATTCG
TGCTGGGAGCTATTGCCCTGGGAGTTGCTACAGCTGCTGCCGTGACAGCCGGAATCGCCATT
GCCAAGACCATCCGGCTGGAAAGCGAAGTGAATGCCATCAAGGGCGCACTGAAGCAGACCAA
CGAAGCCGTGTCTACCCTCGGCAACGGTGTTAGAGTGCTGGCCACAGCAGTGCGCGAGCTGA
AAGAATTTTGCTCCAAGAACCTGACCAGCGCCATCAACCGGAACAAGTGCGACATTGCCGAC
CTGAAGATGGCCGTGTCCTTCAGCCAGTTCAACCGGCGGTTTCTGAACGTCGTGCGGCAGTT
TTCTGACAACGCCGGCATCACACCCGCCATTAGCCTGGACCTGATGACAGATGCTGAGCTGG
CTCGGGCCGTGTCTTACATGCCTACATCTGCCGGCCAGATCAAGCTGATGCTGGAAAACAGA
GCCATGGTCCGACGGAAAGGCTTCGGCATCCTGATCGGCGTGTACGGCAGCTCCGTGATCTA
TATGGTGCAGCTGCCTATCTTCGGCGTGATCGACACCCCTTGCTGGATCATTAAGGCCGCTC
CTAGCTGCAGCGAGAAGAACGGCAATTACGCCTGCCTGCTGAGAGAGGACCAAGGCTGGTAC
TGCAAGAATGCCGGCAGCACCGTGTACTACCCCAACGAGAAGGATTGCGAGACACGGGGCGA
TCACGTGTTCTGTGATACAGCCGCCGGAATCAATGTGGCCGAGCAGAGCAGAGAGTGCAACA
TCAACATCAGCACCACAAACTACCCCTGCAAGGTGTCCACAGGCAGACACCCTATCTCTATG
GTGGCCCTGTCTCCTCTGGGAGCCCTGGTTGCTTGTTACAAGGGCGTGTCCTGTAGCATCGG
CAGCAACTGGGTCGGAATCATCAAGCAGCTGCCCAAGGGCTGCTCCTACATCACCAATCAGG
ACGCCGACACCGTGACCATCGACAACACAGTGTATCAGCTGAGCAAGGTGGAAGGCGAGCAG
CACGTTATCAAGGGCAGACCTGTGTCCAGCAGCTTCGACCCCATCAAGTTCCCCGAGGATCA
GTTCAACGTGGCCCTGGATCAGGTGTTCGAGAGCATCGAGAATAGCCAGGCTCTGGTGGACC
AGTCCAACAAGATCCTGAATAGCGCCGAGAAGGGCAACACCGGCTTCATCATCGTGGTTATC
CTGGTGGCTGTGCTGGGCCTGACAATGATCAGCGTGTCCATCATCATTATC
SEQ ID NO:60 
ATGAGCTGGAAAGTCATGATCATCATCAGCCTGCTGATCACCCCTCAGCACGGCCTGAAAGA
GAGCTACCTGGAAGAGTCCTGCAGCACCATCACAGAGGGCTACCTGTCTGTGCTGAGAACCG
GCTGGTACACCAACGTGTTCACACTGGAAGTGGGCGACGTGGAAAACTGCACCTGTACAGAT
GGCCCCAGCCTGATCAAGACCGAGCTGGATCTGACAAAGAGCGCCCTGAGAGAACTGAAAAC
CGTGTCTGCCGATCAGCTGGCCAGAGAGGAACAGATCGAGAACCCCAGACAGAGCAGATTCG
TGCTGGGAGCTATTGCCCTGGGAGTTGCTACAGCTGCTGCCGTGACAGCCGGAATCGCCATT
GCCAAGACCATCCGGCTGGAAAGCGAAGTGAATGCCATCAAGGGCGCACTGAAGCAGACCAA
CGAAGCCGTGTCTACCCTCGGCAACGGTGTTAGAGTGCTGGCCACAGCAGTGCGCGAGCTGA
AAGAATTTGTGTCCAAGAACCTGTGCAGCGCCATCAACCGGAACAAGTGCGACATTGCCGAC
CTGAAGATGGCCGTGTCCTTCAGCCAGTTCAACCGGCGGTTTCTGAACGTCGTGCGGCAGTT
TTCTGACAACGCCGGCATCACACCCGCCATTAGCCTGGACCTGATGACAGATGCTGAGCTGG
CTCGGGCCGTGTCTTACATGCCTACATCTGCCGGCCAGATCAAGCTGATGCTGGAAAACAGA
GCCATGGTCCGACGGAAAGGCTTCGGCATCCTGATCGGCGTGTACGGCAGCTCCGTGATCTA
TATGGTGCAGCTGCCTATCTTCGGCGTGATCGACACCCCTTGCTGGATCATTAAGGCCGCTC
CTAGCTGCAGCGAGAAGAACGGCAATTACGCCTGCCTGCTGAGAGAGGACCAAGGCTGGTAC
TGCAAGAATGCCGGCAGCACCGTGTACTACCCCAACGAGAAGGATTGCGAGACACGGGGCGA
TCACGTGTTCTGTGATACAGCCGCCGGAATCAATGTGGCCGAGCAGAGCAGAGAGTGCAACA
TCAACATCAGCACCACAAACTACCCCTGCAAGGTGTCCACAGGCAGACACCCTATCTCTATG
 
GTGGCCCTGTCTCCTCTGGGAGCCCTGGTTGCTTGTTACAAGGGCGTGTCCTGTAGCATCGG
CAGCAACTGGGTCGGAATCATCAAGCAGCTGCCCAAGGGCTGCTCCTACATCACCAATCAGG
ACGCCGACACCGTGACCATCGACAACACAGTGTATCAGCTGAGCAAGGTGGAAGGCGAGCAG
CACGTTATCAAGGGCAGACCTGTGTCCAGCAGCTTCGACCCCATCAAGTTCCCCGAGGATCA
GTTCAACGTGGCCCTGGATCAGGTGTTCGAGAGCATCGAGAATAGCCAGGCTCTGGTGGACC
AGTCCAACAAGATCCTGAATAGCGCCGAGAAGGGCAACACCGGCTTCATCATCGTGGTTATC
CTGGTGGCTGTGCTGGGCCTGACAATGATCAGCGTGTCCATCATCATTATC
SEQ ID NO:61 
ATGAGCTGGAAGGTGGTCATCATCTTCAGCCTGCTGATCACCCCTCAGCACGGCCTGAAAGA
GAGCTACCTGGAAGAGTCCTGCAGCACCATCACAGAGGGCTACCTGTCTGTGCTGAGAACCG
GCTGGTACACCAACGTGTTCACACTGGAAGTGGGCGACGTGGAAAACTGCACATGCTCTGAT
GGCCCCAGCCTGATCAAGACCGAGCTGGATCTGACAAAGAGCGCCCTGAGAGAACTGAAAAC
CGTGTCTGCCGATCAGCTGGCCAGAGAGGAACAGATCGAGAACCCCAGACAGAGCAGATTCG
TGCTGGGAGCTATTGCCCTGGGAGTTGCTACAGCTGCTGCTGTGACAGCTGGCGTGGCCATT
GCCAAGACCATCAGACTGGAAAGCGAAGTGACCGCCATCAAGAACGCCCTGAAAACCACCAA
CGAGGCCGTGTCTACACTCGGCAATGGCGTTAGAGTGCTGGCCACAGCAGTGCGCGAGCTGA
AGGACTTCGTGTCCAAGAACCTGTGCAGAGCCATCAACAAGAACAAGTGCGACATCGACGAC
CTGAAGATGGCCGTGTCCTTTAGCCAGTTCAACCGGCGGTTTCTGAACGTCGTGCGGCAGTT
TTCTGACAACGCCGGCATCACACCAGCCATCAGCCTGGACCTGATGACAGATGCTGAGCTGG
CTAGAGCCGTGTCCAACATGCCTACATCTGCCGGCCAGATCAAGCTGATGCTGGAAAACAGA
GCCATGGTCCGACGGAAAGGCTTCGGCATCCTGATCGGCGTGTACGGCAGCAGCGTGATCTA
TATGGTGCAGCTGCCTATCTTCGGCGTGATCGACACCCCTTGCTGGATTGTGAAGGCCGCTC
CTAGCTGTAGCGGCAAGAAGGGCAATTACGCCTGCCTGCTGAGAGAGGACCAAGGCTGGTAT
TGTCAGAATGCCGGCAGCACCGTGTACTACCCCAACGAGAAGGATTGCGAGACACGGGGCGA
TCACGTGTTCTGTGATACAGCCGCCGGAATCAATGTGGCCGAGCAGAGCAAAGAGTGCAACA
TCAACATCAGCACCACGAACTACCCCTGCAAGGTGTCCACAGGCAGACACCCTATCTCTATG
GTGGCCCTGTCTCCTCTGGGAGCCCTGGTTGCTTGTTACAAGGGCGTGTCCTGTAGCATCGG
CAGCAACAGAGTGGGCATCATCAAGCAGCTGAACAAGGGCTGCAGCTACATCACCAACCAGG
ACGCCGATACCGTGACCATCGACAACACCGTGTATCAGCTGAGCAAGGTGGAAGGCGAACAG
CACGTGATCAAGGGCAGACCTGTGTCCAGCAGCTTCGACCCCATCAAGTTCCCCGAGGATCA
GTTCAACGTGGCCCTGGACCAGGTGTTCGAGAACATCGAGAATAGCCAGGCTCTGGTGGACC
AGTCCAACAGAATCCTGTCTAGCGCCGAGAAGGGAAACACCGGCTTCATCATCGTGATCATC
CTGATTGCCGTGCTGGGCTCCAGCATGATCCTGGTGTCCATCTTTATCATC
SEQ ID NO:62 
ATGAGCTGGAAGGTGGTCATCATCTTCAGCCTGCTGATCACCCCTCAGCACGGCCTGAAAGA
GAGCTACCTGGAAGAGTCCTGCAGCACCATCACAGAGGGCTACCTGTCTGTGCTGAGAACCG
GCTGGTACACCAACGTGTTCACACTGGAAGTGGGCGACGTGGAAAACCTGACATGCTCTGAT
GGCCCCAGCCTGATCAAGACCGAGCTGGATCTGACAAAGAGCGCCCTGAGAGAACTGAAAAC
CGTGTCTGCCGATCAGCTGGCCAGAGAGGAACAGATCGAGAACCCCAGACAGAGCAGATTCG
TGCTGGGAGCTATTGCCCTGGGAGTTGCTACAGCTGCTGCTGTGACAGCTGGCGTGGCCATT
GCCAAGACCATCAGACTGGAAAGCGAAGTGACCGCCATCAAGAACGCCCTGAAAACCACCAA
CGAGGCCTGCTCTACACTCGGCAATGGCGTTAGAGTGTGCGCCACAGCAGTGCGCGAGCTGA
AGGACTTCGTGTCCAAGAACCTGACCAGAGCCATCAACAAGAACAAGTGCGACATCGACGAC
CTGAAGATGGCCGTGTCCTTTAGCCAGTTCAACCGGCGGTTTCTGAACGTCGTGCGGCAGTT
TTCTGACAACGCCGGCATCACACCAGCCATCAGCCTGGACCTGATGACAGATGCTGAGCTGG
CTAGAGCCGTGTCCAACATGCCTACATCTGCCGGCCAGATCAAGCTGATGCTGGAAAACAGA
GCCATGGTCCGACGGAAAGGCTTCGGCATCCTGATCGGCGTGTACGGCAGCAGCGTGATCTA
TATGGTGCAGCTGCCTATCTTCGGCGTGATCGACACCCCTTGCTGGATTGTGAAGGCCGCTC
CTAGCTGTAGCGGCAAGAAGGGCAATTACGCCTGCCTGCTGAGAGAGGACCAAGGCTGGTAT
TGTCAGAATGCCGGCAGCACCGTGTACTACCCCAACGAGAAGGATTGCGAGACACGGGGCGA
TCACGTGTTCTGTGATACAGCCGCCGGAATCAATGTGGCCGAGCAGAGCAAAGAGTGCAACA
TCAACATCAGCACCACGAACTACCCCTGCAAGGTGTCCACAGGCAGACACCCTATCTCTATG
GTGGCCCTGTCTCCTCTGGGAGCCCTGGTTGCTTGTTACAAGGGCGTGTCCTGTAGCATCGG
CAGCAACAGAGTGGGCATCATCAAGCAGCTGAACAAGGGCTGCAGCTACATCACCAACCAGG
ACGCCGATACCGTGACCATCGACAACACCGTGTATCAGCTGAGCAAGGTGGAAGGCGAACAG
CACGTGATCAAGGGCAGACCTGTGTCCAGCAGCTTCGACCCCATCAAGTTCCCCGAGGATCA
GTTCAACGTGGCCCTGGACCAGGTGTTCGAGAACATCGAGAATAGCCAGGCTCTGGTGGACC
 
AGTCCAACAGAATCCTGTCTAGCGCCGAGAAGGAAACACCGGCTTCATCATCGTGATCATC
CTGATTGCCGTGCTGGGCTCCAGCATGATCCTGGTGTCCATCTTTATCATC
SEQ ID NO:63 
ATGAGCTGGAAAGTCATGATCATCATCAGCCTGCTGATCACCCCTCAGCACGGCCTGAAAGA
GAGCTACCTGGAAGAGTCCTGCAGCACCATCACAGAGGGCTACCTGTCTGTGCTGAGAACCG
GCTGGTACACCAACGTGTTCACACTGGAAGTGGGCGACGTGGAAAACCTGACCTGTACAGAT
GGCCCCAGCCTGATCAAGACCGAGCTGGATCTGACAAAGAGCGCCCTGAGAGAACTGAAAAC
CGTGTCTGCCGATCAGCTGGCCAGAGAGGAACAGATCGAGAACCCCAGACAGAGCAGATTCG
TGCTGGGAGCTATTGCCCTGGGAGTTGCTACAGCTGCTGCCGTGACAGCCGGAATCGCCATT
GCCAAGACCATCCGGCTGGAAAGCGAAGTGAATGCCATCAAGGGCGCACTGAAGCAGACCAA
CGAAGCCTGCTCTACCCTCGGCAACGGTGTTAGAGTGTGCGCCACAGCAGTGCGCGAGCTGA
AAGAATTTGTGTCCAAGAACCTGACCAGCGCCATCAACCGGAACAAGTGCGACATTGCCGAC
CTGAAGATGGCCGTGTCCTTCAGCCAGTTCAACCGGCGGTTTCTGAACGTCGTGCGGCAGTT
TTCTGACAACGCCGGCATCACACCCGCCATTAGCCTGGACCTGATGACAGATGCTGAGCTGG
CTCGGGCCGTGTCTTACATGCCTACATCTGCCGGCCAGATCAAGCTGATGCTGGAAAACAGA
GCCATGGTCCGACGGAAAGGCTTCGGCATCCTGATCGGCGTGTACGGCAGCTCCGTGATCTA
TATGGTGCAGCTGCCTATCTTCGGCGTGATCGACACCCCTTGCTGGATCATTAAGGCCGCTC
CTAGCTGCAGCGAGAAGAACGGCAATTACGCCTGCCTGCTGAGAGAGGACCAAGGCTGGTAC
TGCAAGAATGCCGGCAGCACCGTGTACTACCCCAACGAGAAGGATTGCGAGACACGGGGCGA
TCACGTGTTCTGTGATACAGCCGCCGGAATCAATGTGGCCGAGCAGAGCAGAGAGTGCAACA
TCAACATCAGCACCACAAACTACCCCTGCAAGGTGTCCACAGGCAGACACCCTATCTCTATG
GTGGCCCTGTCTCCTCTGGGAGCCCTGGTTGCTTGTTACAAGGGCGTGTCCTGTAGCATCGG
CAGCAACTGGGTCGGAATCATCAAGCAGCTGCCCAAGGGCTGCTCCTACATCACCAATCAGG
ACGCCGACACCGTGACCATCGACAACACAGTGTATCAGCTGAGCAAGGTGGAAGGCGAGCAG
CACGTTATCAAGGGCAGACCTGTGTCCAGCAGCTTCGACCCCATCAAGTTCCCCGAGGATCA
GTTCAACGTGGCCCTGGATCAGGTGTTCGAGAGCATCGAGAATAGCCAGGCTCTGGTGGACC
AGTCCAACAAGATCCTGAATAGCGCCGAGAAGGGCAACACCGGCTTCATCATCGTGGTTATC
CTGGTGGCTGTGCTGGGCCTGACAATGATCAGCGTGTCCATCATCATTATC
SEQ ID NO:64 
MSWKVVIIFSLLITPQHGLKESYLEESCSTITEGYLSVLRTGWYTNVFTLEVGDVENLTCSD
GPSLIKTELDLTKSALRELKTVSADQLAREEQIENPRQSRFVLGAIALGVATAAAVTAGVAI
AKTIRLESEVTAIKNALKTTNEAVSTLGNGVRVLATAVRELKDFVSKNLTRAINKNKCDIDD
LKMAVSFSQFNRRFLNVVRQFSDNAGITPAISLDLMTDAELARAVSNMPTSAGQIKLMLENR
AMVRRKGFGILIGVYGSSVIYMVQLPIFGVIDTPCWIVKAAPSCSGKKGNYACLLREDQGWY
CQNAGSTVYYPNEKDCETRGDHVFCDTAAGINVAEQSKECNINISTTNYPCKVSTGRHPISM
VALSPLGALVACYKGVSCSIGSNRVGIIKQLNKGCSYITNQDADTVTIDNTVYQLSKVEGEQ
HVIKGRPVSSSFDPIKFPEDQFNVALDQVFENIENSQALVDQSNRILSSAEKGNTGFIIVII
LIAVLGSSMILVSIFIIIKKTKKPTGAPPELSGVTNNGFIPHS
SEQ ID NO:65 
MSWKVMIIISLLITPQHGLKESYLEESCSTITEGYLSVLRTGWYTNVFTLEVGDVENLTCTD
GPSLIKTELDLTKSALRELKTVSADQLAREEQIENPRQSRFVLGAIALGVATAAAVTAGIAI
AKTIRLESEVNAIKGALKQTNEAVSTLGNGVRVLATAVRELKEFVSKNLTSAINRNKCDIAD
LKMAVSFSQFNRRFLNVVRQFSDNAGITPAISLDLMTDAELARAVSYMPTSAGQIKLMLENR
AMVRRKGFGILIGVYGSSVIYMVQLPIFGVIDTPCWIIKAAPSCSEKNGNYACLLREDQGWY
CKNAGSTVYYPNEKDCETRGDHVFCDTAAGINVAEQSRECNINISTTNYPCKVSTGRHPISM
VALSPLGALVACYKGVSCSIGSNWVGIIKQLPKGCSYITNQDADTVTIDNTVYQLSKVEGEQ
HVIKGRPVSSSFDPIKFPEDQFNVALDQVFESIENSQALVDQSNKILNSAEKGNTGFIIVVI
LVAVLGLTMISVSIIIIIKKTRKPTGAPPELNGVTNGGFIPHS
SEQ ID NO:66
   GYIPEAPRDGQAYVRKDGEWVLLSTFL
SEQ ID NO:67 
MESQVRQQFSKDIEKLLNEQVNKEMNSSNLYMSMSSWCYTHSLDGAGLFLFDHAAEEYEHAK
KLIIFLNENNVPVQLTSISAPEHKFEGLTQIFQKAYEHEQHISESINNIVDHAIKSKDHATF
NFLQWYVAEQHEEEVLFKDILDKIELIGNENHGLYLADQYVKGIAKSRKS
SEQ ID NO:68
   LVPRGS
SEQ ID NO:69
   HHHHHH
SEQ ID NO:70
   WSHPGFEK
SEQ ID NO:71 
MSWKVVIIFSLLITPQHGLKESYLEESCSTITEGYLSVLRTGWYTNVFTLEVGDVENLTCSD
GPSLIKTELDLTKSALRELKTVSADQLAREEQIENPRQSKKRKRRKTIRLESEVTAIKNALK
TTNEAVSTLGNGVRVLATAVRELKDFVSKNLTRAINKNKCDIDDLKMAVSFSQFNRRFLNVV
RQFSDNAGITPAISLDLMTDAELARAVSNMPTSAGQIKLMLENRAMVRRKGFGILIGVYGSS
VIYMVQLPIFGVIDTPCWIVKAAPSCSEKKGNYACLLREDQGWYCQNAGSTVYYPNEKDCET
RGDHVFCDTAAGINVAEQSKECNINISTTNYPCKVSTGRHPISMVALSPLGALVACYKGVSC
SIGSNRVGIIKQLNKGCSYITNQDADTVTIDNTVYQLSKVEGEQHVIKGRPVSSSFDPIKFP
EDQFNVALDQVFENIENSQALVDQSNRILSSAEKGNTSAIGGYIPEAPRDGQAYVRKDGEWV
LLSTFL
SEQ ID NO:72 
MSWKVMIIISLLITPQHGLKESYLEESCSTITEGYLSVLRTGWYTNVFTLEVGDVENLTCTD
GPSLIKTELDLTKSALRELKTVSADQLAREEQIENPRQSKKRKRRKTIRLESEVNAIKGALK
QTNEAVSTLGNGVRVLATAVRELKEFVSKNLTSAINRNKCDIADLKMAVSFSQFNRRFLNVV
RQFSDNAGITPAISLDLMTDAELARAVSYMPTSAGQIKLMLENRAMVRRKGFGILIGVYGSS
VIYMVQLPIFGVIDTPCWIIKAAPSCSEKNGNYACLLREDQGWYCKNAGSTVYYPNEKDCET
RGDHVFCDTAAGINVAEQSRECNINISTTNYPCKVSTGRHPISMVALSPLGALVACYKGVSC
SIGSNWVGIIKQLPKGCSYITNQDADTVTIDNTVYQLSKVEGEQHVIKGRPVSSSFDPIKFP
EDQFNVALDQVFESIENSQALVDQSNKILNSAEKGNTSAIGGYIPEAPRDGQAYVRKDGEWV
LLSTFL
SEQ ID NO:73 
MSWKVVIIFSLLITPQHGLKESYLEESCSTITEGYLSVLRTGWYTNVFTLEVGDVENLTCSD
GPSLIKTELDLTKSALRELKTVSADQLAREEQIENPRRRRFVLGAIACGVATAAAVTAGVAI
AKCIRLESEVTAIKNCLKTTNECVSTLGCGVRVLATAVRELKDFVSKNLTRAINKNKCDIPD
LKMAVSFSQFNRRFLNVVRQFSDNAGITPAISKDLMTDAELARAISNMPTSAGQIKLMLENR
AMVRRKGFGILIGVYGSSVIYMVQLPIFGVIDTPCWIVKAAPSCSEKKGNYACLLREDQGWY
CQNAGSTVYYPCEKDCETRGDHVFCDTAAGINVAEQSKECNINISTTNYPCKVSCGRHPISM
VALSPLGALVACYKGVSCSIGSNRVGIIKQLNKGCSYITNQDADTVTIDNTVYQLSKVEGEQ
HVIKGRPVSSSFDPIKFPQDQFNVALDQCFENIENSQALVDQSNRILSSAEKGNTSAIGGYI
PEAPRDGQAYVRKDGEWVLLSTFL
SEQ ID NO:74 
MSWKVMIIISLLITPQHGLKESYLEESCSTITEGYLSVLRTGWYTNVFTLEVGDVENLTCTD
GPSLIKTELDLTKSALRELKTVSADQLAREEQIENPRRRRFVLGAIACGVATAAAVTAGIAI
AKCIRLESEVNAIKGCLKQTNECVSTLGCGVRVLATAVRELKEFVSKNLTSAINRNKCDIPD
LKMAVSFSQFNRRFLNVVRQFSDNAGITPAISKDLMTDAELARAISYMPTSAGQIKLMLENR
AMVRRKGFGILIGVYGSSVIYMVQLPIFGVIDTPCWIIKAAPSCSEKNGNYACLLREDQGWY
CKNAGSTVYYPCEKDCETRGDHVFCDTAAGINVAEQSRECNINISTTNYPCKVSCGRHPISM
VALSPLGALVACYKGVSCSIGSNWVGIIKQLPKGCSYITNQDADTVTIDNTVYQLSKVEGEQ
HVIKGRPVSSSFDPIKFPQDQFNVALDQCFESIENSQALVDQSNKILNSAEKGNTSAIGGYI
PEAPRDGQAYVRKDGEWVLLSTFL
SEQ ID NO:75 
ATGAGCTGGAAGGTGGTCATCATCTTCAGCCTGCTGATCACCCCTCAGCACGGCCTGAAAGA
GAGCTACCTGGAAGAGTCCTGCAGCACCATCACAGAGGGCTACCTGTCTGTGCTGAGAACCG
GCTGGTACACCAACGTGTTCACACTGGAAGTGGGCGACGTGGAAAACCTGACATGCTCTGAT
GGCCCCAGCCTGATCAAGACCGAGCTGGATCTGACAAAGAGCGCCCTGAGAGAACTGAAAAC
 
CGTGTCTGCCGATCAGCTGGCCAGAGAGGAACAGATCGAGAACCCAGACAGAGCAAGAAGC
GGAAGCGGAGAAAGACCATCAGACTGGAAAGCGAAGTGACCGCCATCAAGAACGCCCTGAAA
ACCACCAACGAGGCCGTGTCTACACTCGGCAATGGCGTTAGAGTGCTGGCCACAGCAGTGCG
CGAGCTGAAGGACTTCGTGTCCAAGAACCTGACCAGAGCCATCAACAAGAACAAGTGCGACA
TCGACGACCTGAAGATGGCCGTGTCCTTTAGCCAGTTCAACCGGCGGTTTCTGAACGTCGTG
CGGCAGTTTTCTGACAACGCCGGCATCACACCAGCCATCAGCCTGGACCTGATGACAGATGC
TGAGCTGGCTAGAGCCGTGTCCAACATGCCTACATCTGCCGGCCAGATCAAGCTGATGCTGG
AAAACAGAGCCATGGTCCGACGGAAAGGCTTCGGCATCCTGATCGGCGTGTACGGCAGCAGC
GTGATCTATATGGTGCAGCTGCCTATCTTCGGCGTGATCGACACCCCTTGCTGGATTGTGAA
GGCCGCTCCTAGCTGTAGCGAGAAGAAGGGCAATTACGCCTGCCTGCTGAGAGAGGACCAAG
GCTGGTATTGTCAGAATGCCGGCAGCACCGTGTACTACCCCAACGAGAAGGATTGCGAGACA
CGGGGCGATCACGTGTTCTGTGATACAGCCGCCGGAATCAATGTGGCCGAGCAGAGCAAAGA
GTGCAACATCAACATCAGCACCACGAACTACCCCTGCAAGGTGTCCACAGGCAGACACCCTA
TCTCTATGGTGGCCCTGTCTCCTCTGGGAGCCCTGGTTGCTTGTTACAAGGGCGTGTCCTGT
AGCATCGGCAGCAACAGAGTGGGCATCATCAAGCAGCTGAACAAGGGCTGCAGCTACATCAC
CAACCAGGACGCCGATACCGTGACCATCGACAACACCGTGTATCAGCTGAGCAAGGTGGAAG
GCGAACAGCACGTGATCAAGGGCAGACCTGTGTCCAGCAGCTTCGACCCCATCAAGTTCCCC
GAGGATCAGTTCAACGTGGCCCTGGACCAGGTGTTCGAGAACATCGAGAATAGCCAGGCTCT
GGTGGACCAGTCCAACAGAATCCTGTCTAGCGCCGAGAAGGGAAACACCAGCGCCATCGGCG
GCTATATCCCTGAAGCTCCTAGAGATGGACAGGCCTATGTGCGGAAGGATGGCGAATGGGTG
CTGCTGTCTACATTCCTC
SEQ ID NO:76 
ATGAGCTGGAAAGTCATGATCATCATCAGCCTGCTGATCACCCCTCAGCACGGCCTGAAAGA
GAGCTACCTGGAAGAGTCCTGCAGCACCATCACAGAGGGCTACCTGTCTGTGCTGAGAACCG
GCTGGTACACCAACGTGTTCACACTGGAAGTGGGCGACGTGGAAAACCTGACCTGTACAGAT
GGCCCCAGCCTGATCAAGACCGAGCTGGATCTGACAAAGAGCGCCCTGAGAGAACTGAAAAC
CGTGTCTGCCGATCAGCTGGCCAGAGAGGAACAGATCGAGAACCCCAGACAGAGCAAGAAGC
GGAAGCGGAGAAAGACCATCCGGCTGGAAAGCGAAGTGAATGCCATCAAGGGCGCACTGAAG
CAGACCAACGAAGCCGTGTCTACCCTCGGCAACGGTGTTAGAGTGCTGGCCACAGCAGTGCG
CGAGCTGAAAGAATTTGTGTCCAAGAACCTGACCAGCGCCATCAACCGGAACAAGTGCGACA
TTGCCGACCTGAAGATGGCCGTGTCCTTCAGCCAGTTCAACCGGCGGTTTCTGAACGTCGTG
CGGCAGTTTTCTGACAACGCCGGCATCACACCCGCCATTAGCCTGGACCTGATGACAGATGC
TGAGCTGGCTCGGGCCGTGTCTTACATGCCTACATCTGCCGGCCAGATCAAGCTGATGCTGG
AAAACAGAGCCATGGTCCGACGGAAAGGCTTCGGCATCCTGATCGGCGTGTACGGCAGCTCC
GTGATCTATATGGTGCAGCTGCCTATCTTCGGCGTGATCGACACCCCTTGCTGGATCATTAA
GGCCGCTCCTAGCTGCAGCGAGAAGAACGGCAATTACGCCTGCCTGCTGAGAGAGGACCAAG
GCTGGTACTGCAAGAATGCCGGCAGCACCGTGTACTACCCCAACGAGAAGGATTGCGAGACA
CGGGGCGATCACGTGTTCTGTGATACAGCCGCCGGAATCAATGTGGCCGAGCAGAGCAGAGA
GTGCAACATCAACATCAGCACCACAAACTACCCCTGCAAGGTGTCCACAGGCAGACACCCTA
TCTCTATGGTGGCCCTGTCTCCTCTGGGAGCCCTGGTTGCTTGTTACAAGGGCGTGTCCTGT
AGCATCGGCAGCAACTGGGTCGGAATCATCAAGCAGCTGCCCAAGGGCTGCTCCTACATCAC
CAATCAGGACGCCGACACCGTGACCATCGACAACACAGTGTATCAGCTGAGCAAGGTGGAAG
GCGAGCAGCACGTTATCAAGGGCAGACCTGTGTCCAGCAGCTTCGACCCCATCAAGTTCCCC
GAGGATCAGTTCAACGTGGCCCTGGATCAGGTGTTCGAGAGCATCGAGAATAGCCAGGCTCT
GGTGGACCAGTCCAACAAGATCCTGAATAGCGCCGAGAAGGGCAACACCAGCGCCATCGGCG
GCTATATCCCTGAAGCTCCTAGAGATGGACAGGCCTATGTGCGGAAGGATGGCGAATGGGTG
CTGCTGTCTACATTCCTC
SEQ ID NO:77 
ATGAGCTGGAAGGTGGTCATCATCTTCAGCCTGCTGATCACCCCTCAGCACGGCCTGAAAGA
GAGCTACCTGGAAGAGTCCTGCAGCACCATCACCGAGGGCTACCTGAGCGTGCTGAGAACCG
GCTGGTACACCAACGTGTTCACCCTGGAAGTGGGCGACGTGGAAAACCTGACATGTAGCGAC
GGCCCCAGCCTGATCAAGACCGAGCTGGATCTGACAAAGAGCGCCCTGCGCGAGCTGAAAAC
CGTGTCTGCAGATCAGCTGGCCAGAGAGGAACAGATCGAGAACCCCAGACGGCGGAGATTTG
TGCTGGGAGCCATTGCTTGCGGAGTGGCTACAGCTGCTGCTGTGACAGCTGGCGTGGCCATT
GCCAAGTGCATCCGGCTGGAAAGCGAAGTGACCGCCATCAAGAACTGCCTGAAAACCACCAA
CGAGTGCGTGTCCACACTCGGCTGTGGCGTTAGAGTGCTGGCCACCGCTGTCAGAGAACTGA
AGGACTTCGTGTCCAAGAACCTGACCAGAGCCATCAACAAGAACAAGTGCGACATCCCCGAC
 
CTGAAGATGGCCGTGTCCTTCAGCCAGTTCAACCGGCGGTTCCTGAATGTCGTGCGGCAGTT
CAGCGATAACGCCGGCATCACACCAGCCATCAGCAAGGACCTGATGACCGATGCCGAACTGG
CCAGGGCCATCTCCAACATGCCTACATCTGCCGGCCAGATCAAGCTGATGCTGGAAAACAGA
GCCATGGTCCGACGGAAAGGCTTCGGCATCCTGATCGGCGTGTACGGCAGCAGCGTGATCTA
CATGGTGCAGCTGCCTATCTTCGGCGTGATCGACACCCCTTGCTGGATCGTGAAAGCCGCTC
CTAGCTGCAGCGAGAAGAAGGGCAATTACGCCTGCCTGCTGAGAGAGGACCAAGGCTGGTAC
TGTCAGAATGCCGGCAGCACCGTGTACTACCCCTGCGAGAAGGACTGCGAGACAAGAGGCGA
TCACGTGTTCTGCGATACCGCCGCTGGAATCAATGTGGCCGAGCAGAGCAAAGAGTGCAACA
TCAACATCAGCACCACCAACTATCCCTGCAAGGTGTCCTGCGGCAGACACCCCATTAGCATG
GTGGCTCTGTCTCCACTGGGAGCCCTGGTGGCTTGTTATAAGGGCGTGTCCTGTAGCATCGG
CAGCAACAGAGTGGGCATCATCAAGCAGCTGAACAAGGGCTGCTCCTACATCACCAACCAGG
ACGCCGATACCGTGACCATCGACAATACCGTGTATCAGCTGAGCAAGGTGGAAGGCGAACAG
CACGTGATCAAGGGCAGACCTGTGTCCAGCAGCTTCGACCCCATCAAGTTCCCTCAGGACCA
GTTTAACGTGGCCCTGGATCAGTGCTTCGAGAACATCGAGAACTCTCAGGCTCTGGTGGACC
AGTCCAACAGAATCCTGAGCAGCGCCGAGAAGGAAACACCAGCGCCATCGGCGGCTATATC
CCTGAAGCTCCTAGAGATGGACAGGCCTATGTGCGGAAGGATGGCGAATGGGTGCTGCTGTC
TACATTCCTC
SEQ ID NO:78 
ATGAGCTGGAAAGTCATGATCATCATCAGCCTGCTGATCACCCCTCAGCACGGCCTGAAAGA
GAGCTACCTGGAAGAGTCCTGCAGCACCATCACCGAGGGCTACCTGAGCGTGCTGAGAACCG
GCTGGTACACCAACGTGTTCACCCTGGAAGTGGGCGACGTGGAAAACCTGACCTGCACAGAT
GGCCCCAGCCTGATCAAGACCGAGCTGGATCTGACAAAGAGCGCCCTGCGCGAGCTGAAAAC
CGTGTCTGCAGATCAGCTGGCCAGAGAGGAACAGATCGAGAACCCCAGACGGCGGAGATTTG
TGCTGGGAGCCATTGCTTGCGGAGTGGCTACAGCTGCTGCTGTGACAGCCGGAATCGCCATT
GCCAAGTGCATCCGGCTGGAAAGCGAAGTGAACGCCATCAAGGGCTGCCTGAAGCAGACCAA
CGAGTGCGTGTCCACACTCGGCTGTGGCGTTAGAGTGCTGGCCACCGCTGTCCGGGAACTGA
AAGAATTCGTGTCCAAGAACCTGACCAGCGCCATCAACCGGAACAAGTGCGACATCCCCGAC
CTGAAGATGGCCGTGTCCTTCAGCCAGTTCAACCGGCGGTTCCTGAATGTCGTGCGGCAGTT
CTCTGACAACGCCGGCATCACACCAGCCATCAGCAAGGACCTGATGACCGATGCCGAACTGG
CCAGGGCCATCAGCTACATGCCTACATCTGCCGGCCAGATCAAGCTGATGCTGGAAAACAGA
GCCATGGTCCGACGGAAAGGCTTCGGCATCCTGATCGGCGTGTACGGCAGCAGCGTGATCTA
CATGGTGCAGCTGCCTATCTTCGGCGTGATCGACACCCCTTGCTGGATCATCAAGGCCGCTC
CTAGCTGCAGCGAGAAGAACGGCAATTACGCCTGCCTGCTGAGAGAGGACCAAGGCTGGTAC
TGCAAGAATGCCGGCAGCACCGTGTACTACCCCTGCGAGAAGGACTGCGAGACAAGAGGCGA
TCACGTGTTCTGCGATACCGCCGCTGGAATCAATGTGGCCGAGCAGAGCAGAGAGTGCAACA
TCAACATCAGCACCACCAACTATCCCTGCAAGGTGTCCTGCGGCAGACACCCCATTAGCATG
GTGGCTCTGTCTCCACTGGGAGCCCTGGTGGCTTGTTATAAGGGCGTGTCCTGTAGCATCGG
CAGCAACTGGGTCGGAATCATCAAGCAGCTGCCCAAGGGCTGTTCCTACATCACCAACCAGG
ACGCCGACACCGTGACCATCGACAATACCGTGTATCAGCTGAGCAAGGTGGAAGGCGAACAG
CACGTGATCAAGGGCAGACCTGTGTCCAGCAGCTTCGACCCCATCAAGTTCCCTCAGGACCA
GTTTAACGTGGCCCTGGATCAGTGCTTCGAGAGCATCGAGAACTCTCAGGCTCTGGTGGACC
AGTCCAACAAGATCCTGAACTCCGCCGAGAAGGGCAACACCAGCGCCATCGGCGGCTATATC
CCTGAAGCTCCTAGAGATGGACAGGCCTATGTGCGGAAGGATGGCGAATGGGTGCTGCTGTC
TACATTCCTC
SEQ ID NO:79 
MSWKVVIIFSLLITPQHGLKESYLEESCSTITEGYLSVLRTGWYTNVFTLEVGDVENLTCSD
GPSLIKTELDLTKSALRELKTVSADQLAREEQIENPRRRRFVLGAIALGVCTAAAVTAGVAI
AKTIRLESEVTAIKNALKTTNEAVSTLGNGVRVLATAVRELKDFVSKNLTRAINKNKCDIPD
LKMAVSFSQFNRRFLNVVRQFSDNAGITPAISLDLMTDAELARAVSNMPTSAGQIKLMLENR
AMVRRKGFGILIGVYGSSVIYMVQLPIFGVIDTPCWIVKAAPSCSEKKGNYACLLREDQGWY
CQNAGSTVYYPNEKDCETRGDHVFCDTACGINVAEQSKECNINISTTNYPCKVSTGRNPISM
VALSPLGALVACYKGVSCSIGSNRVGIIKQLNKGCSYITNQDADTVTIDNTVYQLSKVEGEQ
HVIKGRPVSSSFDPIKFPEDQFNVALDQVFENIENSQALVDQSNRILSSAEKGNTSAIGGYI
PEAPRDGQAYVRKDGEWVLLSTFL
SEQ ID NO:80 MSWKVMIIISLLITPQHGLKESYLEESCSTITEGYLSVLRTGWYTNVFTLEVGDVENLTCTD 
GPSLIKTELDLTKSALRELKTVSADQLAREEQIENPRRRRFVLGAIALGVCTAAAVTAGIAI
AKTIRLESEVNAIKGALKQTNEAVSTLGNGVRVLATAVRELKEFVSKNLTSAINRNKCDIPD
LKMAVSFSQFNRRFLNVVRQFSDNAGITPAISLDLMTDAELARAVSYMPTSAGQIKLMLENR
AMVRRKGFGILIGVYGSSVIYMVQLPIFGVIDTPCWIIKAAPSCSEKNGNYACLLREDQGWY
CKNAGSTVYYPNEKDCETRGDHVFCDTACGINVAEQSRECNINISTTNYPCKVSTGRNPISM
VALSPLGALVACYKGVSCSIGSNWVGIIKQLPKGCSYITNQDADTVTIDNTVYQLSKVEGEQ
HVIKGRPVSSSFDPIKFPEDQFNVALDQVFESIENSQALVDQSNKILNSAEKGNTSAIGGYI
PEAPRDGQAYVRKDGEWVLLSTFL
SEQ ID NO:81 
MSWKVVIIFSLLITPQHGLKESYLEESCSTITEGYLSVLRTGWYTNVFTLEVGDVENLTCSD
GPSLIKTELDLTKSALRELKTVSADQLAREEQIENPRQSRFVLGAIALGVATAAAVTAGVAI
AKTIRLESEVTAIKNALKTTNECVSTLGNGVRVLCTAVRELKDFVSKNLTRAINKNKCDIDD
LKMAVSFSQFNRRFLNVVRQFSDNAGITPAISLDLMTDAELARAVSNMPTSAGQIKLMLENR
AMVRRKGFGILIGVYGSSVIYMVQLPIFGVIDTPCWIVKAAPSCSGKKGNYACLLREDQGWY
CQNAGSTVYYPNEKDCETRGDHVFCDTAAGINVAEQSKECNINISTTNYPCKVSTGRHPISM
VALSPLGALVACYKGVSCSIGSNRVGIIKQLNKGCSYITNQDADTVTIDNTVYQLSKVEGEQ
HVIKGRPVSSSFDPIKFPEDQFNVALDQVFENIENSQALVDQSNRILSSAEKGNTGFIIVII
LIAVLGSSMILVSIFIIIKKTKKPTGAPPELSGVTNNGFIPHS
SEQ ID NO:82 
MSWKVMIIISLLITPQHGLKESYLEESCSTITEGYLSVLRTGWYTNVFTLEVGDVENLTCTD
GPSLIKTELDLTKSALRELKTVSADQLAREEQIENPRQSRFVLGAIALGVATAAAVTAGIAI
AKTIRLESEVNAIKGALKQTNECVSTLGNGVRVLCTAVRELKEFVSKNLTSAINRNKCDIAD
LKMAVSFSQFNRRFLNVVRQFSDNAGITPAISLDLMTDAELARAVSYMPTSAGQIKLMLENR
AMVRRKGFGILIGVYGSSVIYMVQLPIFGVIDTPCWIIKAAPSCSEKNGNYACLLREDQGWY
CKNAGSTVYYPNEKDCETRGDHVFCDTAAGINVAEQSRECNINISTTNYPCKVSTGRHPISM
VALSPLGALVACYKGVSCSIGSNWVGIIKQLPKGCSYITNQDADTVTIDNTVYQLSKVEGEQ
HVIKGRPVSSSFDPIKFPEDQFNVALDQVFESIENSQALVDQSNKILNSAEKGNTGFIIVVI
LVAVLGLTMISVSIIIIIKKTRKPTGAPPELNGVTNGGFIPHS
SEQ ID NO:83 
MSWKVVIIFSLLITPQHGLKESYLEESCSTITEGYLSVLRTGWYTNVFTLEVGDVENLTCSD
GPSLIKTELDLTKSALRELKTVSADQLAREEQIENPRQSRFVLGAIALGVAAAAAVTAGVAI
AKTIRLESEVTAINNALKKTNECVSTLGNGVRVLCTAVRELKDFVSKNLTRAINKNKCDIDD
LKMAVSFSQFNRRFLNVVRQFSDNAGITPAISLDLMTDAELARAVPNMPTSAGQIKLMLENR
AMVRRKGFGILIGVYGSSVIYMVQLPIFGVIDTPCWIVKAAPSCSEKKGNYACLLREDQGWY
CQNAGSTVYYPNEKDCETRGDHVFCDTAAGINVAEQSKECNINISTTNYPCKVSTGRHPISM
VALSPLGALVACYKGVSCSIGSNRVGIIKQLNKGCSYITNQDADTVTIDNTVYQLSKVEGEQ
HVIKGRPVSSSFDPIKFPEDQFNVALDQVFENIENSQALVDQSNRILSSAEKGNTGFIIVII
LIAVLGSSMILVSIFIIIKKTKKPTGAPPELSGVTNNGFIPHN
SEQ ID NO:84 
MSWKVVIIFSLLITPQHGLKESYLEESCSTITEGYLSVLRTGWYTNVFTLEVGDVENLTCSD
GPSLIKTELDLTKSALRELKTVSADQLAREEQIENPRQSRFVLGAIALGVAAAAAVTAGVAI
AKTIRLESEVTAINNALKKTNEAVSTLGNGVRVLATAVRECKDFVSKNLTRAINKNKCDIDD
LKMAVSFSQCNRRFLNVVRQFSDNAGITPAISLDLMTDAELARAVPNMPTSAGQIKLMLENR
AMVRRKGFGILIGVYGSSVIYMVQLPIFGVIDTPCWIVKAAPSCSEKKGNYACLLREDQGWY
CQNAGSTVYYPNEKDCETRGDHVFCDTAAGINVAEQSKECNINISTTNYPCKVSTGRHPISM
VALSPLGALVACYKGVSCSIGSNRVGIIKQLNKGCSYITNQDADTVTIDNTVYQLSKVEGEQ
HVIKGRPVSSSFDPIKFPEDQFNVALDQVFENIENSQALVDQSNRILSSAEKGNTGFIIVII
LIAVLGSSMILVSIFIIIKKTKKPTGAPPELSGVTNNGFIPHN
SEQ ID NO:85 
MSWKVVIIFSLLITPQHGLKESYLEESCSTITEGYLSVLRTGWYTNVFTLEVGDVENLTCSD
GPSLIKTELDLTKSALRELKTVSADQLAREEQIENPRQSRFVLGAIALGVAAAAAVTAGVAI
AKTIRLESEVTAINNALKKTCEAVSTLGNGVRVLATCVRELKDFVSKNLTRAINKNKCDIDD
LKMAVSFSQFNRRFLNVVRQFSDNAGITPAISLDLMTDAELARAVPNMPTSAGQIKLMLENR
AMVRRKGFGILIGVYGSSVIYMVQLPIFGVIDTPCWIVKAAPSCSEKKGNYACLLREDQGWY
 
CQNAGSTVYYPNEKDCETRGDHVFCDTAAGINVAEQSKECNINISTTNYPCKVSTGRHPISM
VALSPLGALVACYKGVSCSIGSNRVGIIKQLNKGCSYITNQDADTVTIDNTVYQLSKVEGEQ
HVIKGRPVSSSFDPIKFPEDQFNVALDQVFENIENSQALVDQSNRILSSAEKGNTGFIIVII
LIAVLGSSMILVSIFIIIKKTKKPTGAPPELSGVTNNGFIPHN
SEQ ID NO:86 
ATGTCCTGGAAGGTGGTGATCATCTTCAGCCTGCTGATCACCCCGCAGCACGGCCTGAAGGA
GTCCTACCTGGAGGAGTCCTGCTCCACCATCACCGAGGGCTACCTGTCCGTGCTGCGGACCG
GGTGGTACACCAACGTGTTCACCCTGGAGGTGGGCGACGTGGAGAACCTGACCTGCAGCGAC
GGCCCCTCCCTGATCAAGACCGAGCTGGACCTGACCAAGTCCGCGCTGCGGGAGCTGAAGAC
CGTGTCCGCCGACCAGCTGGCCCGGGAGGAGCAGATCGAGAACCCTCGGCAGAGCCGGTTCG
TGCTGGGCGCCATCGCCCTGGGCGTGGCCACCGCCGCGGCCGTGACCGCCGGCGTGGCCATC
GCCAAGACCATCCGGCTGGAGAGCGAGGTGACCGCCATCAAGAACGCCCTGAAGACCACCAA
CGAGTGCGTGAGCACCCTGGGCAACGGCGTGCGGGTGCTGTGCACCGCGGTGCGGGAGCTGA
AGGACTTCGTGTCCAAGAACCTGACCCGGGCCATCAACAAGAACAAGTGCGACATCGACGAC
CTGAAGATGGCCGTGAGCTTCAGCCAGTTCAACCGGCGGTTCCTGAACGTGGTGCGCCAGTT
CTCCGACAACGCCGGCATCACCCCGGCCATCAGCCTGGACCTGATGACCGACGCCGAGCTGG
CCCGGGCGGTGTCCAACATGCCCACCTCCGCGGGCCAGATCAAGCTGATGCTGGAGAACAGG
GCGATGGTGCGGCGGAAGGGCTTCGGCATCCTGATCGGCGTGTACGGGTCCAGCGTGATCTA
TATGGTGCAGCTGCCCATCTTCGGGGTGATCGACACCCCTTGCTGGATCGTGAAGGCCGCTC
CCTCCTGCAGCGGCAAGAAGGGCAACTACGCCTGCCTGCTGCGGGAGGACCAGGGCTGGTAC
TGCCAGAACGCCGGCAGCACCGTGTACTACCCCAACGAGAAGGACTGCGAGACGCGGGGCGA
CCACGTGTTCTGCGACACCGCCGCGGGCATCAACGTGGCGGAGCAGTCCAAGGAGTGCAACA
TCAACATCAGCACCACCAACTACCCCTGCAAGGTGTCCACCGGCCGGCACCCCATCTCTATG
GTGGCCCTGAGCCCTCTGGGCGCCCTGGTGGCCTGCTACAAGGGCGTGAGCTGCTCCATCGG
CAGCAACCGGGTGGGCATCATCAAGCAGCTGAACAAGGGCTGCAGCTACATCACCAACCAGG
ACGCCGACACCGTGACCATCGACAACACCGTGTACCAGCTGTCCAAGGTGGAGGGCGAGCAG
CACGTGATCAAGGGCCGGCCGGTGAGCTCCTCCTTCGACCCCATCAAGTTCCCGGAGGACCA
GTTCAACGTGGCCCTGGACCAGGTGTTCGAGAACATCGAGAACAGCCAGGCCCTGGTGGACC
AGAGCAACCGCATCCTGTCCTCCGCCGAGAAGGGCAACACCGGCTTCATCATCGTGATCATC
CTGATCGCGGTGCTGGGCAGCTCCATGATCCTGGTGTCCATCTTCATCATC
SEQ ID NO:87 
ATGTCCTGGAAGGTGATGATCATCATCTCCCTGCTGATCACCCCGCAGCACGGCCTGAAGGA
GTCCTACCTGGAGGAGTCCTGCTCCACCATCACCGAGGGCTACCTGTCCGTGCTGCGGACCG
GCTGGTACACCAACGTGTTCACCCTGGAGGTGGGCGACGTGGAGAACCTGACCTGCACCGAC
GGCCCCTCCCTGATCAAGACCGAGCTGGACCTGACCAAGTCCGCCCTGCGGGAGCTGAAGAC
CGTGTCCGCCGACCAGCTGGCCCGGGAGGAGCAGATCGAGAACCCTCGGCAGTCCCGGTTCG
TGCTGGGCGCCATCGCCCTGGGCGTGGCCACCGCCGCCGCCGTGACCGCCGGCATCGCCATC
GCCAAGACCATCCGGCTGGAGTCCGAGGTGAACGCCATCAAGGGCGCCCTGAAGCAGACCAA
CGAGTGCGTGTCCACCCTGGGCAACGGCGTGCGGGTGCTGTGCACCGCCGTGCGGGAGCTGA
AGGAGTTCGTGTCCAAGAACCTGACCTCCGCCATCAACCGGAACAAGTGCGACATCGCCGAC
CTGAAGATGGCCGTGTCCTTCTCCCAGTTCAACCGGCGGTTCCTGAACGTGGTGCGGCAGTT
CTCCGACAACGCCGGCATCACCCCTGCCATCTCCCTGGACCTGATGACCGACGCCGAGCTGG
CCCGGGCCGTGTCCTACATGCCCACCTCCGCCGGCCAGATCAAGCTGATGCTGGAGAACCGG
GCGATGGTGCGGCGGAAGGGCTTCGGCATCCTGATCGGCGTGTACGGCTCCTCCGTGATCTA
TATGGTGCAGCTGCCCATCTTCGGCGTGATCGACACCCCTTGCTGGATCATCAAGGCCGCTC
CCTCCTGCTCCGAGAAGAACGGCAACTACGCCTGCCTGCTGCGGGAGGACCAGGGCTGGTAC
TGCAAGAACGCCGGCTCCACCGTGTACTACCCCAACGAGAAGGACTGCGAGACTCGGGGCGA
CCACGTGTTCTGCGACACCGCCGCCGGCATCAACGTGGCCGAGCAGTCCCGGGAGTGCAACA
TCAACATCTCCACCACCAACTACCCCTGCAAGGTGTCCACCGGCCGGCACCCCATCTCTATG
GTGGCCCTGAGCCCTCTGGGCGCCCTGGTGGCCTGCTACAAGGGCGTGTCCTGCTCCATCGG
CTCCAACTGGGTGGGCATCATCAAGCAGCTGCCCAAGGGCTGCTCCTACATCACCAACCAGG
ACGCCGACACCGTGACCATCGACAACACCGTGTACCAGCTGTCCAAGGTGGAGGGCGAGCAG
CACGTGATCAAGGGCCGGCCCGTGTCCTCCTCCTTCGACCCCATCAAGTTCCCCGAGGACCA
GTTCAACGTGGCCCTGGACCAGGTGTTCGAGTCCATCGAGAACTCCCAGGCCCTGGTGGACC
AGTCCAACAAGATCCTGAACTCCGCCGAGAAGGGCAACACCGGCTTCATCATCGTGGTGATC
CTGGTGGCCGTGCTGGGCCTGACTATGATCTCCGTGTCCATCATCATCATC
SEQ ID NO:88 
ATGTCCTGGAAGGTGGTGATCATCTTCAGCCTGCTGATCACCCCGCAGCACGGCCTGAAGGA
GTCCTACCTGGAGGAGTCCTGCTCCACCATCACCGAGGGCTACCTGTCCGTGCTGCGGACCG
GGTGGTACACCAACGTGTTCACCCTGGAGGTGGGCGACGTGGAGAACCTGACCTGCAGCGAC
GGCCCCTCCCTGATCAAGACCGAGCTGGACCTGACCAAGTCCGCGCTGCGGGAGCTGAAGAC
CGTGTCCGCCGACCAGCTGGCCCGGGAGGAGCAGATCGAGAACCCCCGGCAGAGCCGGTTCG
TGCTGGGCGCCATCGCCCTGGGCGTGGCCACCGCCGCGGCCGTGACCGCCGGCGTGGCCATC
GCCAAGACCATCCGGCTGGAGAGCGAGGTGACCGCCATCAAGAACGCCCTGAAGACCACCAA
CGAGTGCGTGAGCACCCTGGGCAACGGCGTGCGGGTGCTGTGCACCGCGGTGCGGGAGCTGA
AGGACTTCGTGTCCAAGAACCTGACCCGGGCCATCAACAAGAACAAGTGCGACATCGACGAC
CTGAAGATGGCCGTGAGCTTCAGCCAGTTCAACCGGCGGTTCCTGAACGTGGTGCGCCAGTT
CTCCGACAACGCCGGCATCACCCCGGCCATCAGCCTGGACCTGATGACCGACGCCGAGCTGG
CCCGGGCGGTGTCCAACATGCCCACCTCCGCGGGCCAGATCAAGCTGATGCTGGAGAACAGG
GCGATGGTGCGGCGGAAGGGCTTCGGCATCCTGATCGGCGTGTACGGGTCCAGCGTGATCTA
TATGGTGCAGCTGCCCATCTTCGGGGTGATCGACACCCCCTGCTGGATCGTGAAGGCCGCCC
CCTCCTGCAGCGGCAAGAAGGGCAACTACGCCTGCCTGCTGCGGGAGGACCAGGGCTGGTAC
TGCCAGAACGCCGGCAGCACCGTGTACTACCCCAACGAGAAGGACTGCGAGACGCGGGGCGA
CCACGTGTTCTGCGACACCGCCGCGGGCATCAACGTGGCGGAGCAGTCCAAGGAGTGCAACA
TCAACATCAGCACCACCAACTACCCCTGCAAGGTGTCCACCGGCCGGCACCCCATCTCTATG
GTGGCCCTGAGCCCCCTGGGCGCCCTGGTGGCCTGCTACAAGGGCGTGAGCTGCTCCATCGG
CAGCAACCGGGTGGGCATCATCAAGCAGCTGAACAAGGGCTGCAGCTACATCACCAACCAGG
ACGCCGACACCGTGACCATCGACAACACCGTGTACCAGCTGTCCAAGGTGGAGGGCGAGCAG
CACGTGATCAAGGGCCGGCCGGTGAGCTCCTCCTTCGACCCCATCAAGTTCCCGGAGGACCA
GTTCAACGTGGCCCTGGACCAGGTGTTCGAGAACATCGAGAACAGCCAGGCCCTGGTGGACC
AGAGCAACCGCATCCTGTCCTCCGCCGAGAAGGGCAACACCGGCTTCATCATCGTGATCATC
CTGATCGCGGTGCTGGGCTCCAGCATGATCCTGGTGTCCATCTTCATCATCATCAAGAAGAC
CAAGAAGCCGACCGGCGCCCCGCCGGAGCTGAGCGGCGTGACCAACAACGGCTTCATCCCGC
ACAGC
SEQ ID NO:89 
ATGTCCTGGAAGGTGATGATCATCATCAGCCTGCTGATCACCCCGCAGCACGGCCTGAAGGA
GTCCTACCTGGAGGAGTCCTGCTCCACCATCACCGAGGGCTACCTGTCCGTGCTGCGGACCG
GGTGGTACACCAACGTGTTCACCCTGGAGGTGGGCGACGTGGAGAACCTGACCTGCACCGAC
GGCCCCTCCCTGATCAAGACCGAGCTGGACCTGACCAAGTCCGCGCTGCGGGAGCTGAAGAC
CGTGAGCGCGGACCAGCTGGCCCGGGAGGAGCAGATCGAGAACCCTCGGCAGAGCCGGTTCG
TGCTGGGCGCCATCGCCCTGGGCGTGGCCACCGCCGCCGCGGTGACCGCCGGCATCGCCATC
GCCAAGACCATCCGGCTGGAGTCCGAGGTGAACGCCATCAAGGGCGCCCTGAAGCAGACCAA
CGAGTGCGTGAGCACCCTGGGCAACGGCGTGCGCGTGCTGTGCACCGCCGTGCGGGAGCTGA
AGGAGTTCGTGTCCAAGAACCTGACCAGCGCCATCAACAGGAACAAGTGCGACATCGCCGAC
CTGAAGATGGCGGTGTCCTTCAGCCAGTTCAACCGGCGGTTCCTGAACGTGGTGCGGCAGTT
CTCCGACAACGCCGGCATCACCCCGGCCATCAGCCTGGACCTGATGACCGACGCCGAGCTGG
CCCGGGCCGTGAGCTACATGCCCACCAGCGCCGGCCAGATCAAGCTGATGCTGGAGAACCGG
GCGATGGTGCGGCGGAAGGGCTTCGGCATCCTGATCGGCGTGTACGGCTCCTCCGTGATCTA
TATGGTGCAGCTGCCCATCTTCGGGGTGATCGACACCCCTTGCTGGATCATCAAGGCCGCTC
CCTCCTGCAGCGAGAAGAACGGCAACTACGCCTGCCTGCTGCGGGAGGACCAGGGCTGGTAC
TGCAAGAACGCCGGCAGCACCGTGTACTACCCGAACGAGAAGGACTGCGAGACGCGGGGCGA
CCACGTGTTCTGCGACACCGCCGCCGGCATCAACGTGGCGGAGCAGTCCCGGGAGTGCAACA
TCAACATCAGCACCACCAACTACCCCTGCAAGGTGAGCACCGGCAGGCACCCCATCTCTATG
GTGGCCCTGTCCCCGCTGGGCGCCCTGGTGGCCTGCTACAAGGGCGTGAGCTGCAGCATCGG
CAGCAACTGGGTGGGCATCATCAAGCAGCTGCCCAAGGGCTGCAGCTACATCACCAACCAGG
ACGCGGACACCGTGACCATCGACAACACCGTGTACCAGCTGTCCAAGGTGGAGGGCGAGCAG
CACGTGATCAAGGGCCGCCCCGTGTCCTCCTCCTTCGACCCCATCAAGTTCCCGGAGGACCA
GTTCAACGTGGCCCTGGACCAGGTGTTCGAGTCCATCGAGAACAGCCAGGCCCTGGTGGACC
AGTCCAACAAGATCCTGAACTCCGCCGAGAAGGGCAACACCGGCTTCATCATCGTGGTGATC
CTGGTGGCCGTGCTGGGCCTGACGATGATCAGCGTGTCCATCATCATCATCATCAAGAAGAC
CCGGAAGCCCACCGGCGCCCCGCCGGAGCTGAACGGCGTGACCAACGGCGGCTTCATCCCTC
ACTCC
SEQ ID NO:90 
ATGTCCTGGAAGGTGGTGATCATCTTCAGCCTGCTGATCACCCCGCAGCACGGCCTGAAGGA
GTCCTACCTGGAGGAGTCCTGCTCCACCATCACCGAGGGCTACCTGTCCGTGCTGCGGACCG
GGTGGTACACCAACGTGTTCACCCTGGAGGTGGGCGACGTGGAGAACCTGACCTGCAGCGAC
GGCCCCTCCCTGATCAAGACCGAGCTGGACCTGACCAAGTCCGCGCTGCGGGAGCTGAAGAC
CGTGTCCGCCGACCAGCTGGCCCGGGAGGAGCAGATCGAGAACCCTCGGCAGAGCCGGTTCG
TGCTGGGCGCCATCGCCCTGGGCGTGGCCGCCGCCGCGGCCGTGACCGCCGGCGTGGCCATC
GCCAAGACCATCCGGCTGGAGTCCGAGGTGACCGCCATCAACAACGCCCTGAAGAAGACCAA
CGAGTGCGTGTCCACCCTGGGCAACGGCGTGCGCGTGCTGTGCACCGCCGTGCGGGAGCTGA
AGGACTTCGTGTCCAAGAACCTGACCCGGGCCATCAACAAGAACAAGTGCGACATCGACGAC
CTGAAGATGGCCGTGAGCTTCAGCCAGTTCAACCGGCGGTTCCTGAACGTGGTGCGCCAGTT
CAGCGACAACGCCGGCATCACCCCGGCCATCAGCCTGGACCTGATGACCGACGCCGAGCTGG
CCAGGGCCGTGCCCAACATGCCCACCAGCGCCGGCCAGATCAAGCTGATGCTGGAGAACCGG
GCGATGGTGCGGCGGAAGGGCTTCGGCATCCTGATCGGCGTGTACGGGTCCAGCGTGATCTA
TATGGTGCAGCTGCCCATCTTCGGGGTGATCGACACCCCTTGCTGGATCGTGAAGGCCGCTC
CCTCCTGCAGCGAGAAGAAGGGCAACTACGCCTGCCTGCTGCGGGAGGACCAGGGCTGGTAC
TGCCAGAACGCCGGCAGCACCGTGTACTACCCCAACGAGAAGGACTGCGAGACGCGGGGCGA
CCACGTGTTCTGCGACACCGCCGCGGGCATCAACGTGGCGGAGCAGTCCAAGGAGTGCAACA
TCAACATCAGCACCACCAACTACCCCTGCAAGGTGTCCACCGGCAGGCACCCCATCTCTATG
GTGGCCCTGAGCCCTCTGGGCGCCCTGGTGGCCTGCTACAAGGGCGTGAGCTGCTCCATCGG
CAGCAACCGGGTGGGCATCATCAAGCAGCTGAACAAGGGCTGCTCCTACATCACCAACCAGG
ACGCCGACACCGTGACCATCGACAACACCGTGTACCAGCTGTCCAAGGTGGAGGGCGAGCAG
CACGTGATCAAGGGCCGGCCCGTGTCCTCCAGCTTCGACCCCATCAAGTTCCCGGAGGACCA
GTTCAACGTGGCCCTGGACCAGGTGTTCGAGAACATCGAGAACAGCCAGGCCCTGGTGGACC
AGAGCAACCGCATCCTGTCCTCCGCCGAGAAGGGCAACACCGGCTTCATCATCGTGATCATC
CTGATCGCCGTGCTGGGCTCCAGCATGATCCTGGTGAGCATCTTCATCATCATCAAGAAGAC
CAAGAAGCCCACCGGCGCCCCGCCGGAGCTGTCCGGCGTGACCAACAACGGCTTCATCCCTC
ACAAC
SEQ ID NO:91 
ATGAGCTGGAAGGTGGTGATTATCTTCAGCCTGCTGATTACACCTCAACACGGCCTGAAGGA
GAGCTACCTGGAAGAGAGCTGCTCCACCATCACCGAGGGCTACCTGAGCGTGCTGCGGACCG
GCTGGTACACCAACGTGTTCACCCTGGAGGTGGGCGACGTGGAGAACCTGACCTGCAGCGAC
GGCCCTAGCCTGATCAAGACCGAGCTGGACCTGACCAAGAGCGCTCTGAGAGAGCTGAAGAC
CGTGTCCGCCGACCAGCTGGCCAGAGAGGAACAGATCGAGAACCCTCGGCAGAGCAGATTCG
TGCTGGGCGCCATCGCTCTGGGAGTCGCCGCTGCCGCTGCAGTGACAGCTGGAGTGGCCATT
GCTAAGACCATCAGACTGGAAAGCGAGGTGACAGCCATCAACAATGCCCTGAAGAAGACCAA
CGAGGCCGTGAGCACCCTGGGCAATGGAGTGAGAGTGCTGGCCACAGCCGTGCGGGAGTGCA
AGGACTTCGTGAGCAAGAACCTGACCAGAGCCATCAACAAGAACAAGTGCGACATCGATGAC
CTGAAGATGGCCGTGAGCTTCTCCCAGTGCAACAGACGGTTCCTGAACGTGGTGAGACAGTT
CTCCGACAACGCTGGAATCACACCTGCCATTAGCCTGGACCTGATGACCGACGCCGAGCTGG
CTAGAGCCGTGCCCAACATGCCCACCAGCGCTGGCCAGATCAAGCTGATGCTGGAGAACAGA
GCCATGGTGCGGAGAAAGGGCTTCGGCATCCTGATTGGGGTGTATGGAAGCTCCGTGATCTA
CATGGTGCAGCTGCCCATCTTCGGCGTGATCGACACACCCTGCTGGATCGTGAAGGCCGCTC
CTAGCTGCTCCGAGAAGAAAGGAAACTATGCCTGTCTGCTGAGAGAGGACCAGGGCTGGTAC
TGCCAGAACGCCGGAAGCACAGTGTACTATCCCAACGAGAAGGACTGCGAAACCAGAGGCGA
CCACGTGTTCTGCGACACCGCTGCCGGAATCAACGTGGCCGAGCAGAGCAAGGAGTGCAACA
TCAACATCAGCACAACCAACTACCCCTGCAAGGTGAGCACCGGACGGCACCCCATCAGCATG
GTGGCTCTGAGCCCTCTGGGCGCTCTGGTGGCCTGCTATAAGGGCGTGTCCTGTAGCATCGG
CAGCAATCGGGTGGGCATCATCAAGCAGCTGAACAAGGGATGCTCCTACATCACCAACCAGG
ACGCCGACACCGTGACCATCGACAACACCGTGTACCAGCTGAGCAAGGTGGAGGGCGAGCAG
CACGTGATCAAGGGCAGACCCGTGAGCTCCAGCTTCGACCCCATCAAGTTCCCTGAGGACCA
GTTCAACGTGGCCCTGGACCAGGTGTTTGAGAACATCGAGAACAGCCAGGCCCTGGTGGACC
AGAGCAACAGAATCCTGTCCAGCGCTGAGAAGGGCAACACCGGCTTCATCATTGTGATCATT
CTGATCGCCGTGCTGGGCAGCTCCATGATCCTGGTGAGCATCTTCATCATTATCAAGAAGAC
CAAGAAACCCACCGGAGCCCCTCCTGAGCTGAGCGGCGTGACCAACAATGGCTTCATTCCCC
ACAAC
SEQ ID NO:92 ATGAGCTGGAAGGTGGTGATTATCTTCAGCCTGCTGATTACACCTCAACACGGCCTGAAGGA 
GAGCTACCTGGAAGAGAGCTGCTCCACCATCACCGAGGCTACCTGAGCGTGCTGCGGACCG
GCTGGTACACCAACGTGTTCACCCTGGAGGTGGGCGACGTGGAGAACCTGACCTGCAGCGAC
GGCCCTAGCCTGATCAAGACCGAGCTGGACCTGACCAAGAGCGCTCTGAGAGAGCTGAAGAC
CGTGTCCGCCGACCAGCTGGCCAGAGAGGAACAGATCGAGAACCCTCGGCAGAGCAGATTCG
TGCTGGGCGCCATCGCTCTGGGAGTCGCCGCTGCCGCTGCAGTGACAGCTGGAGTGGCCATT
GCTAAGACCATCAGACTGGAAAGCGAGGTGACAGCCATCAACAATGCCCTGAAGAAGACCAA
CGAGTGCGTGAGCACCCTGGGCAATGGAGTGAGAGTGCTGTGCACAGCCGTGCGGGAGCTGA
AGGACTTCGTGAGCAAGAACCTGACCAGAGCCATCAACAAGAACAAGTGCGACATCGATGAC
CTGAAGATGGCCGTGAGCTTCTCCCAGTTCAACAGACGGTTCCTGAACGTGGTGAGACAGTT
CTCCGACAACGCTGGAATCACACCTGCCATTAGCCTGGACCTGATGACCGACGCCGAGCTGG
CTAGAGCCGTGCCCAACATGCCCACCAGCGCTGGCCAGATCAAGCTGATGCTGGAGAACAGA
GCCATGGTGCGGAGAAAGGGCTTCGGCATCCTGATTGGGGTGTATGGAAGCTCCGTGATCTA
CATGGTGCAGCTGCCCATCTTCGGCGTGATCGACACACCCTGCTGGATCGTGAAGGCCGCTC
CTAGCTGCTCCGAGAAGAAAGGAAACTATGCCTGTCTGCTGAGAGAGGACCAGGGCTGGTAC
TGCCAGAACGCCGGAAGCACAGTGTACTATCCCAACGAGAAGGACTGCGAAACCAGAGGCGA
CCACGTGTTCTGCGACACCGCTGCCGGAATCAACGTGGCCGAGCAGAGCAAGGAGTGCAACA
TCAACATCAGCACAACCAACTACCCCTGCAAGGTGAGCACCGGACGGCACCCCATCAGCATG
GTGGCTCTGAGCCCTCTGGGCGCTCTGGTGGCCTGCTATAAGGGCGTGTCCTGTAGCATCGG
CAGCAATCGGGTGGGCATCATCAAGCAGCTGAACAAGGGATGCTCCTACATCACCAACCAGG
ACGCCGACACCGTGACCATCGACAACACCGTGTACCAGCTGAGCAAGGTGGAGGGCGAGCAG
CACGTGATCAAGGGCAGACCCGTGAGCTCCAGCTTCGACCCCATCAAGTTCCCTGAGGACCA
GTTCAACGTGGCCCTGGACCAGGTGTTTGAGAACATCGAGAACAGCCAGGCCCTGGTGGACC
AGAGCAACAGAATCCTGTCCAGCGCTGAGAAGGGCAACACCGGCTTCATCATTGTGATCATT
CTGATCGCCGTGCTGGGCAGCTCCATGATCCTGGTGAGCATCTTCATCATTATCAAGAAGAC
CAAGAAACCCACCGGAGCCCCTCCTGAGCTGAGCGGCGTGACCAACAATGGCTTCATTCCCC
ACAAC
SEQ ID NO:93 
ATGAGCTGGAAGGTGGTGATTATCTTCAGCCTGCTGATTACACCTCAACACGGCCTGAAGGA
GAGCTACCTGGAAGAGAGCTGCTCCACCATCACCGAGGGCTACCTGAGCGTGCTGCGGACCG
GCTGGTACACCAACGTGTTCACCCTGGAGGTGGGCGACGTGGAGAACCTGACCTGCAGCGAC
GGCCCTAGCCTGATCAAGACCGAGCTGGACCTGACCAAGAGCGCTCTGAGAGAGCTGAAGAC
CGTGTCCGCCGACCAGCTGGCCAGAGAGGAACAGATCGAGAACCCTCGGCAGAGCAGATTCG
TGCTGGGCGCCATCGCTCTGGGAGTCGCCGCTGCCGCTGCAGTGACAGCTGGAGTGGCCATT
GCTAAGACCATCAGACTGGAAAGCGAGGTGACAGCCATCAACAATGCCCTGAAGAAGACCTG
CGAGGCCGTGAGCACCCTGGGCAATGGAGTGAGAGTGCTGGCCACATGCGTGCGGGAGCTGA
AGGACTTCGTGAGCAAGAACCTGACCAGAGCCATCAACAAGAACAAGTGCGACATCGATGAC
CTGAAGATGGCCGTGAGCTTCTCCCAGTTCAACAGACGGTTCCTGAACGTGGTGAGACAGTT
CTCCGACAACGCTGGAATCACACCTGCCATTAGCCTGGACCTGATGACCGACGCCGAGCTGG
CTAGAGCCGTGCCCAACATGCCCACCAGCGCTGGCCAGATCAAGCTGATGCTGGAGAACAGA
GCCATGGTGCGGAGAAAGGGCTTCGGCATCCTGATTGGGGTGTATGGAAGCTCCGTGATCTA
CATGGTGCAGCTGCCCATCTTCGGCGTGATCGACACACCCTGCTGGATCGTGAAGGCCGCTC
CTAGCTGCTCCGAGAAGAAAGGAAACTATGCCTGTCTGCTGAGAGAGGACCAGGGCTGGTAC
TGCCAGAACGCCGGAAGCACAGTGTACTATCCCAACGAGAAGGACTGCGAAACCAGAGGCGA
CCACGTGTTCTGCGACACCGCTGCCGGAATCAACGTGGCCGAGCAGAGCAAGGAGTGCAACA
TCAACATCAGCACAACCAACTACCCCTGCAAGGTGAGCACCGGACGGCACCCCATCAGCATG
GTGGCTCTGAGCCCTCTGGGCGCTCTGGTGGCCTGCTATAAGGGCGTGTCCTGTAGCATCGG
CAGCAATCGGGTGGGCATCATCAAGCAGCTGAACAAGGGATGCTCCTACATCACCAACCAGG
ACGCCGACACCGTGACCATCGACAACACCGTGTACCAGCTGAGCAAGGTGGAGGGCGAGCAG
CACGTGATCAAGGGCAGACCCGTGAGCTCCAGCTTCGACCCCATCAAGTTCCCTGAGGACCA
GTTCAACGTGGCCCTGGACCAGGTGTTTGAGAACATCGAGAACAGCCAGGCCCTGGTGGACC
AGAGCAACAGAATCCTGTCCAGCGCTGAGAAGGGCAACACCGGCTTCATCATTGTGATCATT
CTGATCGCCGTGCTGGGCAGCTCCATGATCCTGGTGAGCATCTTCATCATTATCAAGAAGAC
CAAGAAACCCACCGGAGCCCCTCCTGAGCTGAGCGGCGTGACCAACAATGGCTTCATTCCCC
ACAAC
SEQ ID NO:94 
MSWKVVIIFSLLITPQHGLKESYLEESCSTITEGYLSVLRTGWYTNVFTLEVGDVENLTCSD
GPSLIKTELDLTKSALRELKTVSADQLAREEQIENPRQSRFVLGAIALGVAAAAAVTAGVAI
 
AKTIRLESEVTAINNALKKTNEAVSTLGNGVRVLATAVRELKDFVSKNLTRAINKNKCDIDD
LKMAVSFSQFNRRFLNVVRQFSDNAGITPAISLDLMTDAELARAVPNMPTSAGQIKLMLENR
AMVRRKGFGILIGVYGSSVIYMVQLPIFGVIDTPCWIVKAAPSCSEKKGNYACLLREDQGWY
CQNAGSTVYYPNEKDCETRGDHVFCDTAAGINVAEQSKECNINISTTNYPCKVSTGRHPISM
VALSPLGALVACYKGVSCSIGSNRVGIIKQLNKGCSYITNQDADTVTIDNTVYQLSKVEGEQ
HVIKGRPVSSSFDPIKFPEDQFNVALDQVFENIENSQALVDQSNRILSSAEKGNTGFIIVII
LIAVLGSSMILVSIFIIIKKTKKPTGAPPELSGVTNNGFIPHN
SEQ ID NO:95 
MSWKVVIIFSLLITPQHGLKESYLEESCSTITEGYLSVLRTGWYTNVFTLEVGDVENLTCSD
GPSLIKTELDLTKSALRELKTVSADQLAREEQIENPRQSRFVLGAIALGVATAAAVTAGVAI
AKTIRLESEVTAIKNALKTTNEAVSTLGNGVRVLATAVRELKDFVSKNLTRAINKNKCDIDD
LKMAVSFSQFNRRFLNVVRQFSDNAGITPAISLDLMTDAELARAVSNMPTSAGQIKLMLENR
AMVRRKGFGILIGVYGSSVIYMVQLPIFGVIDTPCWIVKAAPSCSGKKGNYACLLREDQGWY
CQNAGSTVYYPNEKDCETRGDHVFCDTAAGINVAEQSKECNINISTTNYPCKVSTGRHPISM
VALSPLGALVACYKGVSCSIGSNRVGIIKQLNKGCSYITNQDADTVTIDNTVYQLSKVEGEQ
HVIKGRPVSSSFDPIKFPEDQFNVALDQVFENIENSQALVDQSNRILSSAEKGNTGFIIVII
LIAVLGSSMILVSIFII
SEQ ID NO:96 
MSWKVMIIISLLITPQHGLKESYLEESCSTITEGYLSVLRTGWYTNVFTLEVGDVENLTCTD
GPSLIKTELDLTKSALRELKTVSADQLAREEQIENPRQSRFVLGAIALGVATAAAVTAGIAI
AKTIRLESEVNAIKGALKQTNEAVSTLGNGVRVLATAVRELKEFVSKNLTSAINRNKCDIAD
LKMAVSFSQFNRRFLNVVRQFSDNAGITPAISLDLMTDAELARAVSYMPTSAGQIKLMLENR
AMVRRKGFGILIGVYGSSVIYMVQLPIFGVIDTPCWIIKAAPSCSEKNGNYACLLREDQGWY
CKNAGSTVYYPNEKDCETRGDHVFCDTAAGINVAEQSRECNINISTTNYPCKVSTGRHPISM
VALSPLGALVACYKGVSCSIGSNWVGIIKQLPKGCSYITNQDADTVTIDNTVYQLSKVEGEQ
HVIKGRPVSSSFDPIKFPEDQFNVALDQVFESIENSQALVDQSNKILNSAEKGNTGFIIVVI
LVAVLGLTMISVSIIII
SEQ ID NO:97 
ATGAGCTGGAAGGTGGTGATTATCTTCAGCCTGCTGATTACACCTCAACACGGCCTGAAGGA
GAGCTACCTGGAAGAGAGCTGCTCCACCATCACCGAGGGCTACCTGAGCGTGCTGCGGACCG
GCTGGTACACCAACGTGTTCACCCTGGAGGTGGGCGACGTGGAGAACCTGACCTGCAGCGAC
GGCCCTAGCCTGATCAAGACCGAGCTGGACCTGACCAAGAGCGCTCTGAGAGAGCTGAAGAC
CGTGTCCGCCGACCAGCTGGCCAGAGAGGAACAGATCGAGAACCCTCGGCAGAGCAGATTCG
TGCTGGGCGCCATCGCTCTGGGAGTCGCCGCTGCCGCTGCAGTGACAGCTGGAGTGGCCATT
GCTAAGACCATCAGACTGGAAAGCGAGGTGACAGCCATCAACAATGCCCTGAAGAAGACCAA
CGAGGCCGTGAGCACCCTGGGCAATGGAGTGAGAGTGCTGGCCACAGCCGTGCGGGAGCTGA
AGGACTTCGTGAGCAAGAACCTGACCAGAGCCATCAACAAGAACAAGTGCGACATCGATGAC
CTGAAGATGGCCGTGAGCTTCTCCCAGTTCAACAGACGGTTCCTGAACGTGGTGAGACAGTT
CTCCGACAACGCTGGAATCACACCTGCCATTAGCCTGGACCTGATGACCGACGCCGAGCTGG
CTAGAGCCGTGCCCAACATGCCCACCAGCGCTGGCCAGATCAAGCTGATGCTGGAGAACAGA
GCCATGGTGCGGAGAAAGGGCTTCGGCATCCTGATTGGGGTGTATGGAAGCTCCGTGATCTA
CATGGTGCAGCTGCCCATCTTCGGCGTGATCGACACACCCTGCTGGATCGTGAAGGCCGCTC
CTAGCTGCTCCGAGAAGAAAGGAAACTATGCCTGTCTGCTGAGAGAGGACCAGGGCTGGTAC
TGCCAGAACGCCGGAAGCACAGTGTACTATCCCAACGAGAAGGACTGCGAAACCAGAGGCGA
CCACGTGTTCTGCGACACCGCTGCCGGAATCAACGTGGCCGAGCAGAGCAAGGAGTGCAACA
TCAACATCAGCACAACCAACTACCCCTGCAAGGTGAGCACCGGACGGCACCCCATCAGCATG
GTGGCTCTGAGCCCTCTGGGCGCTCTGGTGGCCTGCTATAAGGGCGTGTCCTGTAGCATCGG
CAGCAATCGGGTGGGCATCATCAAGCAGCTGAACAAGGGATGCTCCTACATCACCAACCAGG
ACGCCGACACCGTGACCATCGACAACACCGTGTACCAGCTGAGCAAGGTGGAGGGCGAGCAG
CACGTGATCAAGGGCAGACCCGTGAGCTCCAGCTTCGACCCCATCAAGTTCCCTGAGGACCA
GTTCAACGTGGCCCTGGACCAGGTGTTTGAGAACATCGAGAACAGCCAGGCCCTGGTGGACC
AGAGCAACAGAATCCTGTCCAGCGCTGAGAAGGGCAACACCGGCTTCATCATTGTGATCATT
CTGATCGCCGTGCTGGGCAGCTCCATGATCCTGGTGAGCATCTTCATCATTATCAAGAAGAC
CAAGAAACCCACCGGAGCCCCTCCTGAGCTGAGCGGCGTGACCAACAATGGCTTCATTCCCC
ACAAC
SEQ ID NO:98 
ATGAGCTGGAAGGTGGTCATCATCTTCAGCCTGCTGATCACCCCTCAGCACGGCCTGAAAGA
GAGCTACCTGGAAGAGTCCTGCAGCACCATCACAGAGGGCTACCTGTCTGTGCTGAGAACCG
GCTGGTACACCAACGTGTTCACACTGGAAGTGGGCGACGTGGAAAACCTGACATGCTCTGAT
GGCCCCAGCCTGATCAAGACCGAGCTGGATCTGACAAAGAGCGCCCTGAGAGAACTGAAAAC
CGTGTCTGCCGATCAGCTGGCCAGAGAGGAACAGATCGAGAACCCCAGACAGAGCAGATTCG
TGCTGGGAGCTATTGCCCTGGGAGTTGCTACAGCTGCTGCTGTGACAGCTGGCGTGGCCATT
GCCAAGACCATCAGACTGGAAAGCGAAGTGACCGCCATCAAGAACGCCCTGAAAACCACCAA
CGAGGCCGTGTCTACACTCGGCAATGGCGTTAGAGTGCTGGCCACAGCAGTGCGCGAGCTGA
AGGACTTCGTGTCCAAGAACCTGACCAGAGCCATCAACAAGAACAAGTGCGACATCGACGAC
CTGAAGATGGCCGTGTCCTTTAGCCAGTTCAACCGGCGGTTTCTGAACGTCGTGCGGCAGTT
TTCTGACAACGCCGGCATCACACCAGCCATCAGCCTGGACCTGATGACAGATGCTGAGCTGG
CTAGAGCCGTGTCCAACATGCCTACATCTGCCGGCCAGATCAAGCTGATGCTGGAAAACAGA
GCCATGGTCCGACGGAAAGGCTTCGGCATCCTGATCGGCGTGTACGGCAGCAGCGTGATCTA
TATGGTGCAGCTGCCTATCTTCGGCGTGATCGACACCCCTTGCTGGATTGTGAAGGCCGCTC
CTAGCTGTAGCGGCAAGAAGGGCAATTACGCCTGCCTGCTGAGAGAGGACCAAGGCTGGTAT
TGTCAGAATGCCGGCAGCACCGTGTACTACCCCAACGAGAAGGATTGCGAGACACGGGGCGA
TCACGTGTTCTGTGATACAGCCGCCGGAATCAATGTGGCCGAGCAGAGCAAAGAGTGCAACA
TCAACATCAGCACCACGAACTACCCCTGCAAGGTGTCCACAGGCAGACACCCTATCTCTATG
GTGGCCCTGTCTCCTCTGGGAGCCCTGGTTGCTTGTTACAAGGGCGTGTCCTGTAGCATCGG
CAGCAACAGAGTGGGCATCATCAAGCAGCTGAACAAGGGCTGCAGCTACATCACCAACCAGG
ACGCCGATACCGTGACCATCGACAACACCGTGTATCAGCTGAGCAAGGTGGAAGGCGAACAG
CACGTGATCAAGGGCAGACCTGTGTCCAGCAGCTTCGACCCCATCAAGTTCCCCGAGGATCA
GTTCAACGTGGCCCTGGACCAGGTGTTCGAGAACATCGAGAATAGCCAGGCTCTGGTGGACC
AGTCCAACAGAATCCTGTCTAGCGCCGAGAAGGGAAACACCGGCTTCATCATCGTGATCATC
CTGATTGCCGTGCTGGGCTCCAGCATGATCCTGGTGTCCATCTTTATCATC
SEQ ID NO:99

ATGAGCTGGAAAGTCATGATCATCATCAGCCTGCTGATCACCCCTCAGCACGGCCTGAAAGA
GAGCTACCTGGAAGAGTCCTGCAGCACCATCACAGAGGGCTACCTGTCTGTGCTGAGAACCG
GCTGGTACACCAACGTGTTCACACTGGAAGTGGGCGACGTGGAAAACCTGACCTGTACAGAT
GGCCCCAGCCTGATCAAGACCGAGCTGGATCTGACAAAGAGCGCCCTGAGAGAACTGAAAAC
CGTGTCTGCCGATCAGCTGGCCAGAGAGGAACAGATCGAGAACCCCAGACAGAGCAGATTCG
TGCTGGGAGCTATTGCCCTGGGAGTTGCTACAGCTGCTGCCGTGACAGCCGGAATCGCCATT
GCCAAGACCATCCGGCTGGAAAGCGAAGTGAATGCCATCAAGGGCGCACTGAAGCAGACCAA
CGAAGCCGTGTCTACCCTCGGCAACGGTGTTAGAGTGCTGGCCACAGCAGTGCGCGAGCTGA
AAGAATTTGTGTCCAAGAACCTGACCAGCGCCATCAACCGGAACAAGTGCGACATTGCCGAC
CTGAAGATGGCCGTGTCCTTCAGCCAGTTCAACCGGCGGTTTCTGAACGTCGTGCGGCAGTT
TTCTGACAACGCCGGCATCACACCCGCCATTAGCCTGGACCTGATGACAGATGCTGAGCTGG
CTCGGGCCGTGTCTTACATGCCTACATCTGCCGGCCAGATCAAGCTGATGCTGGAAAACAGA
GCCATGGTCCGACGGAAAGGCTTCGGCATCCTGATCGGCGTGTACGGCAGCTCCGTGATCTA
TATGGTGCAGCTGCCTATCTTCGGCGTGATCGACACCCCTTGCTGGATCATTAAGGCCGCTC
CTAGCTGCAGCGAGAAGAACGGCAATTACGCCTGCCTGCTGAGAGAGGACCAAGGCTGGTAC
TGCAAGAATGCCGGCAGCACCGTGTACTACCCCAACGAGAAGGATTGCGAGACACGGGGCGA
TCACGTGTTCTGTGATACAGCCGCCGGAATCAATGTGGCCGAGCAGAGCAGAGAGTGCAACA
TCAACATCAGCACCACAAACTACCCCTGCAAGGTGTCCACAGGCAGACACCCTATCTCTATG
GTGGCCCTGTCTCCTCTGGGAGCCCTGGTTGCTTGTTACAAGGGCGTGTCCTGTAGCATCGG
CAGCAACTGGGTCGGAATCATCAAGCAGCTGCCCAAGGGCTGCTCCTACATCACCAATCAGG
ACGCCGACACCGTGACCATCGACAACACAGTGTATCAGCTGAGCAAGGTGGAAGGCGAGCAG
CACGTTATCAAGGGCAGACCTGTGTCCAGCAGCTTCGACCCCATCAAGTTCCCCGAGGATCA
GTTCAACGTGGCCCTGGATCAGGTGTTCGAGAGCATCGAGAATAGCCAGGCTCTGGTGGACC
AGTCCAACAAGATCCTGAATAGCGCCGAGAAGGGCAACACCGGCTTCATCATCGTGGTTATC

CTGGTGGCTGTGCTGGGCCTGACAATGATCAGCGTGTCCATCATCATTATC

## Claims

1. A nucleic acid comprising a polynucleotide encoding a mutant of MPV F protein, the mutant comprising an F1 polypeptide and an F2 polypeptide, the mutant comprising disulfide bond mutations relative to a wild-type MPV F protein, the disulfide bond mutations comprising one or more of:
A147C and A159C, E146C and T160C, F168C and F196C, L165C and F196C, N145C and A161C, S149C and V157C, T59C and N180C, T150C and R156C, V52C and L165C, V55C and V169C, L58C and T174C, V148C and L158C;
preferably, the disulfide bond mutations comprise one or more of: A147C and A159C, L165C and F196C, N145C and A161C, S149C and V157C, T59C and N180C;
preferably, the disulfide bond mutations are selected from one of: A147C and A159C, L165C and F196C, N145C and A161C.

2. The nucleic acid according to claim 1, wherein the mutant is selected from one of the following (1) to (38):
(1) a mutant comprising an amino acid sequence set forth in SEQ ID NO: 1 or 2;
(2) a mutant comprising mutations A147C and A159C and comprising an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to the amino acid sequence set forth in SEQ ID NO: 1 or 2;
(3) a mutant comprising an amino acid sequence set forth in SEQ ID NO: 3 or 4;
(4) a mutant comprising mutations E146C and T160C and comprising an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to the amino acid sequence set forth in SEQ ID NO: 3 or 4;
(5) a mutant comprising an amino acid sequence set forth in SEQ ID NO: 5 or 6;
(6) a mutant comprising mutations F168C and F196C and comprising an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to the amino acid sequence set forth in SEQ ID NO: 5 or 6;
(7) a mutant comprising an amino acid sequence set forth in SEQ ID NO: 7 or 8;
(8) a mutant comprising mutations L165C and F196C and comprising an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to the amino acid sequence set forth in SEQ ID NO: 7 or 8;
(9) a mutant comprising an amino acid sequence set forth in SEQ ID NO: 9 or 10;
(10) a mutant comprising mutations N145C and A161C and comprising an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to the amino acid sequence set forth in SEQ ID NO: 9 or 10;
(11) a mutant comprising an amino acid sequence set forth in SEQ ID NO: 11 or 12;
(12) a mutant comprising mutations S149C and V157C and comprising an amino acid sequence having at least 85%, 88%, 90%, 91%, 92%, 93%, 94%, 95%, 95.5%, 96%, 96.5%, 97%, 97.5%, 98%, 98.5%, 99%, or 99.5% identity to the amino acid sequence set forth in SEQ ID NO: 11 or 12;
(13) a mutant comprising an amino acid sequence set forth in SEQ ID NO: 13 or 14;
(14) a mutant comprising mutations T59C and N180C and comprising an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to the amino acid sequence set forth in SEQ ID NO: 13 or 14;
(15) a mutant comprising an amino acid sequence set forth in SEQ ID NO: 15 or 16;
(16) a mutant comprising mutations T59C and N180C and comprising an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to the amino acid sequence set forth in SEQ ID NO: 15 or 16;
(17) a mutant comprising an amino acid sequence set forth in SEQ ID NO: 17 or 18;
(18) a mutant comprising mutations T59C and N180C and comprising an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to the amino acid sequence set forth in SEQ ID NO: 17 or 18;
(19) a mutant comprising an amino acid sequence set forth in SEQ ID NO: 19 or 20;
(20) a mutant comprising mutations T59C and N180C and comprising an amino acid sequence having at least 85%, 88%, 90%, 91%, 92%, 93%, 94%, 95%, 95.5%, 96%, 96.5%, 97%, 97.5%, 98%, 98.5%, 99%, or 99.5% identity to the amino acid sequence set forth in SEQ ID NO: 19 or 20;
(21) a mutant comprising an amino acid sequence set forth in SEQ ID NO: 21 or 22;
(22) a mutant comprising mutations T150C and R156C and comprising an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to the amino acid sequence set forth in SEQ ID NO: 21or 22;
(23) a mutant comprising an amino acid sequence set forth in SEQ ID NO: 23 or 24;
(24) a mutant comprising mutations V52C and L165C and comprising an amino acid sequence having at least 85%, 88%, 90%, 91%, 92%, 93%, 94%, 95%, 95.5%, 96%, 96.5%, 97%, 97.5%, 98%, 98.5%, 99%, or 99.5% identity to the amino acid sequence set forth in SEQ ID NO: 23 or 24;
(25) a mutant comprising an amino acid sequence set forth in SEQ ID NO: 25 or 26;
(26) a mutant comprising mutations V55C and V169C and comprising an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to the amino acid sequence set forth in SEQ ID NO: 25 or 26;
(27) a mutant comprising an amino acid sequence set forth in SEQ ID NO: 27 or 28;
(28) a mutant comprising mutations L58C and T174C and comprising an amino acid sequence having at least 85%, 88%, 90%, 91%, 92%, 93%, 94%, 95%, 95.5%, 96%, 96.5%, 97, 97.5%, 98%, 98.5%, 99%, or 99.5% identity to the amino acid sequence set forth in SEQ ID NO: 27 or 28;
(29) a mutant comprising an amino acid sequence set forth in SEQ ID NO: 29 or 30;
(30) a mutant comprising mutations V148C and L158C and comprising an amino acid sequence having at least 85%, 88%, 90%, 91%, 92%, 93%, 94%, 95%, 95.5%, 96%, 96.5%, 97, 97.5%, 98%, 98.5%, 99%, or 99.5% identity to the amino acid sequence set forth in SEQ ID NO: 29 or 30;
(31) a mutant comprising an amino acid sequence set forth in SEQ ID NO: 81 or 82;
(32) a mutant comprising mutations V148C and L158C and comprising an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to the amino acid sequence set forth in SEQ ID NO: 81 or 82;
(33) a mutant comprising an amino acid sequence set forth in SEQ ID NO: 83;
(34) a mutant comprising mutations A147C and A159C and comprising an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to the amino acid sequence set forth in SEQ ID NO: 83;
(35) a mutant comprising an amino acid sequence set forth in SEQ ID NO: 84;
(36) a mutant comprising mutations L165C and F196C and comprising an amino acid sequence having at least 85%, 88%, 90%, 91%, 92%, 93%, 94%, 95%, 95.5%, 96, 96.5%, 97, 97.5%, 98%, 98.5%, 99%, or 99.5% identity to the amino acid sequence set forth in SEQ ID NO: 84;
(37) a mutant comprising the amino acid sequence set forth in SEQ ID NO: 85; and
(38) a mutant comprising mutations N145C and A161C and comprising an amino acid sequence having at least 85%, 88%, 90%, 91%, 92%, 93%, 94%, 95%, 95.5%, 96%, 96.5%, 97%, 97.5%, 98%, 98.5%, 99% or 99.5% identity to the amino acid sequence set forth in SEQ ID NO: 85.

3. The nucleic acid according to any one of claims 1-2, wherein the wild-type MPV is subtype A or subtype B;
preferably, the wild-type MPV is wild-type hMPV;
preferably, the MPV F protein is hMPV F protein.

4. The nucleic acid according to any one of claims 1-3, wherein the nucleic acid is RNA; preferably, the RNA is mRNA; more preferably, the mRNA comprises at least one of 5'-cap structure, 5'-UTR, 3'-UTR and poly(A) tail;
or, the nucleic acid is DNA; preferably, the DNA can be transcribed into RNA.

5. The nucleic acid according to claim 4, wherein the DNA sequence corresponding to the 5'-UTR is set forth in SEQ ID NO: 31;
and/or, the DNA sequence corresponding to the 3'-UTR is set forth in SEQ ID NO: 32;
and/or, the nucleotides constituting the poly(A) tail comprise at least 20, at least 40, at least 80, at least 100 or at least 120 A nucleotides; preferably, the nucleotides constituting the poly(A) tail comprise at least 20, at least 40, at least 80, at least 100 or at least 120 consecutive A nucleotides; preferably, the nucleotides constituting the poly(A) tail comprise one or more nucleotides other than the A nucleotides; more preferably, the DNA sequence corresponding to the poly(A) tail is set forth in SEQ ID NO: 33.

6. The nucleic acid according to any one of claims 1-5, comprising a modified nucleotide;
preferably, the nucleic acid contains a modified nucleoside; preferably, the modified nucleoside includes at least one of modified uridine, modified cytidine, modified adenosine and modified guanosine.

7. The nucleic acid of any one of claims 1-6, comprising a polynucleotide encoding one or more mutants of MPV F protein.

8. The nucleic acid according to any one of claims 1-7, further comprising a polynucleotide encoding a protein or polypeptide other than a mutant of MPV F protein.

9. The nucleic acid of any one of claims 1-8, further comprising one or more of a polynucleotide encoding the transmembrane domain of MPV F protein, a polynucleotide encoding a ferritin polypeptide, a polynucleotide encoding a signal peptide, and a polynucleotide encoding a trimerization domain.

10. A genetic engineering vector comprising the nucleic acid according to any one of claims 1-9, or comprising a polynucleotide capable of being transcribed into the nucleic acid according to any one of claims 1-9.

11. A host cell comprising the genetic engineering vector of claim 10.

12. A mutant of MPV F protein encoded by the nucleic acid according to any one of claims 1-19;
preferably, the mutant is a trimer.

13. A delivery carrier comprising a nucleic acid composition, wherein the nucleic acid composition comprises a first nucleic acid or a first genetic engineering vector, the first nucleic acid is the nucleic acid according to any one of claims 1-9, and the first genetic engineering vector is the genetic engineering vector according to claim 10;
or, the nucleic acid composition comprises a plurality of first nucleic acids or a plurality of first genetic engineering vectors, the first nucleic acids are the nucleic acids according to any one of claims 1-9, the first genetic engineering vectors are the genetic engineering vectors according to claim 10, and the plurality of first nucleic acids are independent of each other or the plurality of first genetic engineering vectors are independent of each other.

14. The delivery carrier according to claim 13, wherein the nucleic acid composition further comprises a second nucleic acid or a second vector, the second nucleic acid comprises a polynucleotide encoding a protein or polypeptide other than a mutant of MPV F protein, and the second vector comprises a polynucleotide encoding a protein or polypeptide other than a mutant of MPV F protein;
and/or, the first nucleic acid and the second nucleic acid are RNA; more preferably, the first nucleic acid and the second nucleic acid are mRNA.

15. The delivery carrier according to any one of claims 13-14, wherein the delivery carrier is lipid nanoparticles (LNP s), cationic liposomes, cationic proteins or lipid polymers (LPP);
preferably, the delivery carrier is a lipid nanoparticle comprising a cationic lipid comprising the following compound (IV), or a pharmaceutically acceptable salt or stereoisomer thereof: 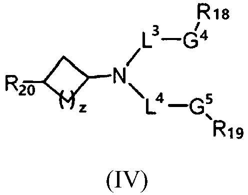 where:
L³ and L⁴ are the same or different, and are each independently C1-C12 alkylene, C2-C12 alkenylene or C2-C12 alkynylene; preferably L³ and L⁴ are the same or different, and are each independently C3-C10 alkylene, C3-C10 alkenylene or C3-C10 alkynylene; further preferably, L³ and L⁴ are the same or different, and are each independently C3-C10 alkylene; most preferably, L³ and L⁴ are the same or different, and are each independently C5-C8 alkylene;
G⁴ and G⁵ are the same or different, and are each independently -O-(C=O)-, -(C=O)-O-, -C(=O)-, -O-, -C(=O)-S- or -S-C(=O)-; preferably, G⁴ and G⁵ are the same or different, and are each independently -O-(C=O)-, -(C=O)-O-, -C(=O)- or -O-; most preferably, G⁴ and G⁵ are the same or different, and are each independently selected from -O-(C=O)- or -(C=O)-O-;
R₁₈ and R₁₉ are the same or different, and are each independently C5-C27 alkyl or C5-C27 alkenyl containing one or more double bonds; preferably, R₁₈ and R₁₉ are the same or different, and are each independently C8-C20 alkyl or C8-C20 alkenyl containing one or more double bonds; further preferably, R₁₈ and R₁₉ are the same or different, and are each independently C9-C17 alkyl or C9-C18 alkenyl containing one or two double bonds; most preferably, R₁₈ and R₁₉ are the same or different, and are each independently 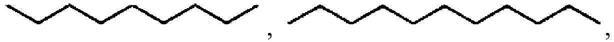 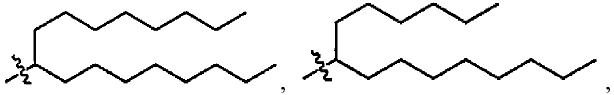 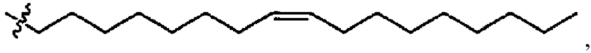
or 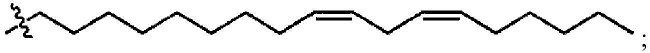
R₂₀ is halogen, hydroxyl, cyano, C1-C6 alkyl, nitro, C1-C6 alkoxy, C1-C6 alkylcarbonyloxy, C1-C6 alkoxycarbonyl, C1-C6 alkylaminocarbonyl or C1-C6 alkylcarbonylamino; preferably, R₂₀ is halogen, hydroxyl, cyano, C1-C6 alkoxy, C1-C6 alkylcarbonyloxy, C1-C6 alkoxycarbonyl, C1-C6 alkylaminocarbonyl or C1-C6 alkylcarbonylamino; further preferably, R₂₀ is halogen, hydroxyl, cyano, C1-C4 alkoxy, C1-C4 alkylcarbonyloxy, C1-C4 alkoxycarbonyl, C1-C4 alkylaminocarbonyl or C1-C4 alkylcarbonylamino; most preferably, R₂₀ is fluorine, hydroxyl, cyano, methoxy, acetoxy, methoxycarbonyl, butylaminocarbonyl or acetamido;
z is 1, 2 or 3.

16. The delivery carrier according to claim 15, wherein the cationic lipid is the following compound (IV-1), or a pharmaceutically acceptable salt or stereoisomer thereof: 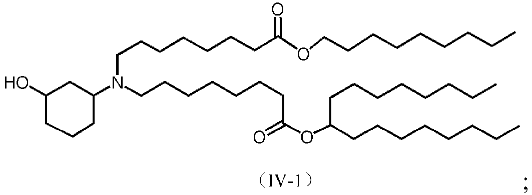 or, the cationic lipid is the following compound, or a pharmaceutically acceptable salt thereof: 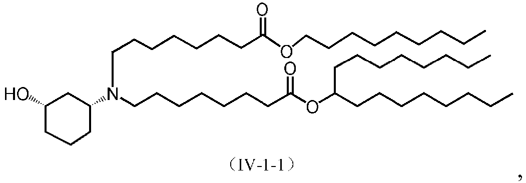 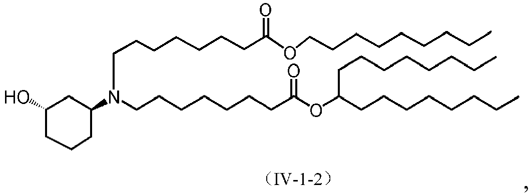 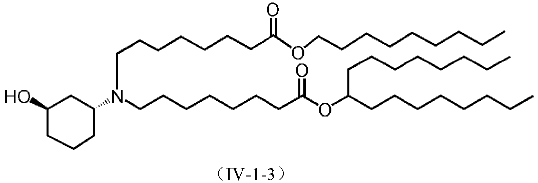 or

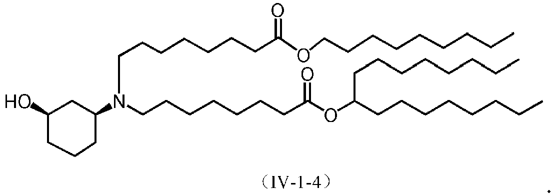

17. A pharmaceutical composition comprising the nucleic acid of any one of claims 1-9, the genetic engineering vector of claim 10, the host cell of claim 11, the mutant of MPV F protein of claim 12, or the delivery carrier of any one of claims 13-16, and a pharmaceutically acceptable carrier.

18. The pharmaceutical composition of claim 17, comprising a plurality of the delivery carriers;
or, the pharmaceutical composition comprises a plurality of the nucleic acids.

19. The pharmaceutical composition according to claim 17 or 18, wherein the pharmaceutical composition comprises two nucleic acids, the two nucleic acids are co-formulated or separately formulated in lipid nanoparticles, the two nucleic acids respectively encode a mutant of subtype A MPV F protein and a mutant of subtype B MPV F protein, and the mutant of subtype A MPV F protein and the mutant of subtype B MPV F protein encoded by the two nucleic acids selected from the group consisting of:
(1) a mutant comprising mutant A147C and A159C and having an amino acid sequence at least 85%, 88%, 90%, 91%, 92%, 93%, 94%, 95%, 95.5%, 96%, 96.5%, 97%, 97.5%, 98%, 98.5%, 99% or 99.5% identity to SEQ ID NO: 1, and a mutant comprising mutant A147C and A159C and having an amino acid sequence at least 85%, 88%, 90%, 91%, 92%, 93%, 94%, 95%, 95.5%, 96%, 96.5%, 97%, 97.5%, 98%, 98.5%, 99% or 99.5% identity to SEQ ID NO: 2; and
(2) a mutant comprising mutations A147C and A159C and having an amino acid sequence at least 85%, 88%, 90%, 91%, 92%, 93, 94, 95, 95.5%, 96, 96.5%, 97, 97.5%, 98, 98.5%, 99, or 99.5% identity to SEQ ID NO: 81, and a mutant comprising mutations A147C and A159C and having an amino acid sequence at least 85%, 88%, 90%, 91%, 92%, 93%, 94%, 95%, 95.5%, 96%, 96.5%, 97%, 97.5%, 98%, 98.5%, 99%, or 99.5% identity to SEQ ID NO: 82.

20. Use of the nucleic acid according to any one of claims 1-9, the genetic engineering vector according to claim 10, the host cell according to claim 11, the mutant of MPV F protein according to claim 12, the delivery carrier according to any one of claims 13-16 or the pharmaceutical composition according to any one of claims 18-19 in the preparation of a medicament;
preferably, the medicament is used for preventing or treating MPV infection or diseases caused by MPV infection;
preferably, the medicament is used for preventing or treating hMPV infection or diseases caused by hMPV infection;
preferably, the medicament is a vaccine.

21. A vaccine comprising the nucleic acid of any one of claims 1-9, the genetic engineering vector of claim 10, the mutant of MPV F protein of claim 12, or the delivery carrier of any one of claims 13-16.
